# EUROPEAN PATENT SPECIFICATION

(11) **EP 3 394 062 B1**
(45) Date of publication and mention of the grant of the patent: **02.10.2019**
(21) Application number: 16825898.6
(22) Date of filing: 21.12.2016
(51) Int. Cl.: C07D 471/06, C07D 491/06, C07D 495/06, C07D 519/00, C07F 7/08, C07F 9/572, C09K 11/06, H01L 51/00, H01L 51/50, H05B 33/20

(54) **HETERO-CONDENSED PHENYLQUINAZOLINES AND THEIR USE IN ELECTRONIC DEVICES**
HETERO-KONDENSIERTE PHENYLCHINAZOLINE UND IHRE VERWENDUNG IN ELEKTRONISCHEN VORRICHTUNGEN
PHENYLQUINAZOLINES HÉTÉRO-CONDENSÉ ET LEUR UTILISATION DANS DES DISPOSITIFS ÉLECTRONIQUES

(30) Priority: 21.12.2015 EP 15201486
(43) Date of publication of application: 31.10.2018
(73) Proprietor: Idemitsu Kosan Co., Ltd, Tokyo 100-8321 (JP)
(72) Inventor: NISHIMAE, Yuichi, 4058 Basel (CH); NAKANO, Yuki, Sodegaura-shi Chiba 299-0293 (JP); NAGASHIMA, Hideaki, 4057 Basel (CH); KAWAMURA, Masahiro, Sodegaura-shi Chiba 299-0293 (JP); HAKETA, Tasuku, Sodegaura-shi Chiba 299-0293 (JP); WOLLEB, Annemarie, 4232 Fehren (CH)
(74) Representative: Hollah, Dorothee
(86) International application number: PCT/IB2016/057889
(87) International publication number: WO 2017/109727

(56) References cited:
- WO-A1-2014/088290
- WO-A1-2016/006791
- KR-A- 20150 111 106
- MARTÍNEZ-VITURRO, C.M. ET AL.: "Synthesis of new chromeno[4,3,2- de ]quinazolin-2-ones, -quinazolines and -pyrrolo[2,1- b]quinazolines", JOURNAL OF HETEROCYCLIC CHEMISTRY, vol. 44, no. 5, 2007, pages 1035-1043, XP055280787, ISSN: 0022-152X, DOI: 10.1002/jhet.5570440510 cited in the application
- HOLLINS, R.A. ET AL.: "A new synthesis of benzothiopyrano[4,3,2- de]quinazolines", JOURNAL OF HETEROCYCLIC CHEMISTRY, vol. 16, no. 4, 1979, pages 679-680, XP055280905, ISSN: 0022-152X, DOI: 10.1002/jhet.5570160413
- EIDEN, F. ET AL.: "Benzothiopyrano[4.3.2-de]-chinazoline", ARCHIV DER PHARMAZIE, vol. 307, no. 9, 1974, pages 701-707, XP055280902,
- BRIEL, D.: "Preparation of benzopyranopyridopyrimidines from 1,3-benzoxazines - An unexpected ring transformation", PHARMAZIE, vol. 46, no. 6, 1991, pages 455-456, XP009190560, ISSN: 0031-7144
- OKABAYASHI, I. ET AL.: "Synthesis of benzothiopyrano[4,3,2- de ]quinoline", JOURNAL OF HETEROCYCLIC CHEMISTRY, vol. 31, no. 4, 1994, pages 733-735, XP055041172, ISSN: 0022-152X, DOI: 10.1002/jhet.5570310407
- WANG, H.-Y. ET AL.: "Novel fluorescence dyes based on entirely new chromeno[4,3,2-de][1,6]naphthyridine framework", DYES AND PIGMENTS, vol. 95, no. 2, 2012, pages 268-274, XP028402238, ISSN: 0143-7208, DOI: 10.1016/J.DYEPIG.2012.05.018 [retrieved on 2012-05-26]
- HOSTYN, S. ET AL.: "Synthesis of the benzo-beta-carboline isoneocryptolepine: the missing indoloquinoline isomer in the alkaloid series cryptolepine, neocryptolepine and isocryptolepine", TETRAHEDRON, vol. 61, no. 6, 2005, pages 1571-1577, XP027860745, ISSN: 0040-4020 [retrieved on 2005-02-07]
- BRY, D. ET AL.: "New pyridoacridine alkaloids from the purple morph of the ascidian", TETRAHEDRON LETTERS, vol. 52, no. 23, 2011, pages 3041-3044, XP028384611, ISSN: 0040-4039, DOI: 10.1016/J.TETLET.2011.04.005 [retrieved on 2011-04-09]
- DELFOURNE, E. ET AL.: "The First Synthesis of the Pentacyclic Pyridoacridine Marine Alkaloids: Arnoamines A and B", THE JOURNAL OF ORGANIC CHEMISTRY, vol. 65, no. 18, 2000, pages 5476-5479, XP055281574, ISSN: 0022-3263, DOI: 10.1021/jo000011a
- GORELIK, M.V. ET AL.: "Syntheses of 7H-pyrido-and 7H-pyrano[2,3,4-kl]acridin-2(3H)-ones from 9-chloroacridines", RUSSIAN CHEMICAL BULLETIN, INTERNATIONAL EDITION, vol. 55, no. 9, 2006, pages 1664-1669, XP019468051, ISSN: 1573-9171, DOI: 10.1007/S11172-006-0471-0
- WU, H. ET AL.: "Silica Gel-Catalyzed One-Pot Syntheses in Water and Fluorescence Properties Studies of 5-Amino-2-aryl-3H-chromeno[4,3,2- de][1,6]naphthyridine-4-carbonitriles and 5-Amino-2-aryl-3H-quinolino[4,3,2- de][1,6]naphthyridine-4-carbonitriles", JOURNAL OF COMBINATORIAL CHEMISTRY, vol. 12, no. 1, 2009, pages 31-34, XP055281588, ISSN: 1520-4766, DOI: 10.1021/cc9001179
- KOELSCH, C.F. ET AL.: "CAMPS REACTION WITH 1-XANTHONAMINE", JOURNAL OF THE AMERICAN CHEMICAL SOCIETY, vol. 71, 1949, pages 3556-3558, XP001022858, ISSN: 0002-7863, DOI: 10.1021/JA01178A521

## Description

The present invention relates to compounds of general formulae (IIb) and (IIc) and to a process for their preparation, to an electronic device comprising at least one of these compounds, to an emitting layer, preferably present in an electronic device, comprising at least one compound of general formula (IIb) or (IIc) and to the use of a compound according to general formula (IIb) or (IIc) in an electronic device as a host material, a charge transporting material, charge and/or exciton blocking material, preferably as a host material or an electron transporting material.

Quinazolines and their use in electronic devices are known from the related art.

KR 20150111106 A discloses compounds according to the following formulae 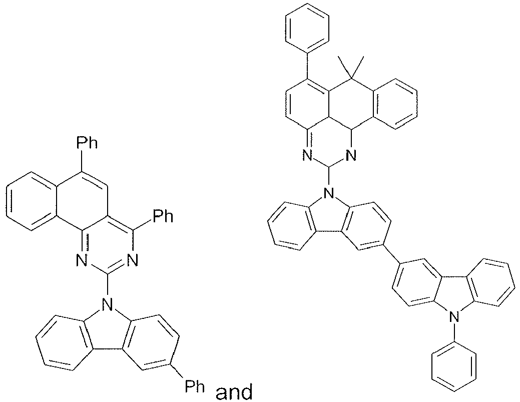 and their use as electroluminescent device materials.

Carlos M. Martinez et al., J. Heterocyclic Chem., 44, 1035 (2007), disclose Quinalozin-derivatives according to the following formula 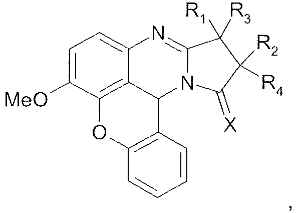 wherein X may be O or H₂ and R₁, R₂, R₃ and R₄ may be hydrogen, R₁ and R₂ may form a six membered ring or R₃ and R₄ may be an additional bond, and a process for their preparation. This document further discloses that the mentioned compounds show activities in a number of pharmaceutical applications based on their sedative, CNS-depressant, neuroleptic, hypnotic, analgesic, diuretic, anthelminthic, antimicrobial, antitubercular, antibiotic antihypertensive, antiinflammatory and antitumoral properties.

There remains a need for electronic devices comprising new materials, especially as a host material, a charge transporting material, charge and/or exciton blocking material to provide improved efficiency, stability, manufacturability, driving voltage and/or spectral characteristics of electronic devices.

Accordingly, it is an object of the present invention, with respect to the aforementioned related art, to provide materials suitable for use in electronic devices, preferably OLEDs, and further applications in organic electronics. More particularly, it should be possible to provide electronic devices comprising new compounds as electron transport materials, as hole transport materials or as host materials. The materials should be suitable especially for OLEDs which comprise at least one emitter, which is preferably a phosphorescence emitter, for example at least one red phosphorescent emitter, especially as a host material. The materials should also be suitable especially for OLEDs which comprise at least one emitter, which is preferably a fluorescence emitter, for example at least one blue fluorescent emitter, especially as an electron transporting material.

Furthermore, the materials should be suitable for providing electronic devices, preferably OLEDs, which ensure good efficiencies and/or a low use and operating voltage of the OLEDs. Said object is solved by a compound of general formula (IIb) or (IIc) 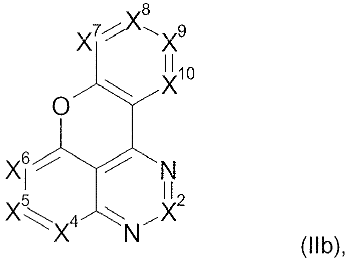 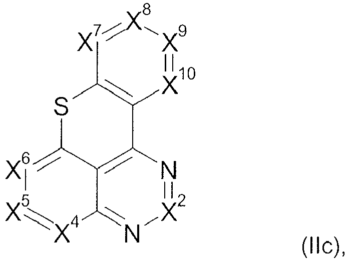 wherein
X² is CR²,
X⁴ is CR⁴,
X⁵ is CR⁵,
X⁶ is CR⁶,
X⁷ is CR⁷,
X⁸ is CR⁸,
X⁹ is CR⁹,
X¹⁰ is CR¹⁰,
R4, R⁵, R⁶, R⁷, R⁸, R⁹ and R¹⁰ are H,
R₂ is a group of formula -(L¹)ₒ-(L²)ₚ-(L³)_{q}-(L⁴)ᵣ-R¹⁴, wherein L¹, L², L³ and L⁴ are independently of each other selected from a C₆-C₄₀aryl group which is unsubstituted or substituted by at least one group E and a C₁-C₂₄heteroaryl group which is unsubstituted or substituted by at least one group E, and R¹⁴ is independently of each other selected from H or a C₁-C₂₅alkyl group which unsubstituted or substituted by at least one group E and/or interrupted by at least one group D,
o is 1, p is independently of each other 0 or 1, q is independently of each other 0 or 1 and r is independently of each other 0 or 1,
D is independently of each other -CO-, -COO-, -S-, -SO-, -SO₂-, -O-,-CR¹⁵=CR¹⁶-, -NR¹⁷-,-SiR²²R²³-, -POR²⁵-, -C≡C-,
E is independently of each other -OR²¹, -SR²¹, -NR¹⁷R¹⁸, -COR²⁰, -COOR¹⁹, -CONR¹⁷R¹⁸, -CN,-SiR²²R²³R²⁴, POR²⁵R²⁷, halogen, a C₆-C₆₀aryl group which is unsubstituted or is substituted by at least one -F, -CF₃, -CF₂CF₃, -CF₂CF₂CF₃, -CF(CF₃)₂, -(CF₂)₃CF₃ or -C(CF₃)₃, a C₁-C₁₈alkyl or a C₁-C₁₈alkyl group which is interrupted by at least one O, a C₁-C₆₀heteroaryl group which is unsubstituted or substituted by at least one -F, -CF₃, -CF₂CF₃, -CF₂CF₂CF₃, -CF(CF₃)₂, -(CF₂)₃CF₃ or -C(CF₃)₃,
R¹⁵ and R¹⁶ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group which is interrupted by at least one O,
R¹⁷ and R¹⁸ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O, or
R¹⁷ and R¹⁸ together form a five or six membered aliphatic, aromatic or heteroaromatic ring,
R¹⁹ is H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O,
R²⁰ is H or a C₆-C₁₈aryl group which is unsubstituted or substituted by at least one C₁-C₁₈alkyl group or at least one C₁-C₁₈alkoxy group, a C₁-C₁₈ alkyl group or a C₁-C₁₈alkyl group, which is interrupted by at least one O,
R²¹ is independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O,
R²², R²³ and R²⁴ are independently of each other H, a C₁-C₁₈alkyl group, a C₆-C₁₈aryl group which is unsubstituted or substituted by at least one C₁-C₁₈alkyl group, and
R²⁵ and R²⁷ are independently of each other H, a C₁-C₁₈alkyl group, a C₆-C₁₈aryl group which is unsubstituted or substituted by at least one C₁-C₁₈alkyl group, a C₇-C₂₅aralkyl which is unsubstituted or substituted by at least one group E, a C₅-C₁₂cycloalkyl group which is unsubstituted or substituted by at least one group E.

The object of the present invention is further solved by a process for the preparation of the compounds of general formula (IIb) and (IIc), by an electronic device comprising at least one of the compounds of general formula (IIb) and (IIc), by an emitting layer, comprising at least one compound of general formula (lib) and (IIc) and by the use of a compound according to general formula (lib) or (IIc) in an electronic device.

The compounds according to the present invention, the process for their preparation, the electronic device according to the present invention and the use of the compounds are explained in detail in the following.

According to the present invention the terms halogen, alkyl, aryl, aryloxy and heteroaryl generally have the following meaning, if said groups are not further specified in specific embodiments mentioned below.

Halogen is fluorine, chlorine, bromine and iodine.

C₁-C₂₅alkyl, preferably C₁-C₁₈alkyl, is typically linear or branched, where possible. Examples are methyl, ethyl, n-propyl, isopropyl, n-butyl, sec.-butyl, isobutyl, tert.-butyl, n-pentyl, 2-pentyl, 3-pentyl, 2,2-dimethylpropyl, 1,1,3,3-tetramethylpentyl, n-hexyl, 1-methylhexyl, 1,1,3,3,5,5-hexamethylhexyl, n-heptyl, isoheptyl, 1,1,3,3-tetramethylbutyl, 1-methylheptyl, 3-methylheptyl, n-octyl, 1,1,3,3-tetramethylbutyl and 2-ethylhexyl, n-nonyl, decyl, undecyl, dodecyl, tridecyl, tetradecyl, pentadecyl, hexadecyl, heptadecyl, or octadecyl. C₁-C₈alkyl is typically methyl, ethyl, n-propyl, isopropyl, n-butyl, sec.-butyl, isobutyl, tert.-butyl, n-pentyl, 2-pentyl, 3-pentyl, 2,2-dimethyl-propyl, n-hexyl, n-heptyl, n-octyl, 1,1,3,3-tetramethylbutyl and 2-ethylhexyl. C₁-C₄alkyl is typically methyl, ethyl, n-propyl, isopropyl, n-butyl, sec.-butyl, isobutyl, tert.-butyl. C₁-C₂₅alkoxy groups, preferably C₁-C₁₈alkoxy groups, are straight-chain or branched alkoxy groups, e.g. methoxy, ethoxy, n-propoxy, isopropoxy, n-butoxy, sec-butoxy, tert-butoxy, amyloxy, isoamyloxy or tert-amyloxy, heptyloxy, octyloxy, isooctyloxy, nonyloxy, decyloxy, undecyloxy, dodecyloxy, tetradecyloxy, pentadecyloxy, hexadecyloxy, heptadecyloxy and octade-cyloxy. Examples of C₁-C₈alkoxy are methoxy, ethoxy, n-propoxy, isopropoxy, n-butoxy, sec.-butoxy, isobutoxy, tert.-butoxy, n-pentyloxy, 2-pentyloxy, 3-pentyloxy, 2,2-dimethylpropoxy, n-hexyloxy, n-heptyloxy, n-octyloxy, 1,1,3,3-tetramethylbutoxy and 2-ethylhexyloxy, preferably C₁-C₄alkoxy such as typically methoxy, ethoxy, n-propoxy, isopropoxy, n-butoxy, sec.-butoxy, isobutoxy, tert.-butoxy.

C₆-C₆₀aryl, preferably C₆-C₂₄aryl, particularly preferably C₆-C₁₈aryl, which optionally can be substituted, is typically phenyl, 4-methylphenyl, 4-methoxyphenyl, naphthyl, especially 1-naphthyl, or 2-naphthyl, biphenylyl, terphenylyl, pyrenyl, 2- or 9-fluorenyl, phenanthryl, or anthryl, which may be unsubstituted or substituted. Phenyl, 1-naphthyl and 2-naphthyl are examples of a C₆-C₁₀aryl group.

C₆-C₂₄aryloxy, which optionally can be substituted, is typically C₆-C₁₀aryloxy, which optionally can be substituted by one, or more C₁-C₈alkyl and/or C₁-C₈alkoxy groups, such as, for example, phenoxy, 1-naphthoxy, or 2-naphthoxy.

C₁-C₆₀heteroaryl, preferably C₂-C₃₀heteroaryl, particularly preferably C₂-C₁₃heteroaryl, represents a ring with five to seven ring atoms or a condensed ring system, wherein nitrogen, oxygen or sulfur are the possible hetero atoms, and is typically a heterocyclic group with 5 to 40 atoms having at least six conjugated π-electrons such as thienyl, benzothiophenyl, dibenzothiophenyl, thianthrenyl, furyl, furfuryl, 2H-pyranyl, benzofuranyl, isobenzofuranyl, dibenzofuranyl, phenoxy-thienyl, pyrrolyl, imidazolyl, pyrazolyl, pyridyl, bipyridyl, triazinyl, pyrimidinyl, pyrazinyl, pyridazinyl, indolizinyl, isoindolyl, indolyl, indazolyl, purinyl, quinolizinyl, chinolyl, isochinolyl, phthalazinyl, naphthyridinyl, chinoxalinyl, chinazolinyl, cinnolinyl, pteridinyl, carbazolyl, carbolinyl, benzotriazolyl, benzoxazolyl, phenanthridinyl, acridinyl, pyrimidinyl, phenanthrolinyl, phenazinyl, isothiazolyl, phenothiazinyl, isoxazolyl, furazanyl, 4-imidazo[1,2-a]benzimidazoyl, 5-benzimidazo[1,2-a]benzimidazoyl, benzimidazolo[2,1-b][1,3]benzothiazolyl, carbazolyl, or phenoxazinyl, which can be unsubstituted or substituted. Benzimidazo[1,2-a]benzimidazo-5-yl, benzimidazo[1,2-a]benzimidazo-2-yl, carbazolyl and dibenzofuranyl are examples of a C₂₋C₁₄heteroaryl group.

C₇-C₂₅aralkyl is for example benzyl, 2-benzyl-2-propyl, β-phenyl-ethyl, α,α-dimethylbenzyl, ω-phenyl-butyl, ω,ω-dimethyl-ω-phenyl-butyl, ω-phenyl-dodecyl, ω-phenyl-octadecyl, ω-phenyl-eicosyl or ω-phenyl-docosyl, preferably C₇-C₁₈aralkyl such as benzyl, 2-benzyl-2-propyl, β-phenyl-ethyl, α,α-dimethylbenzyl, ω-phenyl-butyl, ω,ω-dimethyl-ω-phenyl-butyl, ω-phenyl-dodecyl or ω-phenyl-octadecyl, and particularly preferred C₇-C₁₂aralkyl such as benzyl, 2-benzyl-2-propyl, β-phenyl-ethyl, α,α-dimethylbenzyl, ω-phenyl-butyl, or ω,ω-dimethyl-ω-phenyl-butyl, in which both the aliphatic hydrocarbon group and aromatic hydrocarbon group may be unsubstituted or substituted. Preferred examples are benzyl, 2-phenylethyl, 3-phenylpropyl, naphthylethyl, naphthylmethyl, and cumyl.

C₅-C₁₂cycloalkyl is for example cyclopentyl, cyclohexyl, cycloheptyl, cyclooctyl, cyclononyl, cyclodecyl, cycloundecyl, cyclododecyl, preferably cyclopentyl, cyclohexyl, cycloheptyl, or cyclooctyl, which may be unsubstituted or substituted.

Possible preferred substituents of the above-mentioned groups are C₁-C₈alkyl, a hydroxyl group, a mercapto group, C₁-C₈alkoxy, C₁-C₈alkylthio, halogen, halo-C₁-C₈alkyl, or a cyano group.

The present invention relates to compounds according to the following general formulae (IIb) and (IIc) 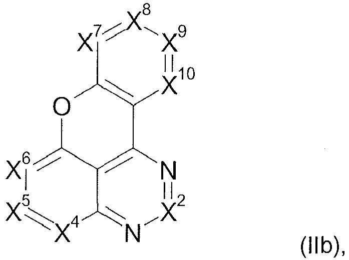 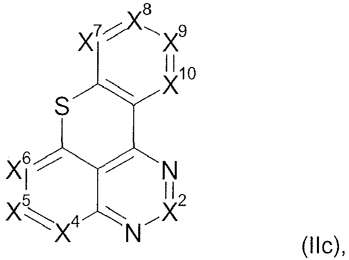 wherein independently of each other X², X⁴, X⁵, X⁶, X⁷, X⁸, X⁹ and X¹⁰ have the same meanings as mentioned above.

R⁴, R⁵, R⁶, R⁷, R⁸, R⁹ and R¹⁰ are H,
R² is a group of formula -(L¹)ₒ-(L²)ₚ-(L³)_{q}-(L⁴)ᵣ-R¹⁴, wherein L¹, L², L³ and L⁴ are independently of each other selected from a C₆-C₄₀aryl group which is unsubstituted or substituted by at least one group E and a C₁-C₂₄heteroaryl group which is unsubstituted or substituted by at least one group E, and R¹⁴ is independently of each other selected from H or a C₁-C₂₅alkyl group which unsubstituted or substituted by at least one group E and/or interrupted by at least one group D, o is 1, p is independently of each other 0 or 1, q is independently of each other 0 or 1 and r is independently of each other 0 or 1,
D is independently of each other -CO-, -COO-, -S-, -SO-, -SO₂-, -O-,-CR¹⁵=CR¹⁶-, -NR¹⁷-,-SiR²²R²³-, -POR²⁵-, -C≡C-, preferably -O-, -NR¹⁷-, -SiR²²R²³-,
E is independently of each other -OR²¹, -SR²¹, -NR¹⁷R¹⁸, -COR²⁰, -COOR¹⁹, -CONR¹⁷R¹⁸, -CN,-SiR²²R²³R²⁴, -POR²⁵R²⁷, halogen, a C₆-C₆₀aryl group which is unsubstituted or is substituted by at least one -F, -CF₃, -CF₂CF₃, -CF₂CF₂CF₃, -CF(CF₃)₂, -(CF₂)₃CF₃ or -C(CF₃)₃, a C₁-C₁₈ alkyl or a C₁-C₁₈alkyl group which is interrupted by at least one O, a C₁-C₆₀heteroaryl group which is unsubstituted or substituted by at least one -F, -CF₃, -CF₂CF₃, -CF₂CF₂CF₃,-CF(CF₃)₂, -(CF₂)₃CF₃ or -C(CF₃)₃, preferably E is independently of each other selected from -NR¹⁷R¹⁸, -CN, -SiR²²R²³R²⁴ or -POR²⁵R²⁷,
R¹⁵ and R¹⁶ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group which is interrupted by at least one O, preferably H,
R¹⁷ and R¹⁸ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O, preferably H, C₁-C₁₈ alkyl group,
R¹⁷ and R¹⁸ together form a five or six membered aliphatic, aromatic or heteroaromatic ring, preferably five or six membered aliphatic ring,
R¹⁹ is H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or a C₁-C₁₈alkyl group,
R²⁰ is H or a C₆-C₁₈aryl group which is unsubstituted or substituted by at least one C₁-C₁₈alkyl group or at least one C₁-C₁₈alkoxy group, a C₁-C₁₈ alkyl group or a C₁-C₁₈alkyl group, which is interrupted by at least one O, preferably H, a C₆-C₁₈aryl group which is unsubstituted or a C₁-C₁₈alkyl group,
R²¹ is independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or a C₁-C₁₈alkyl group,
R^{22,} R²³ and R²⁴ are independently of each other H, a C₁-C₁₈alkyl group, a C₆-C₁₈aryl group which is unsubstituted or substituted by at least one C₁-C₁₈alkyl group, preferably H, a C₆-C₁₈aryl group which is unsubstituted or a C₁-C₁₈alkyl group,
and
R²⁵ and R²⁷ are independently of each other H, a C₁-C₁₈alkyl group, a C₆-C₁₈aryl group which is unsubstituted or substituted by at least one C₁-C₁₈alkyl group, a C₇-C₂₅aralkyl which is unsubstituted or substituted by at least one group E, a C₅-C₁₂cycloalkyl group which is unsubstituted or substituted by at least one group E, preferably H, a C₆-C₁₈aryl group which is unsubstituted or a C₁-C₁₈alkyl group.

Preferably, in formula -(L¹)ₒ₋(L²)ₚ-(L³)_{q}-(L⁴)ᵣ-R¹⁴ o is independently of each other 1, p is independently of each other 1, q is independently of each other 1 and r is independently of each other 1. This preferred embodiment means that L¹ is present, followed by L², followed by L³, followed by L⁴, followed by R¹⁴.

According to a further preferred embodiment, o is independently of each other 1, p is independently of each other 1, q is independently of each other 1 and r is independently of each other 0. This preferred embodiment means that L¹ is present, followed by L², followed by L³, followed by R¹⁴, wherein L⁴ is not present.

According to a further preferred embodiment, o is independently of each other 1, p is independently of each other 1, q is independently of each other 0 and r is independently of each other 0. This preferred embodiment means that L¹ is present, followed by L², followed by R¹⁴, wherein L³ and L⁴ are not present.

According to a further preferred embodiment, o is independently of each other 1, p is independently of each other 0, q is independently of each other 0 and r is independently of each other 0. This preferred embodiment means that L¹ is present, followed by R¹⁴, wherein L⁴, L³ and L² are not present.

L ¹, L², L³ and L⁴ are independently of each other, if present, selected from a C₆-C₄₀aryl group which is unsubstituted or substituted by at least one group E or from a C₁-C₂₄heteroaryl group which is unsubstituted or substituted by at least one group E. According to a further preferred embodiment, L¹. L², L³ and L⁴ are independently of each other, if present, selected from a C₆-C₄₀aryl group which is unsubstituted or from a C₁-C₂₄heteroary! group which is unsubstituted.

According to a preferred embodiment, wherein o is independently of each other 1, p is independently of each other 0, q is independently of each other 0 and r is independently of each other 0, L¹ is selected from a C₆-C₄₀aryl group which is unsubstituted or substituted by at least one group E, preferably unsubstituted, or from a C₁-C₂₄heteroaryl group which is unsubstituted or substituted by at least one group E, preferably unsubstituted.

According to a further preferred embodiment, wherein o is independently of each other 1, p is independently of each other 1, q is independently of each other 0 and r is independently of each other 0, L¹ is selected from a C₆-C₄₀aryl group which is unsubstituted or substituted by at least one group E and L² is selected from a C₁-C₂₄heteroaryl group which is unsubstituted or substituted by at least one group E.

According to a further preferred embodiment, wherein o is independently of each other 1, p is independently of each other 1, q is independently of each other 1 and r is independently of each other 0, L¹ is selected from a C₆-C₄₀aryl group which is unsubstituted or substituted by at least one group E, preferably unsubstituted, L² is selected from a C₁-C₂₄heteroaryl group which is unsubstituted or substituted by at least one group E, preferably unsubstituted, and L³ is selected from a C₁-C₂₄heteroaryl group which is unsubstituted or substituted by at least one group E, preferably unsubstituted.

In general R¹⁴ can be selected from the group as mentioned above. Preferably, R¹⁴ is hydrogen.

Preferred C₆-C₁₈aryl or C₁-C₁₈heteroaryl rings or ring systems, preferably C₆-C₁₈aryl or C₂-C₁₈heteroaryl ring or ring system are for example fused phenylene or naphthylene rings, five or six membered fused C₁-C₁₈heteroarylene rings or ringsystems, preferably fused phenylene or naphthylene rings, five or six membered fused C₂-C₁₈heteroarylene rings or ringsystems.

Further preferred, the present invention relates to compounds according to the present invention, wherein R² is a N-heteroaryl group according to general formula (XII) 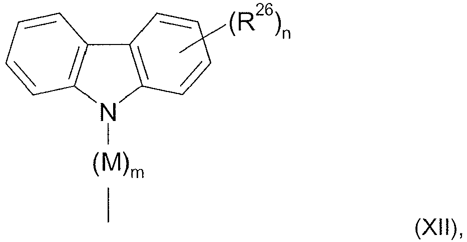 wherein
n is an integer of 0 to 8,
m is an integer of 0 to 4,
M is a C₆-C4₀ arylene group which is unsubstituted or substituted by at least one group E, a C₁-C₂₄heteroarylene group which is unsubstituted or substituted by at least one group E or a C₁-C₂₅ alkylene group which unsubstituted or substituted by at least one group E and/or interrupted by at least one group D,
R²⁶ is independently of each other selected from E, a group of formula -(L¹)ₒ-(L²)ₚ₋(L³)_{q}-(L⁴)ᵣ-R¹⁴, a C₁-C₂₅alkyl group, which is unsubstituted or substituted by at least one group E and/or interrupted by at least one group D, wherein o, p, q, r, L¹, L², L³, L⁴, R¹⁴, E and D have the meanings as mentioned above,
or
at least two of R²⁶, if present at adjacent carbon atoms, may form at least one five or six membered, substituted or unsubstituted, saturated, unsaturated, aromatic or heteroaromatic ring or ring system, to which at least one further aromatic an /or heteroaromatic ring or ringsystem may be fused.

According to this preferred embodiment of the present invention the N-heteroaryl group according to general formula (XII) is a very specific embodiment of the group of formula -(L¹)ₒ-(L²)ₚ-(L³)_{q}-(L⁴)ᵣ-R¹⁴ as defined above.

In particular, the N-heteroaryl group according to general formula (XII) is defined as follows.

In general, n is an integer of 0 to 8, wherein n describes the number of substituents R²⁶ present. If n is 0, no substituent R²⁶ is present, but all eight positions at the fused phenyl rings carry a hydrogen. In case that at least one substituent R²⁶ is present at least one hydrogen at the fused phenyl rings is replaced by this at least one substituent R²⁶.

According to a further embodiment of the present invention at least two of R²⁶, if present at adjacent carbon atoms, may form at least one five or six membered, substituted or unsubstituted, saturated, unsaturated, aromatic or heteroaromatic ring or ring system, to which at least one further aromatic or heteroaromatic ring or ringsystem may be fused to Preferably, the at least at least one further aromatic ring or ringsystem may comprise 5 to 40 carbon atoms, and the at least at least one further heteroaromatic ring or ringsystem may comprise 1 to 40 carbon atoms and heteroatoms like N, O, P or S.

According to this embodiment, at least two of R²⁶, if present at adjacent carbon atoms, may form at least one five or six membered, substituted or unsubstituted, saturated, unsaturated, aromatic or heteroaromatic ring or ring system, in combination with further substituents R²⁶ selected from E, a group of formula -(L¹)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, wherein o, p, q, r, L¹, L², L³, L⁴, R¹⁴. E and D have the meanings as mentioned above,
or
at least two of R²⁶, if present at adjacent carbon atoms, may form at least one five or six membered, substituted or unsubstituted, saturated, unsaturated, aromatic or heteroaromatic ring or ring system, to which at least one further aromatic and/or heteroaromatic ring or ringsystem may be fused, without further substituents R²⁶.

According to this embodiment at least two substituents R²⁶, if present at adjacent carbon atoms, may form at least one five or six membered, substituted or unsubstituted, saturated, unsaturated, aromatic or heteroaromatic ring or ring system, i.e. that two substituents that are present at adjacent carbon atoms, may form a five or six membered, substituted or unsubstituted, saturated, unsaturated, aromatic or heteroaromatic ring or ring system, and that further substituents R²⁶, that are present at further two adjacent carbon atoms, may form a further five or six membered, substituted or unsubstituted, saturated, unsaturated, aromatic or heteroaromatic ring or ring system. Therefore, one, two, three or four, preferably one or two, five or six membered, substituted or unsubstituted, saturated, unsaturated, aromatic or heteroaromatic ring(s) or ring system(s) may be present at the N-heteroaryl group according to general formula (XII).

According to a further preferred embodiment of the present invention at least two substituents R²⁶, if present at adjacent carbon atoms, may form a structure according to general formula (XVII) 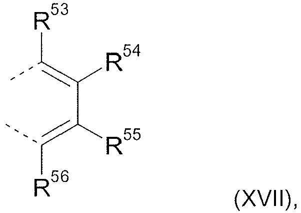 wherein R⁵³, R⁵⁴, R⁵⁵ and R⁵⁶ are independently of each other selected from H, E, a group of formula -(L¹)ₒ-(L²)ₚ-(L³)_{q}-(L⁴)ᵣR¹⁴, a C₁-C₂₅alkyl group, which is unsubstituted or substituted by at least one group E and/or interrupted by at least one group D, wherein o, p, q, r, L¹, L², L³, L⁴, R¹⁴, E and D have the meanings as mentioned above, preferably H.
or
at least two of R⁵³, R⁵⁴, R⁵⁵ or R⁵⁶, if present at adjacent carbon atoms, may form at least one five or six membered, substituted or unsubstituted, saturated, unsaturated, aromatic or heteroaromatic ring or ring system, to which further aromatic and /or heteroaromatic rings or ringsystems may be fused.

Particularly preferred, two of R²⁶, if present at adjacent carbon atoms, form a fused phenyl ring, a fused naphthyl ring, a fused phenanthryl ring, a fused carbazol ring, a fused dizenzofuran ring, a fused dibenzothiophene ring, a fused fluorene ring, and a fused fluoranthene ring, wherein, if a fused fluoranthene ring is present it is built up by three of R²⁶.

In formula (XVII), the dashed bindings are bond to the compound of general formula (XII).

Examples of substituents according to general formula (XII), in which at least two of R²⁶ form at least one five or six membered, substituted or unsubstituted, saturated, unsaturated, aromatic or heteroaromatic ring or ring system, to which at least one further aromatic and/or heteroaromatic ring or ringsystem may be fused, are shown in the following:

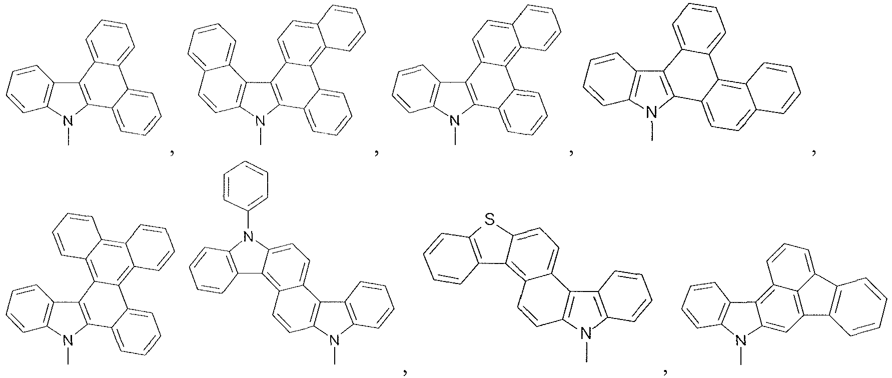

According to a preferred embodiment of the present invention the N-heteroaryl group according to general formula (XII) corresponds to a group according to general formula (XX) 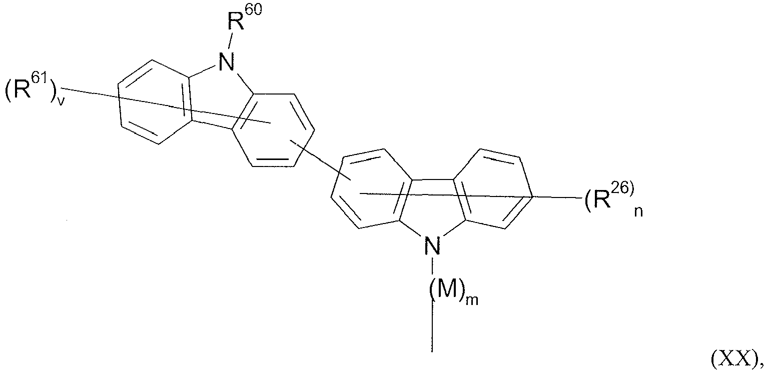 wherein
m, M, R²⁶ have the same meanings as defined above,
n is an integer of 0 to 7,
v is an integer of 0 to 7,
R⁶⁰ is independently of each other selected from H, E, a group of formula -(L¹)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, R²⁶ is independently of each other selected from E, a group of formula -(L¹)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, wherein o, p, q, r, L¹, L², L³, L⁴, R¹⁴, E and D have the meanings as defined above,
R⁶¹ is independently of each other selected from E, a group of formula -(L¹)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, wherein o, p, q, r, L¹, L², L³, L⁴, R¹⁴, E and D have the meanings as defined above,
or at least two of R⁶¹, if present at adjacent carbon atoms, may form at least one five or six membered, substituted or unsubstituted, saturated, unsaturated, aromatic or heteroaromatic ring or ring system, to which at least one further aromatic and/or heteroaromatic ring or ring system may be fused.

In the N-heteroaryl group according to general formula (XX) according to the present invention, n is in general an integer of 0 to 7, for example 0, 1, 2, 3, 4, 5, 6 or 7, wherein n describes the number of substituents R²⁶ present. If n is 0, no substituent R²⁶ is present, but all seven positions at the fused phenyl rings carry a hydrogen and at least one position is substituted by 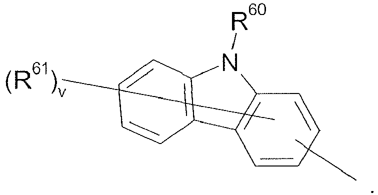 In case that at least one substituent R²⁶ is present, at least one hydrogen at the fused phenyl rings is replaced by this at least one substituent R²⁶.

In the N-heteroaryl group according to general formula (XX) according to the present invention, v is in general an integer of 0 to 7, for example 0, 1, 2, 3, 4, 5, 6 or 7, wherein v describes the number of substituents R⁶¹ present. If v is 0, no substituent R⁶¹ is present, but all seven positions at the substituent 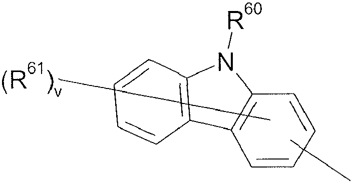 carry a hydrogen atom.

R⁶⁰, if present, are independently of each other selected from a group of formula -(L¹)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, wherein o is independently of each other 0 or 1, p is independently of each other 0 or 1, q is independently of each other 0 or 1 and r is independently of each other 0 or 1, and L¹, L², L³ and L⁴ are independently of each other selected from a C₆-C₄₀aryl group which is unsubstituted or substituted by at least one group E, C₁-C₂₄heteroary! group which is unsubstituted or substituted by at least one group E, wherein R¹⁴ has the meanings as mentioned above.

Preferably, if R⁶⁰ is -(L¹)ₒ-(L²)ₚ-(L³)_{q}-(L⁴)ᵣ-R¹⁴, o is independently of each other 1, p is independently of each other 1, q is independently of each other 1 and r is independently of each other 1. This preferred embodiment means that L¹ is present, followed by L², followed by L³, followed by L⁴, followed by R¹⁴.

According to a further preferred embodiment, if R⁶⁰ is -(L¹)ₒ-(L²)ₚ-(L³)_{q}-(L⁴)ᵣ-R¹⁴, o is independently of each other 1, p is independently of each other 1, q is independently of each other 1 and r is independently of each other 0. This preferred embodiment means that L¹ is present, followed by L², followed by L³, followed by R¹⁴, wherein L⁴ is not present.

According to a further preferred embodiment, if R⁶⁰ is -(L¹)ₒ-(L²)ₚ-(L³)_{q}-(L⁴)ᵣ-R¹⁴, o is independently of each other 1, p is independently of each other 1, q is independently of each other 0 and r is independently of each other 0. This preferred embodiment means that L¹ is present, followed by L², followed by R¹⁴, wherein L³ and L⁴ are not present.

According to a further preferred embodiment, if R⁶⁰ is -(L¹)ₒ-(L²)ₚ-(L³)_{q}-(L⁴)ᵣ-R¹⁴, o is independently of each other 1, p is independently of each other 0, q is independently of each other 0 and r is independently of each other 0. This preferred embodiment means that L¹ is present, followed by R¹⁴, wherein L⁴, L³ and L² are not present.

According to a further preferred embodiment, if R⁶⁰ is -(L¹)ₒ-(L²)ₚ-(L³)_{q}-(L⁴)ᵣ-R¹⁴, o is independently of each other 0, p is independently of each other 0, q is independently of each other 0 and r is independently of each other 0. This preferred embodiment means that the compounds are substituted by R¹⁴, wherein L⁴, L³, L² and L¹ are not present.

Preferably, if R⁶⁰ is -(L¹)ₒ-(L2)ₚ-(L³)_{q}-(L⁴)ᵣ-R¹⁴, L¹, L², L³ and L⁴ are independently of each other, if present, selected from a C₆-C₄₀aryl group which is unsubstituted or substituted by at least one group E or from a C₁-C₂₄heteroaryl group which is unsubstituted or substituted by at least one group E. According to a further preferred embodiment, L¹. L², L³ and L⁴ are independently of each other, if present, selected from a C₆-C₄₀aryl group which is unsubstituted or from a C₁-C₂₄heteroaryl group which is unsubstituted.

According to a preferred embodiment, if R⁶⁰ is -(L¹)ₒ-(L²)ₚ-(L³)_{q}-(L⁴)ᵣ-R¹⁴, wherein o is independently of each other 1, p is independently of each other 0, q is independently of each other 0 and r is independently of each other 0, L¹ is selected from a C₆-C₄₀aryl group which is unsubstituted or substituted by at least one group E, preferably unsubstituted, or from a C₁-C₂₄heteroaryl group which is unsubstituted or substituted by at least one group E, preferably unsubstituted. According to a further preferred embodiment, if R⁶⁰ is -(L¹)ₒ-(L²)ₚ-(L³)_{q}-(L⁴)ᵣ-R¹⁴, wherein o is independently of each other 1, p is independently of each other 1, q is independently of each other 0 and r is independently of each other 0, L¹ is selected from a C₆-C₄₀aryl group which is unsubstituted or substituted by at least one group E and L² is selected from a C₁-C₂₄heteroary! group which is unsubstituted or substituted by at least one group E.

According to a further preferred embodiment, if R⁶⁰ is -(L¹)ₒ-(L²)ₚ-(L³)_{q}-(L⁴)ᵣ-R¹⁴, wherein o is independently of each other 1, p is independently of each other 1, q is independently of each other 1 and r is independently of each other 0, L¹ is selected from a C₆-C₄₀aryl group which is unsubstituted or substituted by at least one group E, preferably unsubstituted, L² is selected from a C₁-C₂₄heteroaryl group which is unsubstituted or substituted by at least one group E, preferably unsubstituted, and L³ is selected from a C₁-C₂₄heteroaryl group which is unsubstituted or substituted by at least one group E, preferably unsubstituted.

If R⁶⁰ is -(L¹)ₒ-(L²)ₚ-(L³)_{q}-(L⁴)ᵣ-R¹⁴ R¹⁴ is independently of each other selected from H, E, a C₆-C₂₄aryl group which is unsubstituted or substituted by at least one group E, C₁-C₂₄heteroaryl group which is unsubstituted or substituted by at least one group E, a C₁-C₂₅alkyl group which unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably R¹⁴ is hydrogen.

Particularly preferred, R⁶⁰ is independently of each other selected from hydrogen, phenyl, phenyl substituted by naphthalene, biphenyl, naphthyl, naphthyl substituted by phenyl ring, phenanthryl, triphenylenyl, fluorenyl, dibenzofuryl or dibenzothienyl.

R⁶¹ is independently of each other selected from E, a group of formula -(L¹)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, wherein o, p, q, r, L¹, L², L³, L⁴, R¹⁴, E and D have the meanings as mentioned above,
or
at least two of R⁶¹, if present at adjacent carbon atoms, may form at least one five or six membered, substituted or unsubstituted, saturated, unsaturated, aromatic or heteroaromatic ring or ring system, to which at least one further aromatic an /or heteroaromatic ring or ringsystem may be fused.

According to a further embodiment of the present invention at least two of R⁶¹, if present at adjacent carbon atoms, may form at least one five or six membered, substituted or unsubstituted, saturated, unsaturated, aromatic or heteroaromatic ring or ring system, to which at least one further aromatic or heteroaromatic ring or ringsystem may be fused to Preferably, the at least at least one further aromatic ring or ringsystem may comprise 5 to 40 carbon atoms, and the at least at least one further heteroaromatic ring or ringsystem may comprise 1 to 40 carbon atoms and heteroatoms like N, O, P or S.

According to this embodiment, at least two of R⁶¹, if present at adjacent carbon atoms, may form at least one five or six membered, substituted or unsubstituted, saturated, unsaturated, aromatic or heteroaromatic ring or ring system, in combination with further substituents R⁶¹ selected from E, a group of formula -(L¹)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, wherein o, p, q, r, L¹, L², L³, L⁴, R¹⁴, E and D have the meanings as mentioned above,
or
at least two of R⁶¹, if present at adjacent carbon atoms, may form at least one five or six membered, substituted or unsubstituted, saturated, unsaturated, aromatic or heteroaromatic ring or ring system, to which at least one further aromatic and/or heteroaromatic ring or ringsystem may be fused, without further substituents R⁶¹.

According to this embodiment at least two substituents R⁶¹, if present at adjacent carbon atoms, may form at least one five or six membered, substituted or unsubstituted, saturated, unsaturated, aromatic or heteroaromatic ring or ring system, i.e. that two substituents that are present at adjacent carbon atoms, may form a five or six membered, substituted or unsubstituted, saturated, unsaturated, aromatic or heteroaromatic ring or ring system, and that further substituents R⁶¹, that are present at further two adjacent carbon atoms, may form a further five or six membered, substituted or unsubstituted, saturated, unsaturated, aromatic or heteroaromatic ring or ring system. Therefore, one, two, three or four, preferably one or two, five or six membered, substituted or unsubstituted, saturated, unsaturated, aromatic or heteroaromatic ring(s) or ring system(s) may be present at the N-heteroaryl group according to general formula (XX).

According to a further preferred embodiment of the present invention at least two substituents R⁶¹, if present at adjacent carbon atoms, may form a structure according to general formula (XIV) 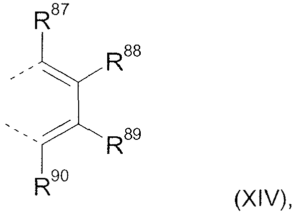 wherein R⁸⁷, R⁸⁸, R⁸⁹ and R⁹⁰ are independently of each other selected from H, E, a group of formula -(L¹)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, wherein o, p, q, r, L¹, L², L³, L⁴, R¹⁴, E and D have the meanings as mentioned above,
or
at least two of R⁸⁷, R⁸⁸, R⁸⁹ or R⁹⁰, if present at adjacent carbon atoms, may form at least one five or six membered, substituted or unsubstituted, saturated, unsaturated, aromatic or heteroaromatic ring or ring system, to which further aromatic and /or heteroaromatic rings or ringsystems may be fused.

Particularly preferred, two of R⁶¹, if present at adjacent carbon atoms, form a fused phenyl ring, a fused naphthyl ring, a fused phenanthryl ring, a fused carbazol ring, a fused dibenzofuran ring, a fused dibenzothiophene ring, a fused fluorene ring, and a fused fluoranthene ring, wherein, if a fused fluoranthene ring is present it is built up by three of R⁶¹.

In formula (XIV), the dashed bindings are bond to the compound of general formula (XX).

The present invention further relates to a compound according to general formula (lib) or (IIc) according to the present invention, wherein R² is a heterocyclic group represented by general formula (XXI) 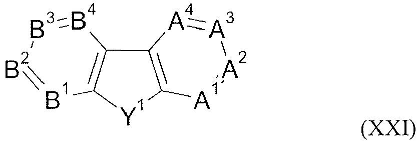 wherein
A¹ is CR⁶² or N,
A² is CR⁶³ or N,
A³ is CR⁶⁴ or N,
A⁴ is CR⁶⁵ or N,
B¹ is CR⁶⁶ or N,
B² is CR⁶⁷ or N,
B³ is CR⁶⁸ or N,
B⁴ is CR⁶⁹ or N,
Y¹ is independently of each other NR⁷⁰, CR⁷¹R⁷², O or S
R⁶², R⁶³, R⁶⁴, R⁶⁵, R⁶⁶, R⁶⁷, R⁶⁸ and R⁶⁹ are independently of each other selected from H, direct bond, E, a group of formula -(L¹)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H, phenyl or carbazolyl,
and/or
at least two of R⁶², R⁶³, R⁶⁴ and R⁶⁵ , if present are directly bonded to the moiety represented by the general formula (XXII) by the two *-locations. 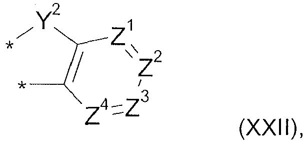 wherein
Y² is independently of each other NR⁷³, CR⁷⁴R⁷⁵, O or S,
Z¹ is CR⁷⁶,
Z² is CR⁷⁷,
Z³ is CR⁷⁸
Z⁴ is CR⁷⁹,
and/or
R62, R⁶³, R⁶⁴ or R⁶⁵, if present at adjacent carbon atoms, together form at least one C₆-C₁₈aryl or C₂-C₁₈heteroaryl ring or ring system,
and/or
at least two of R⁶⁶, R⁶⁷, R⁶⁸ and R⁶⁹, if present are directly bonded to the moiety represented by the general formula (XXIII) by the two *-locations. 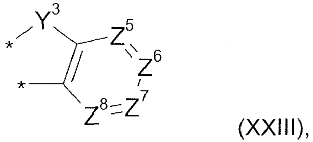 wherein
Y³ is independently of each other NR⁸⁰, CR⁸¹R⁸², O or S,
Z⁵ is CR⁸³,
Z⁶ is CR⁸⁴,
Z⁷ is CR⁸⁵,
Z⁸ is CR⁸⁶,
and/or
at least two of R⁶⁶, R⁶⁷, R⁶⁸ or R⁶⁹, if present at adjacent carbon atoms, together form at least one C₆-C₁₈aryl or C₂-C₁₈heteroaryl ring or ring system,
R⁷⁰, R⁷³ and R⁸⁰ are independently of each other selected from direct bond, H, E, a group of formula -(L¹)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
R⁷¹, R⁷², R⁷⁴, R⁷⁵, R⁸¹ and R⁸² are, independently of each other selected from H, direct bond, E, a group of formula -(L¹)ₒ-(L²)ₚ₋(L³)_{q}-(L⁴)ᵣ-R¹⁴, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
or R⁷¹ and R⁷², R⁷⁴ and R⁷⁵ and/or R⁸¹ and R⁸² together form at least one C₃-C₁₈-alkyl ring or ring system to which at least one C₆-C₁₈aryl ring or ring system may be attached,
R⁷⁶, R⁷⁷, R⁷⁸, R⁷⁹, R⁸³, R⁸⁴, R⁸⁵ and R⁸⁶ are independently of each other selected from H, direct bond, E, a group of formula -(L¹)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
and/or
at least two of R⁷⁶, R⁷⁷, R⁷⁸, R⁷⁹, R⁸³, R⁸⁴, R⁸⁵ or R⁸⁶, if present at adjacent carbon atoms, together form at least one C₆-C₁₈aryl or C₂-C₁₈heteroaryl ring or ring system,
wherein the substituent according to general formula (XXI) is connected to the compound according to general formulae (lib) and (IIc) via one of R⁷⁰, R⁷³, R⁸⁰, R⁶², R⁶³, R⁶⁴, R⁶⁵, R⁶⁶, R⁶⁷, R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁸⁴, R⁸⁵ or R⁸⁶, if present, preferably R⁷⁰, R⁷³ or R⁸⁰, if present, wherein this respective R⁷⁰, R⁷³, R⁸⁰, R⁶², R⁶³, R⁶⁴, R⁶⁵, R⁶⁶, R⁶⁷, R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁸⁴, R⁸⁵ or R⁸⁶, preferably R⁷⁰, R⁷³ or R⁸⁰, is a direct bond, optionally interrupted by a group of formula -(M)ₘ-, in this case,
m is an integer of 0 to 4,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which unsubstituted or substituted by at least one group E and/or interrupted by at least one group D, o is independently of each other 0 or 1, p is independently of each other 0 or 1, q is independently of each other 0 or 1 and r is independently of each other 0 or 1,
L¹, L², L³ and L⁴ are independently of each other selected from a C₆-C₄₀aryl group which is unsubstituted or substituted by at least one group E, C₁-C₂₄heteroary! group which is unsubstituted or substituted by at least one group E,
R¹⁴ is independently of each other selected from H, E, a C₆-C₂₄aryl group which is unsubstituted or substituted by at least one group E, C₁-C₂₄heteroaryl group which is unsubstituted or substituted by at least one group E, a C₁-C₂₅alkyl group which unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
D is independently of each other -CO-, -COO-, -S-, -SO-, -SO₂-, -O-,-CR¹⁵=CR¹⁶-, -NR¹⁷-,-SiR²²R²³-, -POR²⁵-, -C≡C-,
E is independently of each other -OR²¹, -SR²¹, -NR¹⁷R¹⁸, -COR²⁰, -COOR¹⁹, -CONR¹⁷R¹⁸, -CN,-SiR²²R²³R²⁴, POR²⁵R²⁷, halogen, a C₆-C₆₀aryl group which is unsubstituted or is substituted by at least one -F, -CF₃, -CF₂CF₃, -CF₂CF₂CF₃, -CF(CF₃)₂, -(CF₂)₃CF₃ or -C(CF₃)₃, a C₁-C₁₈alkyl or a C₁-C₁₈alkyl group which is interrupted by at least one O, a C₁-C₆₀heteroaryl group which is unsubstituted or substituted by at least one -F, -CF₃, -CF₂CF₃, -CF₂CF₂CF₃, -CF(CF₃)₂, -(CF₂)₃CF₃ or -C(CF₃)₃,
R¹⁵ and R¹⁶ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group which is interrupted by at least one O,
R¹⁷ and R¹⁸ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O, or
R¹⁷ and R¹⁸ together form a five or six membered aliphatic, aromatic or heteroaromatic ring,
R¹⁹ is H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O,
R²⁰ is H or a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O,
R²¹ is independently of each other H, a C₆-C₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O,
R^{22,} R²³ and R²⁴ are independently of each other H, a C₁-C₁₈alkyl group, a C₆-C₁₈aryl group which is unsubstituted or substituted by at least one C₁-C₁₈alkyl group, and
R²⁵ and R²⁷ are independently of each other H, a C₁-C₁₈alkyl group, a C₆-C₁₈aryl group which is unsubstituted or substituted by at least one C₁-C₁₈alkyl group, a C₇-C₂₅aralkyl which is unsubstituted or substituted by at least one group E, a C₅-C₁₂cycloalkyl group which is unsubstituted or substituted by at least one group E.

According to the present invention the heterocyclic group according to general formula (XXI), that may be for example a dibenzofuran, dibenzothiophene or benzofurodibenzofuran group, can be attached to the compound of general formula (IIb) or (IIc) via any atom present in the heterocyclic ring system. Therefore, any of R⁷⁰, R⁷³, R⁸⁰, R⁶², R⁶³, R⁶⁴, R⁶⁵, R⁶⁶, R⁶⁷, R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁸⁴, R⁸⁵ or R⁸⁶, if present, can be a direct bond, optionally interrupted by a group of formula -(M)m-, with which the heterocyclic group according to formula (XXI) can be attached to the compound of general formula (lib) or (IIc).

According to a preferred embodiment of the present invention, the heterocyclic group according to general formula (XXI) is represented by any one of general formula (XXIa), (XXIb) or (XXIc) 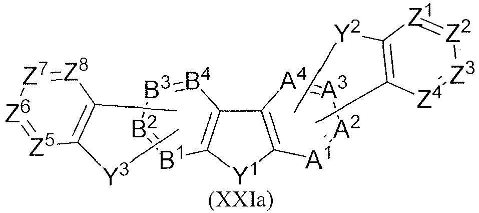 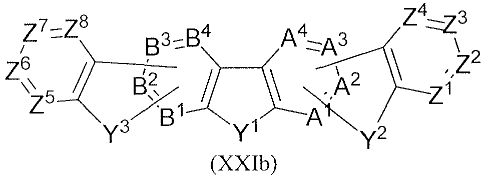 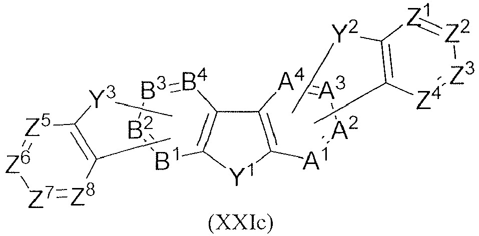 wherein A¹, A², A³, A⁴, B¹ B², B³, B⁴, Z¹, Z², Z³, Z⁴, Z⁵, Z⁶, Z⁷, Z⁸, Y¹, Y² and Y³ have the same meanings as defined above.

According to a further preferred embodiment of the present invention one of Y¹, Y² and Y³ in the compound according general formulae (XXI), (XXIa), (XXIb) and/or (XXIc) is NR⁷⁰, NR⁷³ or NR⁸⁰ respectively.

According to a further preferred embodiment of the present invention two of Y¹, Y² and Y³ in the compound according to general formulae (XXI), (XXIa), (XXIb) and/or (XXIc) are NR⁷⁰, NR⁷³ or NR⁸⁰ respectively.

According to a preferred embodiment of the present invention the N-heteroaryl group according to general formula (XII) corresponds to a N-heteroaryl group according to general formula (XXI) 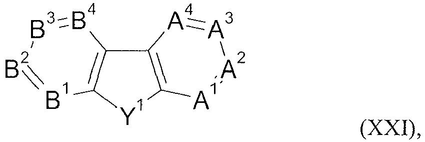
wherein A¹ is CR⁶²,
A² is CR⁶³ or N preferably CR⁶³,
A³ is CR⁶⁴ or N, preferably CR⁶⁴,
A⁴ is CR⁶⁵ or N, preferably CR⁶⁵,
B¹ is CR⁶⁶ or N, preferably CR⁶⁶,
B² is CR⁶⁷ or N, preferably CR⁶⁷,
B³ is CR⁶⁸ or N, preferably CR⁶⁸,
B⁴ is CR⁶⁹ or N, preferably CR⁶⁹,
Y¹ is independently of each other NR⁷⁰,
R⁶⁶, R⁶⁷, R⁶⁸ and R⁶⁹ are independently of each other selected from direct bond, H, E, a group of formula -(L1)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H, phenyl or carbazolyl,
and
at least two of R⁶², R⁶³, R⁶⁴ and R⁶⁵ are direct bond and, if present at adjacent carbon atoms, are directly connected to the two *-locations in the moiety of general formula (XXII) 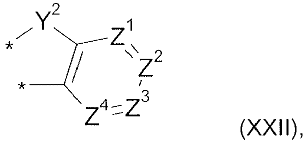 wherein
Y² is independently of each other NR⁷³, CR⁷⁴R⁷⁵, O or S,
Z¹ is CR⁷⁶,
Z² is CR⁷⁷,
Z³ is CR⁷⁸,
Z⁴ is CR⁷⁹,
and the remaining of R⁶², R⁶³, R⁶⁴ and R⁶⁵ that are not direct bond are independently of each other selected from direct bond, H, E, a group of formula -(L¹)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₂cycloalkyl group which is unsubstituted or substituted by at least one group E, preferably H, phenyl or carbazolyl,
wherein R⁷⁶, R⁷⁷, R⁷⁸ and R⁷⁹ are independently of each other selected from H, E, a group of formula -(L¹)ₒ₋(L²)ₚ-(L³)_{q}-(L⁴)ᵣ₋R¹⁴, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably hydrogen,
R⁷³, R⁷⁴ and R⁷⁵ have the same meanings as mentioned above,
and the substituent according to general formula (XXI) is connected to the compound according to general formula (IIb) or (IIc) via R⁷⁰ being a direct bond, which is optionally interrupted by a group of formula -(M)ₘ-, wherein M and m have the same meanings as mentioned above, preferably m is 0 or 1.

According to a preferred embodiment of the present invention the N-heteroaryl group according to general formula (XII) corresponds to a N-heteroaryl group according to general formula (XXI) 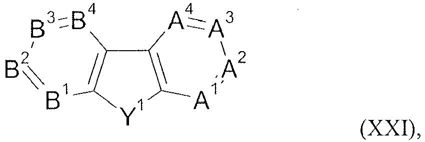 wherein
A² is CR⁶³ or N preferably CR⁶³,
A³ is CR⁶⁴ or N, preferably CR⁶⁴,
A⁴ is CR⁶⁵ or N, preferably CR⁶⁵,
B¹ is CR⁶⁶ or N, preferably CR⁶⁶,
B² is CR⁶⁷ or N, preferably CR⁶⁷,
B³ is CR⁶⁸ or N, preferably CR⁶⁸,
B⁴ is CR⁶⁹ or N, preferably CR⁶⁹,
Y¹ is independently of each other NR⁷⁰, CR⁷¹R⁷², O or S
at least two of R⁶², R⁶³, R⁶⁴ and R⁶⁵ are direct bond and, if present at adjacent carbon atoms, are directly connected to the two *-locations in the moiety of general formula (XXII) 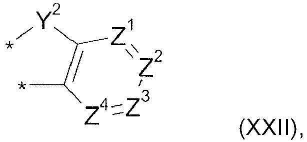 wherein
Y² is independently of each other NR⁷³, CR⁷⁴R⁷⁵, O or S,
Z¹ is CR⁷⁶,
Z² is CR⁷⁷,
Z³ is CR⁷⁸
Z⁴ is CR⁷⁹,
and the remaining of R⁶², R⁶³, R⁶⁴ and R⁶⁵ that are not direct bond are independently of each other selected from direct bond, H, E, a group of formula -(L¹)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H or phenyl,
and
at least two of R⁶⁶, R⁶⁷, R⁶⁸ and R⁶⁹, if present at adjacent carbon atoms, are directly connected to the two *-locations in the moiety of general formula (XXIII) 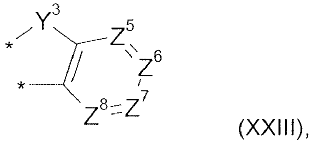 wherein
Y³ is independently of each other NR⁸⁰,
Z⁵ is CR⁸³,
Z⁶ is CR⁸⁴,
Z⁷ is CR⁸⁵,
Z⁸ is CR⁸⁶,
and the remaining of R⁶⁶, R⁶⁷, R⁶⁸ and R⁶⁹ that are not direct bond are independently of each other selected from direct bond, H, E, a group of formula -(L)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H or phenyl,
R⁷⁶, R⁷⁷, R⁷⁸, R⁷⁹, R⁸³, R⁸⁴, R⁸⁵ and R⁸⁶ are independently of each other selected from direct bond, H, E, a group of formula -(L¹)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂Cycloalkyl group which is unsubstituted or substituted by at least one group E, preferably H or phenyl,
R⁷⁰, R⁷¹, R⁷², R⁷³, R⁷⁴ and R⁷⁵ have the same meanings as mentioned above,
and the substituent according to general formula (XXI) is connected to the compound according to general formula (IIb) or (IIc) via R⁸⁰ being a direct bond, which is optionally interrupted by a group of formula -(M)ₘ-, wherein M and m have the same meanings as mentioned above, preferably m is 0 or 1.

According to a preferred embodiment of the present invention the N-heteroaryl group according to general formula (XII) corresponds to a N-heteroaryl group according to general formula (XXI) 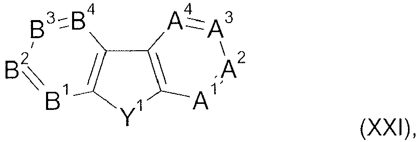 wherein
A² is CR⁶³ or N preferably CR⁶³,
A³ is CR⁶⁴ or N, preferably CR⁶⁴,
A⁴ is CR⁶⁵ or N, preferably CR⁶⁵,
B¹ is CR⁶⁶ or N, preferably CR⁶⁶,
B² is CR⁶⁷ or N, preferably CR⁶⁷,
B³ is CR⁶⁸ or N, preferably CR⁶⁸,
B⁴ is CR⁶⁹ or N, preferably CR⁶⁹,
Y¹ is independently of each other NR⁷⁰,
at least two of R⁶², R⁶³, R⁶⁴ and R⁶⁵ are direct bond and, if present at adjacent carbon atoms, are directly connected to the two *-locations in the moiety of general formula (XXII) 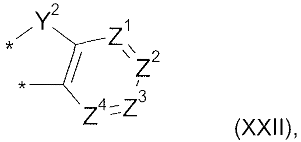 wherein
Y² is independently of each other NR⁷³, CR⁷⁴R⁷⁵, O or S,
Z¹ is CR⁷⁶,
Z² is CR⁷⁷,
Z³ is CR⁷⁸
Z⁴ is CR⁷⁹,
and the remaining of R⁶², R⁶³, R⁶⁴ and R⁶⁵ that are not direct bond are independently of each other selected from direct bond, H, E, a group of formula -(L¹)ₒ-(L²)ₚ-(L³)_{q}-(L⁴)ᵣ-R¹⁴, a C₁₋C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H or phenyl,
and
at least two of R⁶⁶, R⁶⁷, R⁶⁸ and R⁶⁹, if present at adjacent carbon atoms, are directly connected to the two *-locations in the moiety of general formula (XXIII) 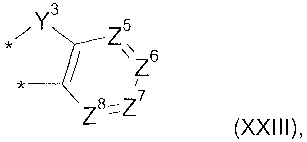 wherein
Y³ is independently of each other NR⁸⁰, CR⁸¹R⁸², O or S,
Z⁵ is CR⁸³,
Z⁶ is CR⁸⁴,
Z⁷ is CR⁸⁵,
Z⁸ is CR⁸⁶,
and the remaining of R⁶⁶, R⁶⁷, R⁶⁸ and R⁶⁹ that are not direct bond are independently of each other selected from direct bond, H, E, a group of formula -(L¹)ₒ₋(L²)ₚ₋(L³)_{q}-(L⁴)ᵣ₋R¹⁴, a C₁₋C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H or phenyl,
R⁷⁶, R⁷⁷, R⁷⁸, R⁷⁹, R⁸³, R⁸⁴, R⁸⁵ and R⁸⁶ are independently of each other selected from direct bond, H, E, a group of formula -(L¹)ₒ-(L²)ₚ-(L³)_{q}-(L⁴)ᵣ-R¹⁴, a C₁₋C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H or phenyl,
R⁷³, R⁷⁴, R⁷⁵, R⁸⁰, R⁸¹ and R⁸² have the same meanings as mentioned above,
and the substituent according to general formula (XXI) is connected to the compound according to general formula (IIb) or (IIc) via R⁷⁰ being a direct bond, which is optionally interrupted by a group of formula -(M)ₘ-, wherein M and m have the same meanings as mentioned above, preferably m is 0 or 1.

According to a preferred embodiment of the present invention the N-heteroaryl group according to general formula (XII) corresponds to the heteroaryl group according to the following formula (XIII) 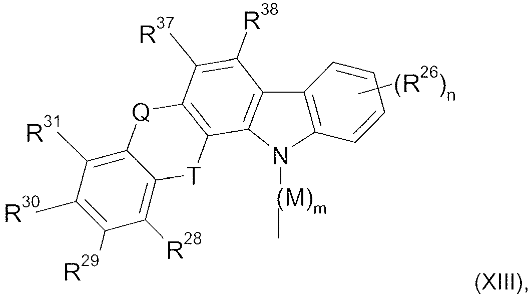
wherein M, m and R²⁶ have the meanings as mentioned above, n is 0 to 4,
R²⁸, R²⁹, R³⁰, R³¹, R³⁷ and R³⁸ are independently of each other selected from H, E, a group of formula -(L¹)ₒ-(L²)ₚ-(L³)_{q}-(L⁴)ᵣR¹⁴, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H, phenyl, biphenyl,
or
at least two of R²⁸, R²⁹, R³⁰, R³¹, R³⁷ or R³⁸, if present at adjacent carbon atoms, together form at least one C₆-C₁₈aryl or C₂-C₁₈heteroaryl ring or ring system, and
Q and T are independently of each other selected from direct bond, S, O, SiR³²R³³, CR³⁴R³⁵ or NR³⁶,
wherein R³² and R³³ are independently of each other H, a C₁-C₁₈alkyl group, a C₆-C₁₈aryl group which is unsubstituted or substituted by at least one C₁-C₁₈alkyl group, preferably H, a C₆-C₁₈aryl group which is unsubstituted or a C₁-C₁₈alkyl group, preferably H, methyl, ethyl, or phenyl,
R³⁴ and R³⁵ are independently of each other H, E, a C₆-C₂₄aryl group which is unsubstituted or substituted by at least one group E, C₁-C₂₄heteroaryl group which is unsubstituted or substituted by at least one group E, a C₁-C₂₅alkyl group which unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, or spiro group, wherein R³⁴ and R³⁵ together form a five or six membered, substituted or unsubstituted, aliphatic ring, preferably H, methyl, ethyl, phenyl, or spiro group, wherein R³⁴ and R³⁵ together form a five or six membered, substituted or unsubstituted, aliphatic ring,
R³⁶ is H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or a C₁-C₁₈alkyl group,
wherein D and E have the same meanings as mentioned above.

According to a particularly preferred embodiment of the present invention, in the compound of formula (XIII), Q is direct bond, T is NR³⁶, R³⁶ is phenyl, , R³⁷ and R³⁸ are H, m is 0, n is 0 and R²⁸, R²⁹, R³⁰ and R³¹ are H.

According to a further preferred embodiment, the heteroaryl group according to general formula (XIII) corresponds to general formula (XIV) 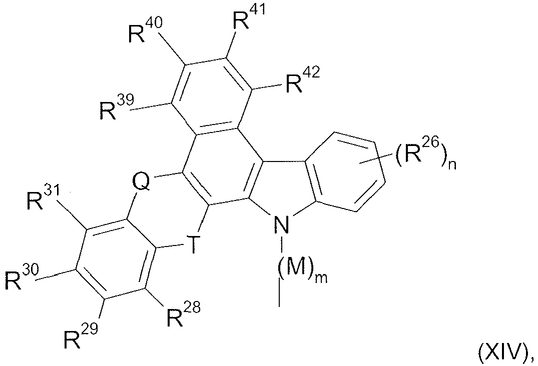
wherein R²⁶, R²⁸, R²⁹, R³⁰, R³¹, M, Q, T, n and m have the same meanings as mentioned above and
R³⁹, R⁴⁰, R⁴¹, R⁴² are independently of each other selected from H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or a C₁-C₁₈alkyl group, preferably R³⁹, R⁴⁰, R⁴¹ and R⁴² are H.

According to a particularly preferred embodiment of the present invention, in the compound of formula (XIV), Q is direct bond, T is S, R³⁹, R⁴⁰, R⁴¹ and R⁴² are H, m is 0 or 1, n is 0 and R²⁸, R²⁹, R³⁰ and R³¹ are H.

According to a further particularly preferred embodiment of the present invention, in the compound of formula (XIV), Q is direct bond, T is NR³⁶, R³⁶ is phenyl, R³⁹, R⁴⁰, R⁴¹ and R⁴² are H, m is 0, n is 0 and R²⁸, R²⁹, R³⁰ and R³¹ are H.

According to a particularly preferred embodiment of the present invention, in the compound of formula (XIV), Q is direct bond, T is CR³⁴R³⁵, R³⁴ and R³⁵ are methyl, R³⁹, R⁴⁰, R⁴¹ and R⁴² are H, m is 0, n is 0 and R²⁸, R²⁹, R³⁰ and R³¹ are H.

According to a further particularly preferred embodiment of the present invention, in the compound of formula (XIV), Q is S, T is direct bond, R³⁹, R⁴⁰, R⁴¹ and R⁴² are H, m is 0, n is 0 and R²⁸, R²⁹, R³⁰ and R³¹ are H.

According to a further particularly preferred embodiment of the present invention, in the compound of formula (XIV), Q is NR³⁶, T is direct bond, R³⁶ is phenyl, R³⁹, R⁴⁰, R⁴¹ and R⁴² are H, m is 0, n is 0 and R²⁸, R²⁹, R³⁰ and R³¹ are H.

According to a further preferred embodiment of the present invention the N-heteroaryl group according to general formula (XII) corresponds to the heteroaryl group according to the following formula (XV) 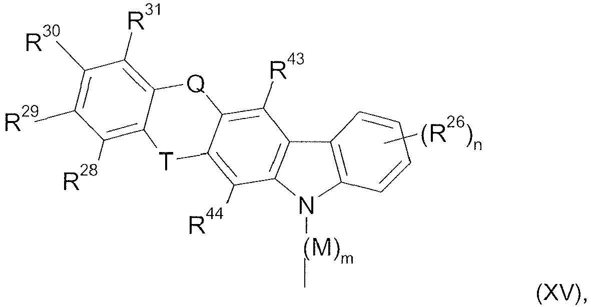
wherein M, m and R²⁶ have the meanings as mentioned above, n is 0 to 4,
R²⁸, R²⁹, R³⁰, R³¹, R⁴³ and R⁴⁴ are independently of each other selected from H, E, a group of formula -(L¹)ₒ-(L²)ₚ₋(L³)_{q}-(L⁴)ᵣ-R¹⁴, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H,
or
at least two of R²⁸, R²⁹, R³⁰ or R³¹, if present at adjacent carbon atoms, together form at least one C₆-C₈aryl or C₂-C₁₈heteroaryl ring or ring system, and
Q and T are independently of each other selected from direct bond, S, O, SiR³²R³³, CR³⁴R³⁵ or NR³⁶,
wherein R³² and R³³ are independently of each other H, a C₁-C₁₈alkyl group, a C₆-C₁₈aryl group which is unsubstituted or substituted by at least one C₁-C₁₈alkyl group, preferably H, a C₆-C₁₈aryl group which is unsubstituted or a C₁-C₁₈alkyl group, preferably H, methyl, ethyl or phenyl,
R³⁴ and R³⁵ are independently of each other H, E, a C₆-C₂₄aryl group which is unsubstituted or substituted by at least one group E, C₁-C₂₄heteroaryl group which is unsubstituted or substituted by at least one group E, a C₁₋C₂₅alkyl group which unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, or spiro group, wherein R³⁴ and R³⁵ together form a five or six membered, substituted or unsubstituted, aliphatic ring, preferably H, ethyl, ethyl, phenyl, and spiro group, wherein R³⁴ and R³⁵ together form a five or six membered, substituted or unsubstituted, aliphatic ring,
R³⁶ is H, a C₆-C₁₈aryl group which is unsubstituted or substituted by at least one C₁-C₁₈alkyl group or at least one C₁₋C₁₈alkoxy group, a C₁-C₁₈alkyl group or a C₁-C₁₈alkyl group, which is interrupted by at least one O, preferably H, a C₆-C₈aryl group which is unsubstituted or a C₁-C₁₈alkyl group, for example phenyl,
wherein D and E have the same meanings as mentioned above.

According to a particular preferred embodiment of the present invention in substituent according to general formula (XV), m is 0, n is 0, R²⁸, R²⁹, R³⁰, R³¹, R⁴³ and R⁴⁴ are H, Q is a direct bond, T is CR³⁴R³⁵, R³⁴ and R³⁵ are methyl.

According to a further particular preferred embodiment of the present invention in substituent according to general formula (XV), m is 0, n is 2, R²⁸, R²⁹, R³⁰, R³¹, R⁴³ and R⁴⁴ are H, two R²⁶ that are present at adjacent carbon atoms together form a fused phenyl ring, which is preferably present in position of R³⁰ and R³¹, Q is a direct bond, T is CR³⁴R³⁵, R³⁴ and R³⁵ are methyl.

According to a further preferred embodiment of the present invention, in substituent according to general formulae (XII), (XIII), (XIV) or (XV) R²⁶ may correspond to the following formula (XVI) 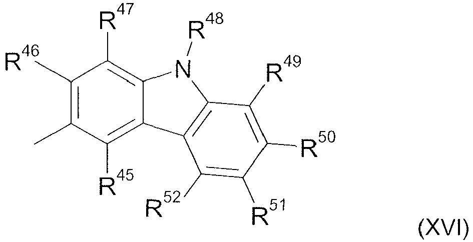
wherein R⁴⁵, R⁴⁶, R⁴⁷, R⁴⁸, R⁴⁹, R⁵⁰, R⁵¹ and R⁵² are independently of each other H, E, a group of formula -(L¹)ₒ-(L²)ₚ-(L³)_{q}-(L⁴)ᵣR¹⁴, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
or
at least two of R⁴⁶, R⁴⁷, R⁴⁹, R⁵⁰, R⁵¹ or R⁵², if present at adjacent carbon atoms, together form at least one C₆-C₁₈aryl or C₁-C₁₈heteroaryl ring or ring system,
wherein E, D, L¹, L², L³, L⁴, o, p, q and r have the same meanings as mentioned above.

Particularly preferred R⁴⁵, R⁴⁶, R⁴⁷, R⁴⁹, R⁵⁰, R⁵¹ and R⁵² are independently of each other H, E, a unsubstituted C₆-C₈aryl group or a C₆-C₁₈aryl group substituted with at least one group E, or a C₁₋C₂₅alkyl group, which is unsubstituted or substituted by at least one group E and/or interrupted by at least one group D, preferably H, phenyl, biphenyl, naphthyl, phenanthryl or dimethylfluorenyl.

R⁴⁸ is particularly preferred H, a unsubstituted C₆-C₁₈aryl group or a C₆-C₈aryl group substituted with at least one group E, preferably phenyl, biphenhyl or naphthyl.

Further preferred at least two of R⁴⁶, R⁴⁷, R⁴⁹, R⁵⁰, R⁵¹ or R⁵², most preferably R⁵¹ and R⁵² if present at adjacent carbon atoms, together form at least one C₆-C₁₈aryl or C₁-C₁₈heteroaryl ring or ring system, most preferably a fused phenyl ring.

According to a particularly preferred embodiment, in general formula (XVI) R⁴⁵, R⁴⁶, R⁴⁷, R⁴⁹, R⁵⁰, R⁵¹ and R⁵² are H and R⁴⁸ is phenyl.

According to a further particularly preferred embodiment, in general formula (XVI) R⁴⁵, R⁴⁶, R⁴⁷, R⁴⁹ and R⁵⁰ are H, R⁵¹ and R⁵² form a fused phenyl ring and R⁴⁸ is phenyl.

In general, m is an integer of 0 to 4, wherein m describes the number of groups M present. If m is 0, no group M is present, but the N-heteroaryl group according to general formula (XII) is directly attached to carbon atom within the skeleton of the compound of general formula (I). Preferably, m is 0, 1, 2 or 3, more preferably, m is 0 or 1.

Preferably, M is a C₆-C₄₀ arylene group which is unsubstituted, a C₁-C₂₄heteroarylene group which is unsubstituted or a C₁-C₂₅ alkylene group which unsubstituted. Particularly preferred, M is a C₆-C₁₈ arylene group which is unsubstituted, most preferably a phenylene group. Preferably, R²⁶ is independently of each other selected from a group of formula -(L¹)ₒ-(L²)ₚ-(L³)_{q}-(L⁴)ᵣ-R¹⁴, wherein o, p, q, r, L¹, L², L³, L⁴ and R¹⁴ have the meanings and preferred meanings as mentioned above.

According to a very preferred embodiment of the present invention, m is 0, and at least two of R²⁶, if present at adjacent carbon atoms, form at least one five or six membered, substituted or unsubstituted, saturated, unsaturated, aromatic or heteroaromatic ring or ring system, without further substituents R²⁶_{.}

According to a further very preferred embodiment of the present invention, m is 1, M is a C₆-C₄₀arylene group which is unsubstituted or substituted by at least one group E, preferably phenylene, and at least two of R²⁶, if present at adjacent carbon atoms, form a five or six membered, substituted or unsubstituted, saturated, unsaturated, aromatic or heteroaromatic ring or ring system, without further substituents R²⁶.

The present invention therefore preferably relates the compound according to the present invention, wherein m is 1, M is a C₆-C₄₀arylene group which is unsubstituted or substituted by at least one group E, preferably phenylene, and at least two of R²⁶, if present at adjacent carbon atoms, form a five or six membered, substituted or unsubstituted, saturated, unsaturated, aromatic or heteroaromatic ring, wherein E has the meanings as mentioned above.

The present invention further preferably relates to the compound according to the present invention, wherein R² is a N-heteroaryl group according to general formula (XII) as defined above.

The present invention therefore relates to compounds according to the following formulae (IIe) and (IIf) 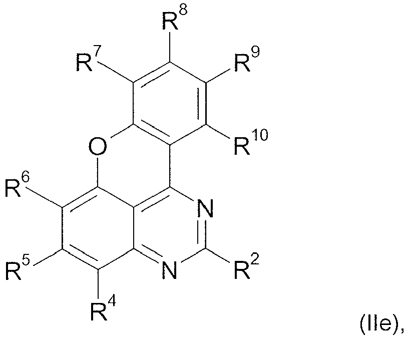 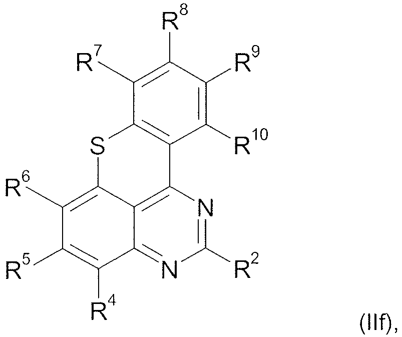 wherein independently of each other R², R⁴, R⁵, R⁶, R⁷, R⁸, R⁹ and R¹⁰ have the same meanings as mentioned above.

Particularly preferred molecules according to general formulae (IIb) and (IIc) according to the present invention are shown in the following.

Preferred examples, wherein Y is O:

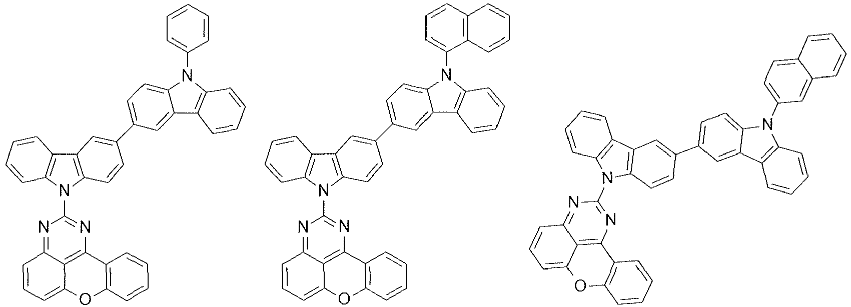

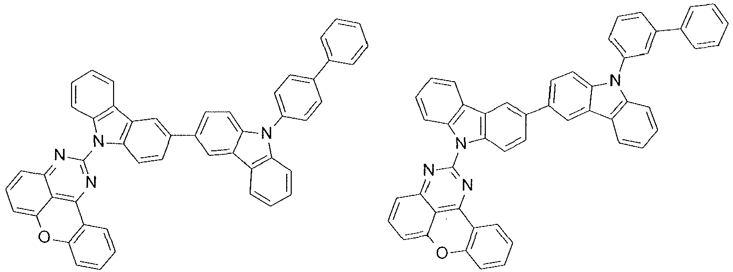

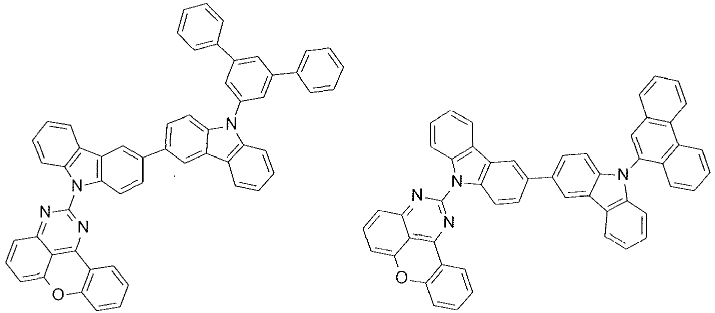

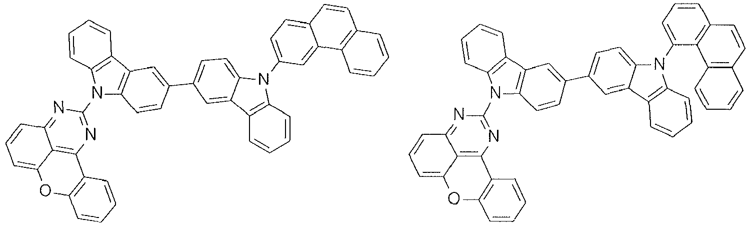

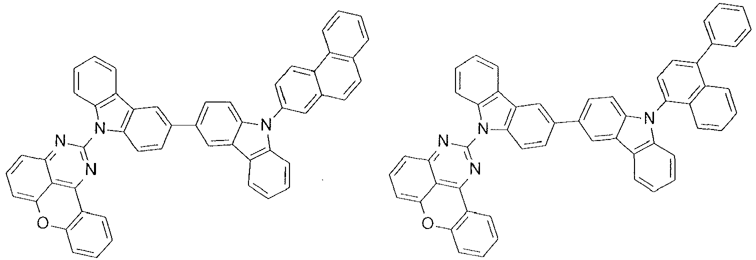

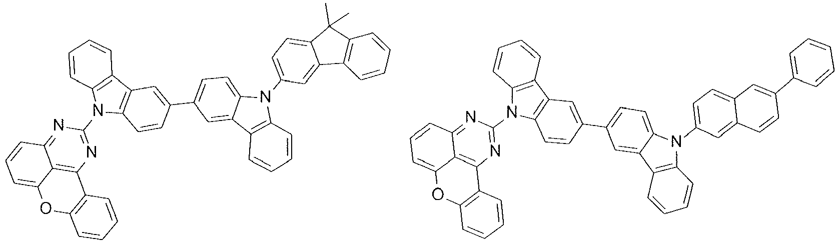

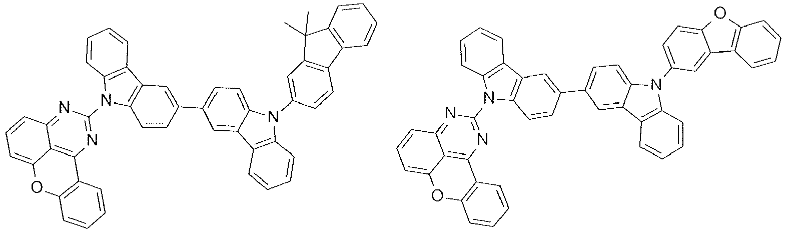

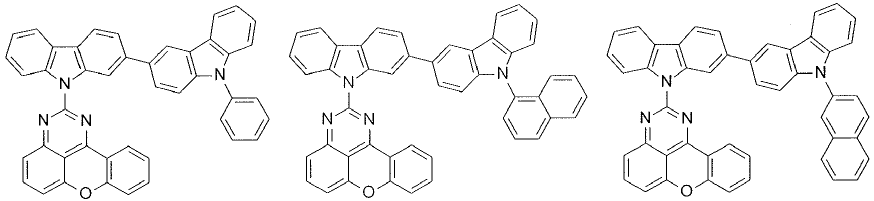

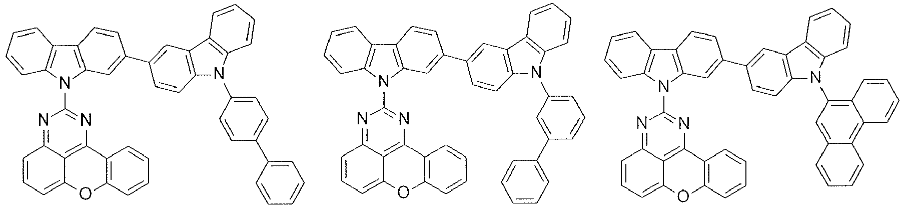

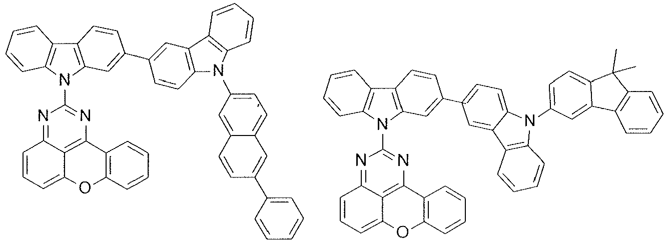

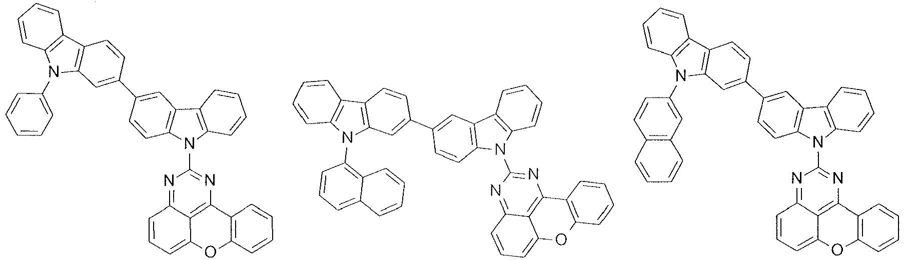

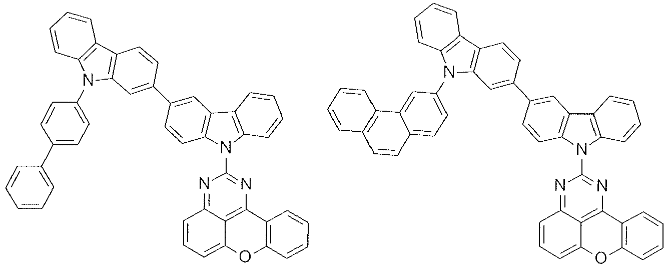

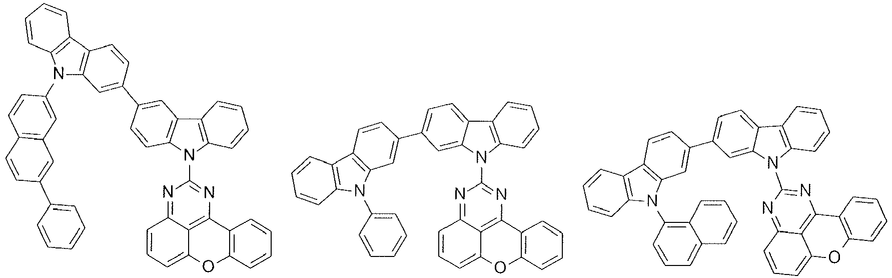

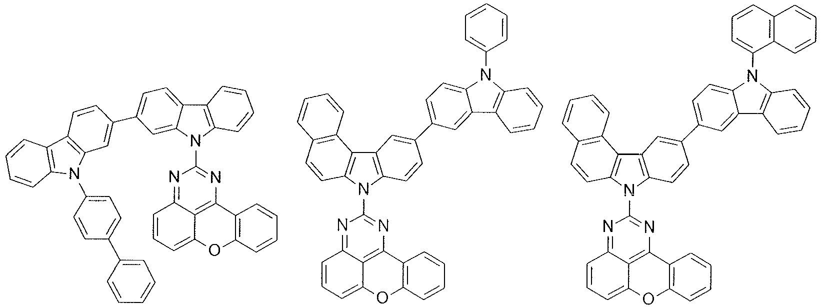

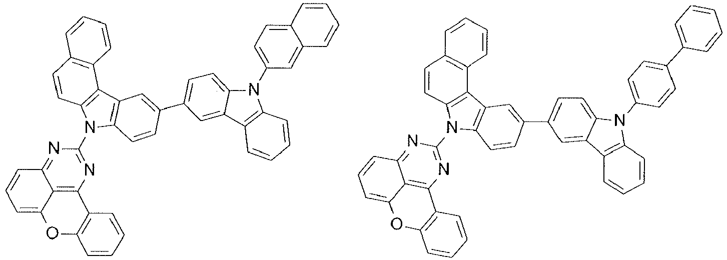

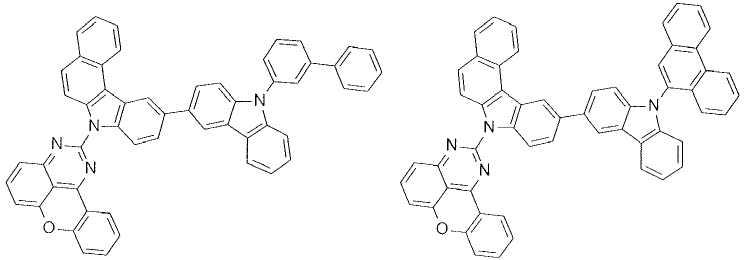

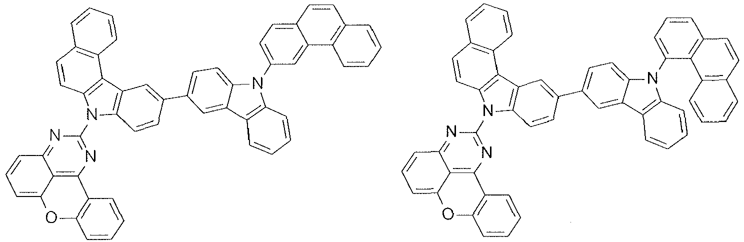

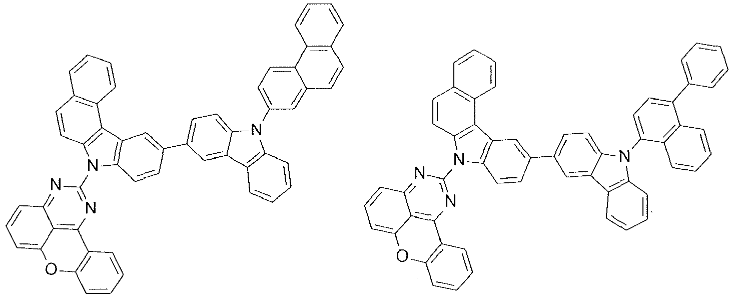

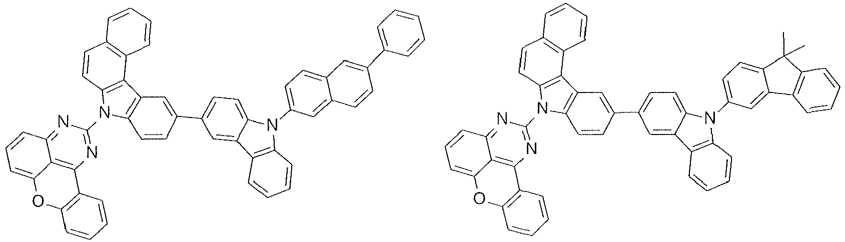

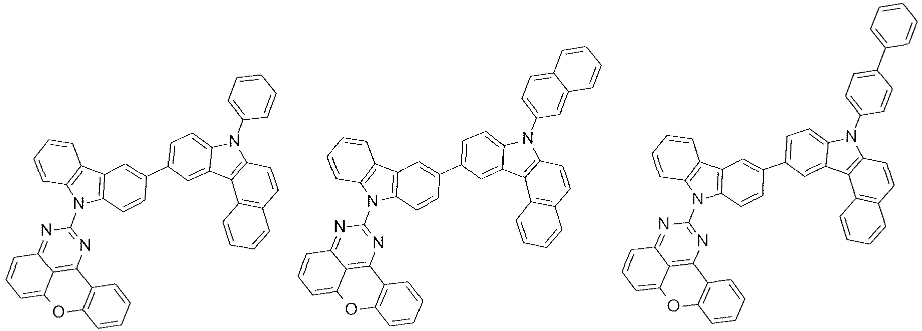

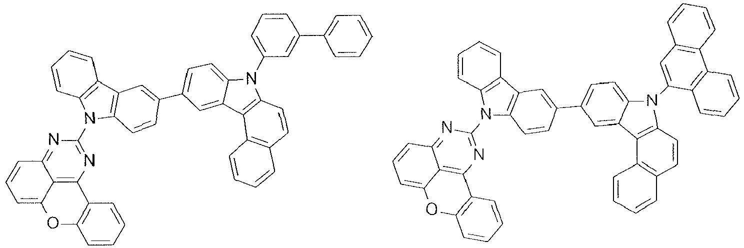

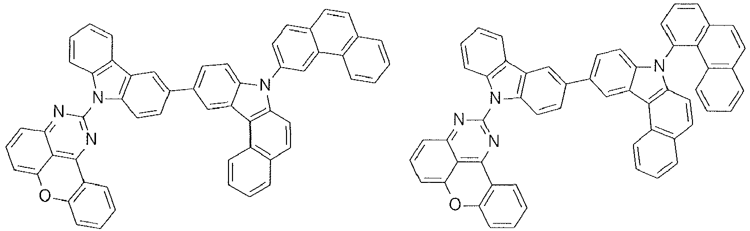

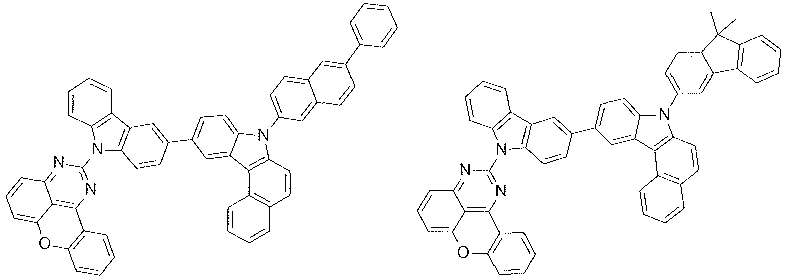

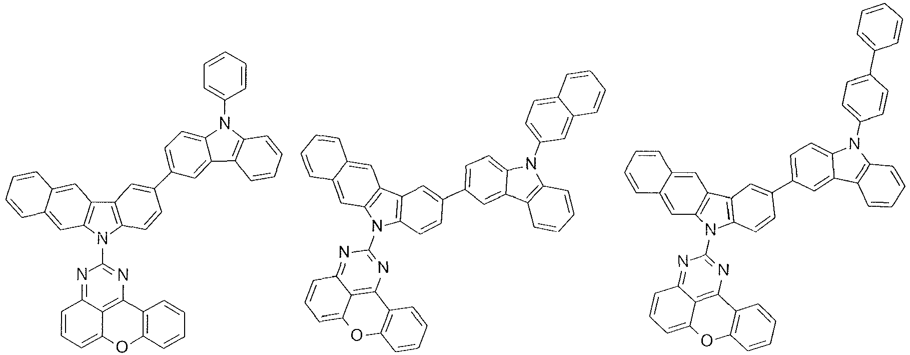

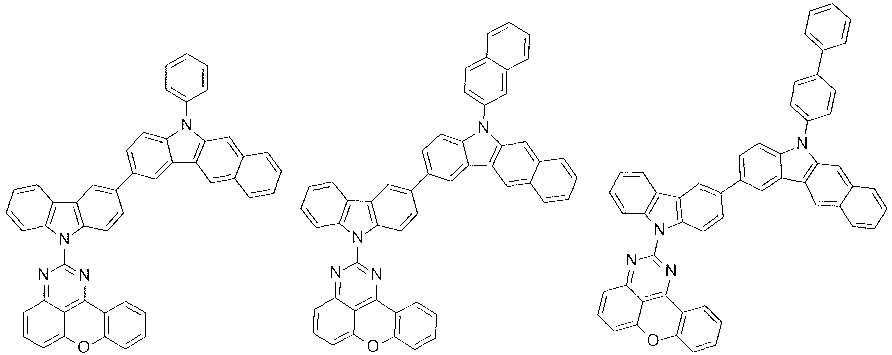

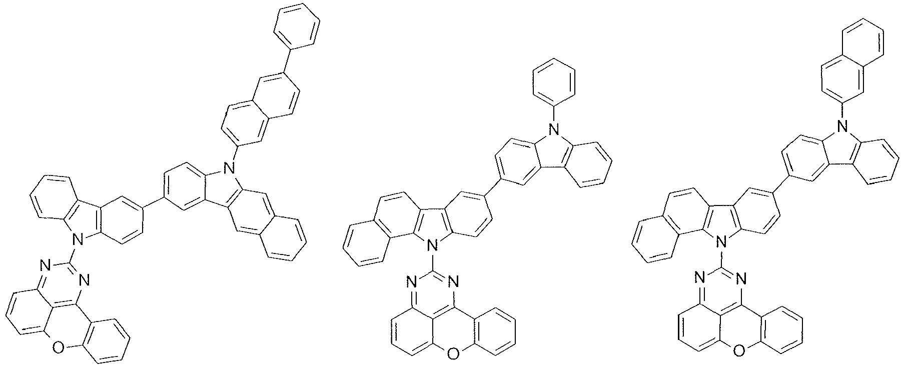

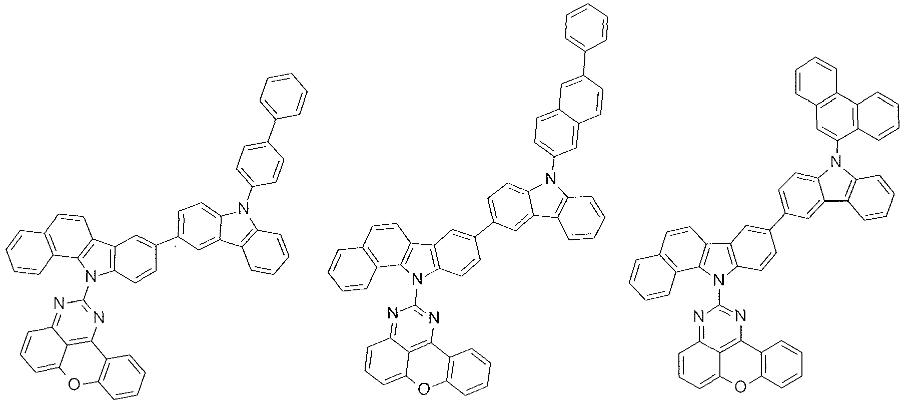

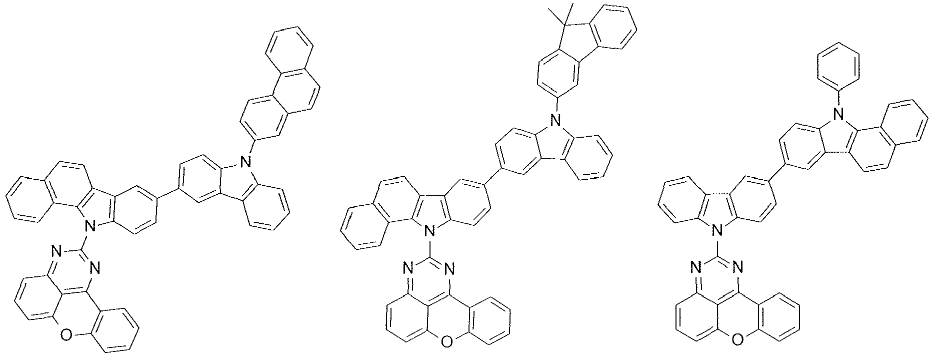

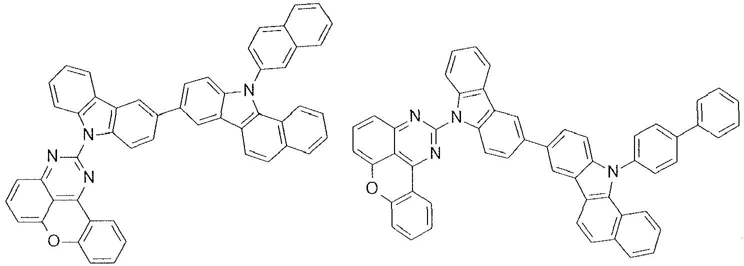

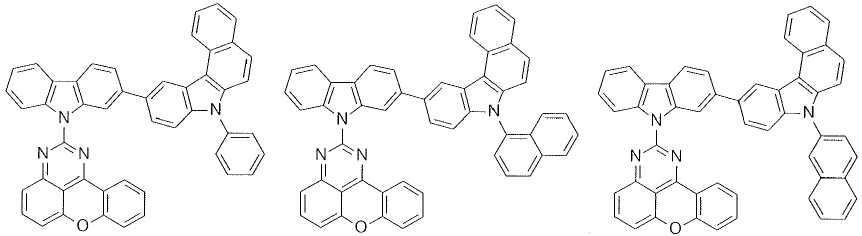

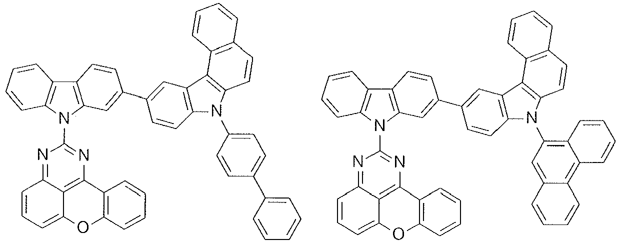

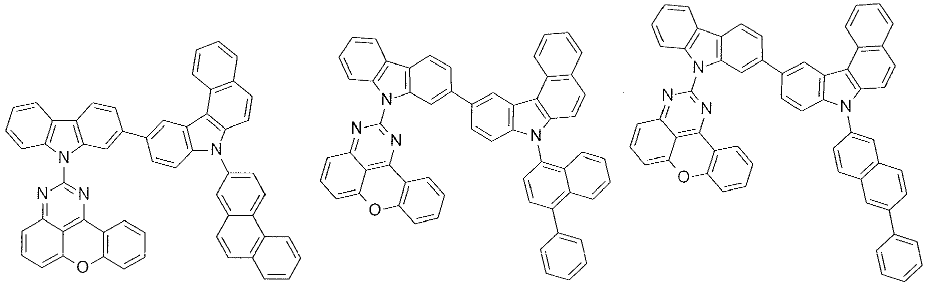

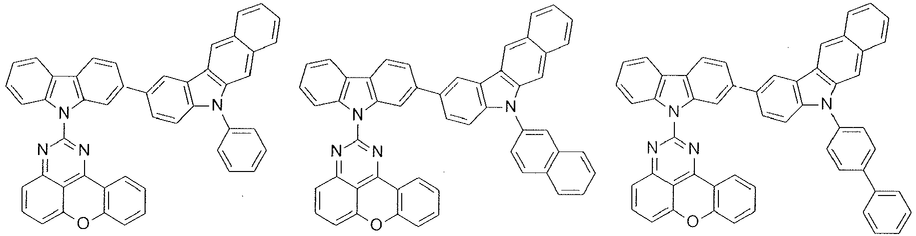

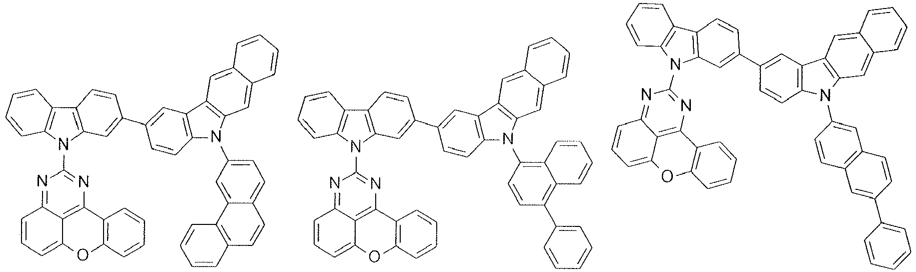

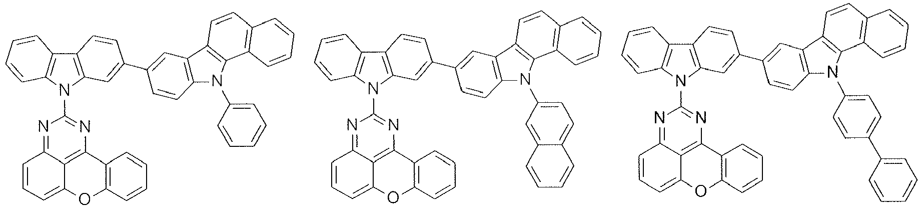

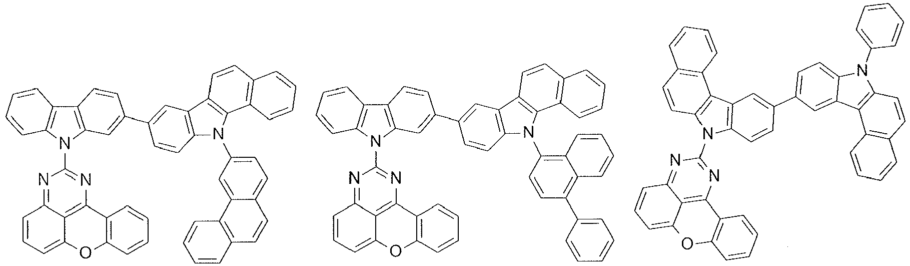

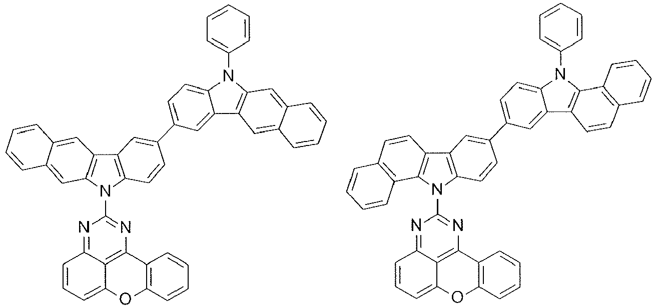

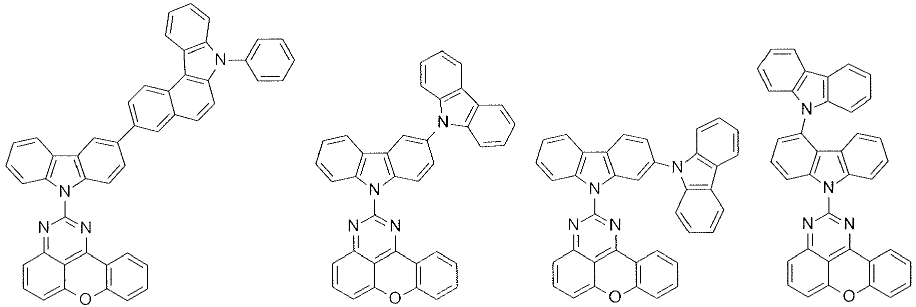

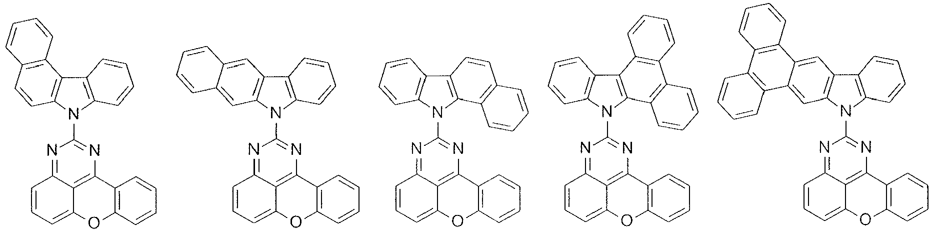

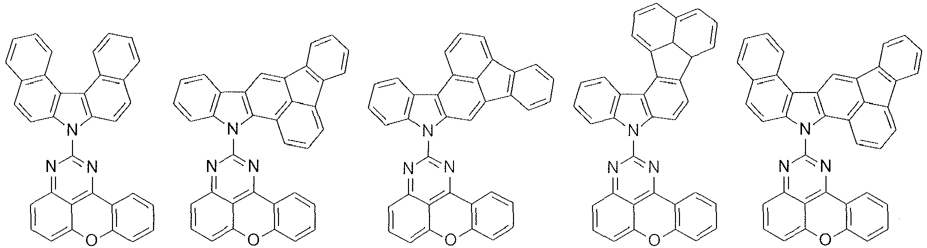

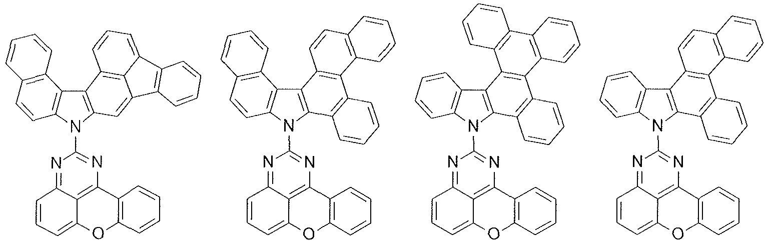

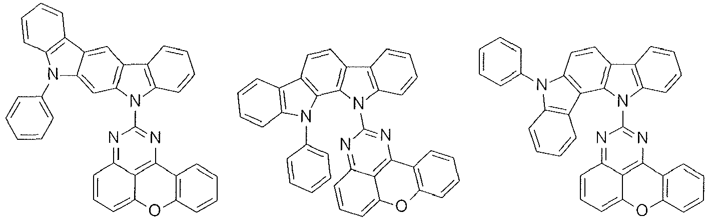

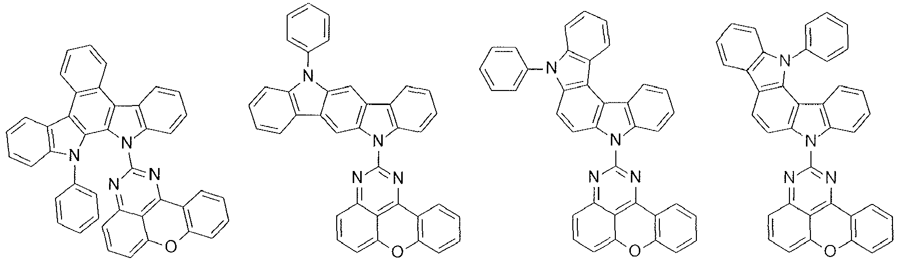

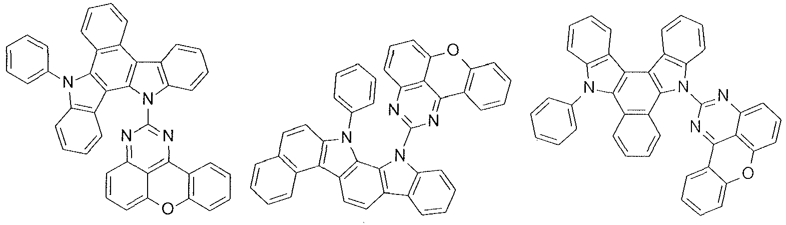

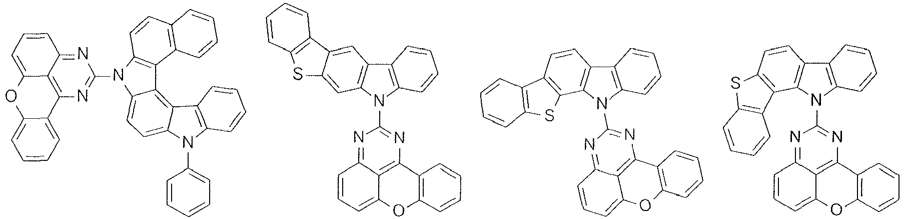

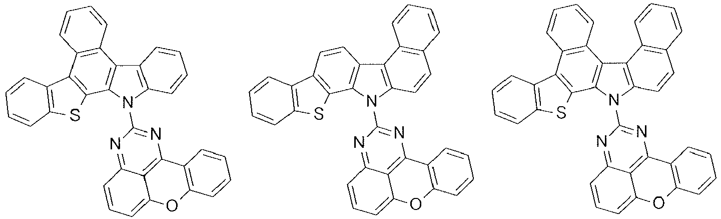

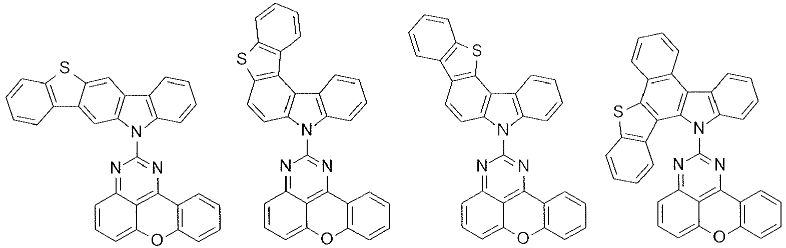

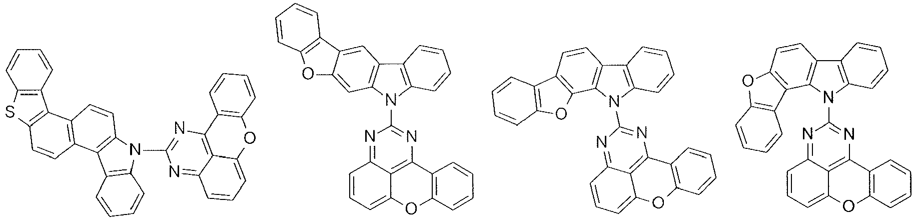

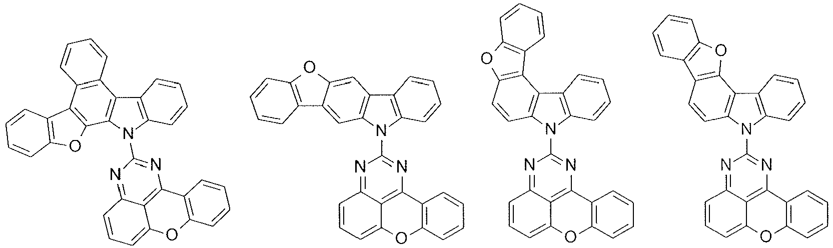

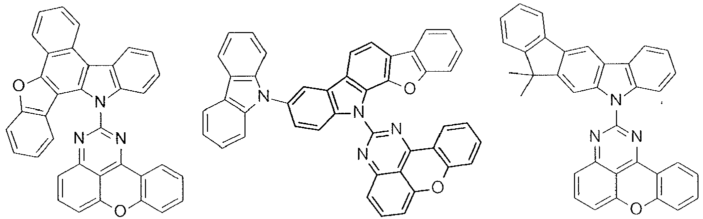

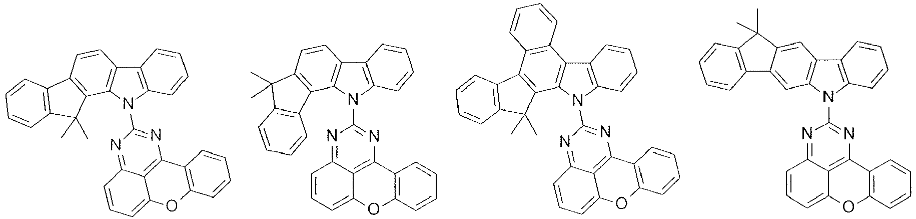

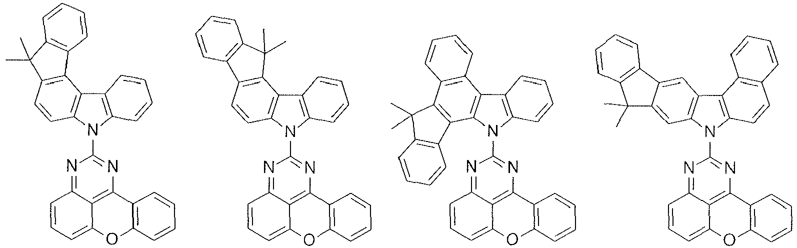

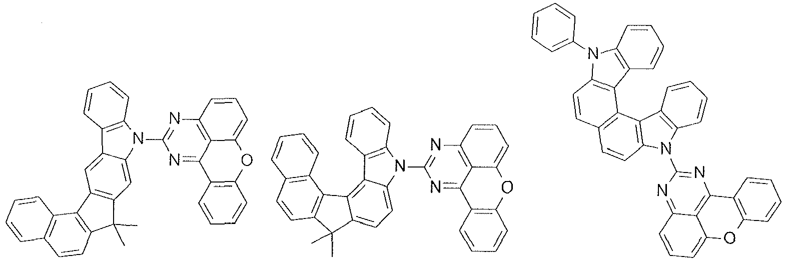

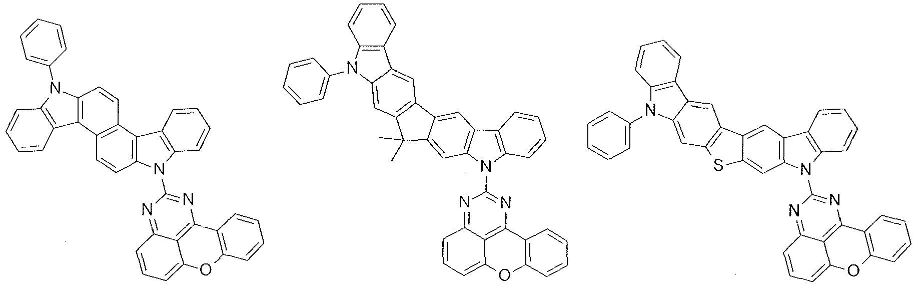

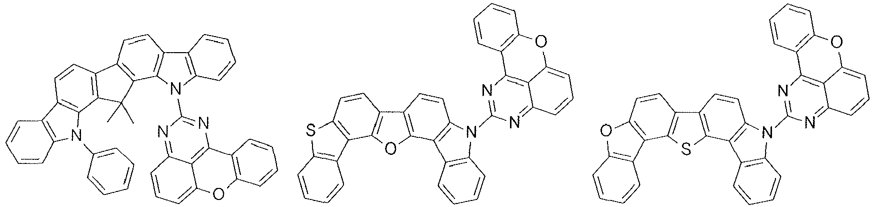

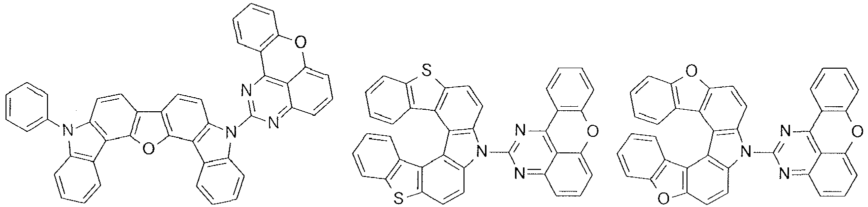

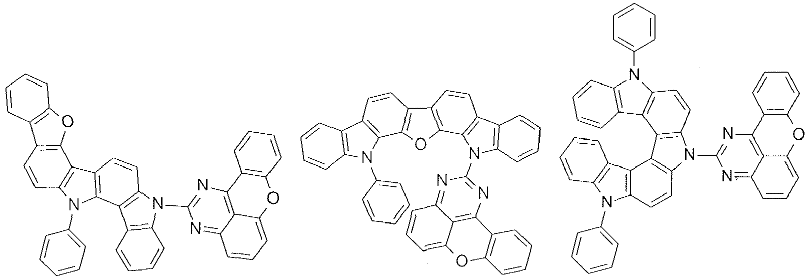

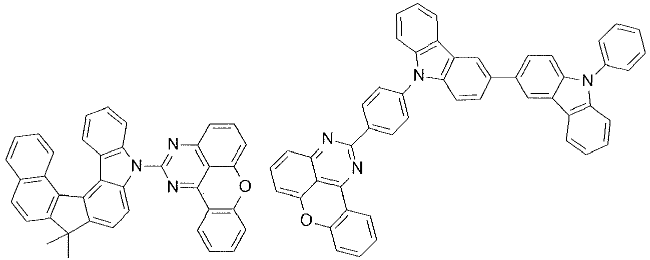

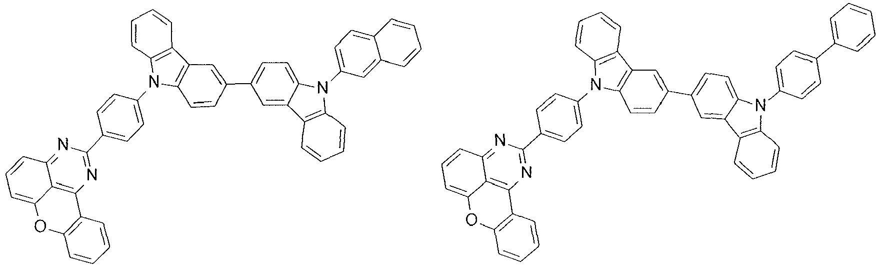

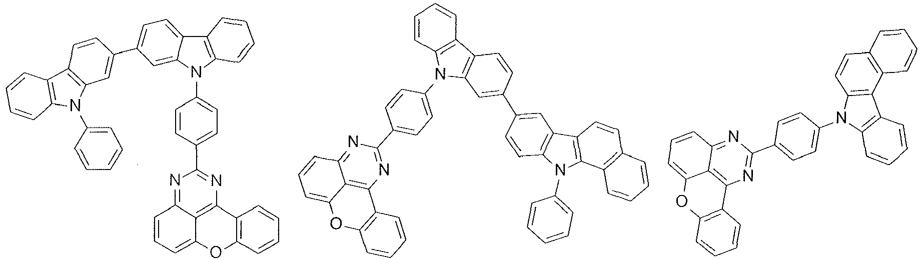

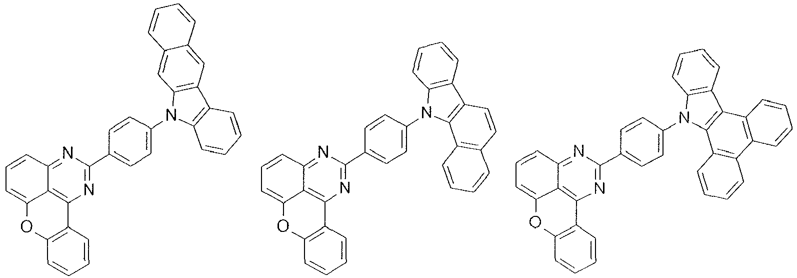

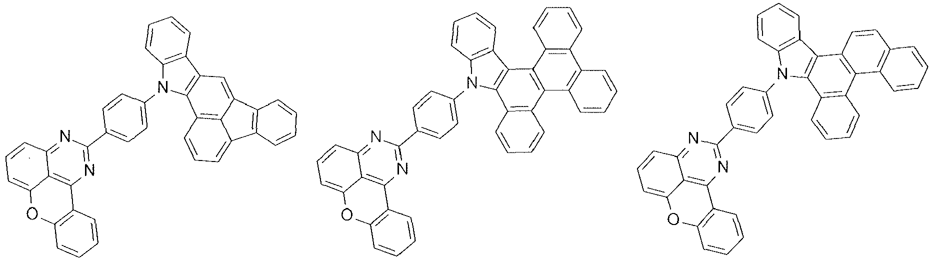

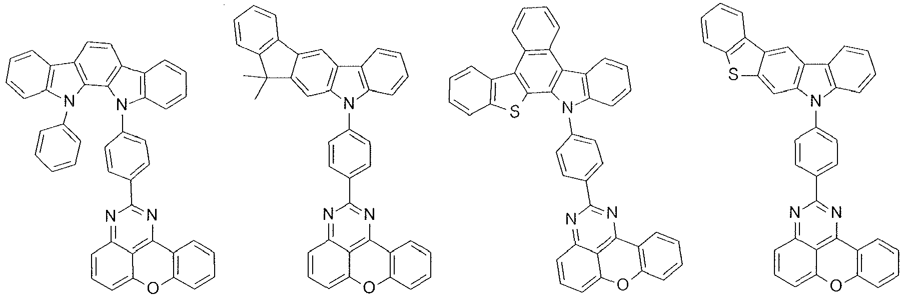

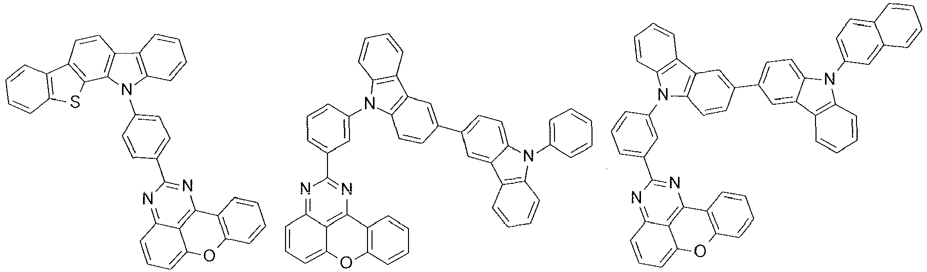

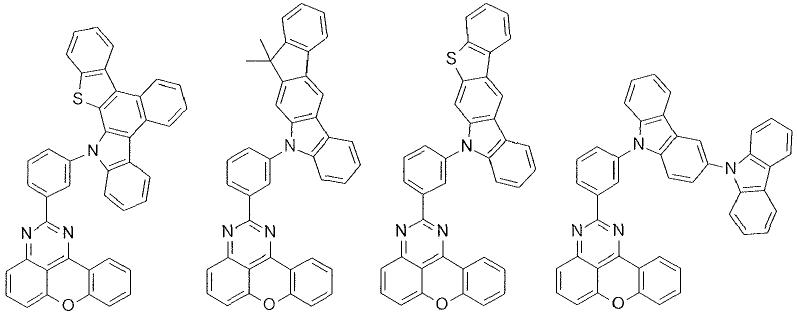

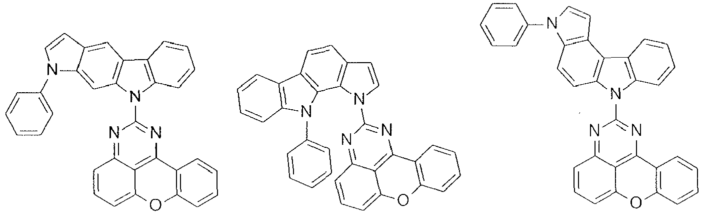

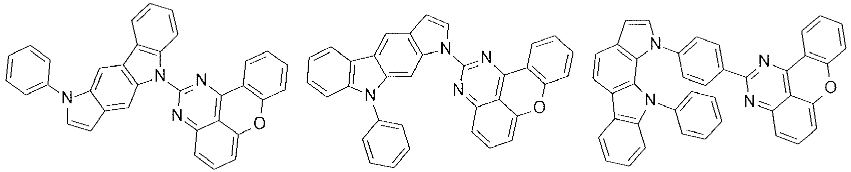

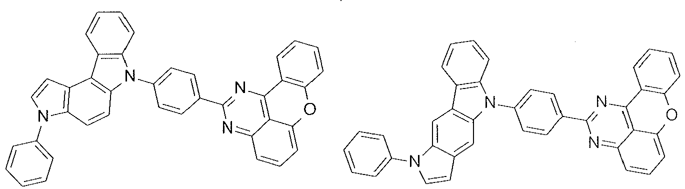

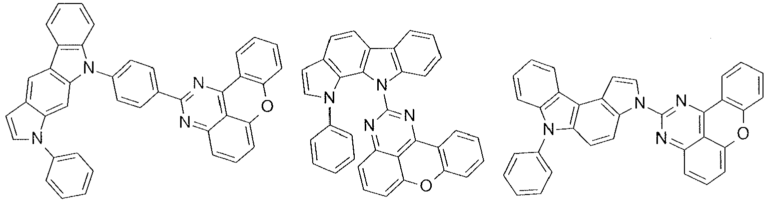

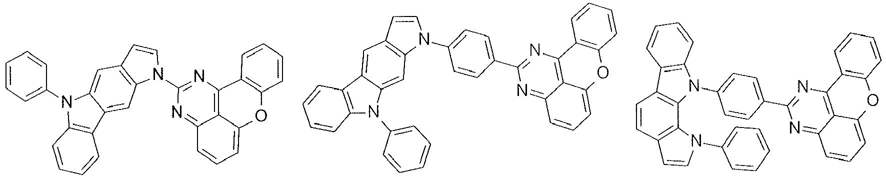

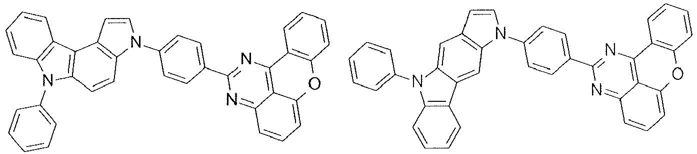

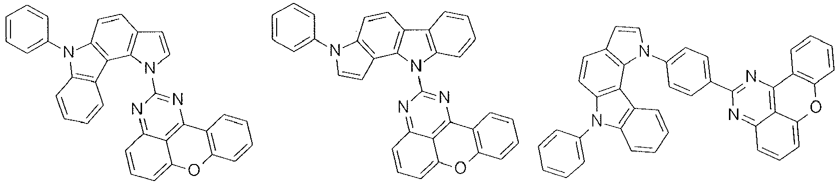

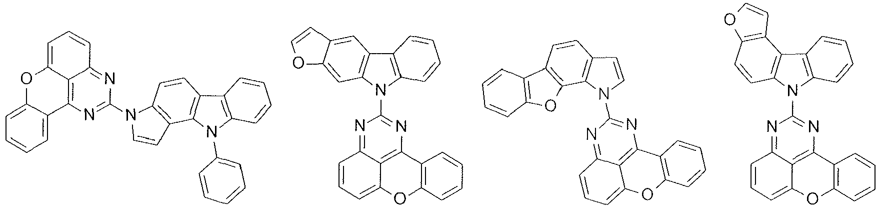

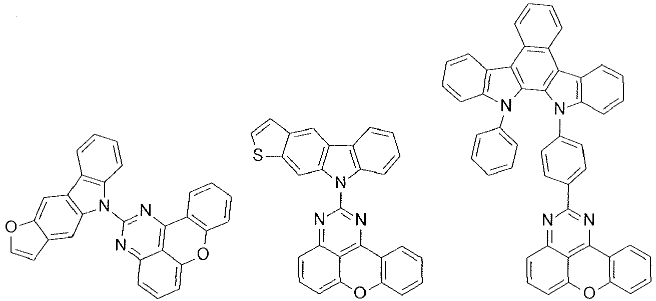

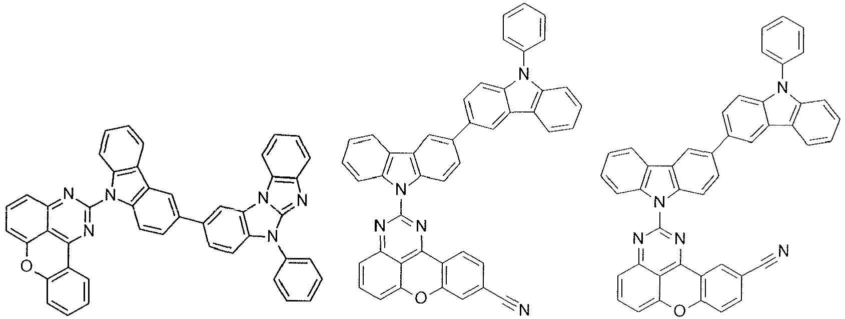

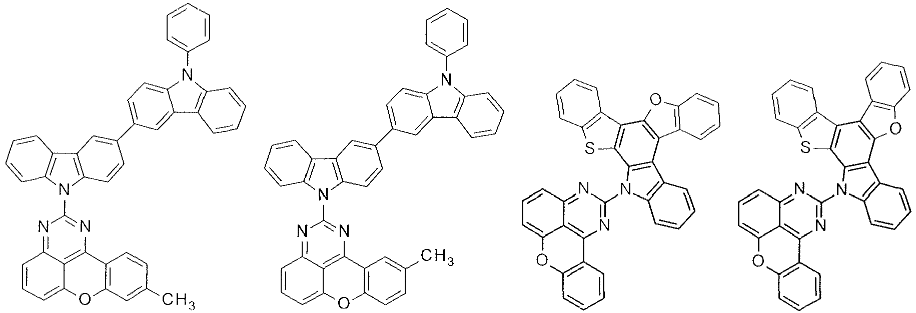

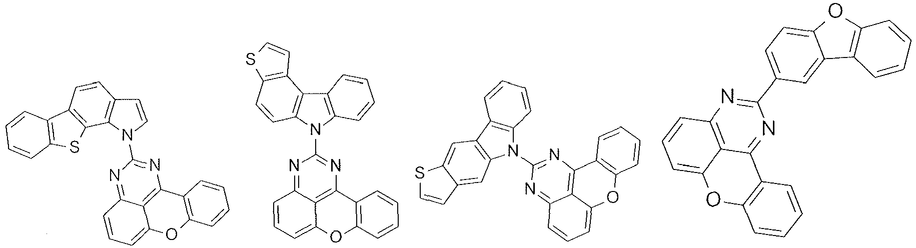

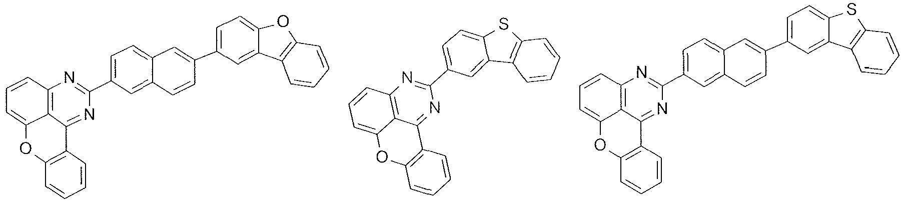

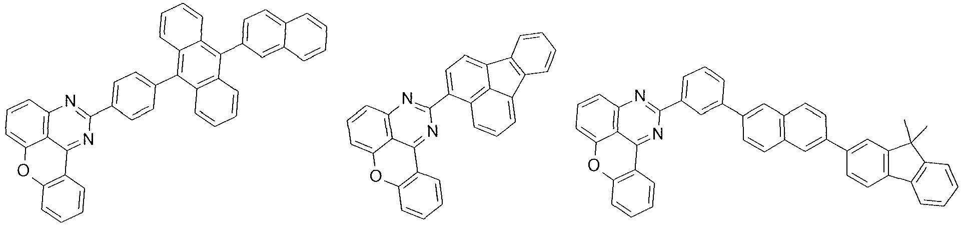

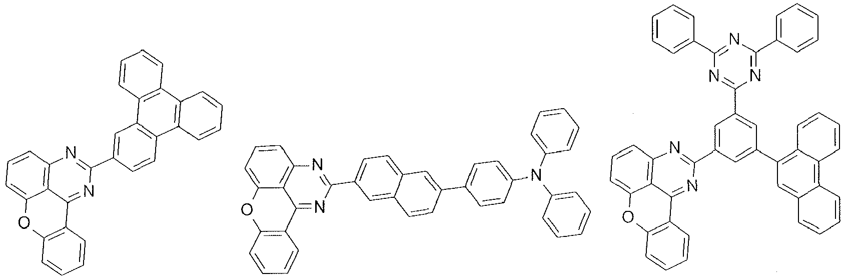

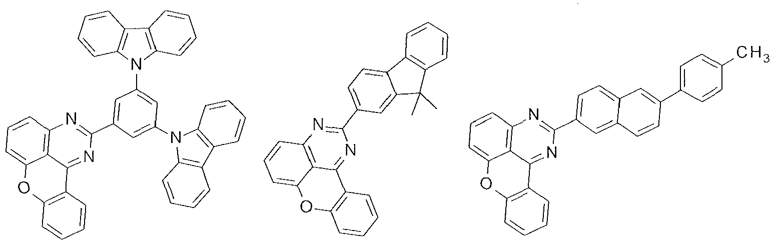

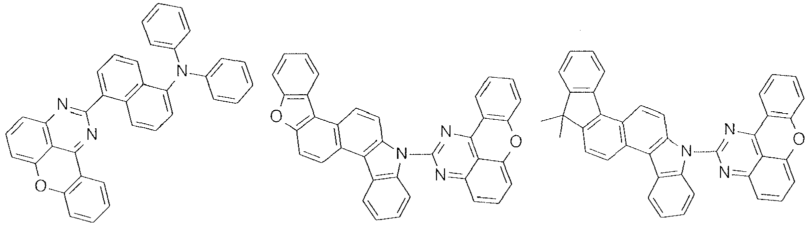

Further preferred examples, wherein Y is O:

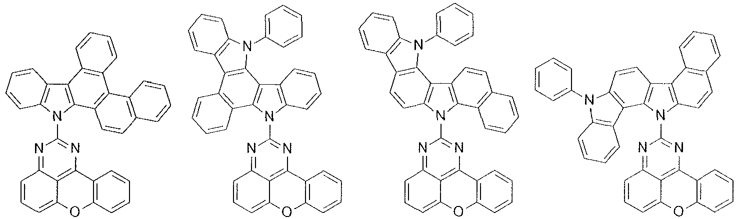

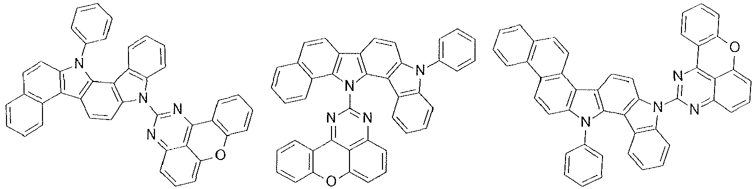

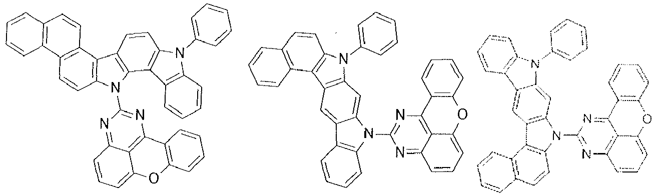

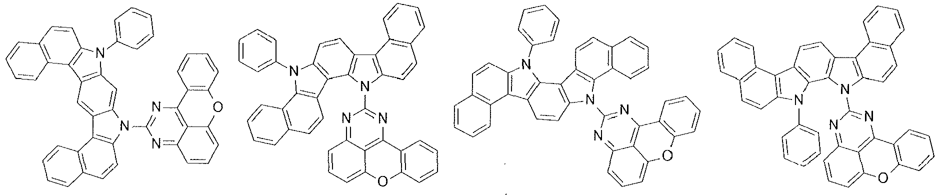

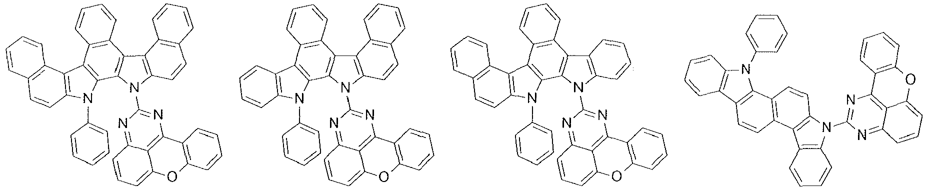

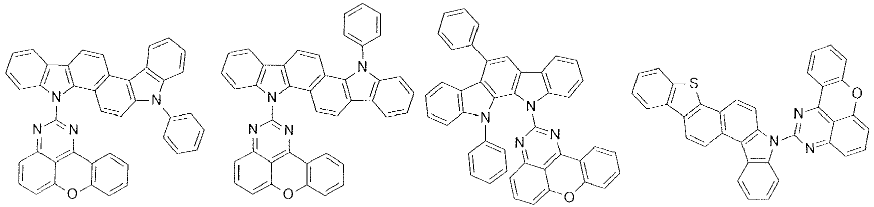

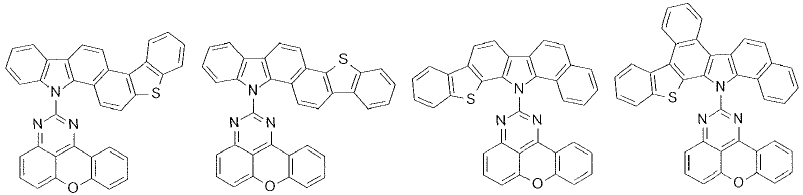

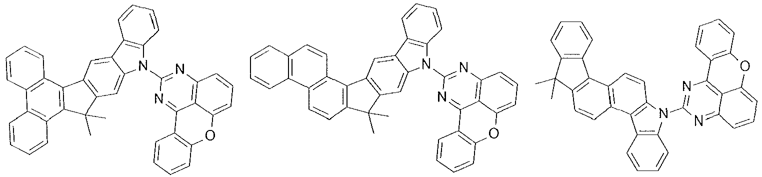

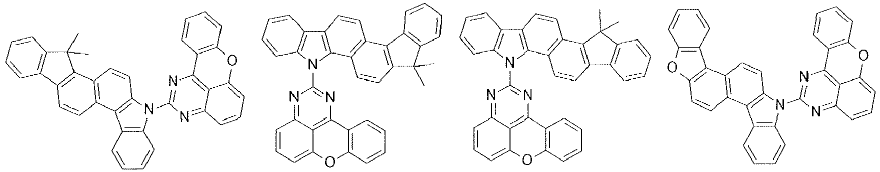

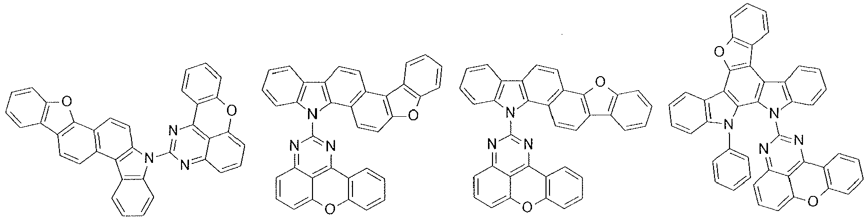

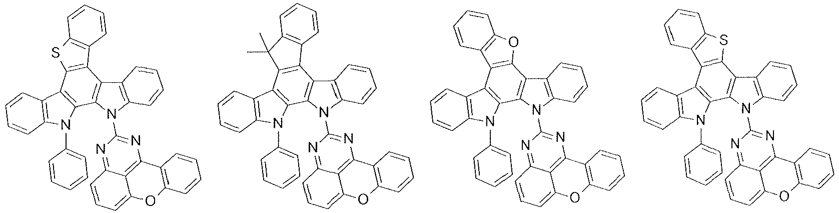

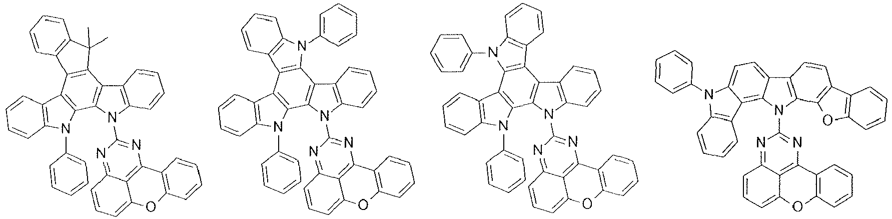

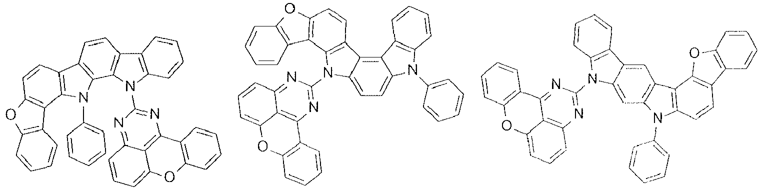

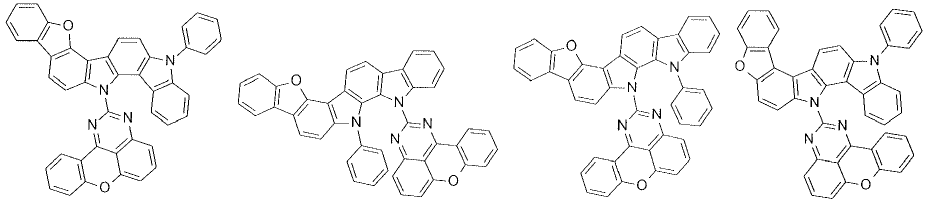

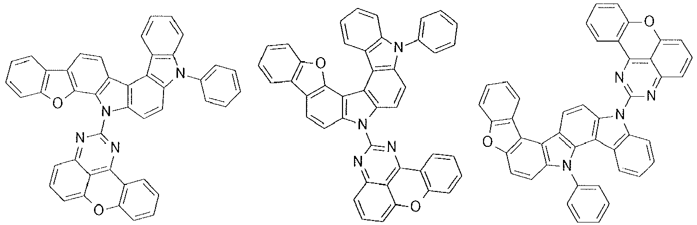

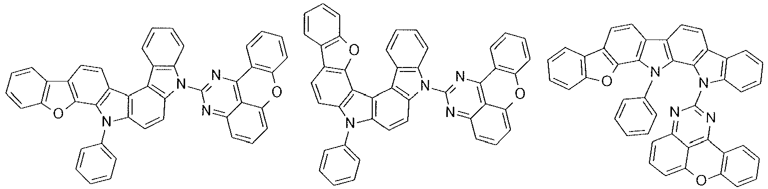

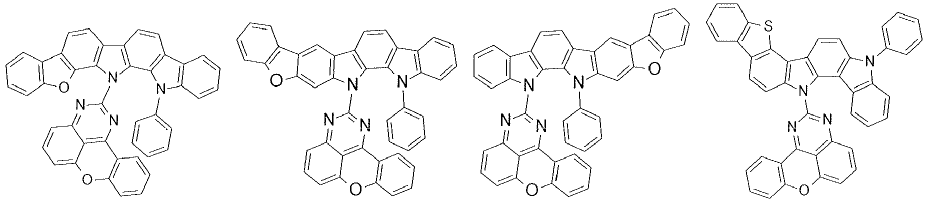

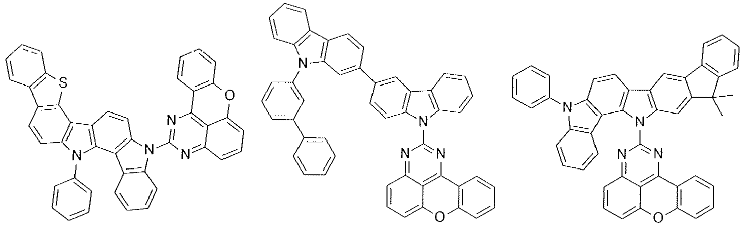

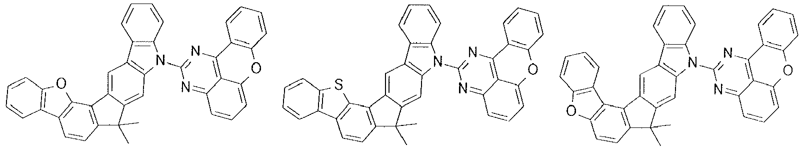

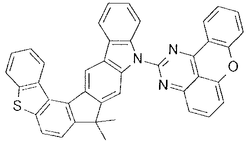

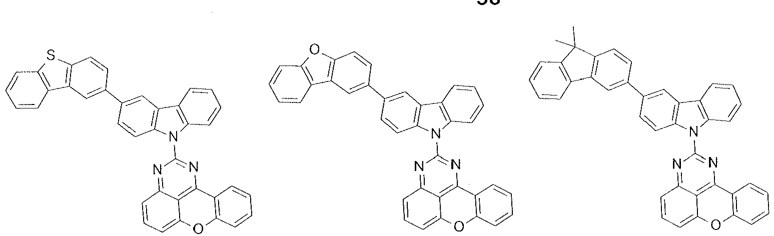

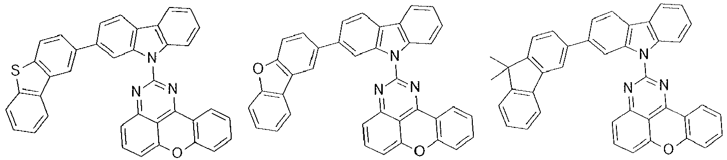

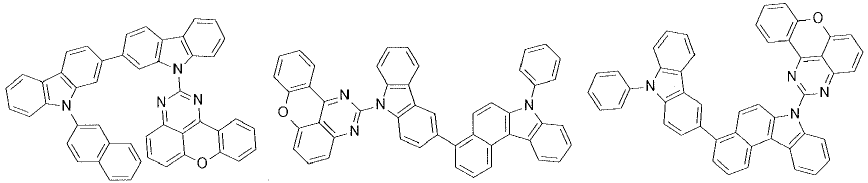

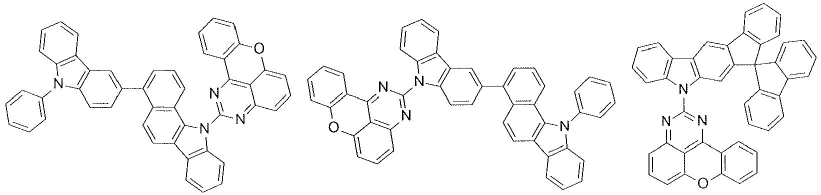

Preferred examples, wherein Y is S:

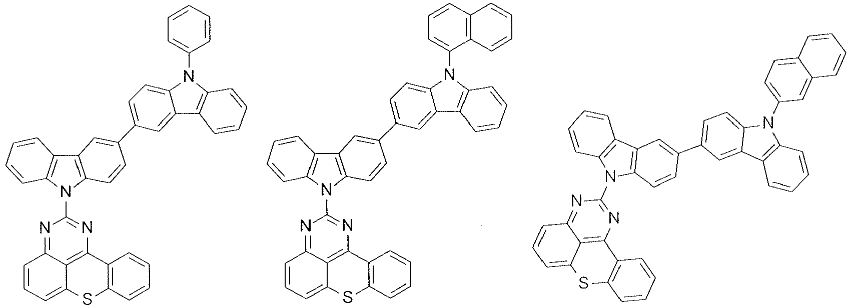

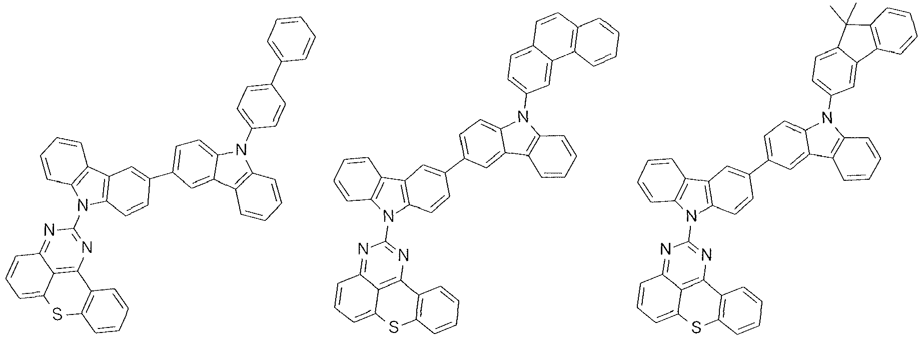

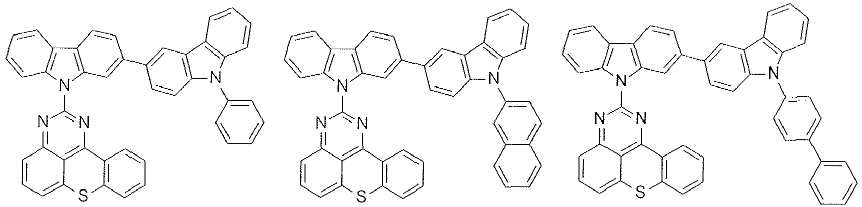

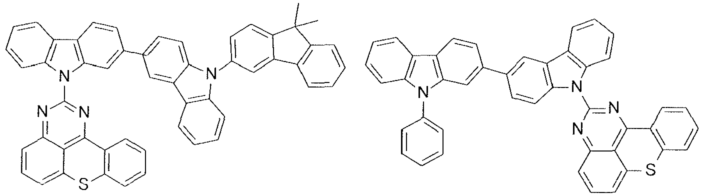

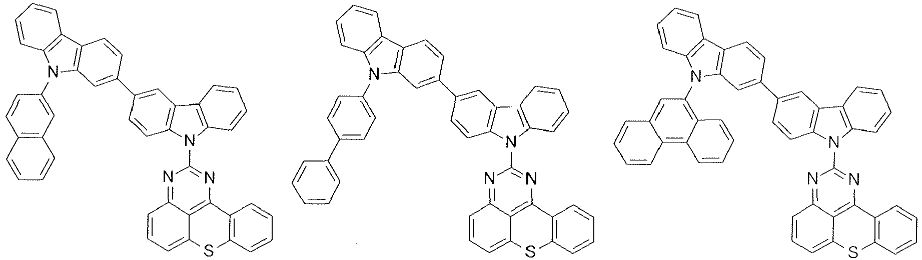

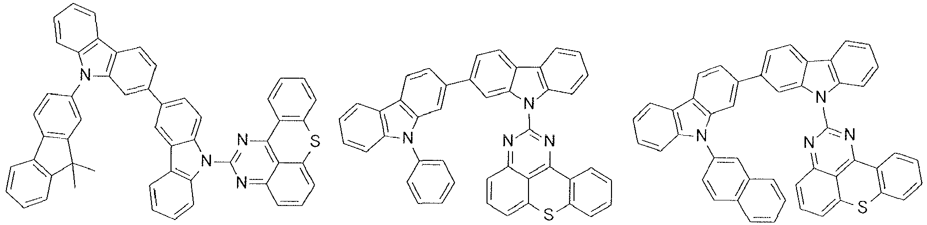

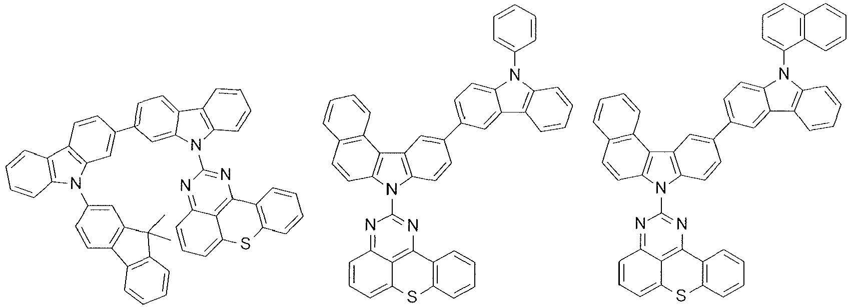

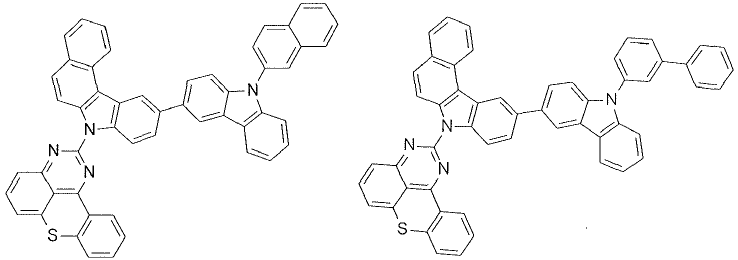

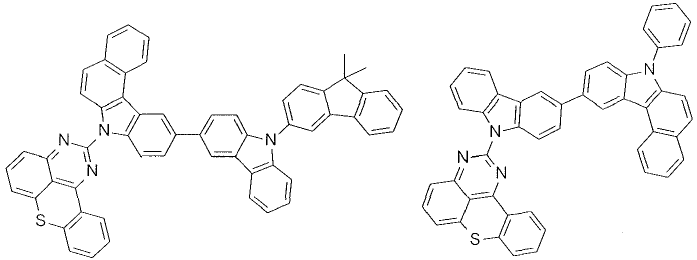

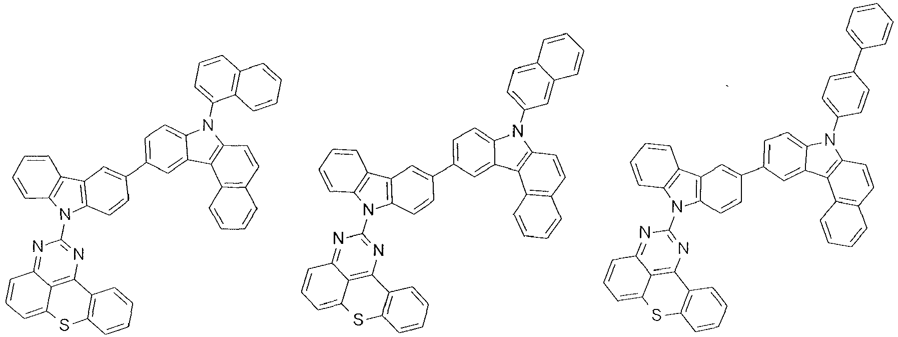

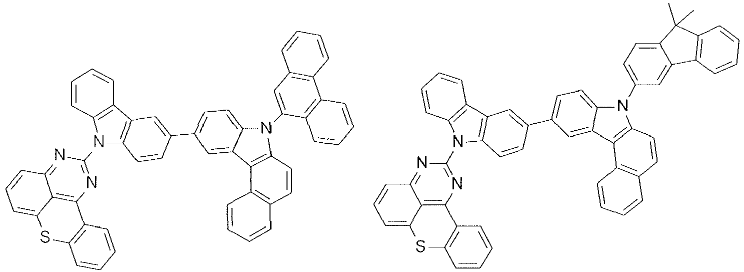

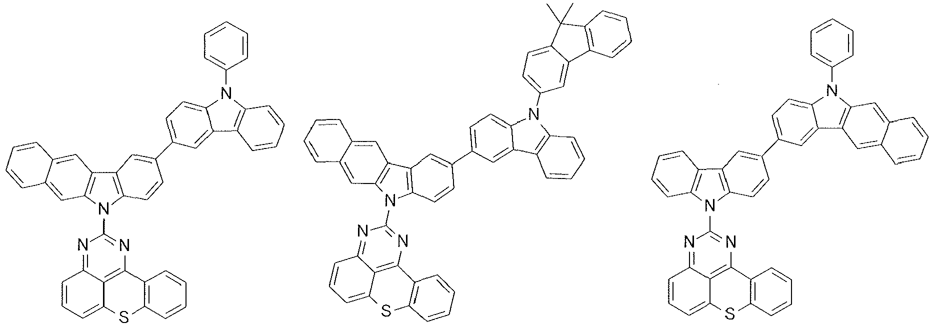

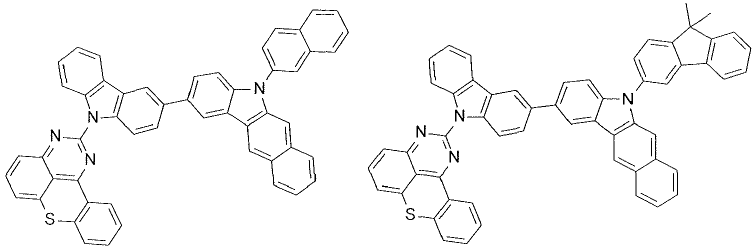

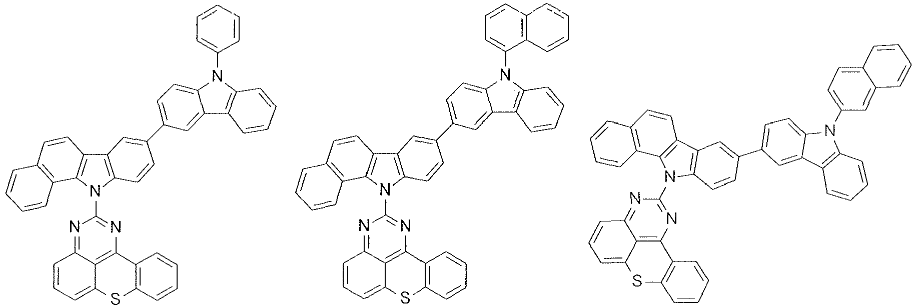

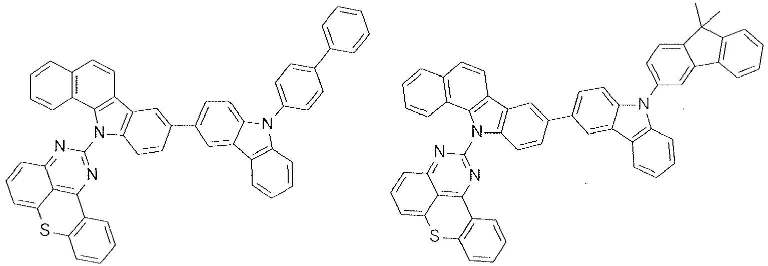

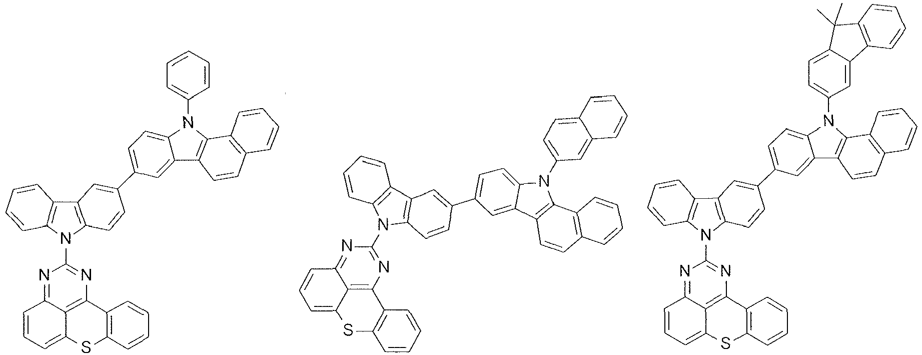

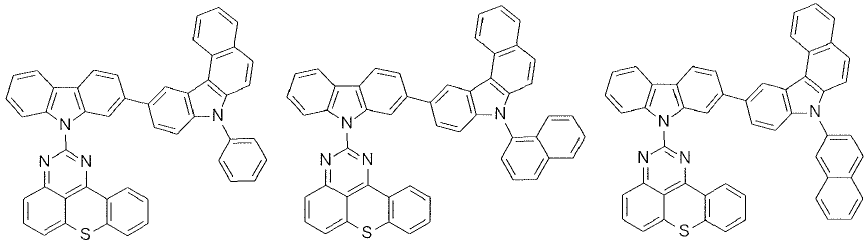

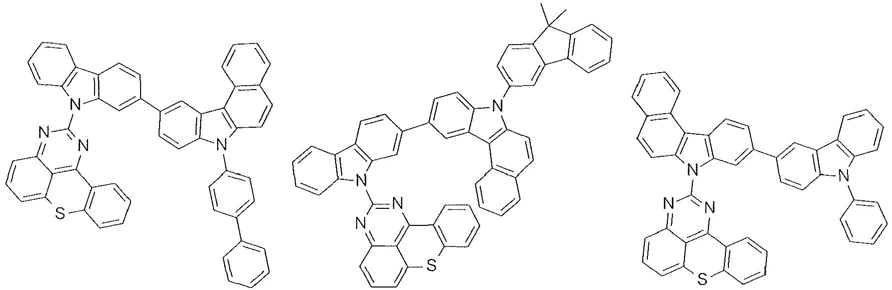

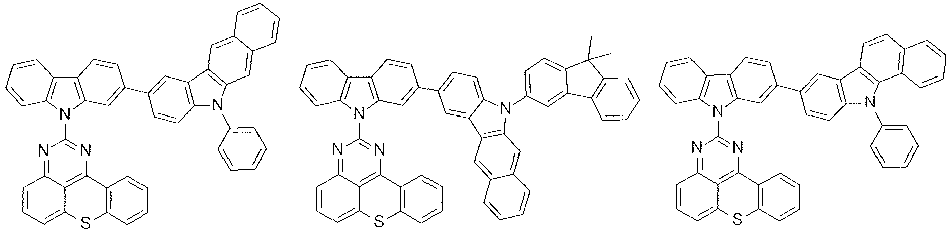

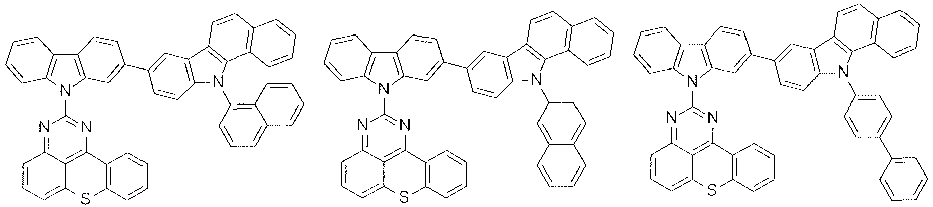

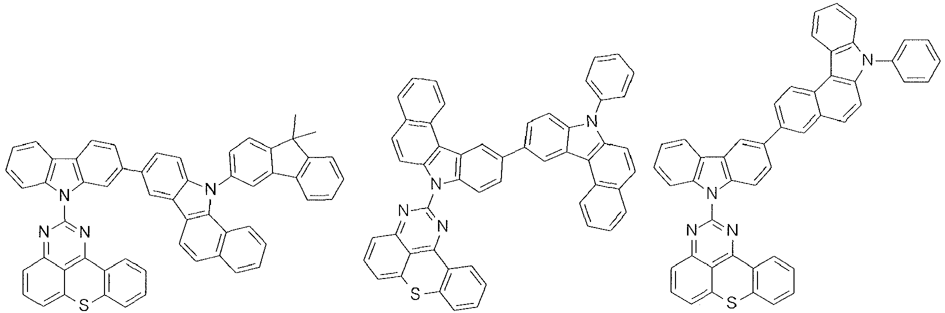

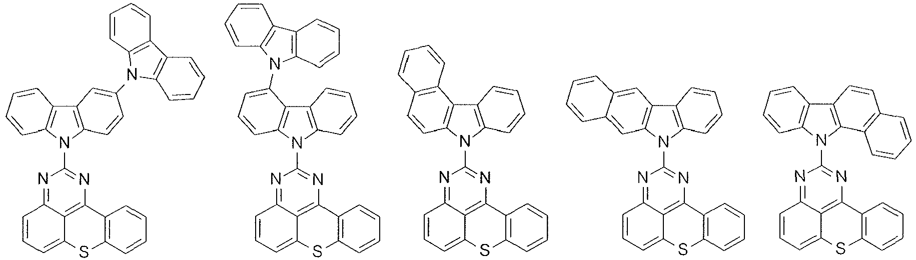

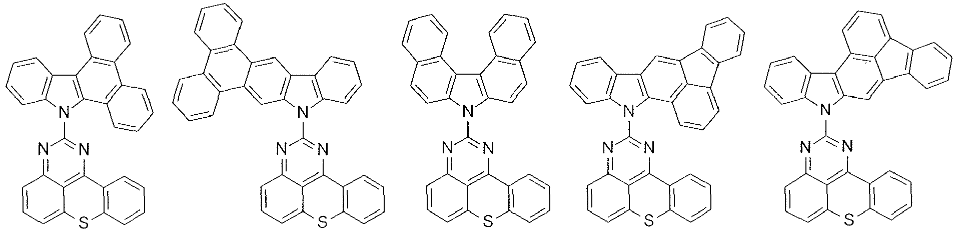

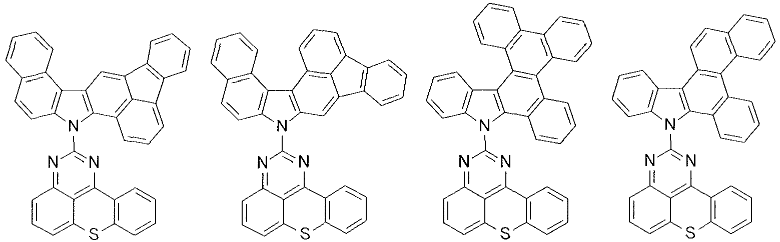

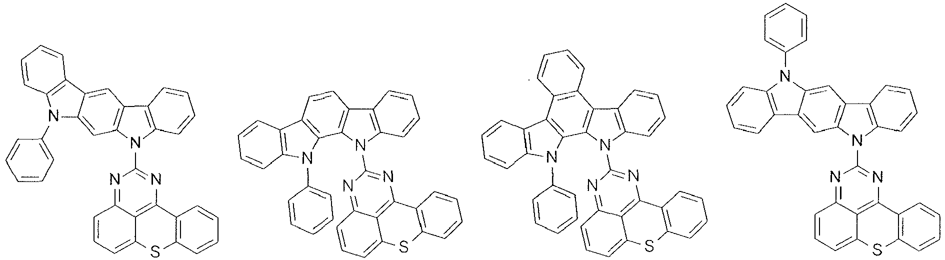

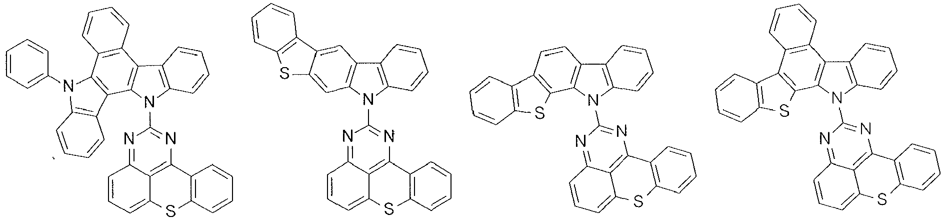

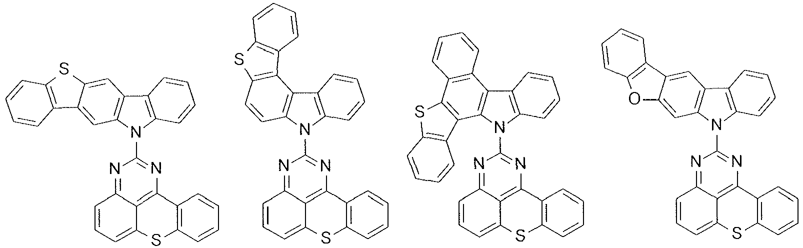

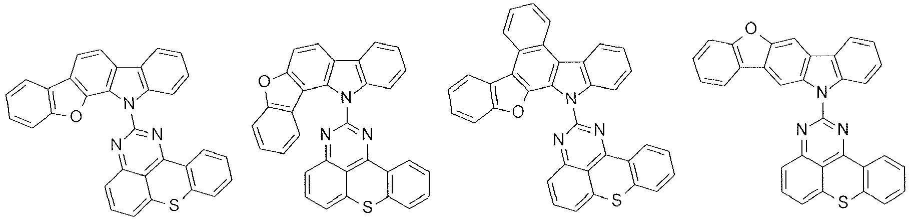

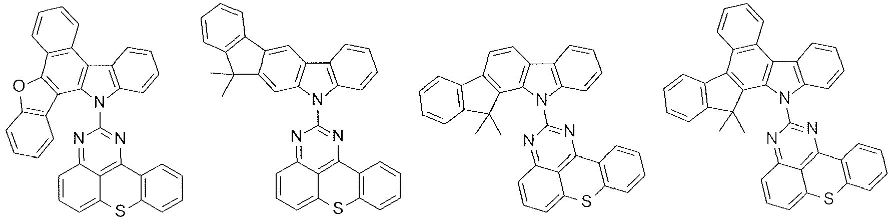

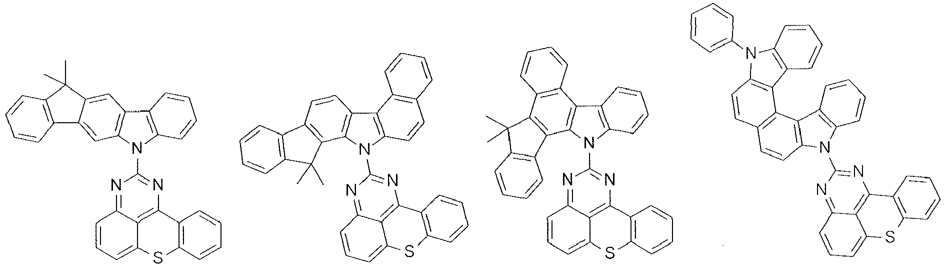

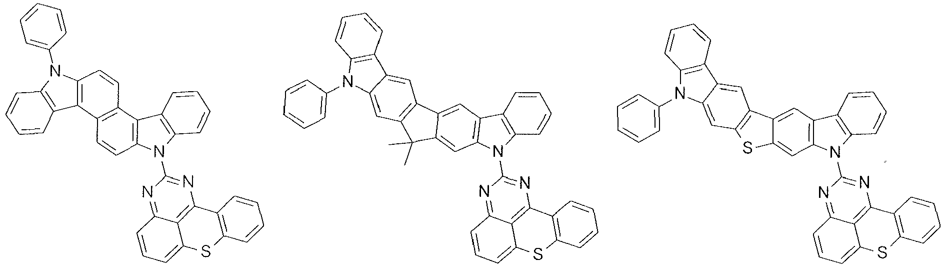

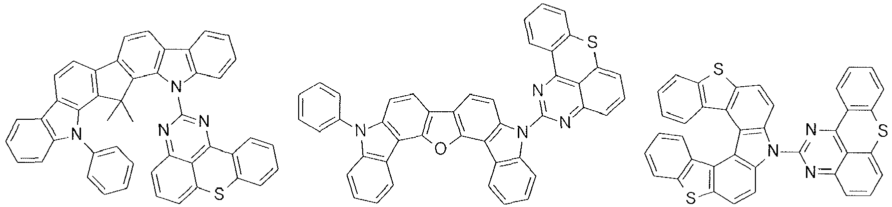

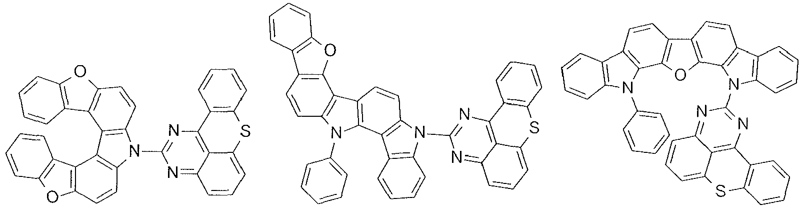

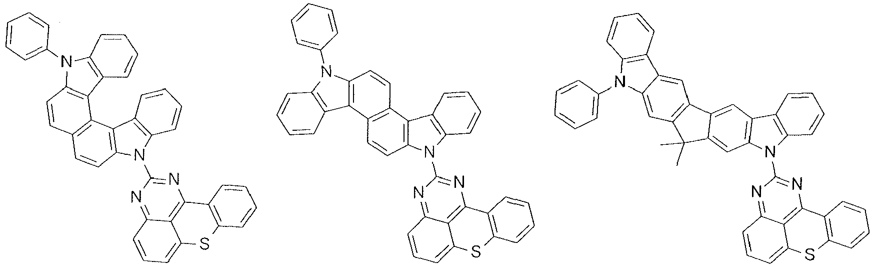

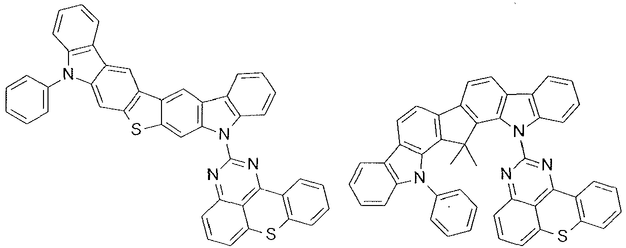

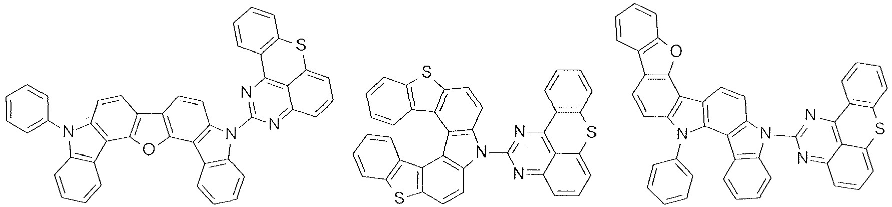

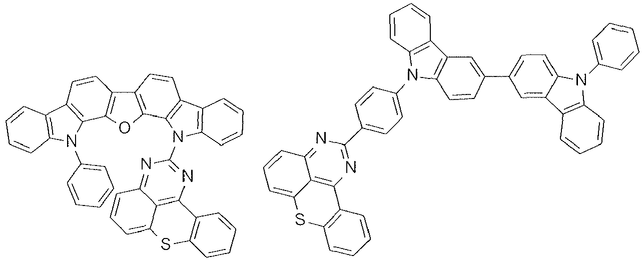

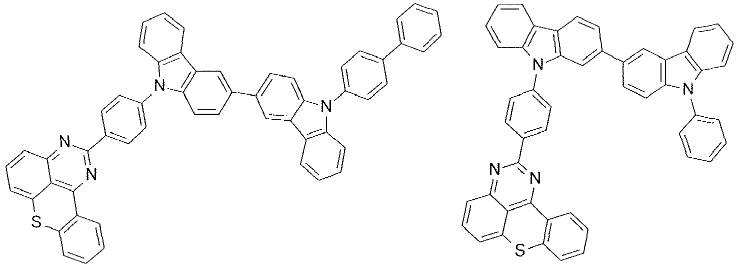

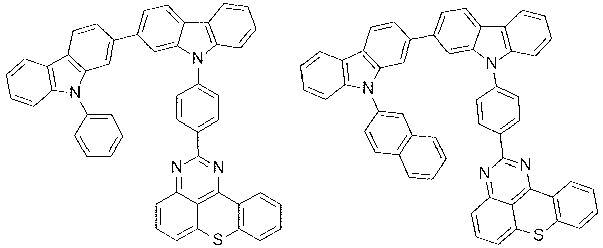

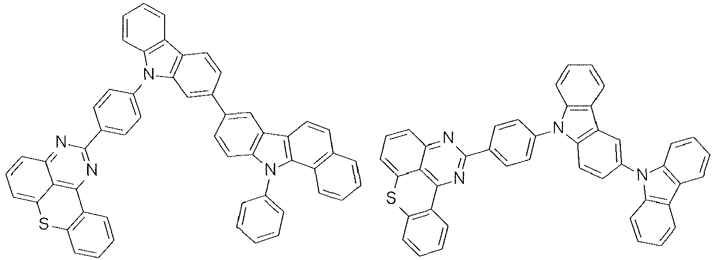

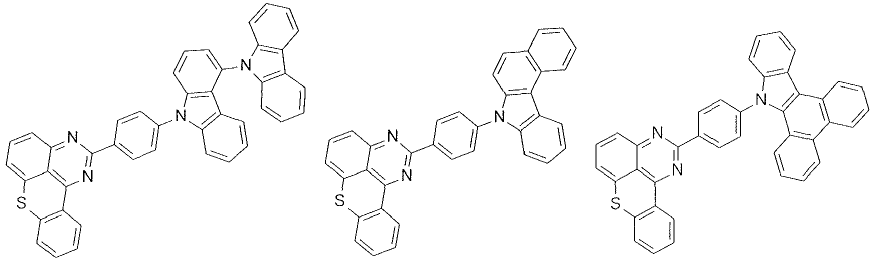

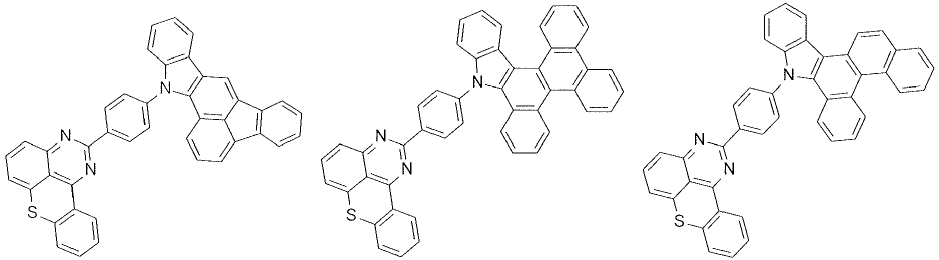

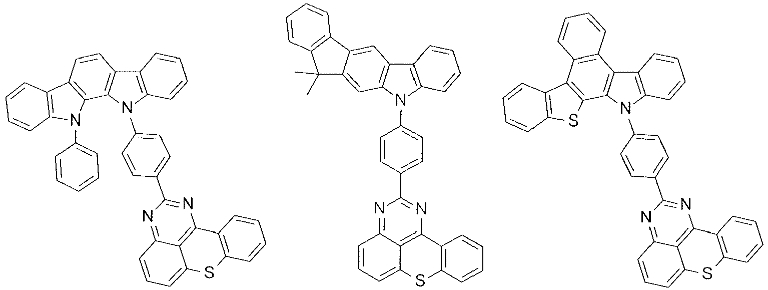

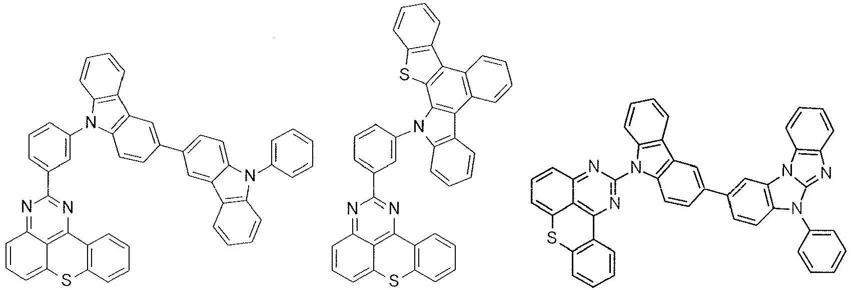

Further preferred examples, wherein Y is S:

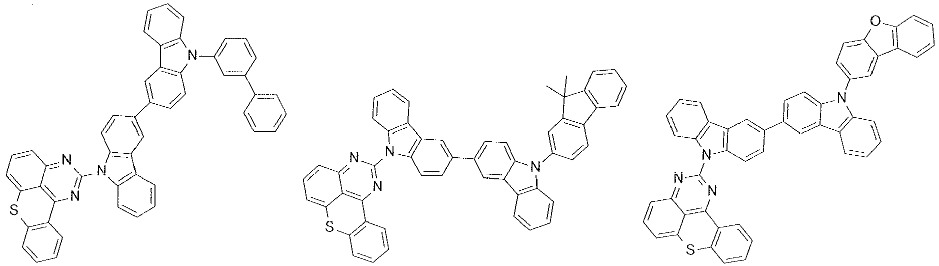

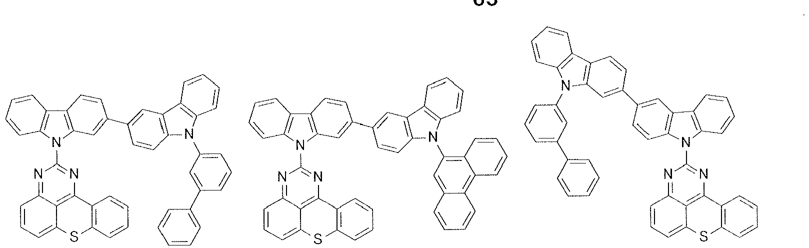

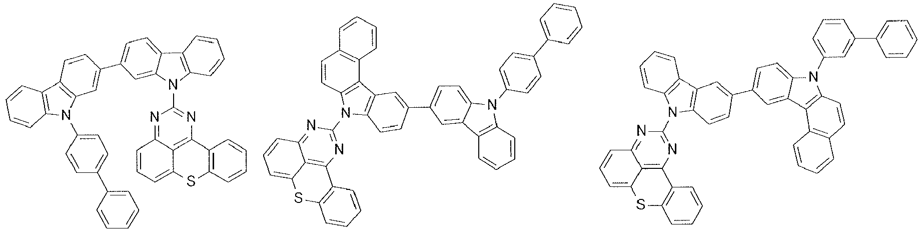

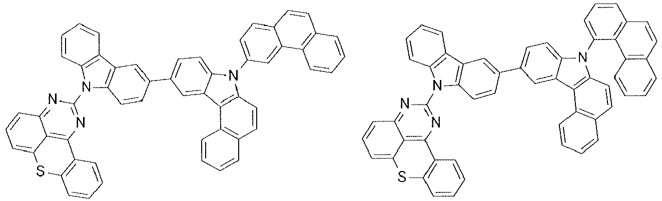

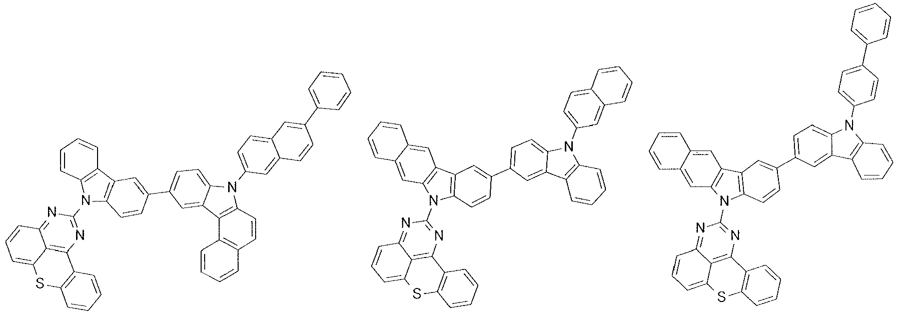

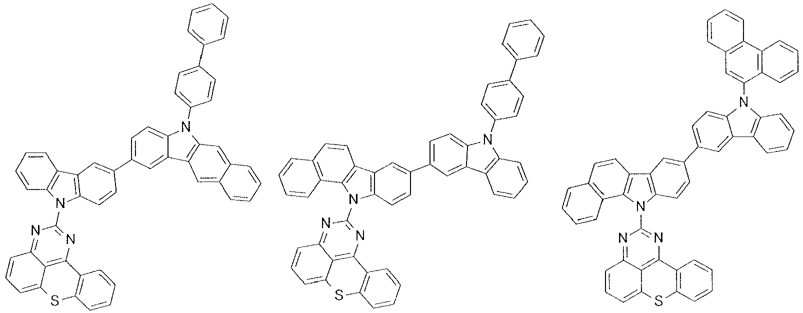

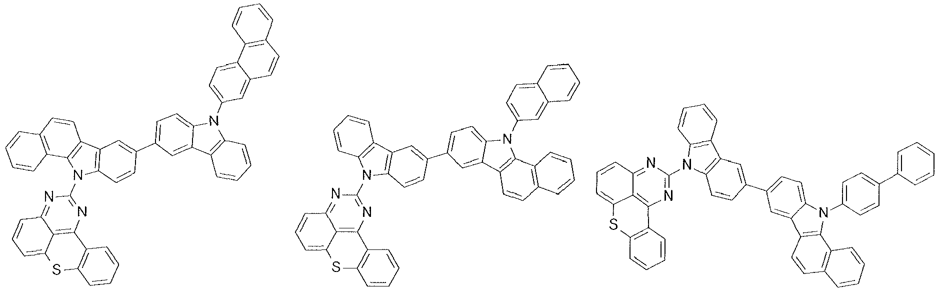

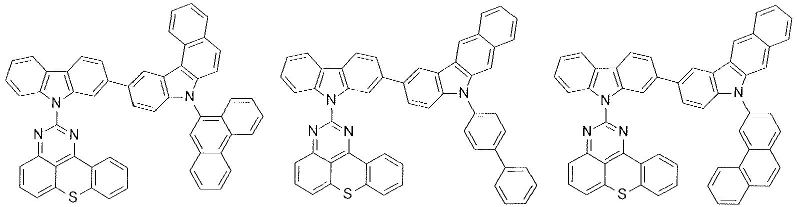

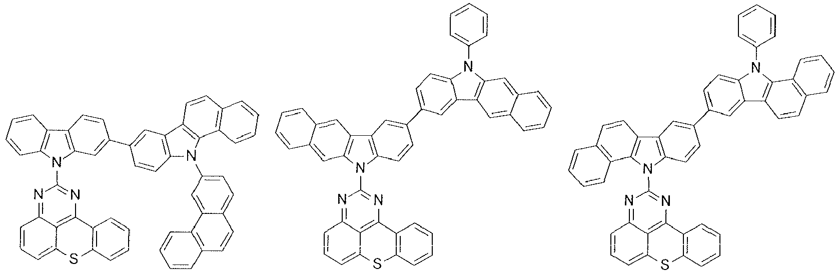

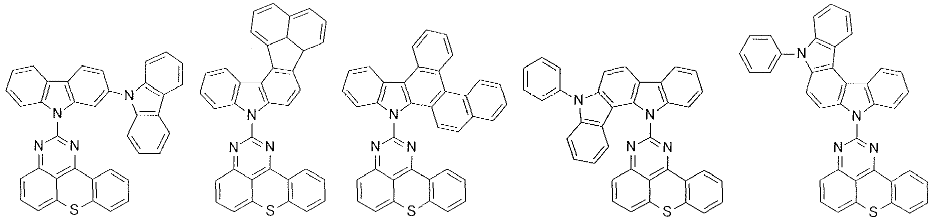

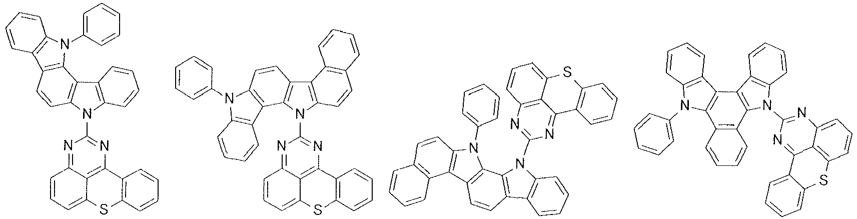

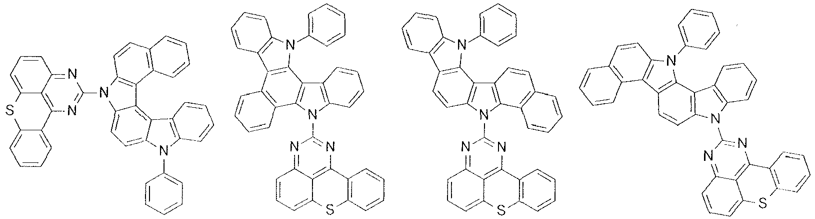

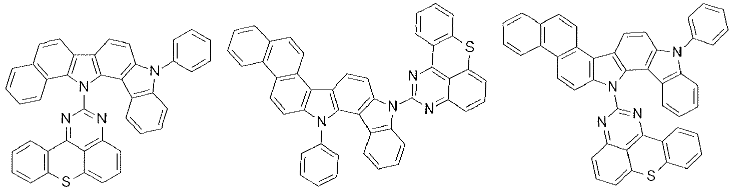

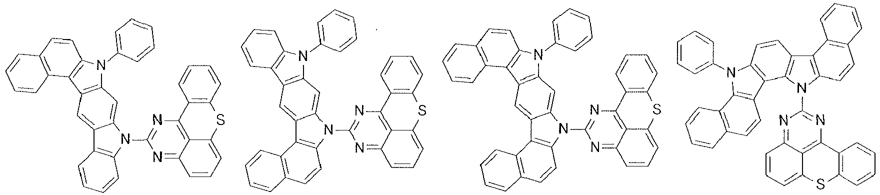

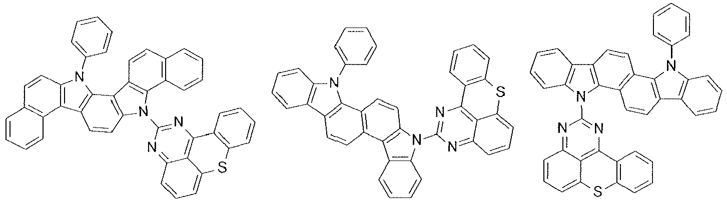

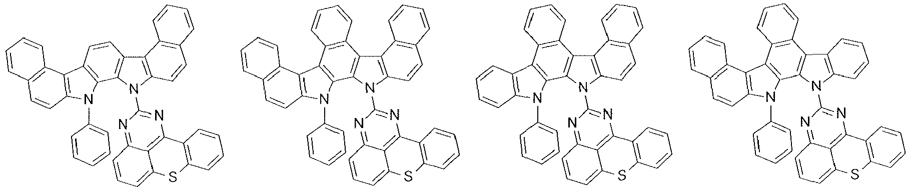

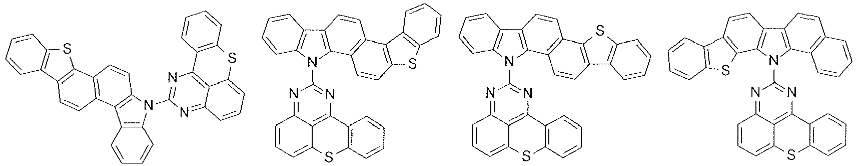

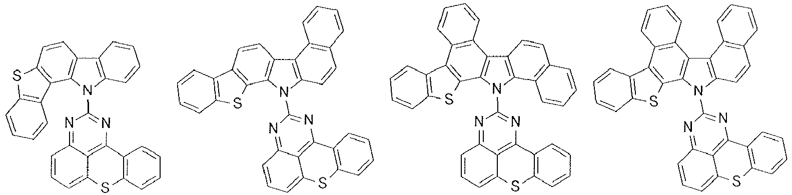

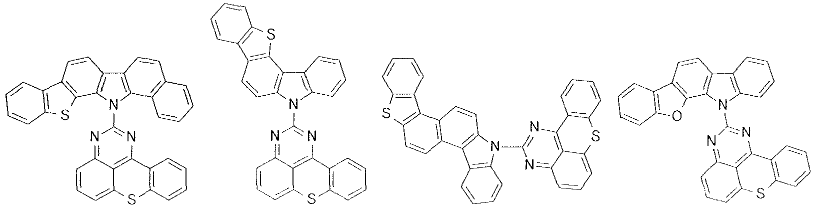

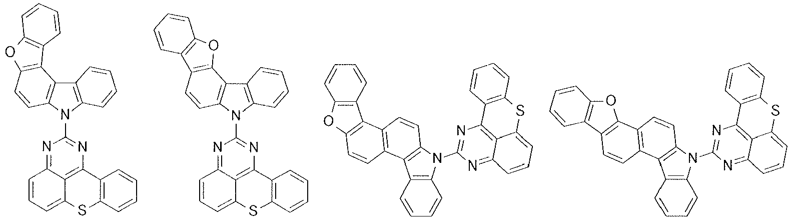

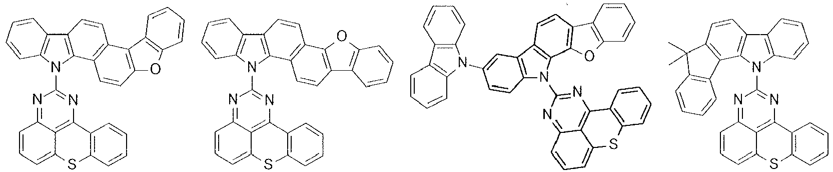

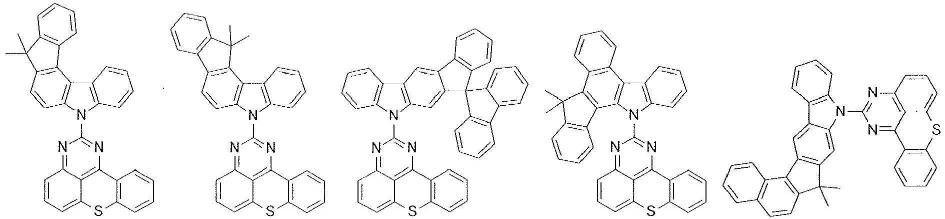

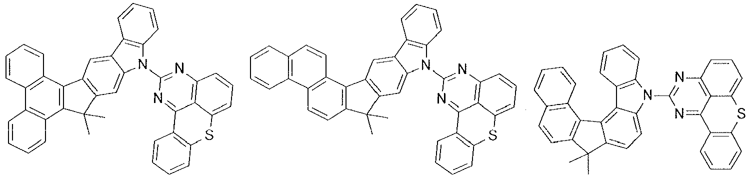

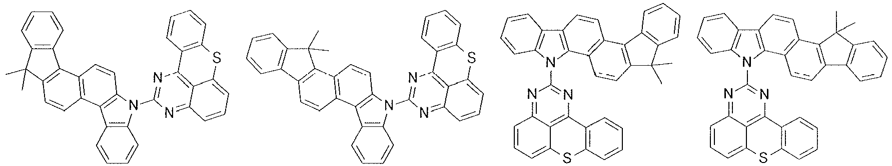

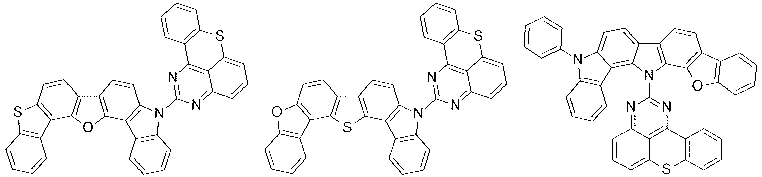

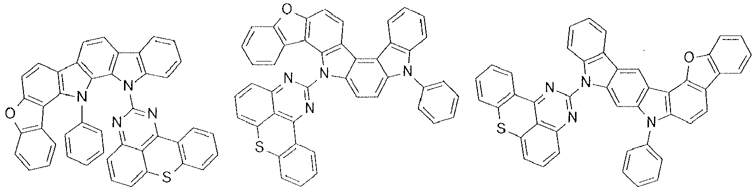

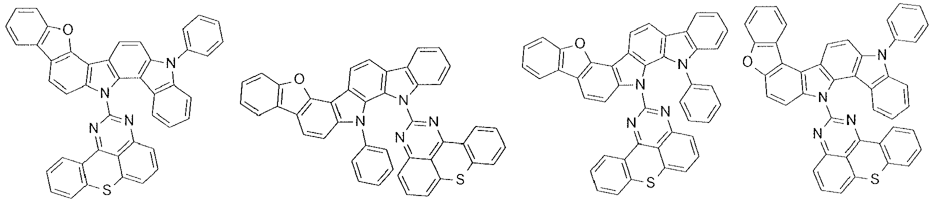

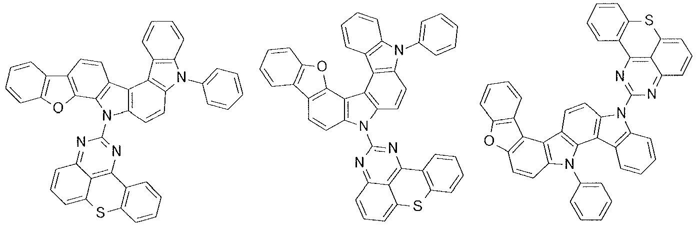

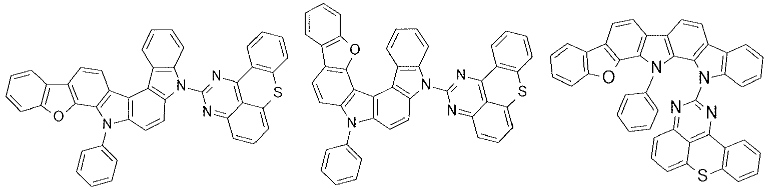

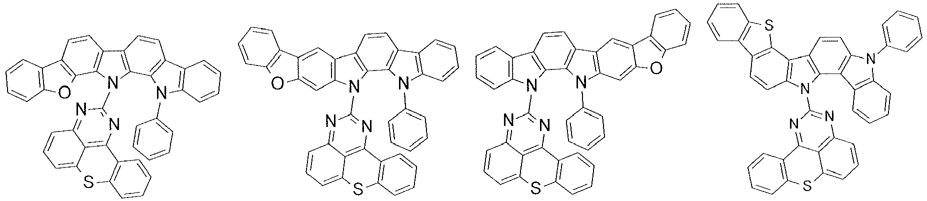

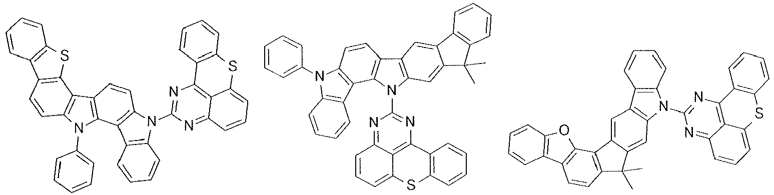

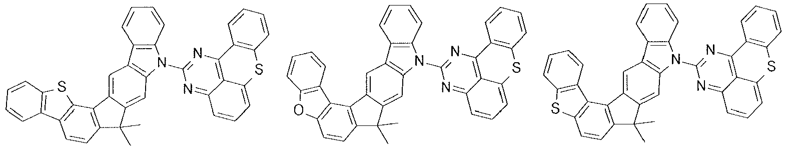

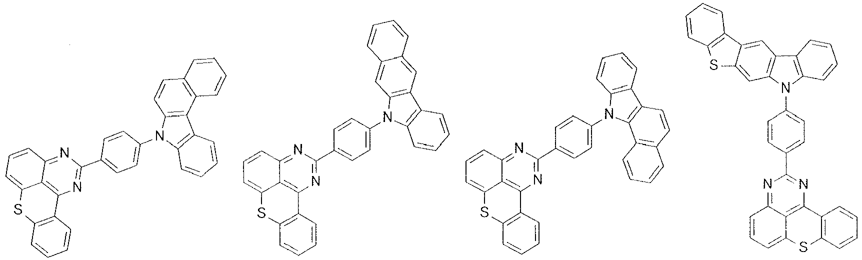

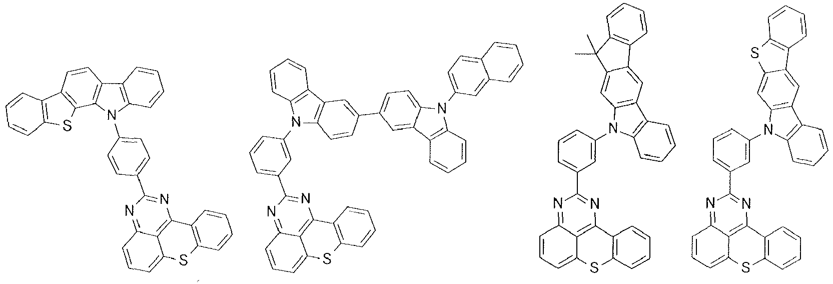

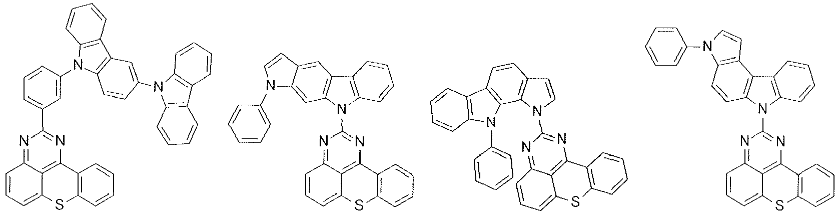

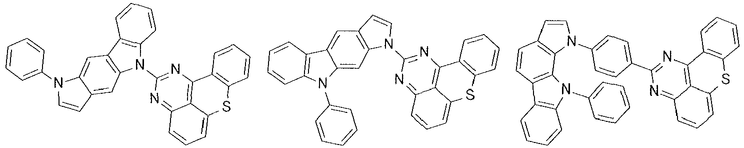

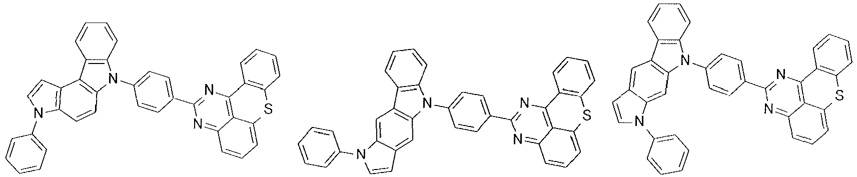

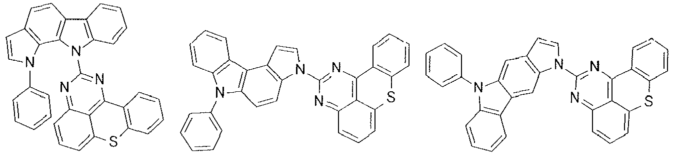

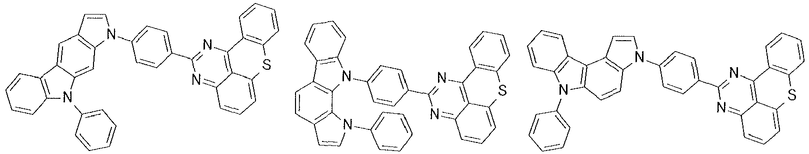

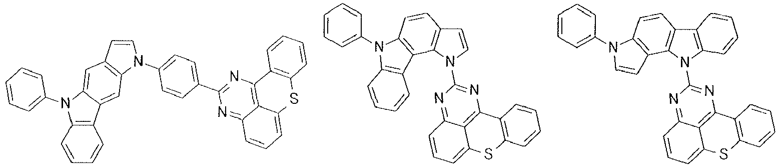

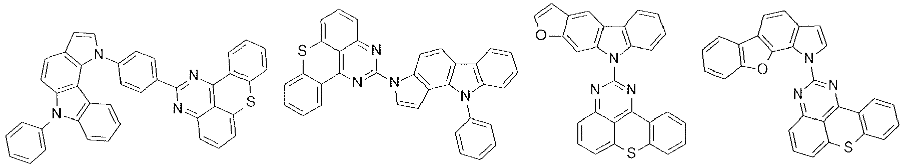

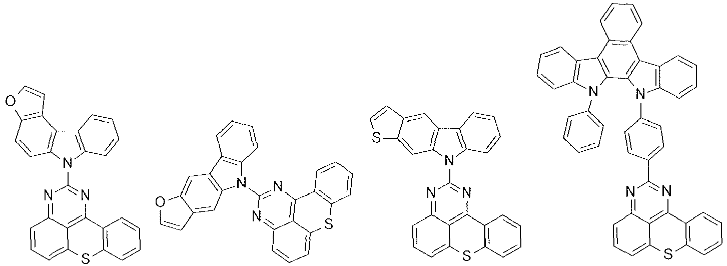

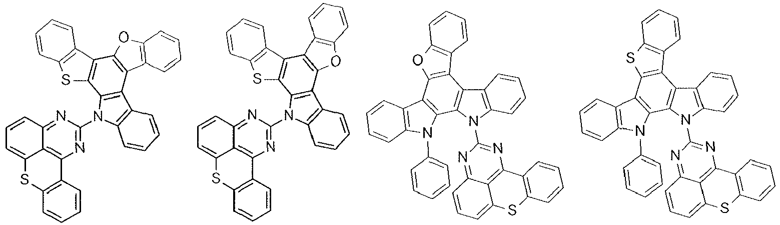

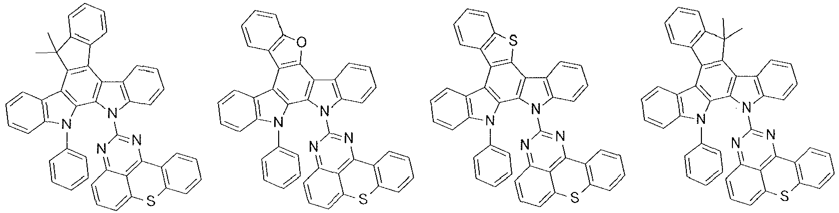

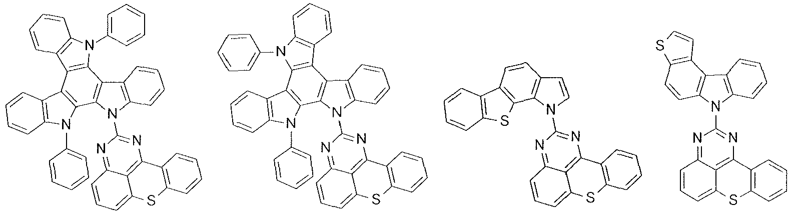

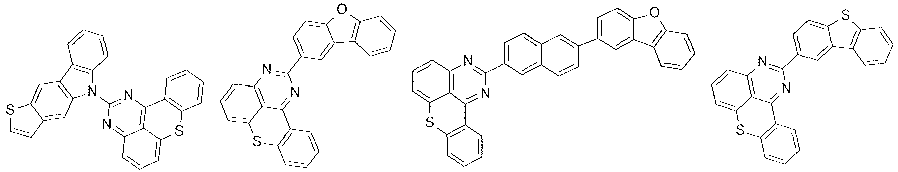

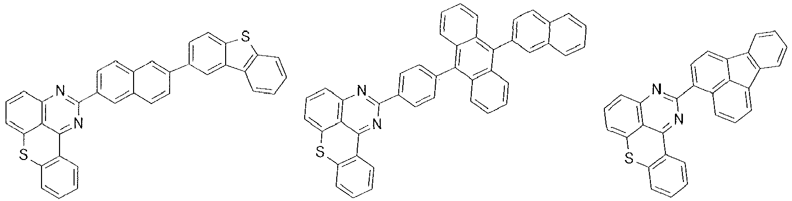

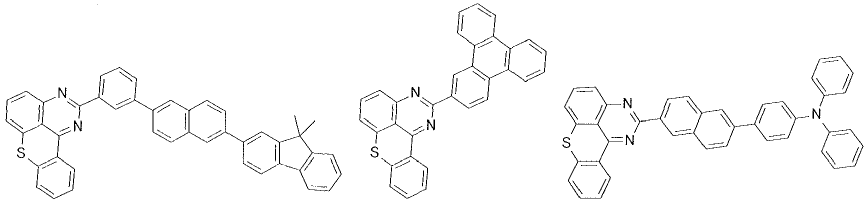

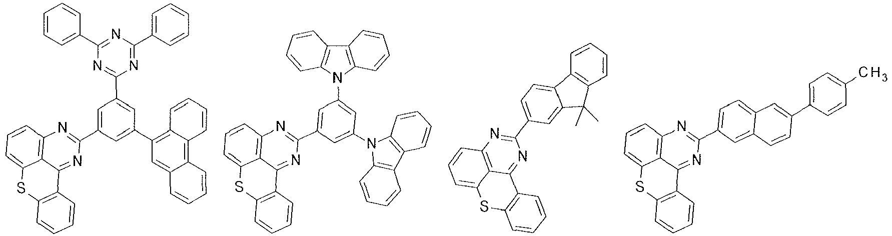

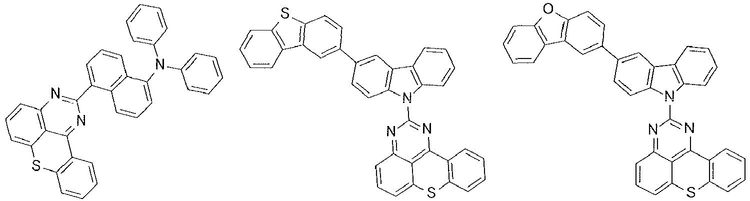

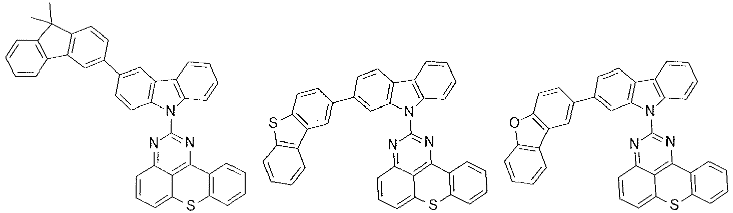

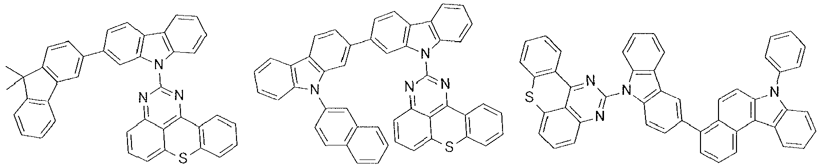

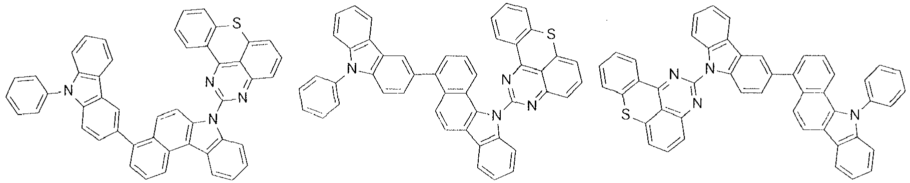

Further preferred examples:

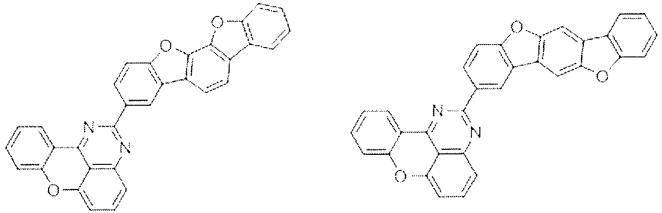

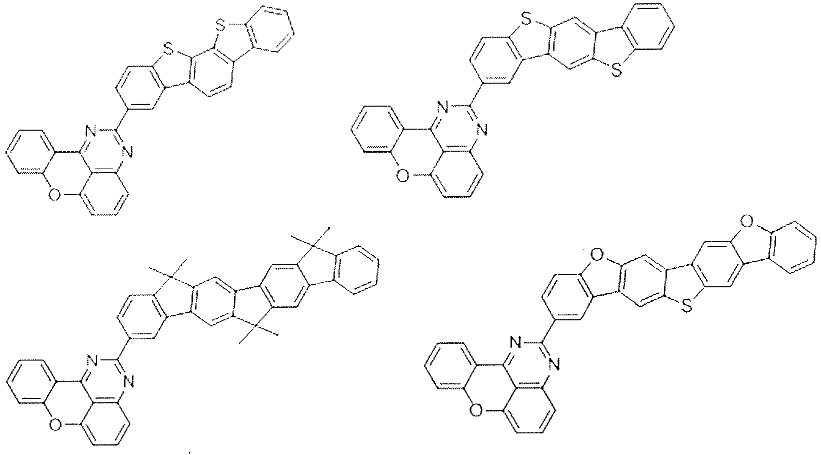

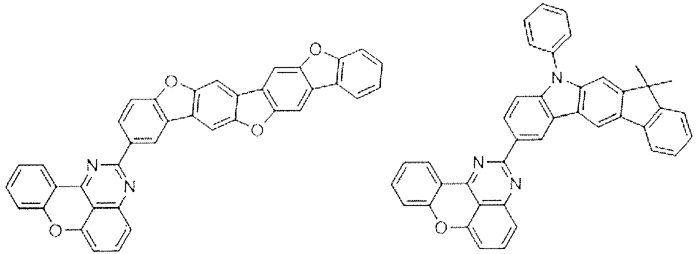

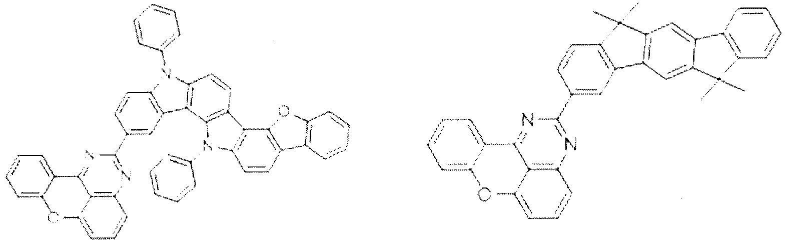

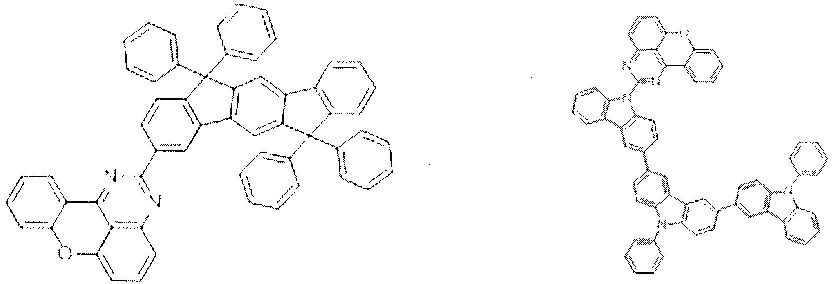

The present invention also relates to a process for the preparation of a compound according to general formula (IIb) and (IIc) as defined above, at least comprising step (A)

### (A) coupling of a compound according to general formula (Vaa) or (Vab)

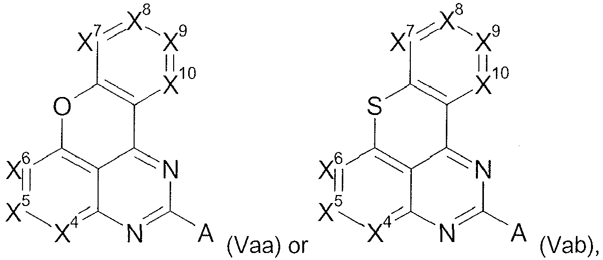

with a compound of formula R²-H to obtain a compound according to general formula (lib) or (IIc), wherein X⁴, X⁵, X⁶, X⁷, X⁸, X⁹, X¹⁰ and R² have the same meanings as defined above and A is a selected from Cl, Br, I, F, OSO₂CH₃, and OSO₂CF₃ or OSO₂C₆H₄CH₃.

Step (A) of the process according to the present invention can in general be conducted by any method and under any conditions that are known to provide the desired product by a person having ordinary skill in the art. For example, step (A) of the process according to the present invention can be coupling reactions that are known to a person having ordinary skill in the art, for example reactions using palladium or copper catalysts.

The present invention therefore preferably relates to the process according to the present invention, wherein step (A) is a coupling reaction that is conducted in the presence of a palladium and/or copper catalyst.

According to one preferred embodiment, step (A) of the process according to the present invention can be conducted using the so called Buchwald-Hartwig reaction which is known to the skilled artisan and which is, for example, mentioned in Adv. Synth. Catal., 2006, 23, J. Organomet. Chem., 1999, 125, Chem. Sci., 2011, 27.

According to another preferred embodiment, step (A) of the process according to the present invention can be conducted using the so called Suzuki reaction which is known to the skilled artisan and which is, for example, mentioned in Chem. Soc. Rev., 2014, 3525, Angew. Chem. Int. Ed., 2009, 6954.

According to another preferred embodiment, step (A) of the process according to the present invention can be conducted using the so called Ulmann reaction which is known to the skilled artisan and which is, for example, mentioned in Chem. Rev., 1995, 2457, "Boronic Acids" Wiley-VCH, 2005.

In particular the coupling according to step (A) of the process according to the present invention is conducted in the presence of at least one basic compound, for example selected from the group consisting of alkali metal salts of alcohols having 1 to 6 carbon atoms, in particular sodium tert-butoxide, potassium tert-butoxide, potassium carbonate, cesium carbonate, rubidium carbonate or potassium phosphate.

The reaction is preferably conducted in at least one aprotic, organic solvent. Preferred are aromatic solvents, for example selected from the group consisting of toluene, benzene, xylene, mesitylene and mixtures thereof.

As a catalyst particularly preferably a combination of at least one Lewis acid and of at least one palladium compound is used. Particularly preferably, a combination of at least one boron comprising complex and at least one palladium salt is used, for example a combination of tert-Bu₃P-HBF₄ and Pd₂(dba)₃, wherein dba means dibenzylideneacetone. Other suitable ligands and/or palladium comprising reagents are mentioned in the above mentioned scientific papers.

The reaction is conducted at a temperature that is high enough to obtain the desired compound in high yield and high selectivity, for example at 40 to 160 °C, preferably at 60 to 140 °C, particularly preferably at 70 to 120 °C.

The reaction is conducted for a time that is long enough to obtain the desired compound in high yield and high selectivity, for example for 1 to 6 h, preferably for 2 to 4 h, particularly preferably for 3 h.

After the reaction is completed, the reaction mixture can be worked up according to methods that are known to the skilled artisan, for example extraction, filtration, recrystallization, chromatography etc.

The reaction product can be analyzed, for example, by proton- or carbon-NMR, mass spectrometry etc.

The substrates that are used in step (A) of the process according to the present invention, i.e. compounds according to general formulae (Vaa) and (Vab) and compounds according to general formula R²-H, wherein R² has the meanings as mentioned above, can be made by methods that are known to a person having ordinary skill in the art. The compound according to general formula R²-H, is, for example, commercially available.

A preferred reaction sequence to obtain the compounds of formulae (Vaa) and (Vab) is shown in the following (the compounds of formulae (Vaa) and (Vab) are summarized as compounds of formula (Va)):
Step (A01): A compound according to general formula (VI) is transferred to a compound of general formula (VII). 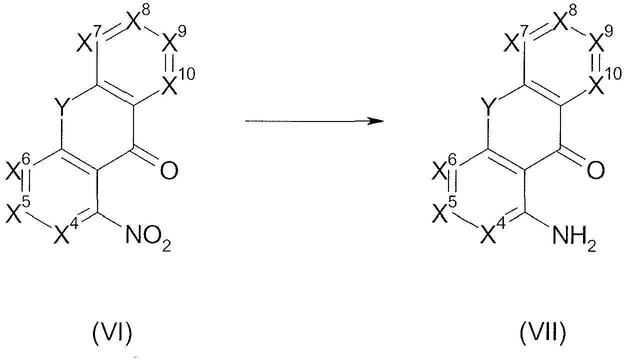 wherein Y is O or S, and, X⁴, X⁵, X⁶, X⁷, X⁸, X⁹ and X¹⁰ have the same meanings as defined above. Y is preferably O.

In particular the reaction according to step (A01) of the process according to the present invention is conducted in the presence of at least one reducing compound, for example selected from the group consisting of HCO₂NH₄ and mixtures thereof.

The reaction is preferably conducted in at least one organic solvent. Preferred are alcohols, for example selected from the group consisting of ethanol, isopropanol and mixtures thereof

Preferably step (A01) is conducted in the presence of a catalyst. As a preferred catalyst palladium is used. Particularly preferably, palladium on carbon is used.

The reaction is conducted at a temperature that is high enough to obtain the desired compound in high yield and high selectivity, for example at 40 to 160 °C, preferably at 60 to 120 °C, particularly preferably at reflux temperature of the solvent.

The reaction is conducted for a time that is long enough to obtain the desired compound in high yield and high selectivity, for example for 0.2 to 6 h, preferably for 0.5 to 3 h.

After the reaction is completed, the reaction mixture can be worked up according to methods that are known to the skilled artisan, for example extraction, filtration, recrystallization, chromatography etc.

Step (A02): The compound according to general formula (VII) is then transferred to a compound of general formula (VIII). 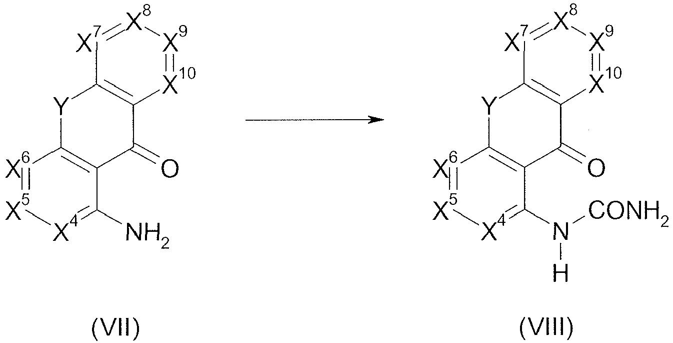 wherein Y is O or S, and, X⁴, X⁵, X⁶, X⁷, X⁸, X⁹ and X¹⁰ have the same meanings as defined above. Y is preferably O.

In particular the reaction according to step (A02) of the process according to the present invention is conducted in the presence of KOCN.

The reaction is preferably conducted in at least one acidic organic solvent. Preferred are carboxylic acids, for example acetic acid.

The reaction is conducted at a temperature that is high enough to obtain the desired compound in high yield and high selectivity, for example at 10 to 40 °C, preferably at room temperature, i.e. 20 °C.

The reaction is conducted for a time that is long enough to obtain the desired compound in high yield and high selectivity, for example for 1 to 6 h, preferably for 2 to 4 h.

After the reaction is completed, the reaction mixture can be worked up according to methods that are known to the skilled artisan, for example extraction, filtration, recrystallization, chromatography etc.

Step (A03): The compound according to general formula (VIII) is then transferred to a compound of general formula (IX). 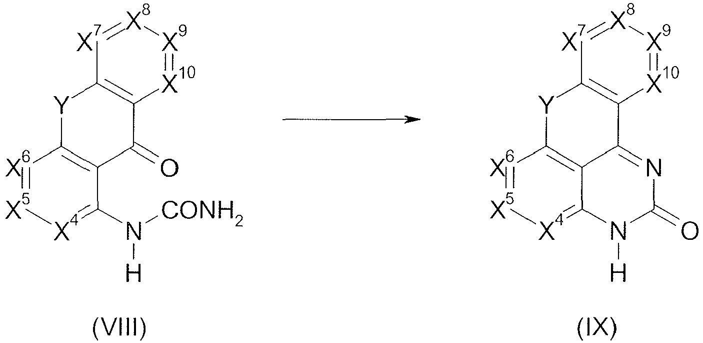 wherein Y is O or S, and, X⁴, X⁵, X⁶, X⁷, X⁸, X⁹ and X¹⁰ have the same meanings as defined above. Y is preferably O.

In particular the reaction according to step (A03) of the process according to the present invention is conducted in the presence of at least one basic compound selected from the group consisting of KOH, NaOH and mixtures thereof.

The reaction is preferably conducted in at least one organic solvent, for example alcohols like ethanol, isopropanol or mixtures thereof

The reaction is conducted at a temperature that is high enough to obtain the desired compound in high yield and high selectivity, for example at 50 to 150 °C, preferably at reflux temperature of the solvent.

The reaction is conducted for a time that is long enough to obtain the desired compound in high yield and high selectivity, for example for 0.1 to 2 h, preferably for 0.3 to 1 h.

After the reaction is completed, the reaction mixture can be worked up according to methods that are known to the skilled artisan, for example extraction, filtration, recrystallization, chromatography etc.

Step (A04): The compound according to general formula (IX) is then transferred to a compound of general formula (X). 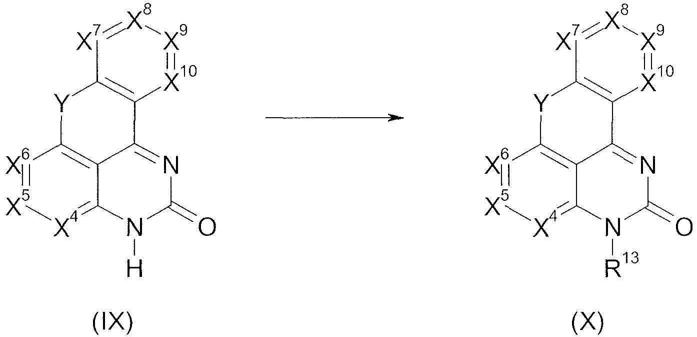 wherein Y is O or S, and, X⁴, X⁵, X⁶, X⁷, X⁸, X⁹ and X¹⁰ have the same meanings as defined above and R¹³ is linear or branched C₁-C₆alkylgroup, preferably methyl. Y is preferably O.

In particular the reaction according to step (A04) of the process according to the present invention is conducted in the presence of at least one strongly basic compound, for example NaH.

The reaction according to step (A04) of the process according to the present invention is further conducted in the presence of at least one compound according to formula R¹³-B, wherein R¹³ has the meanings as mentioned above and B is selected from I, Br or Cl.

The reaction is preferably conducted in at least one organic solvent, for example dimethylformamide or mixtures thereof

The reaction is conducted at a temperature that is high enough to obtain the desired compound in high yield and high selectivity, for example at -20 to 20 °C, preferably at -10 to 10 °C, most preferably at 0 °C.

The reaction is conducted for a time that is long enough to obtain the desired compound in high yield and high selectivity, for example for 0.5 to 4 h, preferably for 1 to 3 h.

After the reaction is completed, the reaction mixture can be worked up according to methods that are known to the skilled artisan, for example extraction, filtration, recrystallization, chromatography etc.

Step (A05): The compound according to general formula (X) is then transferred to a compound of general formula (Va). 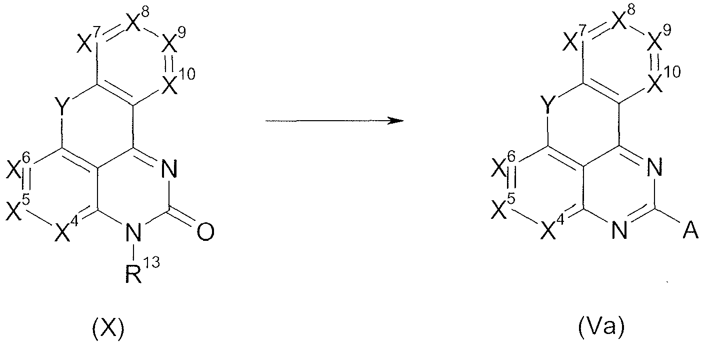 wherein Y is O or S, and, X⁴, X⁵, X⁶, X⁷, X⁸, X⁹, X¹⁰ and R¹² have the same meanings as defined above. Y is preferably O.

In particular, the reaction according to step (A05) according to the present invention is conducted in the presence of at least one compound, which is able to introduce the moiety A into the molecule. According to the preferred case that A is Cl, chlorination agents are used, for example phosphorous comprising chlorination agents like POCl₃ or PCl₅ in step (A05) of the process according to the present invention

The reaction according to step (A05) is preferably conducted without any solvent, i.e. in substance.

The reaction is conducted at a temperature that is high enough to obtain the desired compound in high yield and high selectivity, for example at 40 to 160 °C, preferably at 60 to 120 °C, particularly preferably at 70 to 90 °C.

The reaction is conducted for a time that is long enough to obtain the desired compound in high yield and high selectivity, for example for 1 to 6 h, preferably for 2 to 4 h.

After the reaction is completed, the reaction mixture can be worked up according to methods that are known to the skilled artisan, for example extraction, filtration, recrystallization, chromatography etc.

Further detailed reaction conditions of this reaction scheme for obtaining the compound of formula (Va) can be taken from Carlos M. Martinez et al., J. Heterocyclic Chem., 44, 1035 (2007).

Particularly preferably, the process according to the present invention for the preparation of compounds according to general formula (IIb) and (IIc) comprises step (A01), followed by step (A02), followed by step (A03), followed by step (A04), followed by step (A05), followed by step (A).

According to another preferred embodiment of the process according to the present invention, the compound according to general formula (Va) can also be obtained directly from compound of general formula (IX) using reaction conditions as mentioned in respect of step (A05). The present invention therefore preferably relates to the process according to the present invention, wherein the compound according to general formula (Va) can also be obtained directly from compound of general formula (IX) as shown in the following (step (A05a):

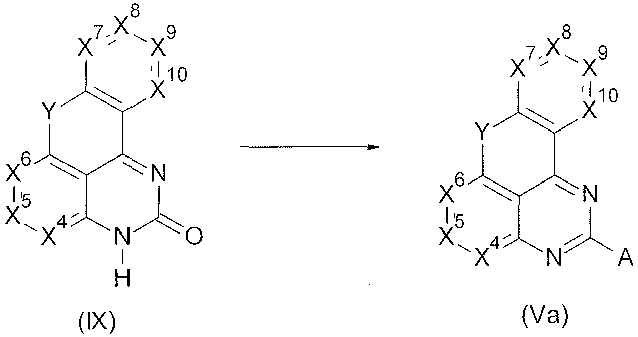

Therefore, particularly preferably, the process according to the present invention for the preparation of compounds according to general formulae (lib) and (IIc) comprises step (A01), followed by step (A02), followed by step (A03), followed by step (A05a), followed by step (A).

According to the preferred embodiment that Y is S, the process according to the present invention for the preparation of compounds according to general formula (IIc) comprises step (A01), followed by step (A02), followed by step (A03), followed by step (A05a), followed by step (A).

Compound (VI) as mentioned above can be prepared in chemical reactions that are known to the skilled artisan. Preferred methods for the preparation of the compound according to general formula (VI) are mentioned in Yamagami, Isao et al., Jpn. Kokai Tokkyo Koh, 2008273906, 2008, Kralj, Ana et al., ChemMedChem, 9(1), 151-168, 2014 and Okabayashi, Ichizo and Iwata, Noriko, Chemical and Pharmaceutical Bulletin, 28(9), 2831-5, 1980.

The compound according to general formula (Va) as mentioned above can also be prepared by further methods that are explained in the following. For example, the compound according to general formula (Va) can be prepared from a compound according to general formula (XI): 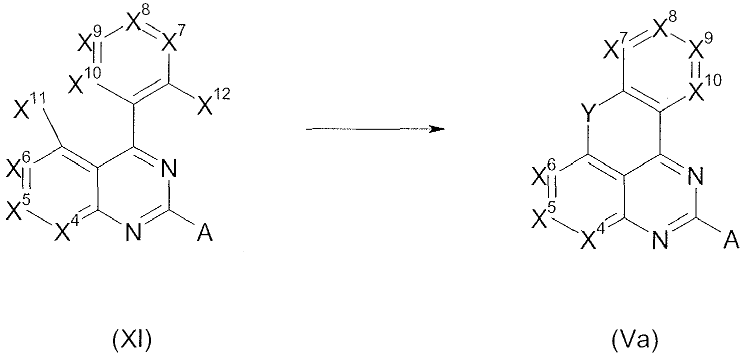 wherein Y is O or S, and, X⁴, X⁵, X⁶, X⁷, X⁸, X⁹, X¹⁰ and A have the same meanings as defined above, X¹¹ and X¹² are independently of each other F or OH, if Y is O, or X¹¹ and X¹² are independently of each other H or S(O)CH₃, if Y is S.

According to the preferred embodiment that Y is O, the following reaction is conducted (step (A06):

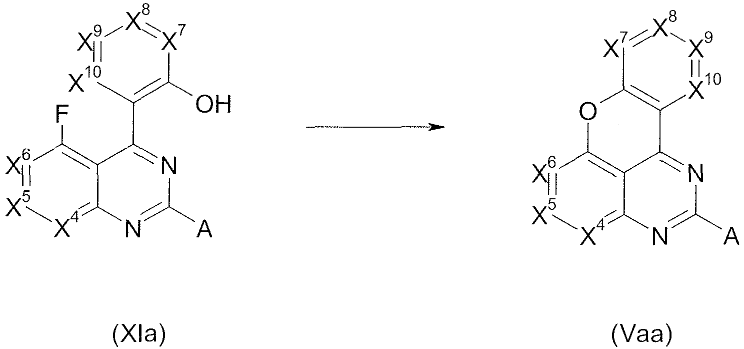

According to this preferred embodiment (step (A06)) the compound according to general formula (Xla) is transferred into the compound according to general formula (Vaa), wherein X⁴, X⁵, X⁶, X⁷, X⁸, X⁹, X¹⁰ and A have the same meanings as defined above.

The reaction is preferably conducted in the presence of at least one basic compound, for example selected from the group consisting of K₂CO₃, NaH and mixtures thereof. Further reaction conditions like temperature, solvent, reaction time etc. are known to the person having ordinary skill in the art.

The compound according to general formula (Xla) can be obtained by any method that is known to the skilled artisan. Preferably the compound according to general formula (Xla) is obtained according to the method described in Kang Hyun-Ju et al., WO 2014088290 or Welsh Dean M et al., Jpn. Kokai Tokkyo Koh, 2014183315, 2014.

According to the preferred embodiment that Y is S, the following reaction is conducted (step (A07):

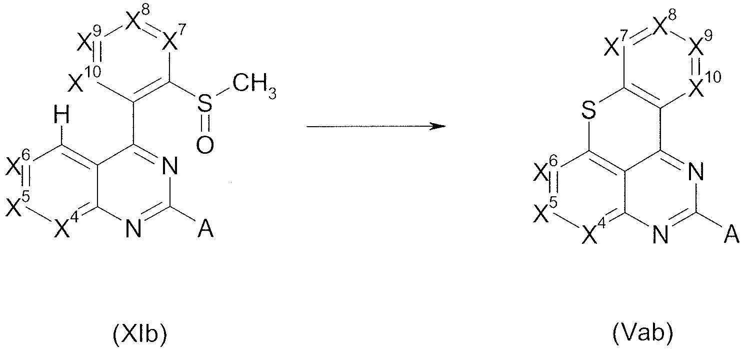

According to this preferred embodiment (step (A07)) the compound according to general formula (Xlb) is transferred into the compound according to general formula (Vab), wherein X⁴, X⁵, X⁶, X⁷, X⁸, X⁹, X¹⁰ and A have the same meanings as defined above.

The reaction is preferably conducted in the presence of at least one acidic compound, for example selected from the group consisting of (CF₃SO₂)₂, CF₃SO_{2O}H, and mixtures thereof.

Further reaction conditions like temperature, solvent, reaction time etc. are known to the person having ordinary skill in the art.

Compounds according to the present invention in organic electronics applications

In the following the use of the compounds according to the present invention in organic electronic applications will be explained. This use will be explained in respect of the compounds according to general formulae (lib) and (IIc) according to the present invention. A person having ordinary skill in the art understands that these explanations also and in particular relate to their preferred embodiments and to the specifically defined molecules that are mentioned in this application.

It has been found that the compounds according to general formulae (lib) and (IIc) are particularly suitable for use in applications in which charge carrier conductivity is required, especially for use in organic electronics applications, for example selected from switching elements such as organic transistors, e.g. organic FETs and organic TFTs, organic solar cells and organic light-emitting diodes (OLEDs).

The present invention therefore also relates to an electronic device comprising at least one compound according to the present invention.

The organic transistor generally includes a semiconductor layer formed from an organic layer with charge transport capacity; a gate electrode formed from a conductive layer; and an insulating layer introduced between the semiconductor layer and the conductive layer. A source electrode and a drain electrode are mounted on this arrangement in order thus to produce the transistor element. In addition, further layers known to those skilled in the art may be present in the organic transistor. The layers with charge transport capacity may comprise the compounds according to general formulae (lib) and (IIc) according to the present invention.

The organic solar cell (photoelectric conversion element) generally comprises an organic layer present between two plate-type electrodes arranged in parallel. The organic layer may be configured on a comb-type electrode. There is no particular restriction regarding the site of the organic layer and there is no particular restriction regarding the material of the electrodes. When, however, plate-type electrodes arranged in parallel are used, at least one electrode is preferably formed from a transparent electrode, for example an ITO electrode or a fluorine-doped tin oxide electrode. The organic layer is formed from two sublayers, i.e. a layer with p-type semiconductor properties or hole transport capacity, and a layer formed with n-type semiconductor properties or charge transport capacity. In addition, it is possible for further layers known to those skilled in the art to be present in the organic solar cell. The layers with charge transport capacity may comprise a compound according to general formulae (IIb) and (IIc) according to the present invention.

The compounds according to general formulae (IIb) and (IIc) being particularly suitable in OLEDs for use as a host (=matrix) material, preferably in a light-emitting layer, and/or a charge transport material, for example as a hole transport material and/or as an electron transporting material, charge and/or exciton blocking material, particularly preferably as a host material or as an electron transporting material.

The present invention also relates to a material for organic electroluminescent device comprising at least one compound according to general formula (IIb) or (IIc).

The present invention therefore preferably relates to the electronic device according to the present invention, preferably an organic electroluminescence device, more preferably an organic light emitting diode (OLED), comprising a cathode, an anode, and a plurality of organic thin film layers provided between the cathode and the anode, the organic thin film layers comprising an emitting layer comprising the at least one compound of general formula (IIb) or (IIc), preferably as a host material, a charge transporting material, charge and/or exciton blocking material, particularly preferably as a host material or as an electron transporting material.

The compounds according to general formulae (IIb) and (IIc) are preferably suitable in OLEDs as host material, preferably in combination with a red phosphorescence emitter. Further preferred, the compounds according to general formulae (lib) and (IIc) are particularly suitable in OLEDs as an electron transporting material, preferably in a light-emitting layer, especially in combination with preferably a blue fluorescence emitter.

The present invention preferably relates to the electronic device, preferably an organic electroluminescence device, more preferably an organic light emitting diode (OLED), according to the present invention, wherein the emitting layer comprises a phosphorescent material, which is an ortho-metallated complex comprising a metal atom selected from iridium (Ir), osmium (Os) and platinum (Pt).

The present invention further relates to an electronic equipment comprising the organic electroluminescence device according to the present invention.

The present invention also relates to an emitting layer, preferably present in an electronic device, more preferably in an electroluminescence device, particularly preferably in an organic light emitting diode (OLED), comprising at least one compound of general formula (IIb) or (IIc) according to the present invention.

The present invention therefore preferably relates to the use of a compound according to general formula (lib) or (IIc) as defined above in an electronic device, preferably in an electroluminescence device, particularly preferably in an organic light emitting diode (OLED), preferably in an emitting layer, as a host material, a charge transporting material, for example as a hole transport material or an electron transport material, preferably as an electron transporting material, as a charge and/or exciton blocking material, preferably as a host material or an electron transporting material.

In the case of use of the inventive compounds according to general formula (IIb) or (IIc) in OLEDs, OLEDs which have good efficiencies and a long lifetime and which can be operated especially at a low use and operating voltage are obtained. The inventive compounds according to general formulae (IIb) and (IIc) are suitable especially for use as matrix and/or charge transport and/or charge blocking materials for green, red and yellow, preferably green and red, more preferably red emitters. The inventive compounds according to general formulae (lib) and (IIc) are further suitable especially for use as electron transporting material for blue emitters. Furthermore, the compounds according to general formulae (lib) and (IIc) can be used as conductor/complementary materials in organic electronics applications selected from switching elements and organic solar cells. According to the present application, the terms matrix and host are used interchangeable.

In the emission layer or one of the emission layers of an OLED, it is also possible to combine an emitter material with at least one matrix material of the compound according to general formula (lib) or (IIc) and one or more, preferably one, further matrix materials (co-host). This may achieve a high quantum efficiency, low driving voltage and/or long lifetime of these devices.

According to one preferred embodiment of the present invention the compounds according to general formulae (IIb) and (IIc) are used as host materials, preferably in emitting layers comprising red light-emitting compounds. According to this embodiment, preferably no further host material is present in the light-emitting layer.

According to another preferred embodiment of the present invention the compounds according to general formulae (lib) and (IIc) are used as host materials, preferably in emitting layers comprising green light-emitting compounds. According to this embodiment, the compounds according to the present invention are preferably used in the presence of at least one further host material, i.e. as a co-host. Further host materials are mentioned in the following.

It is likewise possible that the compounds according to general formula (lib) or (IIc) are present in two or three of the following layers: in the light-emitting layer (preferably as host material) and/or in the transport layer (as electron transport material).

When a compound according to general formula (lib) or (IIc) is used as matrix (host) material in an emission layer and additionally as electron transport material, owing to the chemical identity or similarity of the materials, an improved interface between the emission layer and the adjacent material, which can lead to a decrease in the voltage with equal luminance and to an extension of the lifetime of the OLED. Moreover, the use of the same material as electron transport material and/or as matrix of an emission layer allows the production process of an OLED to be simplified, since the same source can be used for the vapor deposition process of the material of one of the compounds of the formula the compound according to general formula (I).

Suitable structures of organic electronic devices, especially organic light-emitting diodes (OLED), are known to those skilled in the art and are specified below.

For example, the electronic device, preferably an organic electroluminescence device, more preferably an organic light emitting diode (OLED), according to the present invention comprises a cathode, an anode, and a plurality of organic thin film layers provided between the cathode and the anode, the organic thin film layers comprising an emitting layer comprising the at least one compound of general formula (lib) or (IIc), preferably as a host material, a charge transporting material, for example as a hole transport material or an electron transport material, preferably as an electron transporting material, and/or exciton blocking material, particularly preferably as a host material or as an electron transporting material.

The present invention therefore preferably relates to the electronic device, preferably an organic electroluminescence device, more preferably an organic light emitting diode (OLED), according to the present invention, comprising a cathode, an anode, and a plurality of organic thin film layers provided between the cathode and the anode, the organic thin film layers comprising an emitting layer comprising the at least one compound of general formula (lib) or (IIc), preferably as a host material, a charge transporting material, for example as a hole transport material or an electron transport material, preferably as an electron transporting material, and/or exciton blocking material, particularly preferably as a host material or as an electron transporting material. More preferably, the present invention provides an organic light-emitting diode (OLED) comprising an anode and a cathode and a light-emitting layer arranged between the anode and the cathode, and if appropriate at least one further layer selected from the group consisting of at least one blocking layer for holes/excitons, at least one blocking layer for electrons/excitons, at least one hole injection layer, at least one hole transport layer, at least one electron injection layer and at least one electron transport layer, wherein the at least one compound according to general formula (lib) or (IIc) is present in the light-emitting layer and/or in at least one of the further layers. The at least one compound according to general formula (IIb) or (IIc) is preferably present in the light-emitting layer and/or hole/exciton blocking layer and/or the charge blocking layer, i.e. the electron or hole transport layer.

In a preferred embodiment of the present invention, at least one compound according to general formula (IIb) or (IIc) is used as electron transport material. Examples of preferred compounds according to general formulae (lib) and (IIc) are shown above.

In another preferred embodiment of the present invention, at least one compound of general formula (lib) or (IIc) is used as charge/exciton blocker material. Examples of preferred compounds according to general formulae (IIb) and (IIc) are shown above.

The present application further relates to a light-emitting layer, preferably present in an electronic device, more preferably in an electroluminescence device, particularly preferably in an organic light emitting diode (OLED), comprising at least one compound of general formula (lib) or (IIc) as defined above, preferably as host material or co-host material. Examples of preferred compounds according to general formulae (lib) and (IIc) are shown above.

Most preferably, the electronic device according to the present invention is an organic light emitting diode (OLED).

### Structure of the inventive OLED

The inventive organic light-emitting diode (OLED) thus generally has the following structure: an anode (a) and a cathode (i) and a light-emitting layer (e) arranged between the anode (a) and the cathode (i).

The inventive OLED may, for example - in a preferred embodiment - be formed from the following layers:
1. Anode (a)
2. Hole transport layer (c)
3. Light-emitting layer (e)
4. Blocking layer for holes/excitons (f)
5. Electron transport layer (g)
6. Cathode (i)

Layer sequences different than the aforementioned structure are also possible, and are known to those skilled in the art. For example, it is possible that the OLED does not have all of the layers mentioned; for example, an OLED with layers (a) (anode), (e) (light-emitting layer) and (i) (cathode) is likewise suitable, in which case the functions of the layers (c) (hole transport layer) and (f) (blocking layer for holes/excitons) and (g) (electron transport layer) are assumed by the adjacent layers. OLEDs which have layers (a), (c), (e) and (i), or layers (a), (e), (f), (g) and (i), are likewise suitable. In addition, the OLEDs may have a blocking layer for electrons/excitons (d) between the hole transport layer (c) and the light-emitting layer (e).

It is additionally possible that a plurality of the aforementioned functions (electron/exciton blocker, hole/exciton blocker, hole injection, hole conduction, electron injection, electron conduction) are combined in one layer and are assumed, for example, by a single material present in this layer. For example, a material used in the hole transport layer, in one embodiment, may simultaneously block excitons and/or electrons.

Furthermore, the individual layers of the OLED among those specified above may in turn be formed from two or more layers. For example, the hole transport layer may be formed from a layer into which holes are injected from the electrode, and a layer which transports the holes away from the hole-injecting layer into the light-emitting layer. The electron transport layer may likewise consist of a plurality of layers, for example a layer in which electrons are injected by the electrode, and a layer which receives electrons from the electron injection layer and transports them into the light-emitting layer. These layers mentioned are each selected according to factors such as energy level, thermal resistance and charge carrier mobility, and also energy difference of the layers specified with the organic layers or the metal electrodes. The person skilled in the art is capable of selecting the structure of the OLEDs such that it is matched optimally to the organic compounds used in accordance with the invention.

In a preferred embodiment the OLED according to the present invention comprises in this order:
(a) an anode,
(b) optionally a hole injection layer,
(c) optionally a hole transport layer,
(d) optionally an exciton blocking layer
(e) an emitting layer,
(f) optionally a hole/ exciton blocking layer
(g) optionally an electron transport layer,
(h) optionally an electron injection layer, and
(i) a cathode.

In a particularly preferred embodiment the OLED according to the present invention comprises in this order:
(a) an anode,
(b) optionally a hole injection layer,
(c) a hole transport layer,
(d) an exciton blocking layer
(e) an emitting layer,
(f) a hole/ exciton blocking layer
(g) an electron transport layer, and
(h) optionally an electron injection layer, and
(i) a cathode.

The properties and functions of these various layers, as well as example materials are known from the prior art and are described in more detail below on basis of preferred embodiments.

### Anode (a):

The anode is an electrode which provides positive charge carriers. It may be composed, for example, of materials which comprise a metal, a mixture of different metals, a metal alloy, a metal oxide or a mixture of different metal oxides. Alternatively, the anode may be a conductive polymer. Suitable metals comprise the metals of groups 11, 4, 5 and 6 of the Periodic Table of the Elements, and also the transition metals of groups 8 to 10. When the anode is to be transparent, mixed metal oxides of groups 12, 13 and 14 of the Periodic Table of the Elements are generally used, for example indium tin oxide (ITO). It is likewise possible that the anode (a) comprises an organic material, for example polyaniline, as described, for example, in Nature, Vol. 357, pages 477 to 479 (June 11, 1992). Preferred anode materials include conductive metal oxides, such as indium tin oxide (ITO) and indium zinc oxide (IZO), aluminum zinc oxide (AlZnO), and metals. Anode (and substrate) may be sufficiently transparent to create a bottom-emitting device. A preferred transparent substrate and anode combination is commercially available ITO (anode) deposited on glass or plastic (substrate). A reflective anode may be preferred for some top-emitting devices, to increase the amount of light emitted from the top of the device. At least either the anode or the cathode should be at least partly transparent in order to be able to emit the light formed. Other anode materials and structures may be used.

### Hole injection layer (b):

Generally, injection layers are comprised of a material that may improve the injection of charge carriers from one layer, such as an electrode or a charge generating layer, into an adjacent organic layer. Injection layers may also perform a charge transport function. The hole injection layer may be any layer that improves the injection of holes from anode into an adjacent organic layer. A hole injection layer may comprise a solution deposited material, such as a spin-coated polymer, or it may be a vapor deposited small molecule material, such as, for example, CuPc or MTDATA. Polymeric hole-injection materials can be used such as poly(N-vinylcarbazole) (PVK), polythiophenes, polypyrrole, polyaniline, self-doping polymers, such as, for example, sulfonated poly(thiophene-3-[2[(2-methoxyethoxy)ethoxy]-2,5-diyl) (Plexcore® OC Conducting Inks commercially available from Plextronics), and copolymers such as poly(3,4-ethylenedioxythiophene)/poly(4-styrenesulfonate) also called PEDOT/PSS.

### Hole transport layer (c):

According to a preferred embodiment the OLED according to the present invention comprises at least one compound according to general formula (IIb) or (IIc) or their preferred embodiments as a charge transporting material, preferably as a hole transporting layer. In addition to the compounds according to general formulae (lib) and (IIc) or without these compounds either hole-transporting molecules or polymers may be used as the hole transport material. Suitable hole transport materials for layer (c) of the inventive OLED are disclosed, for example, in Kirk-Othmer Encyclopedia of Chemical Technology, 4th Edition, Vol. 18, pages 837 to 860, 1996, US20070278938, US2008/0106190, US2011/0163302 (triarylamines with (di)benzothiophen/(di)benzofuran; Nan-Xing Hu et al. Synth. Met. 111 (2000) 421 (indolocarba-zoles), WO2010002850 (substituted phenylamine compounds) and WO2012/16601 (in particular the hole transport materials mentioned on pages 16 and 17 of WO2012/16601). Combination of different hole transport material may be used. Reference is made, for example, to WO2013/022419, wherein 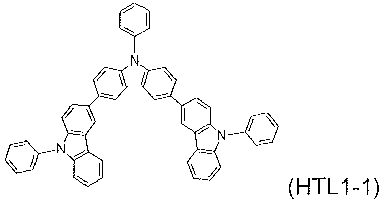 (HTL1-1) and 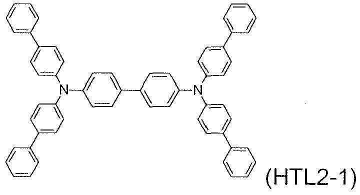 constitute the hole transport layer. Customarily used hole-transporting molecules are selected from the group consisting of 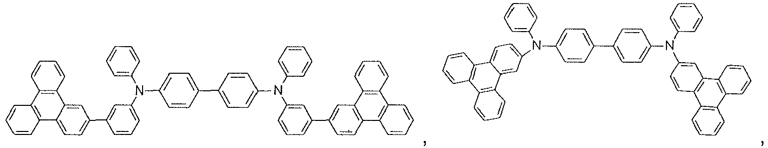 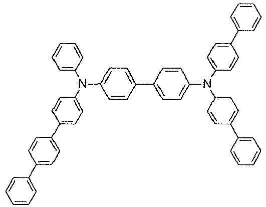 (4-phenyl-N-(4-phenylphenyl)-N-[4-[4-(N-[4-(4-phenyl-phenyl)phenyl]anilino)phenyl]phenyl]aniline), 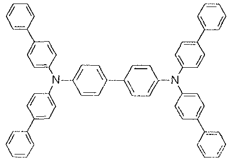 (4-phenyl-N-(4-phenylphenyl)-N-[4-[4-(4-phenyl-N-(4-phenylphenyl)anilino)phenyl]phenyl]aniline), 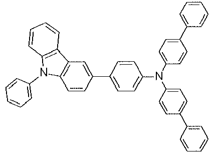 (4-phenyl-N-[4-(9-phenylcarbazol-3-yl)phenyl]-N-(4-phenylphenyl)aniline), 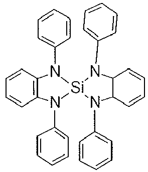 (1,1',3,3'-tetraphenylspiro[1,3,2-benzodiazasilole-2,2'-3a,7a-dihydro-1,3,2-benzodiazasilole]), 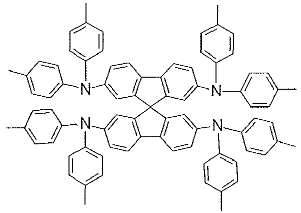 (N2,N2,N2',N2',N7,N7,N7',N7'-octakis(p-tolyl)-9,9'-spirobi[fluorene]-2,2',7,7'-tetramine),4,4'-bis[N-(1-naphthyl)-N-phenylamino]biphenyl (α-NPD), N,N'-diphenyl-N,N'-bis(3-methylphenyl)-[1,1'-biphenyl]-4,4'-diamine (TPD), 1,1-bis[(di-4-tolylamino)phenyl]cyclohexane (TAPC), N,N'-bis(4-methylphenyl)-N,N'-bis(4-ethylphenyl)-[1,1'-(3,3'-dimethyl)biphenyl]-4,4'-diamine (ETPD), tetrakis(3-methylphenyl)-N,N,N',N'-2,5-phenylenediamine (PDA), α-phenyl-4-N,N-diphenylaminostyrene (TPS), p-(diethylamino)benzaldehyde diphenylhydrazone (DEH), triphenylamine (TPA), bis[4-(N,N-diethylamino)2-methylphenyl](4-methylphenyl)methane (MPMP), 1-phenyl-3-[p-(diethylamino)styryl]5-[p-(diethylamino)phenyl]pyrazoline (PPR or DEASP), 1,2-trans-bis(9H-carbazol9-yl)-cyclobutane (DCZB), N,N,N',N'-tetrakis(4-methylphenyl)-(1,1'-biphenyl)-4,4'-diamine (TTB), fluorine compounds such as 2,2',7,7'-tetra(N,N-di-tolyl)amino9,9-spirobifluorene (spiro-TTB), N,N'-bis(naphthalen-1-yl)-N,N'-bis(phenyl)9,9-spirobifluorene (spiro-NPB) and 9,9-bis(4-(N,N-bis-biphenyl-4-yl-amino)phenyl-9Hfluorene, benzidine compounds such as N,N'-bis(naphthalen-1-yl)-N,N'-bis(phenyl)benzidine and porphyrin compounds such as copper phthalocyanines. In addition, polymeric hole-injection materials can be used such as poly(N-vinylcarbazole) (PVK), polythiophenes, polypyrrole, polyaniline, self-doping polymers, such as, for example, sulfonated poly(thiophene-3-[2[(2-methoxyethoxy)ethoxy]-2,5-diyl) (Plexcore® OC Conducting Inks commercially available from Plextronics), and copolymers such as poly(3,4-ethylenedioxythiophene)/poly(4-styrenesulfonate) also called PEDOT/PSS. Preferred examples of a material of the hole injecting layer are a porphyrin compound, an aromatic tertiary amine compound, or a styrylamine compound. Particularly preferable examples include an aromatic tertiary amine compound such as hexacyanohexaazatriphenylene (HAT).

The hole-transporting layer may also be electronically doped in order to improve the transport properties of the materials used, in order firstly to make the layer thicknesses more generous (avoidance of pinholes/short circuits) and in order secondly to minimize the operating voltage of the device. Electronic doping is known to those skilled in the art and is disclosed, for example, in W. Gao, A. Kahn, J. Appl. Phys., Vol. 94, 2003, 359 (p-doped organic layers); A. G. Werner, F. Li, K. Harada, M. Pfeiffer, T. Fritz, K. Leo, Appl. Phys. Lett., Vol. 82, No. 25, 2003, 4495 and Pfeiffer et al., Organic Electronics 2003, 4, 89 - 103 and K. Walzer, B. Maennig, M. Pfeiffer, K. Leo, Chem. Soc. Rev. 2007, 107, 1233. For example it is possible to use mixtures in the hole-transporting layer, in particular mixtures which lead to electrical p-doping of the hole-transporting layer. p-Doping is achieved by the addition of oxidizing materials. These mixtures may, for example, be the following mixtures: mixtures of the abovementioned hole transport materials with at least one metal oxide, for example MoO₂, MoO₃, WOₓ, ReO₃ and/or V₂O₅, preferably MoO₃ and/or ReO₃, more preferably MoO₃, or mixtures comprising the aforementioned hole transport materials and one or more compounds selected from 7,7,8,8-tetracyanoquinodimethane (TCNQ), 2,3,5,6-tetrafluoro-7,7,8,8-tetracyanoquinodimethane (F₄-TCNQ), 2,5-bis(2-hydroxyethoxy)-7,7,8,8-tetracyanoquinodimethane, bis(tetra-n-butylammonium)tetracyanodiphenoquinodimethane, 2,5-dimethyl-7,7,8,8-tetracyanoquinodimethane, tetracyanoethylene, 11,11,12,12-tetracyanonaphtho2,6-quinodimethane, 2-fluoro-7,7,8,8-tetracyanoquino-dimethane, 2,5-difluoro-7,7,8,8etracyanoquinodimethane, dicyanomethylene-1,3,4,5,7,8-hexafluoro-6Hnaphthalen-2-ylidene)malononitrile (F₆-TNAP), Mo(tfd)₃ (from Kahn et al., J. Am. Chem. Soc. 2009, 131 (35), 12530-12531), compounds as described in EP1988587, US2008265216, EP2180029, US20100102709, WO2010132236, EP2180029 and quinone compounds as mentioned in EP2401254.

### Exciton blocking layer (d):

Blocking layers may be used to reduce the number of charge carriers (electrons or holes) and/or excitons that leave the emissive layer. An electron/exciton blocking layer (d) may be disposed between the first emitting layer (e) and the hole transport layer (c), to block electrons from emitting layer (e) in the direction of hole transport layer (c). Blocking layers may also be used to block excitons from diffusing out of the emissive layer.

Suitable metal complexes for use as electron/exciton blocker material are, for example, carbene complexes as described in WO2005/019373A2, WO2006/056418A2, WO2005/113704,, WO2007/115970, WO2007/115981, WO2008/000727 and WO2014147134.

According to a preferred embodiment of the present invention, at least one compound of general formula (IIb) or (IIc) is present in the exciton blocking layer of the OLED according to the present invention.

### Emitting layer (e)

The light emitting layer is an organic layer having a light emitting function and is formed from one or more layers, wherein one of the layers comprises a host material (first host material), optionally a second host material, and the light emitting material as described below.

When the light emitting layer is composed of two or more layers, the light emitting layer or layers other than that mentioned above contains or contain a host material and a dopant material when a doping system is employed. The major function of the host material is to promote the recombination of electrons and holes and confine excitons in the light emitting layer. The dopant material causes the excitons generated by recombination to emit light efficiently.

In case of a phosphorescent device, the major function of the host material is to confine the excitons generated on the dopant in the light emitting layer.

The light emitting layer may be made into a double dopant layer, in which two or more kinds of dopant materials having high quantum yield are used in combination and each dopant material emits light with its own color. For example, to obtain a yellow emission, a light emitting layer formed by co-depositing a host, a red-emitting dopant and a green-emitting dopant is used.

In a laminate of two or more light emitting layers, electrons and holes are accumulated in the interface between the light emitting layers, and therefore, the recombination region is localized in the interface between the light emitting layers, to improve the quantum efficiency.

The light emitting layer may be different in the hole injection ability and the electron injection ability, and also in the hole transporting ability and the electron transporting ability each being expressed by mobility.

The light emitting layer is formed, for example, by a known method, such as a vapor deposition method, a spin coating method, and LB method. Alternatively, the light emitting layer may be formed by making a solution of a binder, such as resin, and the material for the light emitting layer in a solvent into a thin film by a method such as spin coating.

The light emitting layer is preferably a molecular deposit film. The molecular deposit film is a thin film formed by depositing a vaporized material or a film formed by solidifying a material in the state of solution or liquid. The molecular deposit film can be distinguished from a thin film formed by LB method (molecular build-up film) by the differences in the assembly structures and higher order structures and the functional difference due to the structural differences.

The light-emitting layer (e) comprises at least one emitter material. In principle, it may be a fluorescence or phosphorescence emitter, suitable emitter materials being known to those skilled in the art. The at least one emitter material is preferably a phosphorescence emitter.

The emission wavelength of the phosphorescent dopant used in the light emitting layer is not particularly limited. In a preferred embodiment, at least one of the phosphorescent dopants used in the light emitting layer has the peak of emission wavelength of in general 430 nm or longer and 780 nm or shorter, preferably 490 nm or longer and 700 nm or shorter and more preferably 490 nm or longer and 650 nm or shorter. Most preferred are green emitter materials (490 to 570 nm). In another preferred embodiment, red emitter materials (570 to 680 nm) are preferred.

The phosphorescent dopant (phosphorescent emitter material) is a compound which emits light by releasing the energy of excited triplet state and preferably a organometallic complex comprising at least one metal selected from Ir, Pt, Pd, Os, Au, Cu, Re, Rh and Ru and a ligand, although not particularly limited thereto as long as emitting light by releasing the energy of excited triplet state. A ligand having an ortho metal bond is preferred. In view of obtaining a high phosphorescent quantum yield and further improving the external quantum efficiency of electroluminescence device, a metal complex comprising a metal selected from Ir, Os, and Pt is preferred, with iridium complex, osmium complex, and platinum, particularly an ortho metallated complex thereof being more preferred, iridium complex and platinum complex being still more preferred, and an ortho metallated iridium complex being particularly preferred.

According to a particularly preferred embodiment of the present invention, the compounds according to general formulae (IIb) and (IIc) can be used as the matrix (=host material) in the light-emitting layer.

Suitable metal complexes for use in the inventive OLEDs, preferably as emitter material, are described, for example, in documents WO 02/60910 A1, US 2001/0015432 A1, US 2001/0019782 A1, US 2002/0055014 A1, US 2002/0024293 A1, US 2002/0048689 A1, EP 1 191 612 A2, EP 1 191 613 A2, EP 1 211 257 A2, US 2002/0094453 A1, WO 02/02714 A2, WO 00/70655 A2, WO 01/41512 A1, WO 02/15645 A1, WO 2005/019373 A2, WO 2005/113704 A2, WO 2006/115301 A1, WO 2006/067074 A1, WO 2006/056418, WO 2006121811 A1, WO 2007095118 A2, WO 2007/115970, WO 2007/115981, WO 2008/000727, WO2010129323, WO2010056669, WO10086089, US2011/0057559, WO2011/106344, US2011/0233528, WO2012/048266 and WO2012/172482.

Further suitable metal complexes are the commercially available metal complexes tris(2-phenylpyridine)iridium(III), iridium(III) tris(2-(4-tolyl)pyridinato-N,C²'), bis(2-phenylpyridine)(acetylacetonato)iridium(lll), iridium(III) tris(1-phenylisoquinoline), iridium(IlI) bis(2,2'-benzothienyl)pyridinato-N,C³')(acetylacetonate), tris(2-phenylquinoline)iridium(III), iridium(III) bis(2-(4,6-difluorophenyl)pyridinato-N,C²)picolinate, iridium(III) bis(1-phenylisoquinoline)(acetylacetonate), bis(2-phenylquinoline)(acetylacetonato)iridium(III), iridium(III) bis(di-benzo[f,h]quinoxaline)(acetylacetonate), iridium(III) bis(2-methyldibenzo[f,h]quinoxaline)(acetylacetonate) and tris(3-methyl-1-phenyl-4-trimethylacetyl-5-pyrazolino)terbium(III), bis[1-(9,9-dimethyl-9H-fluoren-2-yl)isoquinoline](acetylacetonato)iridium(III), bis(2-phenylbenzothiazolato)(acetylacetonato)iridium(III), bis(2-(9,9-dihexylfluorenyl)-1-pyridine)(acetylacetonato)iridium(III), bis(2-benzo[b]thiophen-2-yl-pyridine)(acetylacetonato)iridium(III).

In addition, the following commercially available materials are suitable:
tris(dibenzoylacetonato)mono(phenanthroline)europium(III), tris(dibenzoylmethane)-mono(phenanthroline)europium(III), tris(dibenzoylmethane)mono(5-aminophenanthroline)-europium(III), tris(di-2-naphthoylmethane)mono(phenanthroline)europium(III), tris(4-bromobenzoylmethane)mono(phenanthroline)europium(III), tris(di(biphenyl)methane)-mono(phenanthroline)europium(III), tris(dibenzoylmethane)mono(4,7-diphenylphenanthroline)europium(III), tris(dibenzoylmethane)mono(4,7-di-methylphenanthroline)europium(III), tris(dibenzoylmethane)mono(4,7-dimethylphenanthrolinedisulfonic acid)europium(III) disodium salt, tris[di(4-(2-(2-ethoxyethoxy)ethoxy)benzoy[methane)lmono-(phenanthroline)europium(III) and tris[di[4-(2-(2-ethoxyethoxy)ethoxy)benzoylmethane)]mono(5-aminophenanthroline)europium(III), osmium(II) bis(3-(trifluoromethyl)-5-(4-tert-butylpyridyl)-1,2,4-triazolato)diphenylmethylphosphine, osmium(II) bis(3-(trifluoromethyl)-5-(2-pyridyl)-1,2,4-triazole)dimethylphenylphosphine, osmium(II) bis(3-(trifluoromethyl)-5-(4-tert-butylpyridyl)-1,2,4-triazolato)dimethylphenylphosphine, osmium(II) bis(3-(trifluoromethyl)-5-(2-pyridyl)-pyrazolato)dimethylphenylphosphine, tris[4,4'-di-tert-butyl(2,2')-bipyridine]ruthenium(III), osmium(II) bis(2-(9,9-dibutylfluorenyl)-1-isoquinoline(acetylacetonate).

Particularly suitable metal complexes are described in US2014048784, US2012223295, US2014367667, US2013234119, US2014001446, US2014231794, US2014008633, WO2012108388 and WO2012108389. The emitters mentioned in US2013234119, paragraph [0222], are exemplified. Selected emitters, especially red emitters, of said emitters mentioned in US2013234119, paragraph [0222], are:

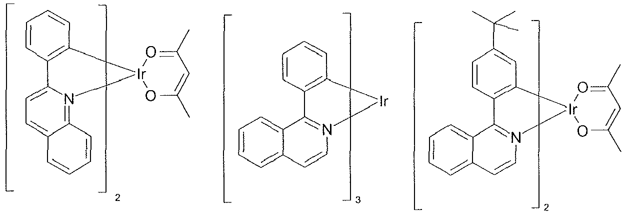

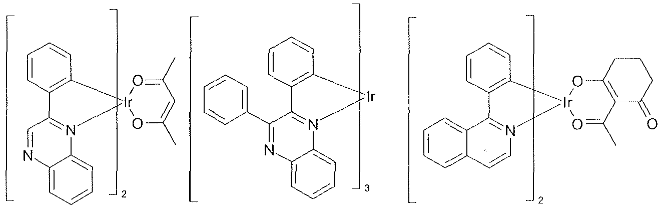

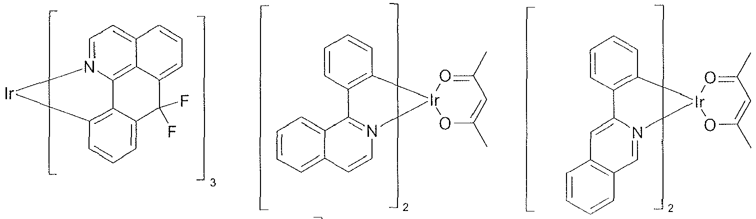

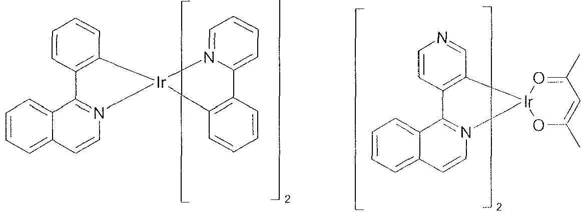

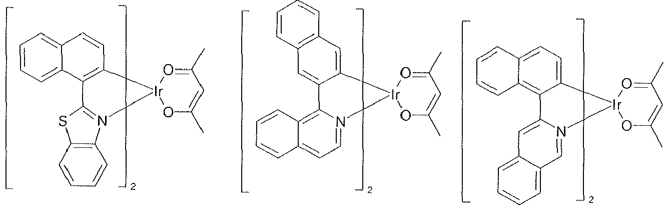

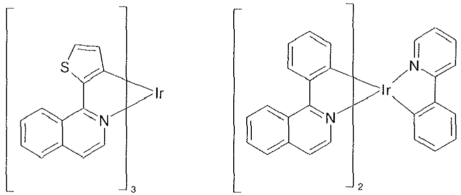

Further suitable Emitters are mentioned in: Mrs Bulletin, 2007, 32, 694:

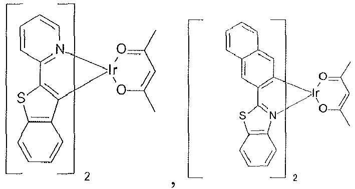

Further suitable Emitters are mentioned in: WO2009100991:

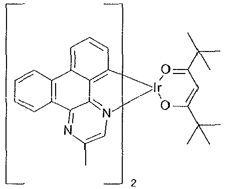

Further suitable Emitters are mentioned in: WO2008101842:

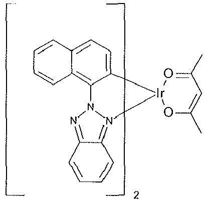

Further suitable Emitters are mentioned in: US 20140048784, especially in paragraph [0159]:

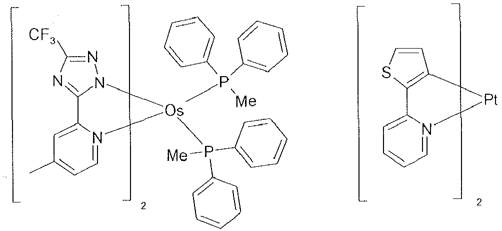

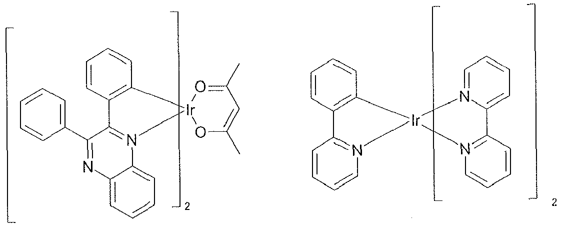

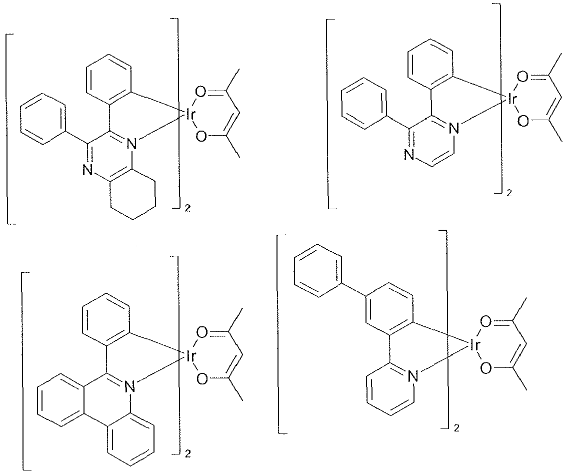

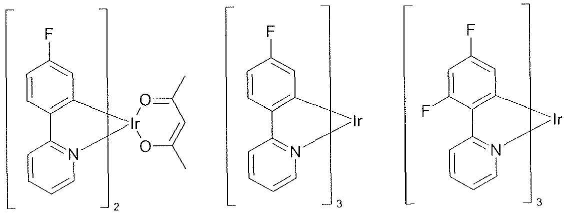

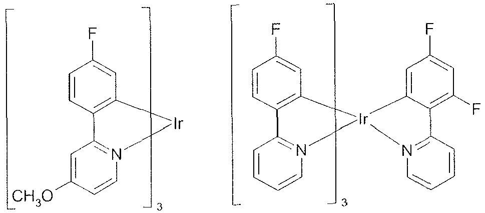

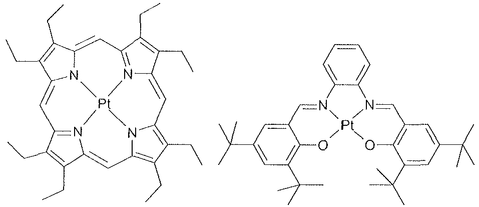

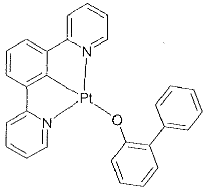

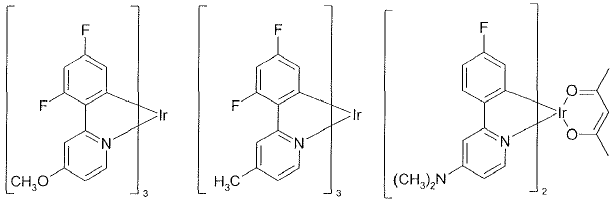

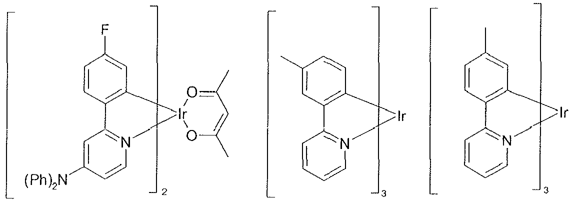

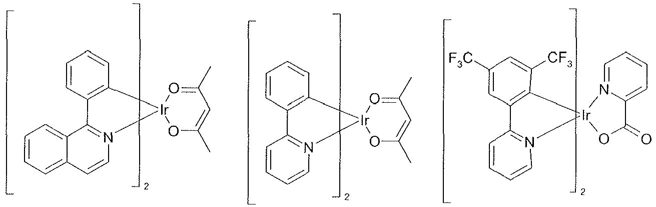

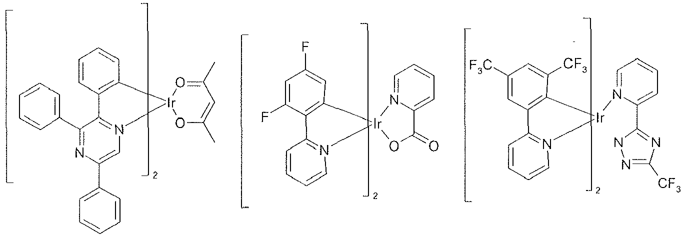

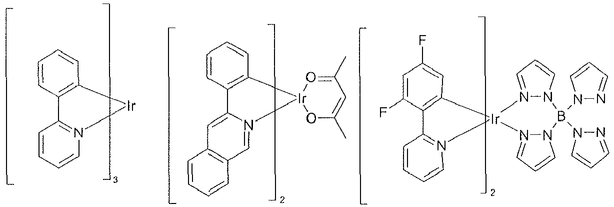

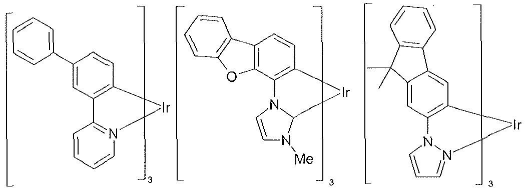

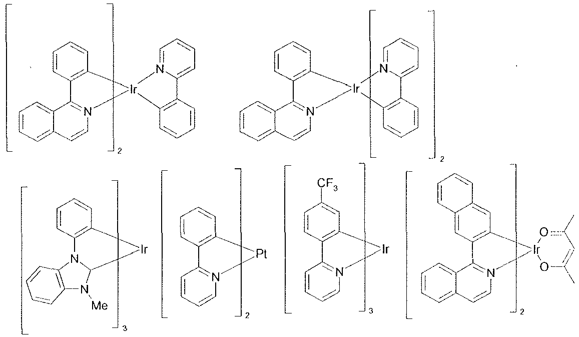

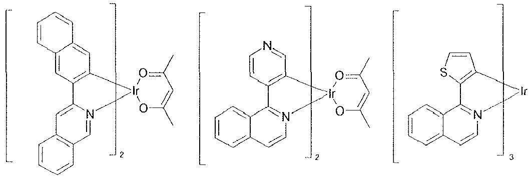

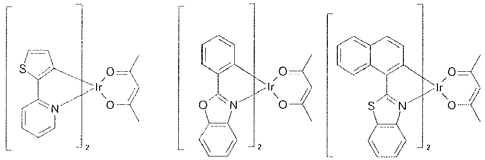

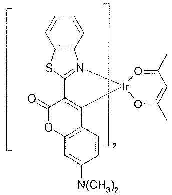

Further suitable red emitters are shown in WO 2008/109824. Preferred red emitters according to this document are the following compounds:

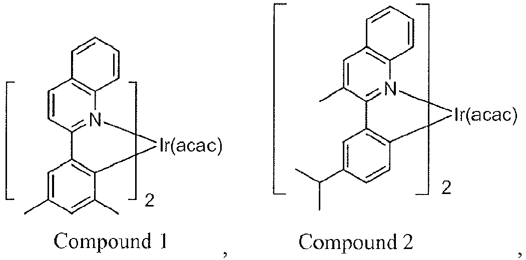

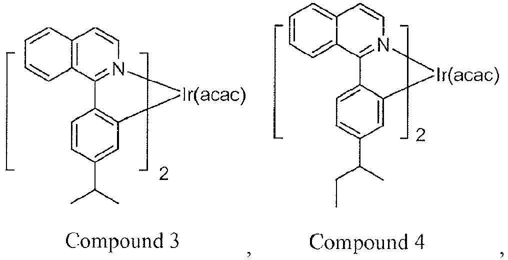

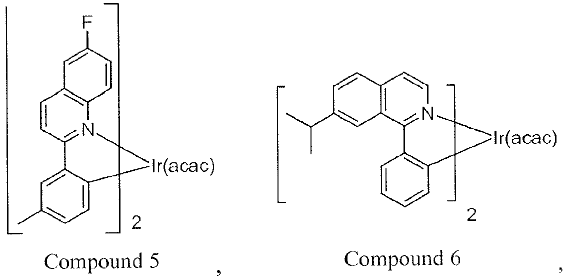

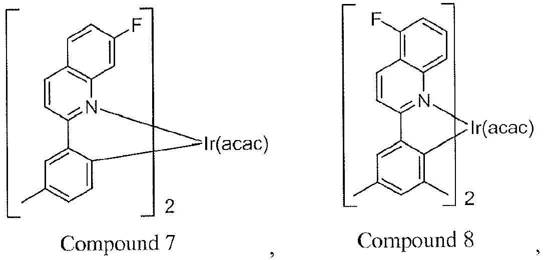

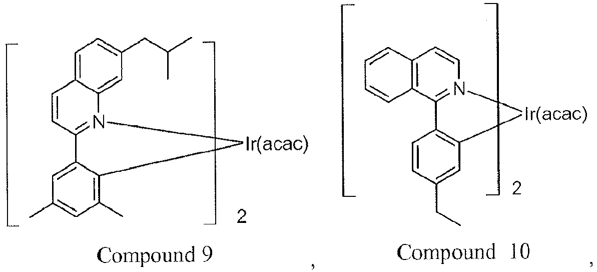

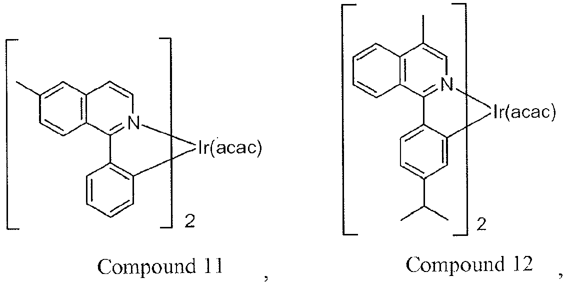

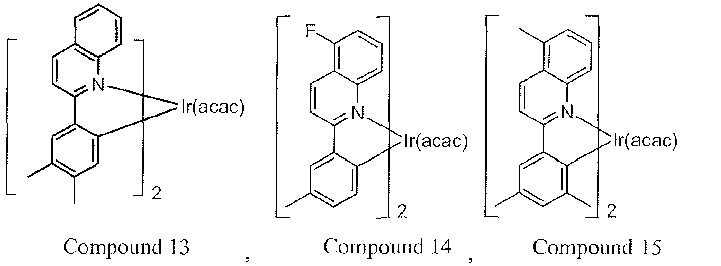

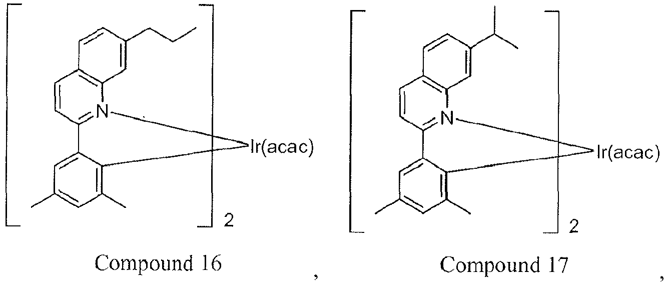

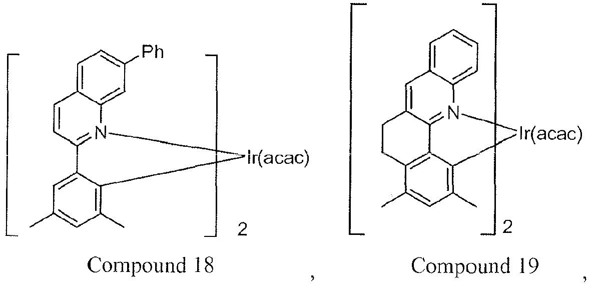

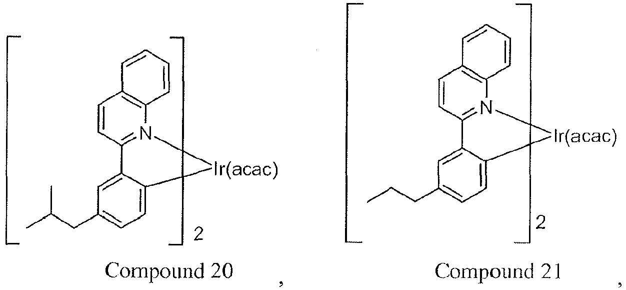

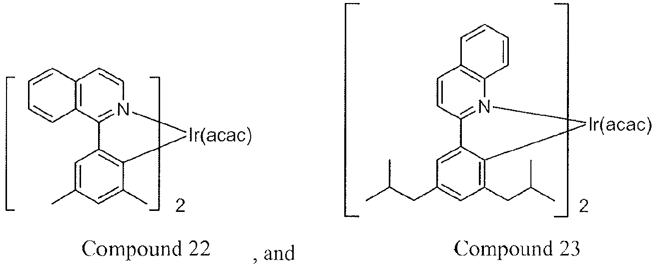

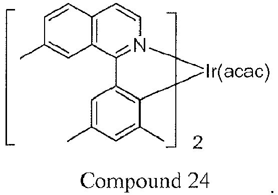

The emitter materials (dopants), preferably the phosphorescent emitter materials, may be used alone or in combination of two or more.

Further red emitters that may be used in the OLEDs according to the present invention are disclosed in US 2013/0299795 and are shown in the following:

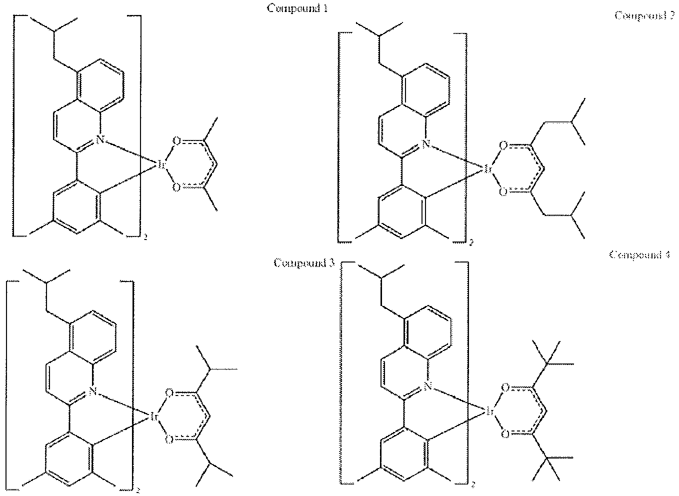

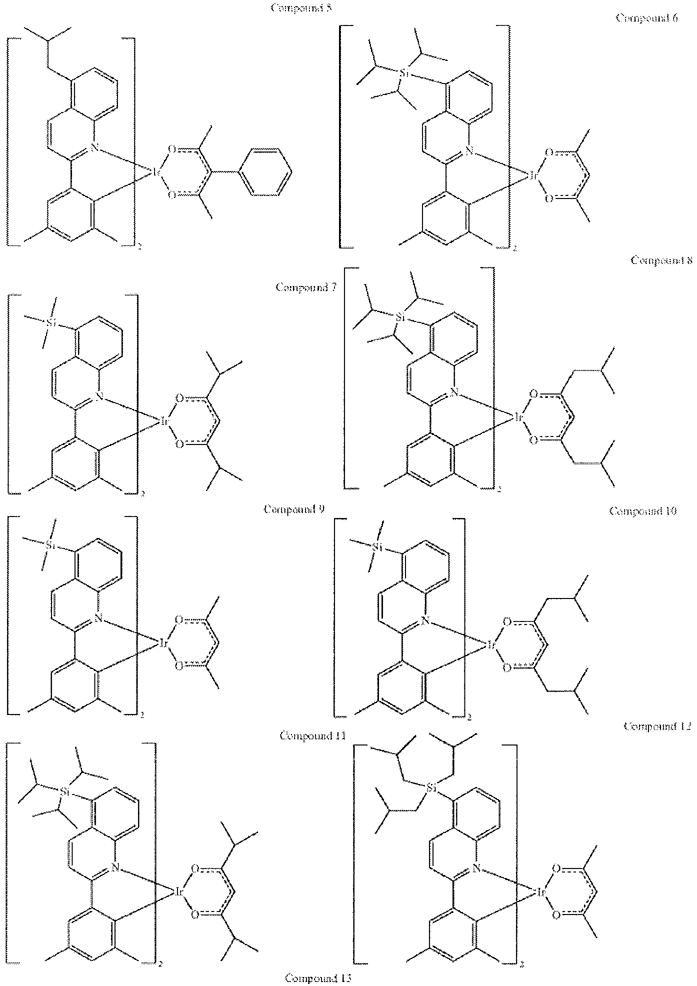

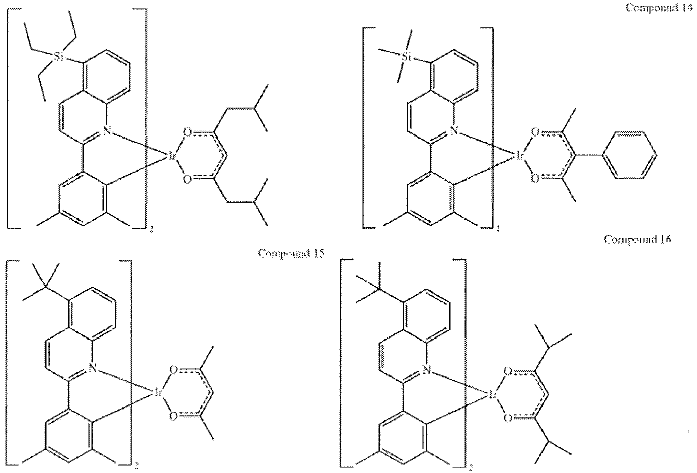

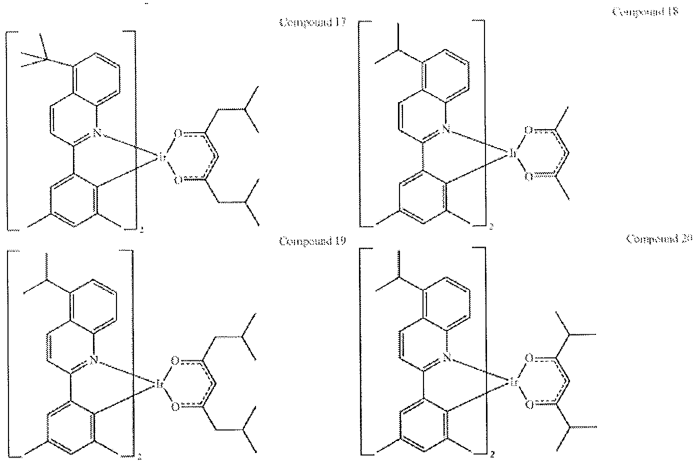

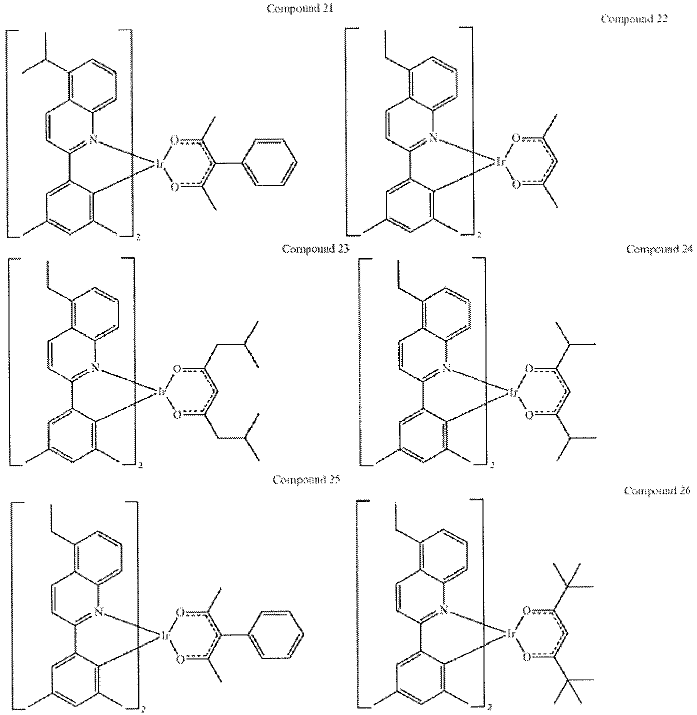

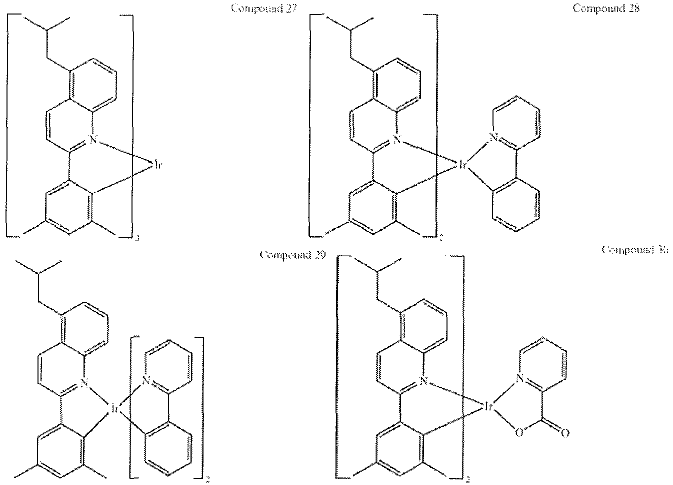

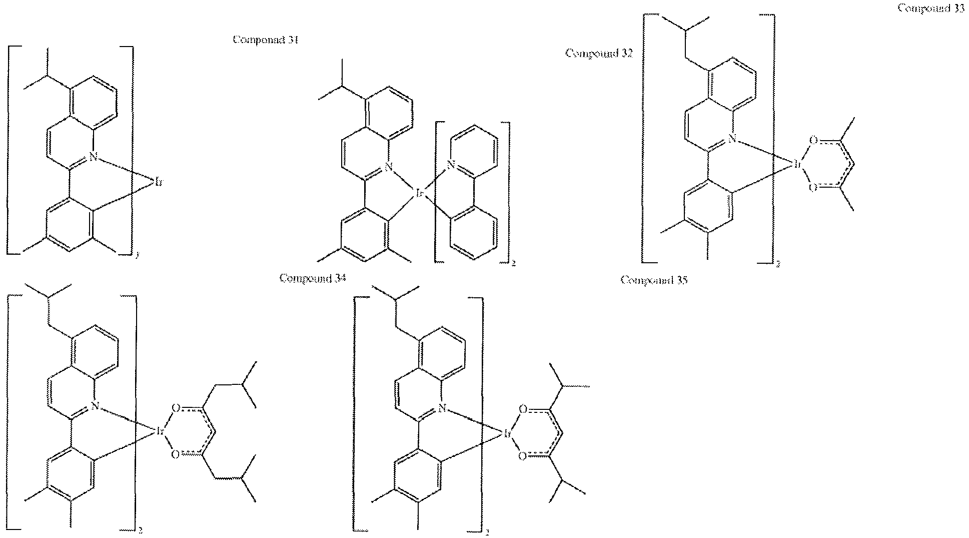

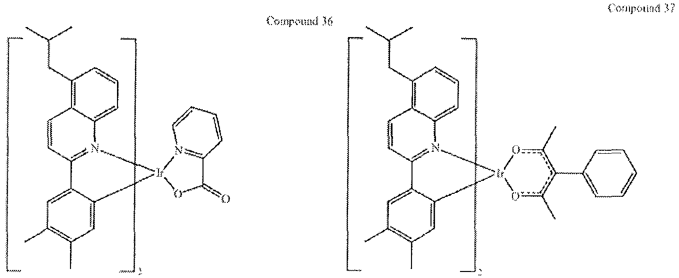

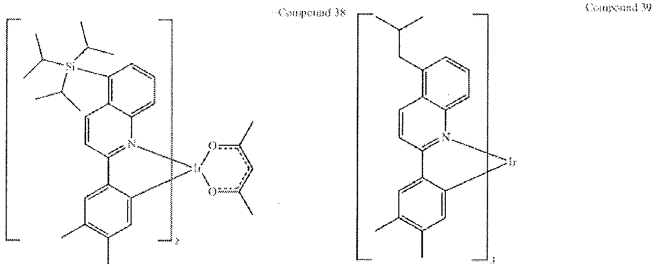

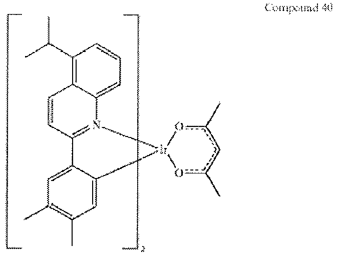

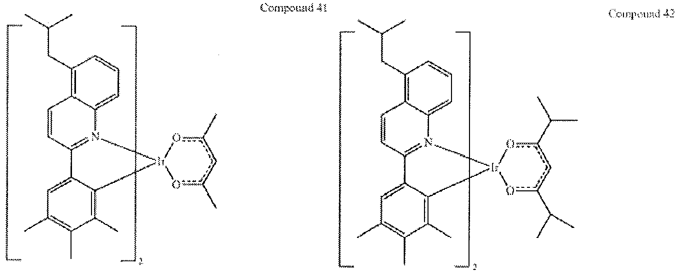

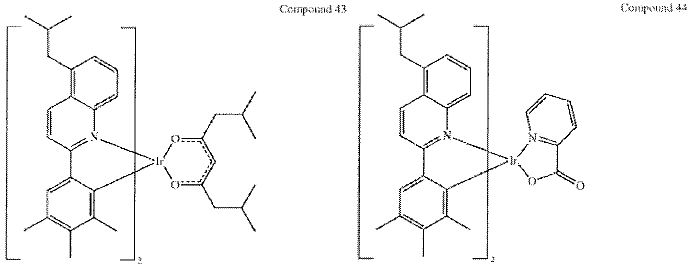

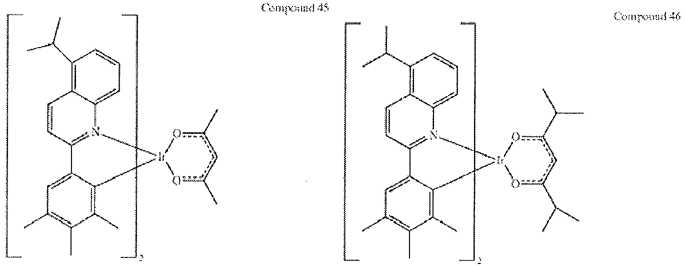

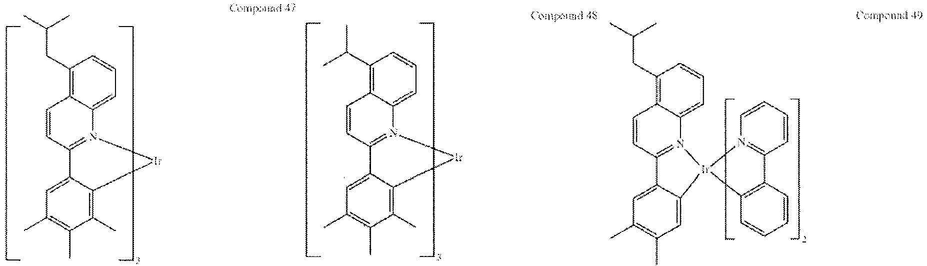

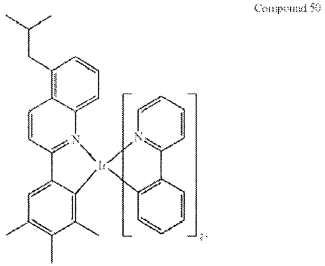

Further red emitters that may be used in the OLEDs according to the present invention are disclosed in US 2013/0146848 and are shown in the following:

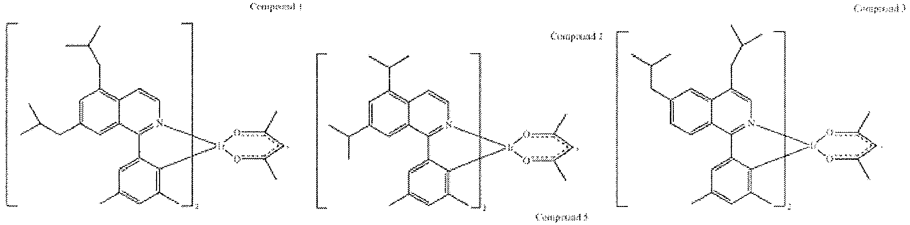

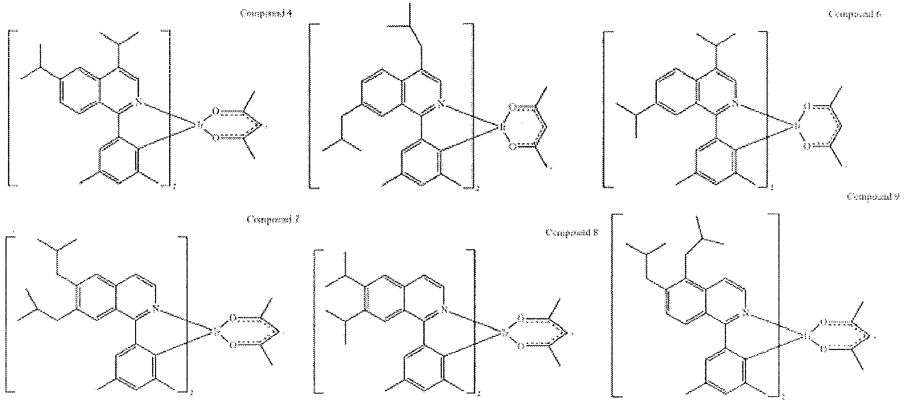

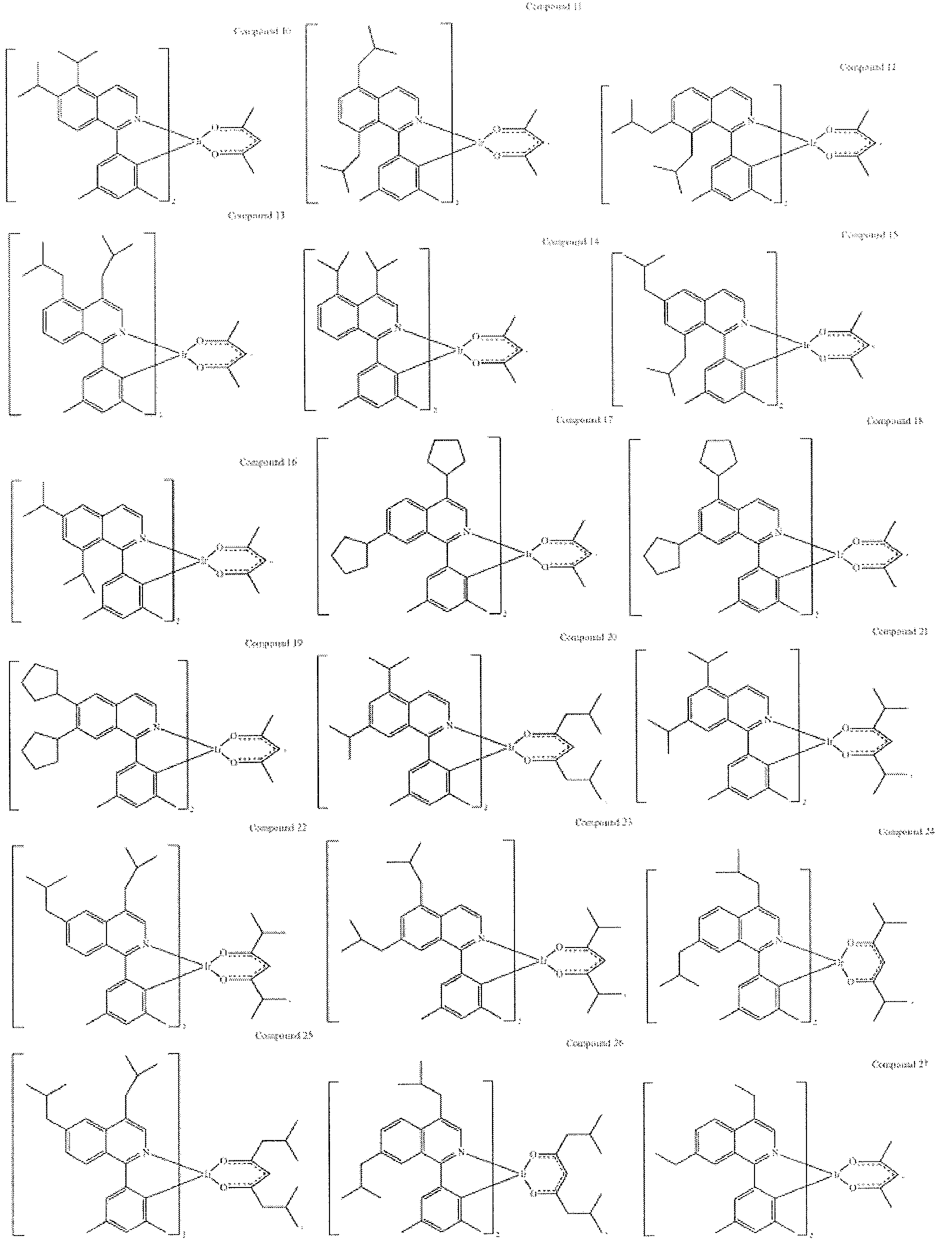

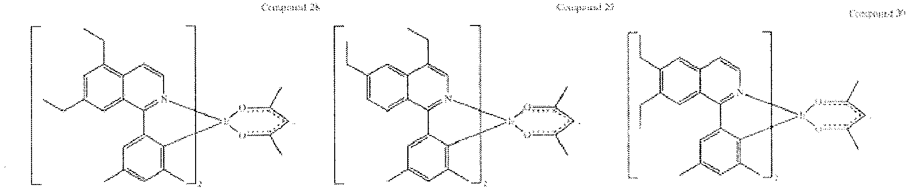

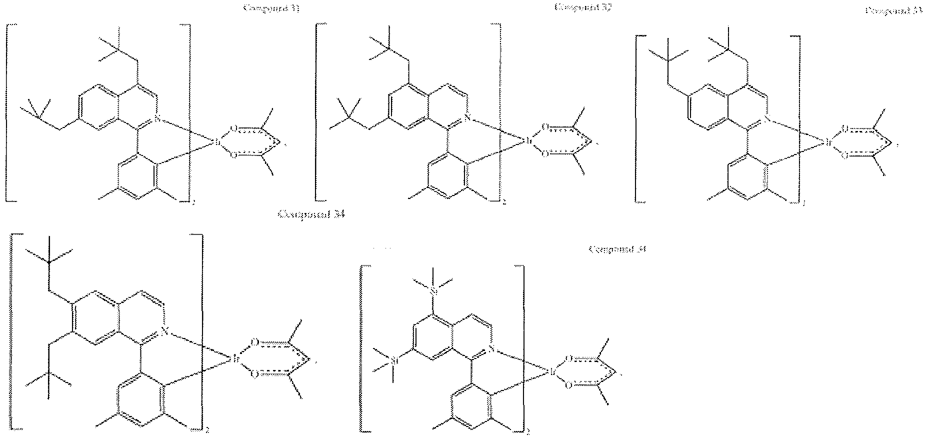

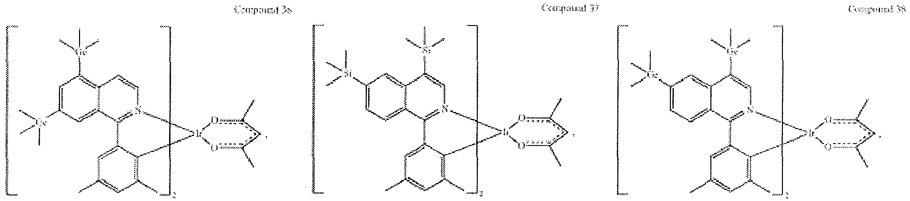

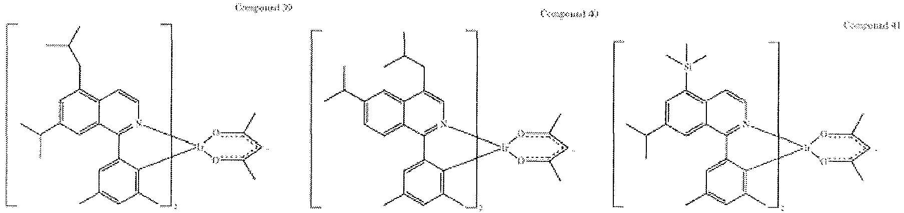

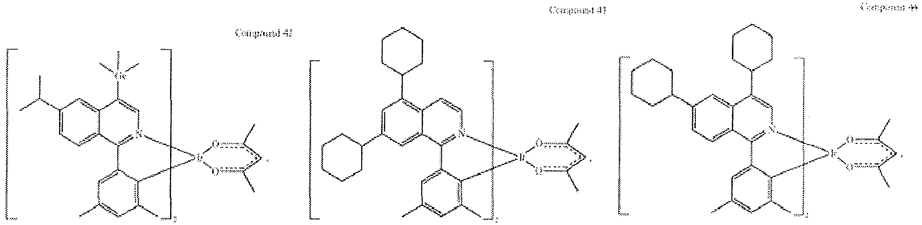

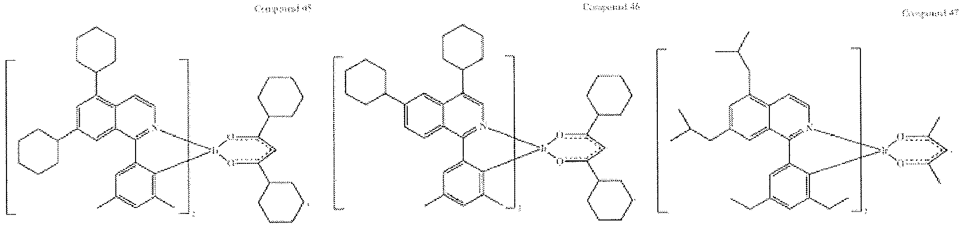

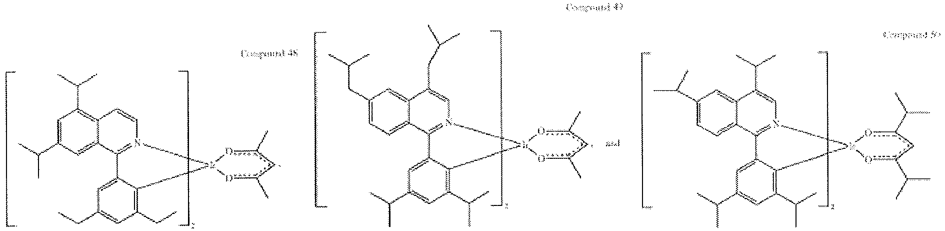

Further suitable red emitters are shown in US 2015/0001472. Preferred red emitters according to this document are the following compounds:

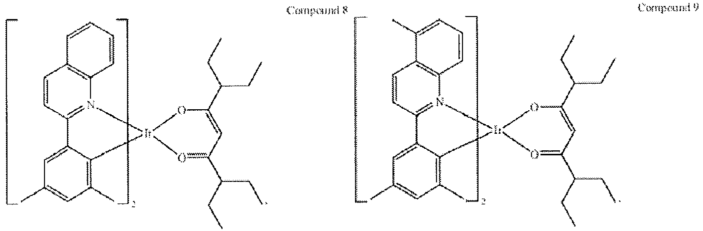

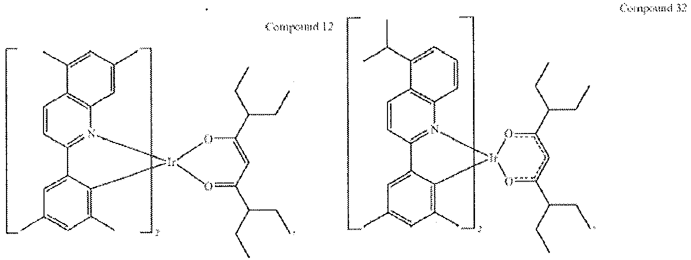

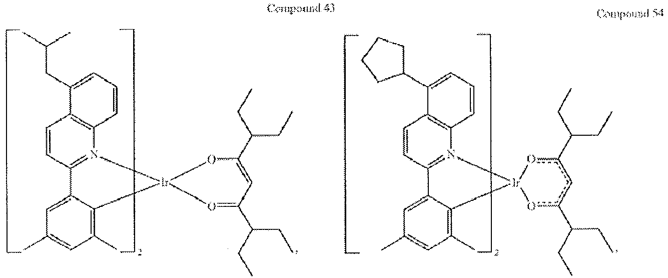

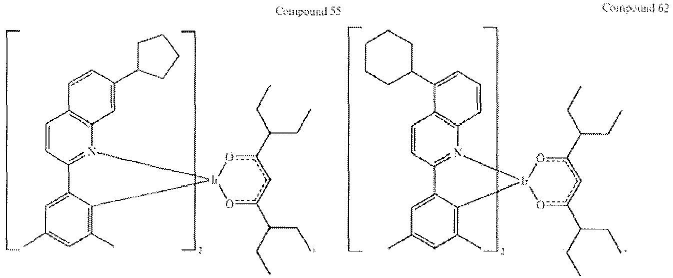

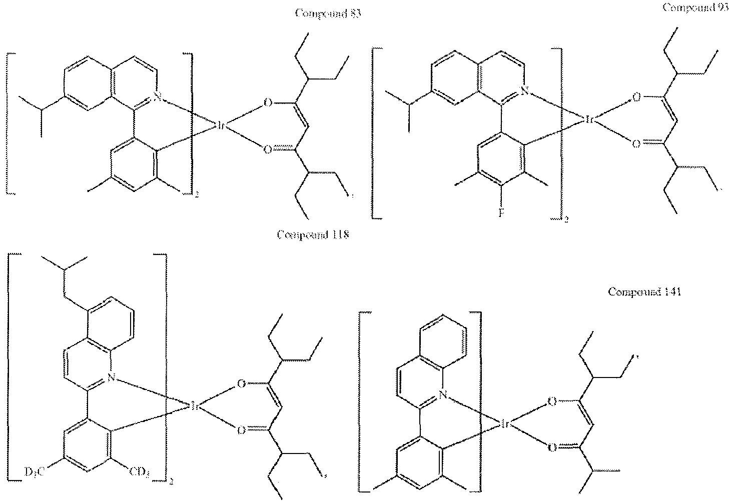

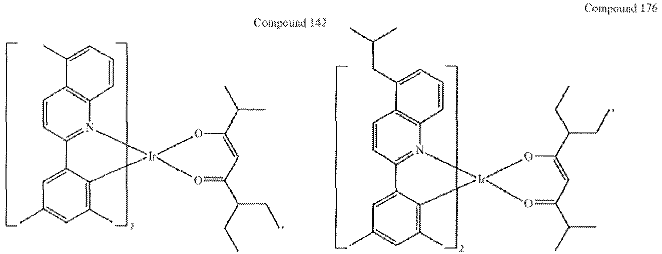

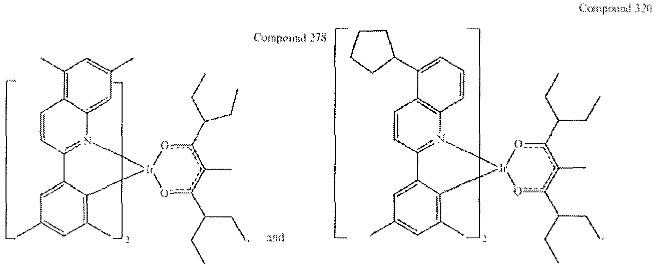

According to a further embodiment of the OLED according to the present invention, the light emitting layer may comprise at least one fluorescent, preferably blue, emitter. Examples of preferred blue dopants that may be present in the light emitting layer of the OLED according to the present invention are polycyclic amine derivatives as mentioned in EP 2924029. Particularly preferred aromatic amine derivatives are selected from compounds according to the following formula (20):

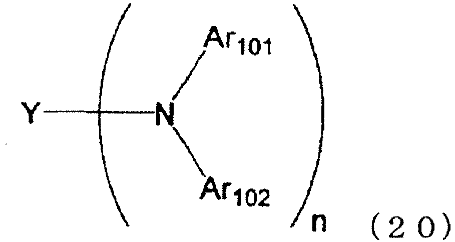

In the formula (20), Y is a substituted or unsubstituted fused aromatic hydrocarbon group including 10 to 50 ring carbon atoms.

Ar₁₀₁, and Ar₁₀₂ are independently a substituted or unsubstituted aryl group including 6 to 50 ring carbon atoms or a substituted or unsubstituted heterocyclic ring group including 5 to 50 ring atoms.

Specific examples of Y include the above-mentioned fused aryl group. Y is preferably a substituted or unsubstituted anthryl group, a substituted or unsubstitued pyrenyl group or a substituted or unsubstituted chrysenyl group.

n is an integer of 1 to 4. It is preferred that n be an integer of 1 to 2.

The above-mentioned formula (20) is preferably one represented by the following formulas (21) to (24).

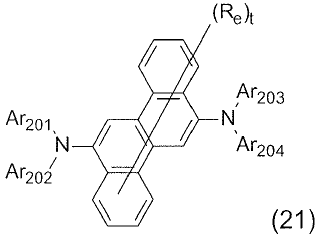

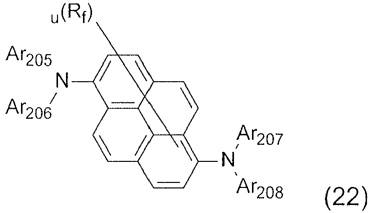

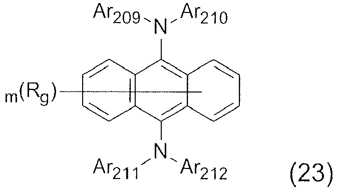

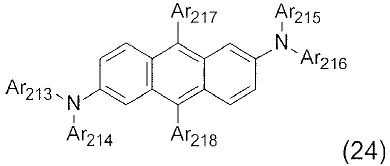

In the formulas (21) to (24), Rₑ, R_{f} and R_{g} are independently a substituted or unsubstituted alkyl group including 1 to 20 carbon atoms, a substituted or unsubstituted alkenyl group including 2 to 50 carbon atoms, a substituted or unsubstituted alkynyl group including 2 to 50 carbon atoms, a substituted or unsubstituted aralykyl group including 1 to 20 carbon atoms, a substituted or unsubstituted cycloalkyl group including 3 to 20 ring carbon atoms, a substituted or unsubstituted alkoxy group including 1 to 20 carbon atoms, a substituted or unsubstituted aryloxy group including 6 to 20 ring carbon atoms, a substituted or unsubstituted aryl group including 6 to 50 ring carbon atoms, a substituted or unsubstituted silyl group, a substituted or unsubstituted alkyl germanium group including 1 to 50 carbon atoms or a substituted or unsubstituted aryl germanium group including 6 to 50 ring carbon atoms. Rₑ, R_{f} and R_{g} may independently be bonded to any of the bonding positions of the benzene rings that constitutes the fused polycyclic skeleton.

As preferable examples of Rₑ, R_{f} and R_{g}, a substituted or unsubstituted aryl group including 6 to 50 ring carbon atoms can be given. More preferably, Rₑ, R_{f} and R_{g} are a substituted or unsubstituted phenyl group, a substituted or unsubstituted naphthyl group, or the like.

t is an integer of 0 to 10. u is an integer of 0 to 8. m is an integer of 0 to 10. Ar₂₀₁ to Ar₂₁₈ are independently an aryl group including 6 to 50 ring carbon atoms or a substituted or unsubstituted heterocyclic group including 5 to 50 ring atoms.

Preferred examples of Ar₂₀₁ to Ar₂₁₈ include a substituted or unsubstituted phenyl group, a substituted or unsubstituted dibenzofuranyl group or the like. As preferable examples of the substituent of Ar₂₀₁ to Ar₂₁₈, an alkyl group, a cyano group and a substituted or unsubstituted silyl group can be given.

In the formulas (21) to (24), as examples of the alkyl group, the alkoxy group, the aryl group, the aryloxy group and the heterocyclic group, those exemplified above can be given.

As the alkenyl group including 2 to 50, preferably 2 to 30, more preferably 2 to 20, and particularly preferably 2 to 10, carbon atoms, a vinyl group, an allyl group, a 1-butenyl group, a 2-butenyl group, a 3-butenyl group, a 1,3-butanedienyl group, a 1-methylvinyl group, a styryl group, a 2,2-diphenylvinyl group, a 1,2-diphenylvinyl group, a 1-methylallyl group, a 1,1-dimethylallyl group, a 2-methylallyl group, a 1-phenylallyl group, a 2-phenylallyl group, a 3-phenylallyl group, a 3,3-diphenylallyl group, a 1,2 - dimethylallyl group, a 1-phenyl-1-butenyl group, a 3-phenyl-1-butenyl group or the like can be given. Preferred are a styryl group, a 2,2-diphenylvinyl group, a 1,2-diphenylvinyl group or the like.

As the alkynyl group including 2 to 50 (preferably 2 to 30, more preferably 2 to 20, particularly preferably 2 to 10) carbon atoms, a propargyl group, a 3-pentynyl group or the like can be given.

As the alkyl germanium group, a methylhydrogermyl group, a trimethylgermyl group, a triethylgermyl group, a tripropylgermyl group, a dimethyl-t-butylgermyl group or the like can be given.

As the aryl germanium group, a phenyldihydrogermyl group, a diphenylhydrogermyl group, a triphenylgermyl group, a tritolylgermyl group, a trinaphthylgermyl group or the like can be given.

As the styrylamine compound and the styryldiamine compound, those represented by the following formulas (17) and (18) are preferable.

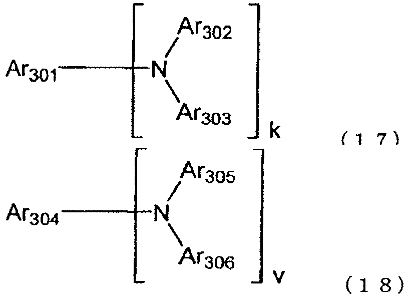

In the formula (17), Ar₃₀₁ is a k-valent group; a k-valent group corresponding to a phenyl group, a naphthyl group, a biphenyl group, a terphenyl group, a stilbene group, a styrylaryl group and a distyrylaryl group. Ar₃₀₂ and Ar₃₀₃ are independently an aryl group including 6 to 20 ring carbon atoms, and Ar₃₀₁, Ar₃₀₂ and Ar₃₀₃ may be substituted.

k is an integer of 1 to 4, with an integer of 1 and 2 being preferable. Any one of Ar₃₀₁ to Ar₃₀₃ is a group including a styryl group. It is further preferred that at least one of Ar₃₀₂ and Ar₃₀₃ be substituted by a styryl group.

As for the aryl group including 6 to 20 ring carbon atoms, the above-mentioned aryl group can be specifically given. Preferable examples include a phenyl group, a naphthyl group, an anthranyl group, a phenanthryl group, a terphenyl group or the like.

In the formula (18), Ar₃₀₄ to Ar₃₀₆ are a v-valent substituted or unsubstituted aryl group including 6 to 40 ring carbon atoms. v is an integer of 1 to 4, with an integer of 1 and 2 being preferable.

Here, as the aryl group including 6 to 40 ring carbon atoms in the formula (18), the above-mentioned aryl group can be specifically given. A naphthyl group, an anthranyl group, a chrysenyl group, a pyrenyl group or an aryl group represented by the formula (20) is preferable.

As preferable substituents that substitute on the aryl group, an alkyl group including 1 to 6 carbon atoms, an alkoxy group including 1 to 6 carbon atoms, an aryl group including 6 to 40 ring carbon atoms, an amino group substituted by an aryl group including 6 to 40 ring carbon atoms, an ester group including an aryl group that includes 5 to 40 ring carbon atoms, an ester group including an alkyl group that includes 1 to 6 carbon atoms, a cyano group, a nitro group, a halogen atom or the like can be given.

The content of the emitter materials (dopants), preferably the phosphorescent emitter materials, in the light emitting layer is not particularly limited and selected according to the use of the device, and preferably 0.1 to 70% by mass, and more preferably 1 to 30% by mass. If being 0.1% by mass or more, the amount of light emission is sufficient. If being 70% by mass or less, the concentration quenching can be avoided. The further component in the emitting layer is usually one or more host material, which is preferably present in an amount of 30 to 99.9 % by mass, more preferably 70 to 99% by mass, wherein the sum of the emitter material(s) and the host material(s) is 100% by mass.

Further possible fluorescent blue emitters that may be present in the emitting layer of the OLED according to the present invention are mentioned in US2012112169.

### Host (matrix) materials

The light-emitting layer may comprise further components in addition to the emitter material. For example, a fluroescent dye may be present in the light-emitting layer in order to alter the emission color of the emitter material. In addition - in a preferred embodiment - a matrix material can be used. This matrix material may be a polymer, for example poly(N-vinylcarbazole) or polysilane. The matrix material may, however, be a small molecule, for example 4,4'-N,N'-dicarbazolebiphenyl (CDP=CBP) or tertiary aromatic amines, for example TCTA.

In the case that one or more phosphorescent emitter materials are used in the light emitting layer, one or more phosphorescent hosts are employed as host material. The phosphorescent host is a compound which confines the triplet energy of the phosphorescent dopant efficiently in the light emitting layer to cause the phosphorescent dopant to emit light efficiently.

In a preferred embodiment, the light-emitting layer is formed of at least one emitter material and of at least one of the matrix materials mentioned in this application. According to a preferred embodiment, the electronic device according to the present invention, preferably the OLED according to the present invention, comprises at least one compound according to general formula (IIb) or (IIc) as matrix (host) material.

According to one embodiment, the light-emitting layer comprises at least one emitter material and at least two matrix materials, wherein one of the matrix materials is a compound according to general formula (IIb) or (IIc) and the other matrix material(s) is/are used as co-host(s). Suitable other host materials than the compounds of general formulae (lib) and (IIc) (co-hosts) are mentioned below. This embodiment is preferably realized with emitter materials that emit red light.

According to another embodiment, the light-emitting layer comprises at least one emitter material and a compound according to general formula (lib) or (IIc) as a single matrix material. Examples of preferred compounds of general formulae (IIb) and (IIc) useful as single host material are shown above. This embodiment is preferably realized with emitter materials that emit red light.

The compounds according to general formulae (IIb) and (IIc) are suitable as single host material as well as host material, together with one or more further host materials (co-host). Suitable further host materials are mentioned below. "Further host materials" means in the sense of the present application, host materials different from the compounds of general formulae (IIb) and (IIc). However, it is also possible to use two or more different compounds of general formulae (lib) and (IIc) as host material in the light-emitting layer in an OLED of the present application.

In a more preferred embodiment, the light-emitting layer is formed from 0.1 to 70% by weight, preferably 1 to 30% by weight, of at least one of the aforementioned emitter materials and 30 to 99.9% by weight, preferably 70 to 99% by weight, of at least one of the matrix materials mentioned in the specification - in one embodiment at least one compound according to general formula (lib) or (IIc) - where the sum total of the emitter material and of the matrix material adds up to 100% by weight.

In a further more preferred embodiment, the light-emitting layer comprises a compound of general formula (lib) or (IIc) as matrix material, at least one further matrix material (co-host) and at least one emitter material. In said embodiment, the light-emitting layer is formed from 0.1 to 70% by weight, preferably 1 to 30% by weight, of the at least one emitter material and 30 to 99.9% by weight, preferably 70 to 99% by weight, of a compound according to general formula (IIb) or (IIc) and the further matrix material, where the sum total of the at least one emitter material, the further matrix material and of the compound of general formula (IIb) or (IIc) adds up to 100% by weight.

The content ratio of the compound according to general formula (lib) or (IIc) as first host material and the second matrix material as co-host in the light emitting layer is not particularly limited and may be selected accordingly, and the ratio of first host material: second host material is preferably 1:99 to 99:1, more preferably 10:90 to 90:10, each based on mass.

In the following host materials are mentioned that may be used in the electronic device according to the present invention as single host materials, if the compounds according to the present invention are used as charge transporting material, i.e. as electron transporting material or hole transporting material. The host materials that are mentioned in the following can also be used as second host materials, if the compounds according to general formula (IIb) or (IIc) are used as first host material and vice versa.

Further suitable host materials, which may be small molecules or (co)polymers of the small molecules mentioned, are specified in the following publications: WO2007108459 (H-1 to H-37), preferably H-20 to H-22 and H-32 to H-37, most preferably H-20, H-32, H-36, H-37, WO2008035571 A1 (Host 1 to Host 6), JP2010135467 (compounds 1 to 46 and Host-1 to Host-39 and Host-43), WO2009008100 compounds No.1 to No.67, preferably No.3, No.4, No.7 to No. 12, No.55, No.59, No. 63 to No.67, more preferably No. 4, No. 8 to No. 12, No. 55, No. 59, No.64, No.65, and No. 67, WO2009008099 compounds No. 1 to No. 110, WO2008140114 compounds 1-1 to 1-50, WO2008090912 compounds OC-7 to OC-36 and the polymers of Mo-42 to Mo-51, JP2008084913 H-1 to H-70, WO2007077810 compounds 1 to 44, preferably 1, 2, 4-6, 8, 19-22, 26, 28-30, 32, 36, 39-44, WO201001830 the polymers of monomers 1-1 to 1-9, preferably of 1-3, 1-7, and 1-9, WO2008029729 the (polymers of) compounds 1-1 to 1-36, WO20100443342 HS-1 to HS-101 and BH-1 to BH-17, preferably BH-1 to BH-17, JP2009182298 the (co)polymers based on the monomers 1 to 75, JP2009170764, JP2009135183 the (co)polymers based on the monomers 1-14, WO2009063757 preferably the (co)polymers based on the monomers 1-1 to 1-26, WO2008146838 the compounds a-1 to a-43 and 1-1 to 1-46, JP2008207520 the (co)polymers based on the monomers 1-1 to 1-26, JP2008066569 the (co)polymers based on the monomers 1-1 to 1-16, WO2008029652 the (co)polymers based on the monomers 1-1 to 1-52, WO2007114244 the (co)polymers based on the monomers 1-1 to 1-18, JP2010040830 the compounds HA-1 to HA-20, HB-1 to HB-16, HC-1 to HC-23 and the (co)polymers based on the monomers HD-1 to HD-12, JP2009021336, WO2010090077 the compounds 1 to 55, WO2010079678 the compounds H1 to H42, WO2010067746, WO2010044342 the compounds HS-1 to HS-101 and Poly-1 to Poly-4, JP2010114180 the compounds PH-1 to PH-36, US2009284138 the compounds 1 to 111 and H1 to H71, WO2008072596 the compounds 1 to 45, JP2010021336 the compounds H-1 to H-38, preferably H-1, WO2010004877 the compounds H-1 to H-60, JP2009267255 the compounds 1-1 to 1-105, WO2009104488 the compounds 1-1 to 1-38, WO2009086028, US2009153034, US2009134784, WO2009084413 the compounds 2-1 to 2-56, JP2009114369 the compounds 2-1 to 2-40, JP2009114370 the compounds 1 to 67, WO2009060742 the compounds 2-1 to 2-56, WO2009060757 the compounds 1-1 to 1-76, WO2009060780 the compounds 1-1 to 1-70, WO2009060779 the compounds 1-1 to 1-42, WO2008156105 the compounds 1 to 54, JP2009059767 the compounds 1 to 20, JP2008074939 the compounds 1 to 256, JP2008021687 the compounds 1 to 50, WO2007119816 the compounds 1 to 37, WO2010087222 the compounds H-1 to H-31, WO2010095564 the compounds HOST-1 to HOST-61, WO2007108362, WO2009003898, WO2009003919, WO2010040777, US2007224446, WO06128800, WO2012014621, WO2012105310, WO2012/130709 and European patent applications EP12175635.7, EP12185230.5 and EP12191408.9 (in particular page 25 to 29 of EP12191408.9).

The above-mentioned small molecules are more preferred than the above-mentioned (co)polymers of the small molecules.

Further suitable host materials, are described in WO2011137072 (for example, 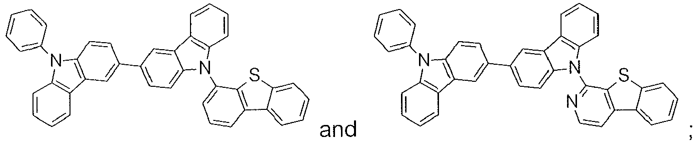 best results are achieved if said compounds are combined with 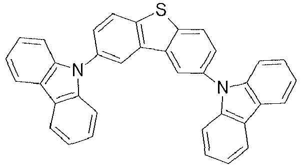 ); WO2012048266 (for example, 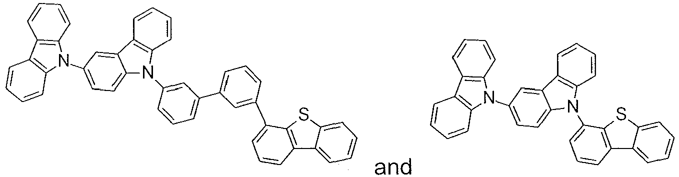 ).

The host materials mentioned above may be used in the OLED of the present invention alone or in combination with the compound of general formula (IIb) or (IIc) as host material. In this case, the compound of general formula (lib) or (IIc) is the host and the host materials mentioned above are the co-hosts.

Further examples of the compounds which are suitable as phosphorescent host, alone or in combination with the compound of general formula (lib) or (IIc) as host material, include a carbazole derivative, a triazole derivative, a oxazole derivative, an oxadiazole derivative, an imidazole derivative, a polyarylalkane derivative, a pyrazoline derivative, a pyrazolone derivative, a phenylenediamine derivative, an arylamine derivative, an amino-substituted chalcone derivative, a styrylanthracene derivative, a fluorenone derivative, a hydrazone derivative, a stilbene derivative, a silazane derivative, an aromatic tertiary amine compound, a styrylamine compound, an aromatic methylidene compound, a porphyrin compound, an anthraquinodimethane derivative, an anthrone derivative, a diphenylquinone derivative, a thiopyran dioxide derivative, a carbodiimide derivative, a fluorenylidenemethane derivative, a distyrylpyrazine derivative, a tetracarboxylic anhydride of fused ring such as naphthalene and perylene, a phthalocyanine derivative, a metal complex of 8-quinolinol derivative, metal phthalocyanine, metal complexes having a ligand such as benzoxazole and benzothiazole, an electroconductive oligomer, such as a polysilane compound, a poly(N-vinylcarbazole) derivative, an aniline copolymer, thiophene oligomer, and a polythiophene, and a polymer such as a polythiophene derivative, a polyphenylene derivative, a polyphenylenevinylene derivative, and a polyfluorene derivative. These phosphorescent hosts may be used alone or in combination of two or more. Specific examples thereof are shown below:

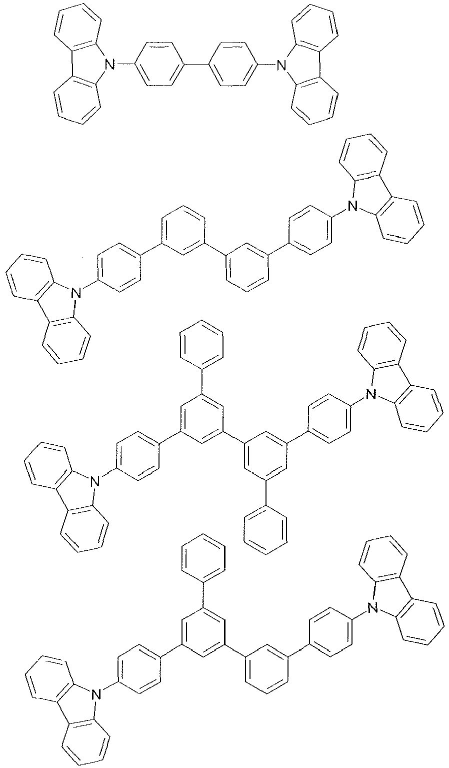

Further suitable hosts, which are especially useful as co-host together with at least one compound of formulae (I), (II) or (III) are the hosts described in US2014048784, US2012223295, US2014367667, US2013234119, US2014001446, US2014231794, US2014008633, WO2012108388, WO2014009317 and WO2012108389, as well as the compounds of formula (1) described in the EP application EP 15187954, filed on October 1, 2015.

Especially preferred are the first and second host materials mentioned in US2013234119, the host materials mentioned in US2014048784 and the compounds of formula (1) described in the EP application EP 15187954, filed on October 1, 2015.

The first host material mentioned in US2013234119 which is preferably used as co-host together with at least one compound of general formula (IIb) or (IIc) in the light emitting layer of an OLED according to the present invention is represented by formula (A). The lifetime of an OLED is increased by using as a first host material at least one compound of general formula (IIb) or (IIc) and as co-host the host material represented by formula (A) in the light emitting layer: 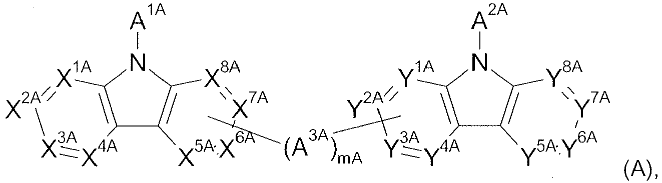 wherein
each of A^{1A} and A^{2A} independently represents an aryl group having 6 to 30 ring carbon atoms, which may be unsubstituted or substituted; or a heterocyclic group having 5 to 30 ring atoms, which may be unsubstituted or substituted;
A^{3A} represents a divalent aryl group having 6 to 30 ring carbon atoms, which may be unsubstituted or substituted; or a divalent heterocyclic group having 5 to 30 ring atoms, which may be unsubstituted or substituted;
mA represents an integer of 0 to 3;
each of X^{1A} to X^{8A} and Y^{1A} to Y^{8A} independently represents N or CR^{a};
each of R^{a} independently represents a hydrogen atom, an aryl group having 6 to 30 ring carbon atoms, which may be unsubstituted or substituted; a heterocyclic group having 5 to 30 ring atoms, which may be unsubstituted or substituted; an alkyl group having 1 to 30 carbon atoms, which may be unsubstituted or substituted for example by E; a silyl group, which may be unsubstituted or substituted; a halogen atom, or a cyano group, provided that when two or more R^{a} groups exist, the R^{a} groups may be the same or different and one of X^{5A} to X^{8A} and one of Y^{1A} to Y^{4A} are bonded to each other via A^{3A;} and
the formula (A) satisfies at least one of the flowing requirements (i) to (v);
(i) at least one of A^{1A} and A^{2A} represents a cyano-substituted aromatic hydrocarbon group having 6 to 30 ring carbon atoms or a cyano-substituted heterocyclic group having 5 to 30 ring atoms;
(ii) at least one of X^{1A} to X^{4A} and Y^{5A} to Y^{8A} represents CR^{a}, and at least one of R^{a} in X^{1A} to X^{4A} and Y^{5A} to Y^{8A} represents a cyano-substituted aromatic hydrocarbon group having 6 to 30 ring carbon atoms or a cyano-substituted heterocyclic group having 5 to 30 ring atoms;
(iii) mA represents an integer of 1 to 3 and at least one of A³ represents a cyano-substituted divalent aromatic hydrocarbon group having 6 to 30 ring carbon atoms or a cyano-substituted divalent heterocyclic group having 5 to 30 ring atoms;
(iv) at least one of X^{5A} to X^{8A} and Y^{1A} to Y^{4A} represents CR^{a}, and at least one of R^{a} in X^{5A} to X^{8A} and Y^{1A} to Y^{4A} represents a cyano-substituted aromatic hydrocarbon group having 6 to 30 ring carbon atoms or a cyano-substituted heterocyclic group having 5 to 30 ring atoms; and
(v) at least one of X^{1A} to X8A and Y^{1A} to Y^{8A} represents C-CN.

The cyano-substituted aromatic hydrocarbon group having 6 to 30 ring carbon atoms and the cyano-substituted heterocyclic group having 5 to 30 ring atoms may be further substituted by a group other than the cyano group.

The subscript mA is preferably 0 to 2 and more preferably 0 or 1. When mA is 0, one of X^{5A} to X^{8A} and one of Y^{1A} to Y^{4A} are bonded to each other via a single bond.

In formula (A), the groups mentioned above have the following meanings:
The aromatic hydrocarbon group having 6 to 30 ring carbon atoms represented by A^{1A}, A^{2A} and R^{a} may be a non-condensed aromatic hydrocarbon group or a condensed aromatic hydrocarbon group. Specific examples thereof include phenyl group, naphthyl group, phenanthryl group, biphenyl group, terphenyl group, quaterphenyl group, fluoranthenyl group, triphenylenyl group, phenanthrenyl group, fluorenyl group, spirofluorenyl group, 9,9-diphenylfluorenyl group, 9,9'-spirobi[9H-fluorene]-2-yl group, 9,9-dimethylfluorenyl group, benzo[c]phenanthrenyl group, benzo[a]triphenylenyl group, naphtho[1,2-c]phenanthrenyl group, naphtho[1,2-a]triphenylenyl group, dibenzo[a,c]triphenylenyl group, and benzo[b]fluoranthenyl group, with phenyl group, naphthyl group, biphenyl group, terphenyl group, phenanthryl group, triphenylenyl group, fluorenyl group, spirobifluorenyl group, and fluoranthenyl group being preferred, and phenyl group, 1-naphthyl group, 2-naphthyl group, biphenyl-2-yl group, biphenyl-3-yl group, biphenyl-4-yl group, phenanthrene-9-yl group, phenanthrene-3-yl group, phenanthrene-2-yl group, triphenylene-2-yl group, 9,9-dimethylfluorene-2-yl group, fluoranthene-3-yl group being more preferred.

Examples of the divalent aromatic hydrocarbon group having 6 to 30 ring carbon atoms represented by A^{3A} include divalent residues of the above aromatic hydrocarbon groups having 6 to 30 ring carbon atoms.

The heterocyclic group having 5 to 30 ring atoms represented by A^{1A}, A^{2A} and R^{a} may be a non-condensed heterocyclic group or a condensed heterocyclic group. Specific examples thereof include the residues of pyrrole ring, isoindole ring, benzofuran ring, isobenzofuran ring, dibenzothiophene ring, isoquinoline ring, quinoxaline ring, phenanthridine ring, phenanthroline ring, pyridine ring, pyrazine ring, pyrimidine ring, pyridazine ring, triazine ring, indole ring, quinoline ring, acridine ring, pyrrolidine ring, dioxane ring, piperidine ring, morpholine ring, piperazine ring, carbazole ring, furan ring, thiophene ring, oxazole ring, oxadiazole ring, benzoxazole ring, thiazole ring, thiadiazole ring, benzothiazole ring, triazole ring, imidazole ring, benzimidazole ring, pyran ring, dibenzofuran ring, and benzo[c]dibenzofuran ring, and the residues of derivatives of these rings, with the residues of dibenzofuran ring, carbazole ring, dibenzothiophene ring, and derivatives of these rings being preferred, and the residues of dibenzofuran-2-yl group, dibenzofuran-4-yl group, 9-phenylcarbazole-3-yl group, 9-phenylcarbazole-2-yl group, dibenzothiophene-2-yl group, and dibenzothiophene-4-yl group being more preferred.

Examples of the divalent heterocyclic group having 5 to 30 ring atoms represented by A^{3A} include divalent residues of the above heterocyclic group having 5 to 30 ring atoms.

Examples of the alkyl group having 1 to 30 carbon atoms represented by R^{a} include methyl group, ethyl group, n-propyl group, isopropyl group, n-butyl group, s-butyl group, isobutyl group, t-butyl group, n-pentyl group, n-hexyl group, n-heptyl group, n-octyl group, n-nonyl group, n-decyl group, n-undecyl group, n-dodecyl group, n-tridecyl group, n-tetradecyl group, n-pentadecyl group, n-hexadecyl group, n-heptadecyl group, n-octadecyl group, neopentyl group, 1-methylpentyl group, cyclopropyl group, cyclobutyl group, cyclopentyl group, cyclohexyl group, cyclooctyl group, and adamantyl group, with methyl group, ethyl group, n-propyl group, isopropyl group, n-butyl group, s-butyl group, isobutyl group, t-butyl group, cyclopentyl group, and cyclohexyl group being preferred.

Examples of the silyl group , which may be unsubstituted or substituted ; represented by R^{a} include trimethylsilyl group, triethylsilyl group, tributylsilyl group, dimethylethylsilyl group, t-butyldimethylsilyl group, vinyldimethylsilyl group, propyldimethylsilyl group, dimethylisopropylsilyl group, dimethylpropylsilyl group, dimethylbutylsilyl group, dimethyltertiarybutylsilyl group, diethylisopropylsilyl group, phenyldimethylsilyl group, diphenylmethylsilyl group, diphenyltertiarybutylsilyl group, and triphenylsilyl group, with trimethylsilyl group, triethylsilyl group, t-butyldimethylsilyl group, vinyldimethylsilyl group, and propyldimethylsilyl group being preferred.

Examples of the halogen atom represented by R^{a} include fluorine, chlorine, bromine, and iodine, with fluorine being preferred.

Also preferred as R^{a} is a hydrogen atom or an aryl group having 6 to 30 ring carbon atoms, which may be unsubstituted or substituted.

Examples of the optional substituent indicated by "substituted or unsubstituted" and "may be substituted" referred to above or hereinafter include a halogen atom (fluorine, chlorine, bromine, iodine), a cyano group, an alkyl group having 1 to 20, preferably 1 to 6 carbon atoms, a cycloalkyl group having 3 to 20, preferably 5 to 12 carbon atoms, an alkoxyl group having 1 to 20, preferably 1 to 5 carbon atoms, a haloalkyl group having 1 to 20, preferably 1 to 5 carbon atoms, a haloalkoxyl group having 1 to 20, preferably 1 to 5 carbon atoms, an alkylsilyl group having 1 to 10, preferably 1 to 5 carbon atoms, an aromatic hydrocarbon group having 6 to 30, preferably 6 to 18 ring carbon atoms, an aryloxy group having 6 to 30, preferably 6 to 18 ring carbon atoms, an arylsilyl group having 6 to 30, preferably 6 to 18 carbon atoms, an aralkyl group having 7 to 30, preferably 7 to 20 carbon atoms, and a heteroaryl group having 5 to 30, preferably 5 to 18 ring atoms.

The optional substituent mentioned above may be further substituted by the optional group mentioned above.

Examples of the optional alkyl group having 1 to 20 carbon atoms include methyl group, ethyl group, n-propyl group, isopropyl group, n-butyl group, s-butyl group, isobutyl group, t-butyl group, n-pentyl group, n-hexyl group, n-heptyl group, n-octyl group, n-nonyl group, n-decyl group, n-undecyl group, n-dodecyl group, n-tridecyl group, n-tetradecyl group, n-pentadecyl group, n-hexadecyl group, n-heptadecyl group, n-octadecyl group, neopentyl group, and 1-methylpentyl group.

Examples of the optional cycloalkyl group having 3 to 20 carbon atoms include cyclopropyl group, cyclobutyl group, cyclopentyl group, cyclohexyl group, cyclooctyl group, and adamantyl group.

Examples of the optional alkoxyl group having 1 to 20 carbon atoms include those having an alkyl portion selected from the alkyl groups mentioned above.

Examples of the optional haloalkyl group having 1 to 20 carbon atoms include the alkyl groups mentioned above wherein the hydrogen atoms thereof are partly or entirely substituted by halogen atoms.

Examples of the optional haloalkoxyl group having 1 to 20 carbon atoms include the alkoxyl group mentioned above wherein the hydrogen atoms thereof are partly or entirely substituted by halogen atoms.

Examples of the optional alkylsilyl group having 1 to 10 carbon atoms include trimethylsilyl group, triethylsilyl group, tributylsilyl group, dimethylethylsilyl group, t-butyldimethylsilyl group, vinyldimethylsilyl group, propyldimethylsilyl group, dimethylisopropylsilyl group, dimethylpropylsilyl group, dimethylbutylsilyl group, dimethyltertiarybutylsilyl group, and diethylisopropylsilyl group.

Examples of the optional aryl group having 6 to 30 ring carbon atoms include those selected from the aryl groups mentioned above with respect to A^{1A}, A^{2A} and R^{a}.

Examples of the optional aryloxy group having 6 to 30 ring carbon atoms include those having an aryl portion selected from the aromatic hydrocarbon groups mentioned above.

Examples of the optional arylsilyl group having 6 to 30 carbon atoms include phenyldimethylsilyl group, diphenylmethylsilyl group, diphenyltertiarybutylsilyl group, and triphenylsilyl group.

Examples of the optional aralkyl group having 7 to 30 carbon atoms include benzyl group, 2-phenylpropane-2-yl group, 1-phenylethyl group, 2-phenylethyl group, 1-phenylisopropyl group, 2-phenylisopropyl group, phenyl-t-butyl group, α-naphthylmethyl group, 1-α-naphthylethyl group, 2-α-naphthylethyl group, 1-α-naphthytisopropyl group, 2-α-naphthylisopropyl group, β-naphthylmethyl group, 1-β-naphthylethyl group, 2-β-naphthylethyl group, 1-β-naphthylisopropyl group, 2-β-naphthylisopropyl group, 1-pyrrolylmethyl group, 2-(1-pyrrolyl)ethyl group, p-methylbenzyl group, m-methylbenzyl group, o-methylbenzyl group, p-chlorobenzyl group, m-chlorobenzyl group, o-chlorobenzyl group, p-bromobenzyl group, m-bromobenzyl group, o-bromobenzyl group, p-iodobenzyl group, m-iodobenzyl group, o-iodobenzyl group, p-hydroxybenzyl group, m-hydroxybenzyl group, o-hydroxybenzyl group, p-aminobenzyl group, m-aminobenzyl group, o-aminobenzyl group, p-nitrobenzyl group, m-nitrobenzyl group, o-nitrobenzyl group, p-cyanobenzyl group, m-cyanobenzyl group, o-cyanobenzyl group, 1-hydroxy-2-phenylisopropyl group, and 1-chloro-2-phenylisopropyl group.

Examples of the optional heteroaryl group having 5 to 30 ring atoms include those selected from the heterocyclic groups mentioned above with respect to A^{1A}, A^{2A} and R^{a}.

The "carbon number of a to b" in the expression of "substituted or unsubstituted X group having carbon number of a to b" is the carbon number of the unsubstituted X group and does not include the carbon atom of the optional substituent.

The hydrogen atom referred to herein includes isotopes different from neutron numbers, i.e., light hydrogen (protium), heavy hydrogen (deuterium) and tritium.

In the host material represented by formula (A), the groups represented by formulae (a) and (b) are bonded to each other via -(A³)_{mA}- at one of X^{5A} to X^{8A} and one of Y^{1A} to Y^{4A}. Specific examples of the bonding manner between formulae (a) and (b) are represented by X^{6A}-(A^{3A})_{mA}-Y^{3A}, X^{6A}-(A^{3A})_{mA}-Y^{2A}, X^{6A}-(A^{3A})_{mA}-Y^{4A}, X^{6A}-(A^{3A})_{mA}-Y^{1A}, X^{7A}-(A^{3A})_{mA}-Y^{3A}, X^{5A}-(A^{3A})_{mA}-Y3A, X^{8A}-(A^{3A})_{mA}-Y3A, X^{7A}-(A^{3A})_{mA}-Y^{2A}, X^{7A}-(A^{3A})_{mA}-Y^{4A}, X^{7A}-(A^{3A})_{mA}-Y^{1A}, X^{5A}-(A^{3A})_{mA}-Y^{2A}, X^{8A}-(A^{3A})_{mA}-Y^{2A}, X^{8A}-(A^{3A})_{mA}-Y^{4A}, X^{8A}-₍A^{3A})_{mA}-Y^{1A}, X^{5A}-(A^{3A})_{mA}-Y^{1A}, and X^{5A}-(A^{3A})_{mA}-Y^{4A}.

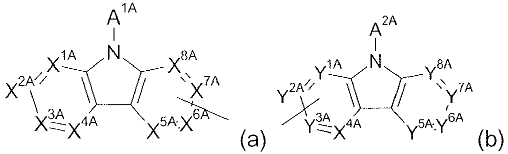

In preferred embodiments of the host material represented by formula (A), the bonding manner between formulae (a) and (b) are represented by X^{6A}-(A^{3A})_{mA}-Y^{3A}, X^{6A}-(A^{3A})_{mA}-Y^{2A}, or X^{7A}-(A^{3A})_{mA}-Y^{3A}, namely the material for organic electroluminescence device is preferably represented by formula (XXII), (XXIII), or (XXIV): 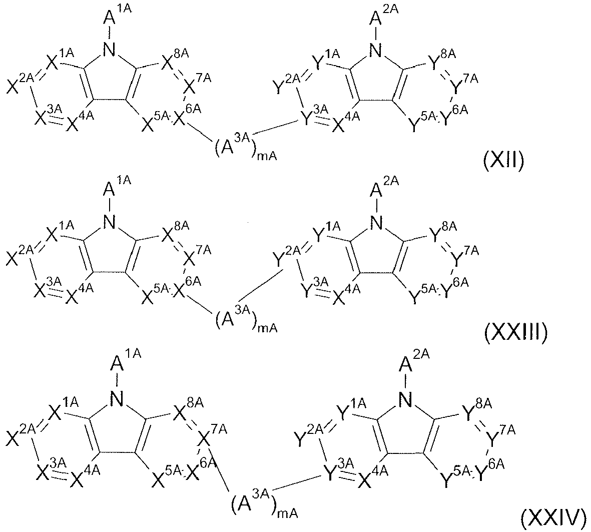 wherein X^{1A} to X^{8A}, Y^{1A} to Y^{8A}, A^{1A} to A^{3A,} and mA are the same as X^{1A} to X^{8A}, Y^{1A} to Y^{8A,} A^{1A} to A^{3A}, mA in formula (A), and each of formulae (XXII), (XXIII), and (XXIV) satisfies at least one of the requirements (i) to (v) as specified in the definition of formula (A).

The host material represented by formula (A) satisfies at least one of the requirements (i) to (v), namely, the host material is a cyano group-introduced biscarbazole derivative having a group represented by formula (a) and a group represented by formula (b) which are linked to each other.

A^{3A} of formula (A) preferably represents a single bond, a substituted or unsubstituted divalent monocyclic hydrocarbon group having 6 or less ring carbon atoms, or a substituted or unsubstituted divalent monocyclic heterocyclic group having 6 or less ring atoms.

Examples of the monocyclic hydrocarbon group having 6 or less ring carbon atoms represented by A^{3A} include phenylene group, cyclopentenylene group, cyclopentadienylene group, cyclohexylene group, and cyclopentylene group, with phenylene group being preferred.

Examples of the monocyclic heterocyclic group having 6 or less ring atoms represented by A^{3A} include pyrrolylene group, pyrazinylene group, pyridinylene group, furylene group, and thiophenylene group.

In a preferred embodiment of formulae (A), (XXII), (XXIII), and (XXIV), mA is 0 and one of X^{5A} to X^{8A} and one of Y^{1A} to Y^{4A} are bonded to each other via a single bond; or A^{3A} represents the substituted or unsubstituted monocyclic hydrocarbon group having 6 or less ring carbon atoms or the substituted or unsubstituted monocyclic heterocyclic group having 6 or less ring atoms.

In more preferred embodiment, mA is 0 and one of X^{5A} to X^{8A} and one of Y^{1A} to Y^{4A} are bonded to each other via a single bond; or A^{3A} represents a substituted or unsubstituted phenylene group.

The host material of formula (A) satisfies preferably at least one of the requirements (i) and (ii);
(i) at least one of A^{1A} and A^{2A} represents a cyano-substituted aromatic hydrocarbon group having 6 to 30 ring carbon atoms or a cyano-substituted heterocyclic group having 5 to 30 ring atoms; and
(ii) at least one of X^{1A} to X^{4A} and Y^{5A} to Y^{8A} represents CR^{a}, and at least one of R^{a} in X^{1A} to X^{4A} and Y^{5A} to Y^{8A} represents a cyano-substituted aromatic hydrocarbon group having 6 to 30 ring carbon atoms or a cyano-substituted heterocyclic group having 5 to 30 ring atoms.

Namely, the host material of formula (A) is preferably any one of the compounds;
(1) satisfying the requirement (i), but not satisfying the requirements (ii) to (v);
(2) satisfying the requirement (ii), but not satisfying the requirements (i) and (iii) to (v); and
(3) satisfying both the requirements (i) and (ii), but not satisfying the requirements (iii) to (v).

The host material of formula (A) satisfying the requirement (i) and/or (ii) has a structure wherein the cyano group-containing aromatic hydrocarbon group or the cyano group-containing heterocyclic group is introduced to the terminal end of the central skeleton comprising the groups represented by formulae (a) and (b).

When the host material of formula (A) satisfies the requirement (i), at least one of A^{1A} and A^{2A} is preferably a cyano-substituted phenyl group, a cyano-substituted naphthyl group, a cyano-substituted phenanthryl group, a cyano-substituted dibenzofuranyl group, a cyano-substituted dibenzothiophenyl group, a cyano-substituted biphenyl group, a cyano-substituted terphenyl group, a cyano-substituted 9,9-diphenylfluorenyl group, a cyano-substituted 9,9'-spirobi[9H-fluorene]-2-yl group, a cyano-substituted 9,9'-dimethylfluorenyl group, or a cyano-substituted triphenylenyl group, and more preferably 3'-cyanobiphenyl-2-yl group, 3'-cyanobiphenyl-3-yl group, 3'-cyanobiphenyl-4-yl group, 4'-cyanobiphenyl-3-yl group, 4'-cyanobiphenyl-4-yl group, 4'-cyanobiphenyl-2-yl group, 6-cyanonaphthalene-2-yl group, 4-cyanonaphthalene-1-yl group, 7-cyanonaphthalene-2-yl group, 8-cyanodibenzofuran-2-yl group, 6-cyanodibenzofuran-4-yl group, 8-cyanodibenzothiophene-2-yl group, 6-cyanodibenzothiophene-4-yl group, 7-cyano-9-phenylcarbazole-2-yl group, 6-cyano-9-phenylcarbazole-3-yl group, 7-cyano-9,9-dimethylfluorene-2-yl group, or 7-cyanotriphenylene-2-yl group.

The host material of formula (A) wherein A^{1A} is substituted by a cyano group and A^{2A} is not substituted by a cyano group is preferred. In this case, the first host material which does not satisfy the requirement (ii) is more preferred.

When the host material of formula (A) satisfies the requirement (ii), at least one of X^{1A} to X^{4A} and Y^{5A} to Y^{8A} is preferably CR^{a}, and one of R^{a} in X^{1A} to X^{4A} and Y^{5A} to Y^{8A} is preferably a cyano-substituted phenyl group, a cyano-substituted naphthyl group, a cyano-substituted phenanthryl group, a cyano-substituted dibenzofuranyl group, a cyano-substituted dibenzothiophenyl group, a cyano-substituted biphenyl group, a cyano-substituted terphenyl group, a cyano-substituted 9,9-diphenylfluorenyl group, a cyano-substituted 9,9'-spirobi[9H-fluorene]-2-yl group, a cyano-substituted 9,9'-dimethylfluorenyl group, or a cyano-substituted triphenylenyl group, and more preferably 3'-cyanobiphenyl-2-yl group, 3'-cyanobiphenyl-3-yl group, 3'-cyanobiphenyl-4-yl group, 4'-cyanobiphenyl-3-yl group, 4'-cyanobiphenyl-4-yl group, 4'-cyanobiphenyl-2-yl group, 6-cyanonaphthalene-2-yl group, 4-cyanonaphthalene-1-yl group, 7-cyanonaphthalene-2-yl group, 8-cyanodibenzofuran-2-yl group, 6-cyanodibenzofuran-4-yl group, 8-cyanodibenzothiophene-2-yl group, 6-cyanodibenzothiophene-4-yl group, 7-cyano-9-phenylcarbazole-2-yl group, 6-cyano-9-phenylcarbazole-3-yl group, 7-cyano-9,9-dimethylfluorene-2-yl group, or 7-cyanotriphenylene-2-yl group.

It is preferred for the host material of formula (A) to satisfy the requirement (ii), but not satisfy the requirement (i).

In formulae (A) and (XXII) to (XXIV), A^{1A} and A^{2A} are preferably different from each other, and more preferably, A^{1A} is substituted by a cyano group but A^{2A} is not substituted by a cyano group. Namely, the host material of formula (A) is preferably structurally asymmetric.

The production method of the first host material is not particularly limited and it is produced according to a known method, for example, by a coupling reaction of a carbazole derivative and an aromatic halogenated compound in the presence of a copper catalyst described in Tetrahedron 40 (1984) 1435 to 1456 or a palladium catalyst described in Journal of American Chemical Society 123 (2001) 7727 to 7729.

Examples of the host material of formula (A) are mentioned in [0145] in US2013234119.

Examples for preferred host materials that are preferably used as co-hosts in the electronic device according to the present invention are mentioned in US2013234119, WO2012108388 and WO2014009317 are:

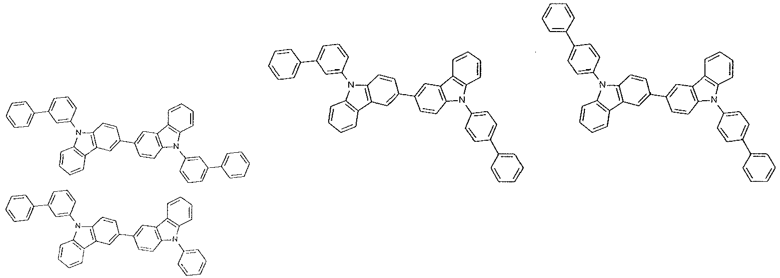

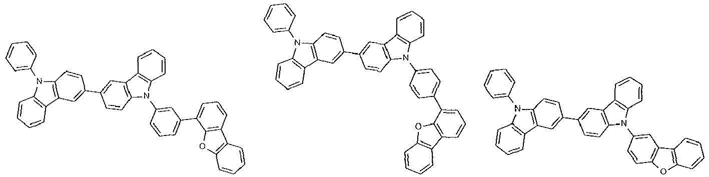

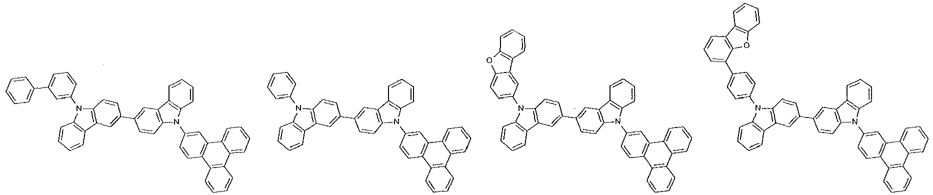

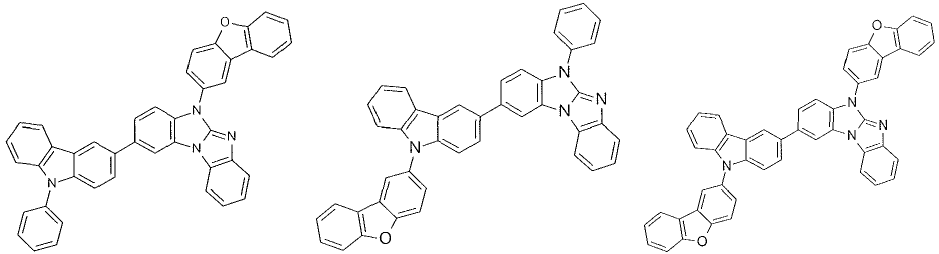

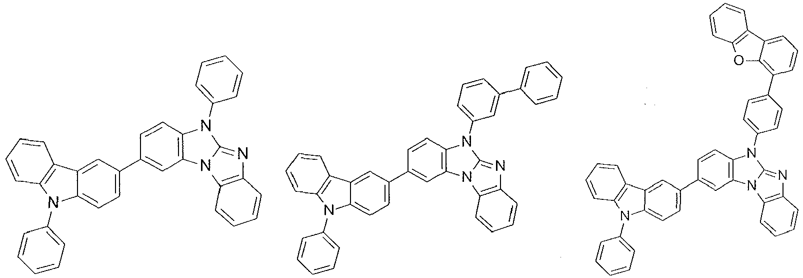

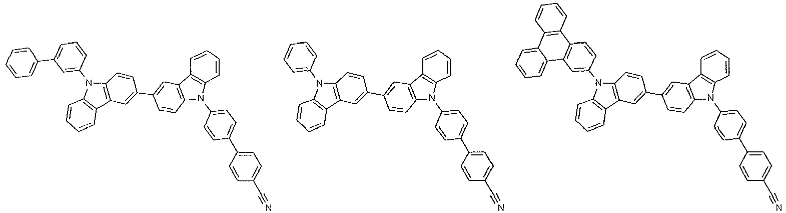

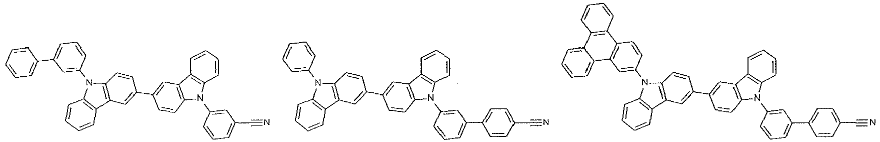

According to the present invention, the compounds according to general formulae (lib) and (IIc) can also be used in combination with host materials that are called "second host materials" in US20130234119, see in particular paragraphs 0146 to 0195 of US20130234119. In addition these compounds according to paragraphs 0146 to 1095 of US20130234119 can also be used as single host material in the electronic device according to the present invention, for example for red emitter material or green emitter material, preferably for red emitter material.. The use of compounds according to general formulae (IIb) and (IIc) according to the present invention in combination with host materials according to paragraphs 0146 to 0195 of US20130234119 as host material for green light emitting materials is preferred.

In particular, compounds according to the formula (1a) can be used as host materials in the electronic device according to the present invention: 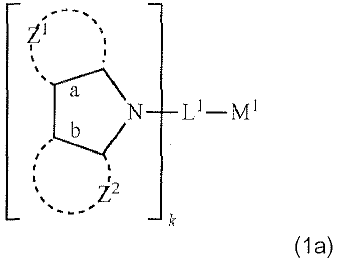 wherein
Z¹ represents a ring structure fused to the side a and represented by formula (1-1) or (1-2), and Z² represents a ring structure fused to the side b and represented by formula (1-1) or (1-2), provided that at least one of Z¹ and Z² is represented by formula (1-1);
M¹ represents a substituted or unsubstituted nitrogen-containing aromatic heteroring having 5 to 30 ring atoms;
L¹ represents a single bond, a substituted or unsubstituted divalent aromatic hydrocarbon group having 6 to 30 ring carbon atoms, a substituted or unsubstituted divalent heterocyclic group having 5 to 30 ring atoms, a cycloalkylene group having 5 to 30 ring atoms, or a group in which the preceding groups are directly linked to each other; and
k represents 1 or 2.

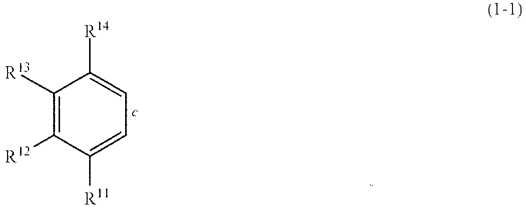

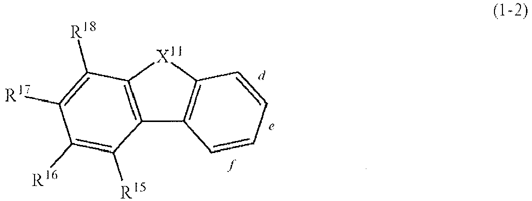

In formula (1-1), a side c is fused to the side a or b of formula (1). In formula (1-2), any one of sides d, e and f is fused to the side a or b of formula (1). In formulae (1-1) and (1-2), X¹¹ represents a sulfur atom, an oxygen atom, N-R¹⁹, or C(R²⁰)(R²¹); and ^{each} of R¹¹ to R²¹ independently represents a hydrogen atom, a heavy hydrogen atom, a halogen atom, a cyano group, a substituted or unsubstituted aromatic hydrocarbon group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 30 ring carbon atoms, a substituted or unsubstituted alkoxy group having 1 to 30 carbon atoms, a substituted or unsubstituted aralkyl group having 6 to 30 ring carbon atoms, or a substituted or unsubstituted aryloxy group having 6 to 30 ring carbon atoms, provided that adjacent groups of R<11 >to R<21 >may be bonded to each other to form a a ring.

The nitrogen-containing aromatic heteroring represented by M¹ of formula (1) includes an azine rings

Examples of the nitrogen-containing aromatic heteroring include pyridine, pyrimidine, pyrazine, triazine, aziridine, azaindolizine, indolizine, imidazole, indole, isoindole, indazole, purine, pteridine, β-carboline, naphthyridine, quinoxaline, terpyridine, bipyridine, acridine, phenanthroline, phenazine, and imidazopyridine, with pyridine, pyrimidine, and triazine being particularly preferred. The formula (1) is preferably represented by formula (2): 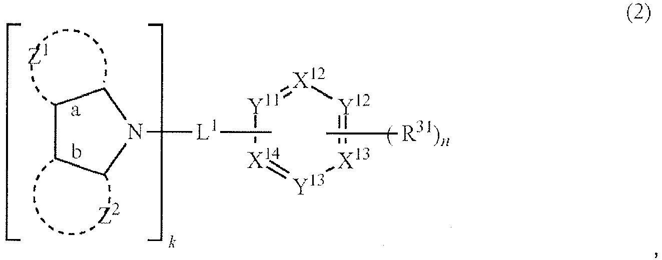
Z¹ represents a ring structure fused to the side a and represented by formula (1-1) or (1-2), and Z² represents a ring structure fused to the side b and represented by formula (1-1) or (1-2), provided that at least one of Z¹ and Z² is represented by formula (1-1);
L¹ is as defined in formula (1);
each of X¹² to X¹⁴ independently represents a nitrogen atom, CH, or a carbon atom bonded to R³¹ or L¹, provided that at least one of X¹² to X¹⁴ represents a nitrogen atom;
^{each} of Y¹¹ to Y¹³ independently represents CH or a carbon atom bonded to R³¹ or L¹;
each of R³¹ independently represents a halogen atom, a cyano group, a substituted or unsubstituted aromatic hydrocarbon group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 30 ring carbon atoms, a substituted or unsubstituted alkoxy group having 1 to 30 carbon atoms, a substituted or unsubstituted aralkyl group having 6 to 30 ring carbon atoms, or a substituted or unsubstituted aryloxy group having 6 to 30 ring carbon atoms;
when two or more R³¹ groups exist, the R³¹ groups may be the same or different and adjacent R³¹ groups may be bonded to each other to form a ring;
k represents 1 or 2, and n represents an integer of 0 to 4;
the side c of formula (1-1) is fused to the side a or b of formula (2); and any one of sides d, e and f of formula (1-2) is fused to the side a or b of formula (2).

Examples of the compound wherein the ring represented by formula (1-1) or (1-2) is fused to the side a or b of formula (2) are shown below.

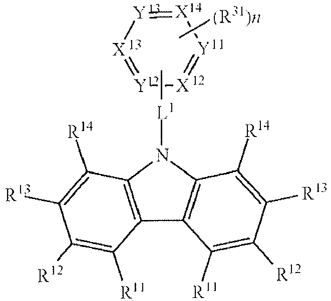

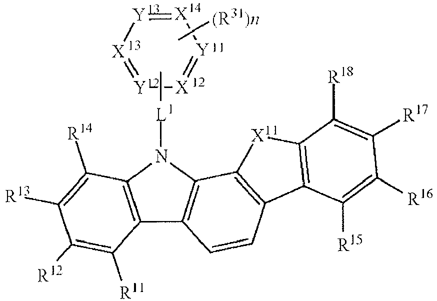

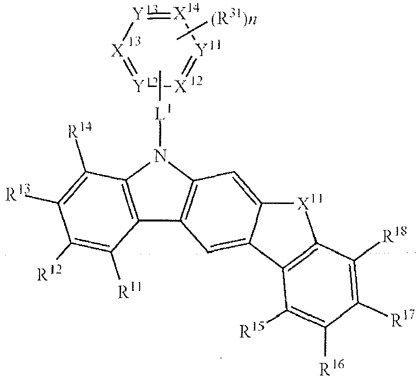

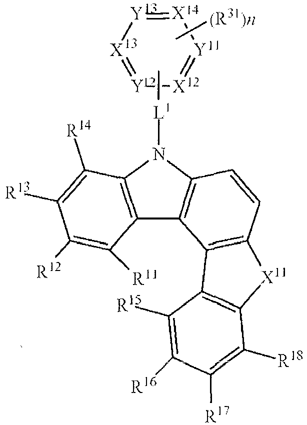

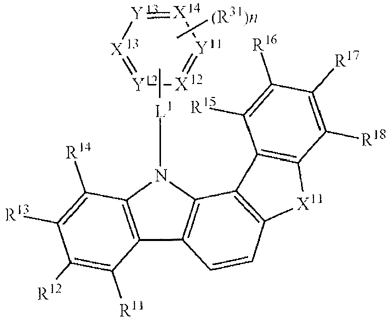

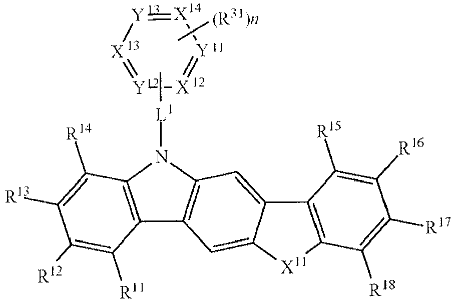

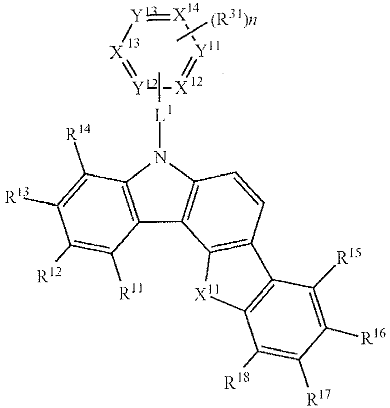

The compound represented by formula (1) or (2) is more preferably represented by formula (3) and particularly preferably represented by formula (4). 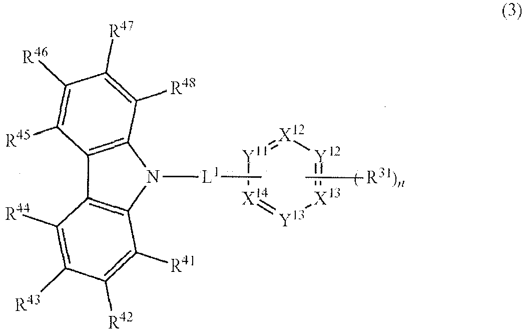
In formula (3), L¹ is as defined in formula (1);
each of X¹² to X¹⁴ independently represents a nitrogen atom, CH, or a carbon atom bonded to R³¹ or L¹, provided that at least one of X¹² to X¹⁴ represents a nitrogen atom;
^{each} of Y¹¹ to Y¹³ independently represents CH or a carbon atom bonded to R³¹ or L¹;
each of R³¹ independently represents a halogen atom, a cyano group, a substituted or unsubstituted aromatic hydrocarbon group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 30 ring carbon atoms, a substituted or unsubstituted alkoxy group having 1 to 30 carbon atoms, a substituted or unsubstituted aralkyl group having 6 to 30 ring carbon atoms, or a substituted or unsubstituted aryloxy group having 6 to 30 ring carbon atoms;
when two or more R³¹ groups exist, the R³¹ groups may be the same or different and adjacent R³¹ groups may be bonded to each other to form a ring;
n represents an integer of 0 to 4;
each of R⁴¹ to R⁴⁸ independently represents a hydrogen atom, a heavy hydrogen atom, a halogen atom, a cyano group, a substituted or unsubstituted aromatic hydrocarbon group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 30 ring carbon atoms, a substituted or unsubstituted alkoxy group having 1 to 30 carbon atoms, a substituted or unsubstituted aralkyl group having 6 to 30 ring carbon atoms, or a substituted or unsubstituted aryloxy group having 6 to 30 ring carbon atoms; and
adjacent groups of R⁴¹ to R⁴⁸ may be bonded to each other to form a ring. 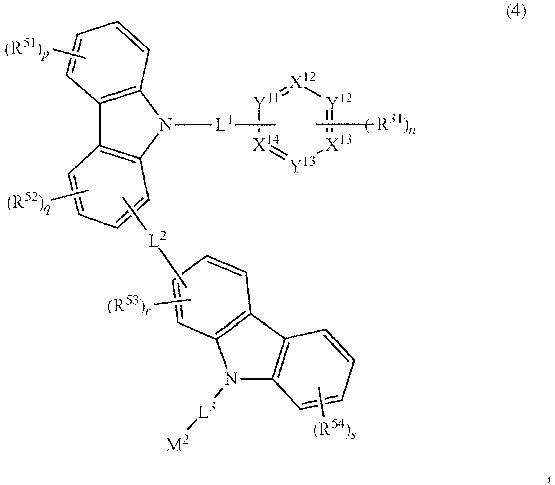
In formula (4), L¹ is as defined in formula (1);
each of X¹² to X¹⁴ independently represents a nitrogen atom, CH, or a carbon atom bonded to R³¹ or L¹, provided that at least one of X¹² to X¹⁴ represents a nitrogen atom;
^{each} of Y¹¹ to Y¹³ independently represents CH or a carbon atom bonded to R³¹ or L¹;
each of R³¹ independently represents a halogen atom, a cyano group, a substituted or unsubstituted aromatic hydrocarbon group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 30 ring carbon atoms, a substituted or unsubstituted alkoxy group having 1 to 30 carbon atoms, a substituted or unsubstituted aralkyl group having 6 to 30 ring carbon atoms, or a substituted or unsubstituted aryloxy group having 6 to 30 ring carbon atoms;
adjacent R³¹ groups may be bonded to each other to form a ring;
n represents an integer of 0 to 4;
each of L² and L³ independently represents a single bond, a substituted or unsubstituted divalent aromatic hydrocarbon group having 6 to 30 ring carbon atoms, a substituted or unsubstituted divalent heterocyclic group having 5 to 30 ring atoms, a cycloalkylene group having 5 to 30 ring atoms, or a group in which the preceding groups are directly linked to each other;
each of R⁵¹ to R⁵⁴ independently represents a halogen atom, a cyano group, a substituted or unsubstituted aromatic hydrocarbon group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 30 ring carbon atoms, a substituted or unsubstituted alkoxy group having 1 to 30 carbon atoms, a substituted or unsubstituted aralkyl group having 6 to 30 ring carbon atoms, or a substituted or unsubstituted aryloxy group having 6 to 30 ring carbon atoms;
when two or more R⁵¹ groups exist, the R⁵¹ groups may be the same or different and adjacent R⁵¹ groups may be bonded to each other to form a ring;
when two or more R⁵² groups exist, the R⁵² groups may be the same or different and adjacent R⁵² groups may be bonded to each other to form a ring;
when two or more R⁵³ groups exist, the R⁵³ groups may be the same or different and adjacent R⁵³ groups may be bonded to each other to form a ring;
when two or more R⁵⁴ groups exist, the R⁵⁴ groups may be the same or different and adjacent R⁵⁴ groups may be bonded to each other to form a ring;
M² represents a substituted or unsubstituted aromatic hydrocarbon group having 6 to 30 ring carbon atoms or a substituted or unsubstituted heterocyclic group having 5 to 30 ring atoms; and
each of p and s independently represents an integer of 0 to 4, and each of q and r independently represents an integer of 0 to 3.

In formulae (1) to (4), (1-1), and (1-2), the groups represented by R¹¹ to R²¹, R³¹, R⁴¹ to R⁴⁸, and R⁵¹ to R⁵⁴ are as defined above with respect to formula (A).

Examples of the divalent aromatic hydrocarbon group having 6 to 30 ring carbon atoms and the divalent heterocyclic group having 5 to 30 ring atoms represented by L¹ to L³ of formulae (1) to (4) includes divalent residues of the corresponding groups described above with respect to formula (A).

According to a further preferred embodiment of the present invention, host materials according to US20140048784, in particular according to paragraphs 0098 to 0154 can be used in the electronic device according to the present invention, in particular, if red light emitting materials are used. The host materials according to US20140048784 can be used as single host materials, which can is preferred, or can be used in combination with compounds (lib) or (IIc) according to the present invention as host material and co-host:
The host material according to US2014048784 is a biscarbazole derivative, having two carbazole structures in a molecule thereof.

The biscarbazole derivative has, at a specific position, a substituted or unsubstituted fluoranthenyl group, a substituted or unsubstituted triphenylenyl group, a substituted or unsubstituted benzophenanthrenyl group, a substituted or unsubstituted benzotriphenylenyl group, a substituted or unsubstituted dibenzotriphenylenyl group, a substituted or unsubstituted chrysenyl group, a substituted or unsubstituted benzochrysenyl group, a substituted or unsubstituted picenyl group, a substituted or unsubstituted benzo[b]fluoranthenyl group, a substituted or unsubstituted benzofuranyl group, a substituted or unsubstituted dibenzofuranyl group, a substituted or unsubstituted benzothiophenyl group, a substituted or unsubstituted dibenzothiophenyl group, a substituted or unsubstituted phenanthrenyl group, a substituted or unsubstituted fluorenyl group, a substituted or unsubstituted binaphthyl group, a substituted or unsubstituted benzonaphthofuranyl group, a substituted or unsubstituted benzonaphthothiophenyl group, a substituted or unsubstituted dibenzophenanthrenyl group, a substituted or unsubstituted naphthotriphenylenyl group, a substituted or unsubstituted benzofluoranthenyl group, a substituted or unsubstituted benzofluorenyl group, or a substituted or unsubstituted phenyl group. Examples thereof include compounds represented by any of formulae (1) to (4), (1'), (1a), and (10). 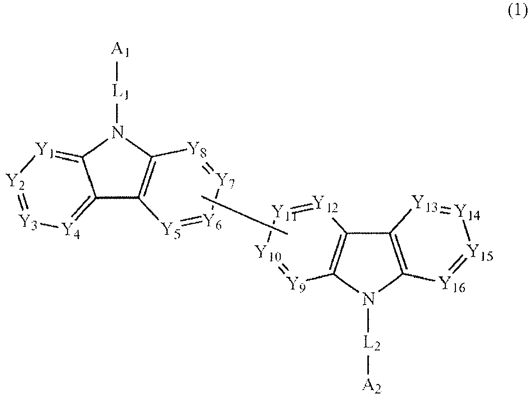 wherein;
each of A1 and A2 independently represents a substituted or unsubstituted aromatic hydrocarbon group having 6 to 30 ring carbon atoms;
each of Y1 to Y16 independently represents C(R) or a nitrogen atom, and each of R groups independently represents a hydrogen atom, a substituent, or a valence bonded to a carbazole skeleton; and
each of L1 and L2 independently represents a single bond, a substituted or unsubstituted, divalent aromatic hydrocarbon group having 6 to 30 ring carbon atoms, or a substituted or unsubstituted, divalent aromatic heterocyclic group having 2 to 30 ring carbon atoms, provided that;
at least one of A1, A2 and R represents a substituted or unsubstituted fluoranthenyl group, a substituted or unsubstituted triphenylenyl group, a substituted or unsubstituted benzophenanthrenyl group, a substituted or unsubstituted benzotriphenylenyl group, a substituted or unsubstituted dibenzotriphenylenyl group, a substituted or unsubstituted chrysenyl group, a substituted or unsubstituted benzochrysenyl group, a substituted or unsubstituted picenyl group, a substituted or unsubstituted benzo[b]fluoranthenyl group, a substituted or unsubstituted phenanthrenyl group, a substituted or unsubstituted binaphthyl group, a substituted or unsubstituted dibenzophenanthrenyl group, a substituted or unsubstituted naphthotriphenylenyl group, a substituted or unsubstituted benzofluorenyl group, or a naphthyl group;
when Y1 to Y16 all represent C(R) wherein R is a hydrogen atom, Y6 and Y11 are bonded to each other via a single bond, each of L1 and L2 represents a single bond, and A1 represents a phenanthrenyl group, A2 represents a phenyl group, a biphenylyl group, or a naphthyl group; and
when Y1 to Y16 all represent C(R) wherein R is a hydrogen atom, Y6 and Y11 are bonded to each other via a single bond, each of L1 and L2 represents a single bond, and A1 represents a naphthyl group, A1 and A2 are different from each other.

In formulae (1) and (1'), at least one of Y1 to Y4 represents C(R), at least one of Y5 to Y8 represents C(R), at least one of Y9 to Y12 represents C(R), and at least one of Y13 to Y16 represent C(R).

In addition, at least one of Y5 to Y8 represents C(R) and at least one of Y9 to Y12 represents C(R), wherein two R groups represent valences which are bonded to each other.

The R groups in formulae (1) and (1') may be the same or different.

In formula (1a), at least one of Y1a to Y4a represents C(R), at least one of Y5a to Y8a represents C(R), at least one of Y9a Y12a represents C(R), and at least one of Y13a to Y16a represents C(R).

In addition, at least one of Y5a to Y8a represents C(R) and at least one of Y9a to Y12a represents C(R), wherein two R groups represent valences which are bonded to each other.

The R groups in formula (1a) may be the same or different.

In formula (10), at least one of Y1' to Y4' represents C(R'), at least one of Y5' to Y8' represents C(R'), at least one of Y9' to Y12' represents C(R'), and at least one of Y13' to Y16' represents C(R').

In addition, at least one of Y5' to Y8' represents C(R') and at least one of Y9' to Y12' represents C(R'), wherein two R' groups represent valences which are bonded to each other.

The R' groups in formula (10) may be the same or different. 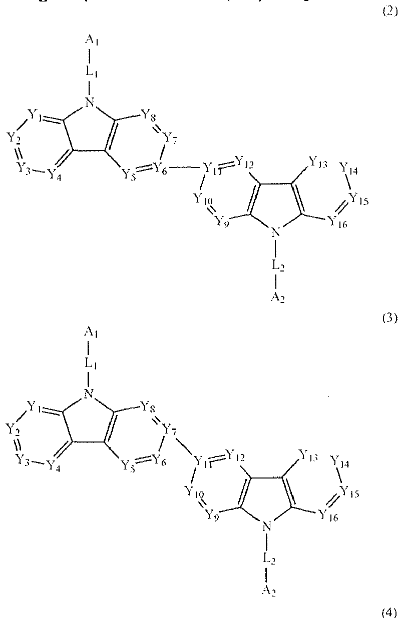 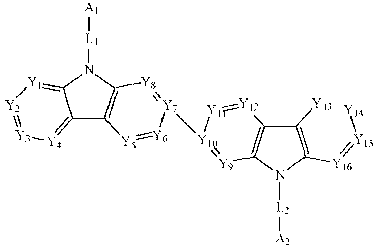 wherein each of A1, A2, Y1 to Y16, L1, and L2 in formulae (2) to (4) is as defined in formula (1). 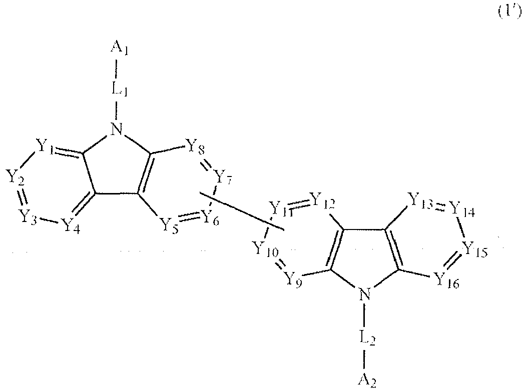 wherein:
each of A1 and A2 independently represents a substituted or unsubstituted aromatic hydrocarbon group having 6 to 30 ring carbon atoms or a substituted or unsubstituted aromatic heterocyclic group having 2 to 30 ring carbon atoms;
each of Y1 to Y16 independently represents C(R) or a nitrogen atom, and each of R groups independently represents a hydrogen atom, a substituent, or a valence bonded to a carbazole skeleton; and
each of L1 and L2 independently represents a single bond, a substituted or unsubstituted, divalent aromatic hydrocarbon group having 6 to 30 ring carbon atoms, or a substituted or unsubstituted, divalent aromatic heterocyclic group having 2 to 30 ring carbon atoms, provided that:
   at least one of A1, A2 and R represents a substituted or unsubstituted fluoranthenyl group, a substituted or unsubstituted triphenylenyl group, a substituted or unsubstituted benzophenanthrenyl group, a substituted or unsubstituted benzotriphenylenyl group, a substituted or unsubstituted dibenzotriphenylenyl group, a substituted or unsubstituted chrysenyl group, a substituted or unsubstituted benzochrysenyl group, a substituted or unsubstituted picenyl group, a substituted or unsubstituted benzo[b]fluoranthenyl group, a substituted or unsubstituted benzofuranyl group, a substituted or unsubstituted dibenzofuranyl group, a substituted or unsubstituted benzothiophenyl group, a substituted or unsubstituted dibenzothiophenyl group, a substituted or unsubstituted phenanthrenyl group, a substituted or unsubstituted fluorenyl group, a substituted or unsubstituted binaphthyl group, a substituted or unsubstituted benzonaphthofuranyl group, a substituted or unsubstituted benzonaphthothiophenyl group, a substituted or unsubstituted dibenzophenanthrenyl group, a substituted or unsubstituted naphthotriphenylenyl group, a substituted or unsubstituted benzofluorenyl group, or a substituted or unsubstituted phenyl group;
   when Y1 to Y16 all represent C(R) wherein R is a hydrogen atom, Y6 and Y11 are bonded to each other via a single bond, each of L1 and L2 represents a single bond, and A1 represents a phenanthrenyl group, A2 does not represent a phenanthrenyl group;
   when Y1 to Y16 all represent C(R), Y6 and Y11 are bonded to each other via a single bond, and each of L1 and L2 represents a single bond, each of R groups does not represent a fluorenyl group; and
   when A1 represents a fluorenyl group, A2 does not represent a phenyl group, a naphthyl group, or a fluorenyl group. 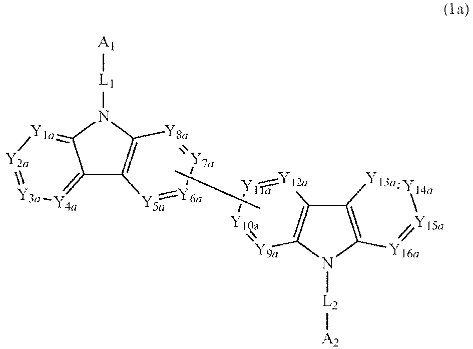
wherein:
one of A1a and A2a represents a group represented by formula (a) and the other represents a substituted or unsubstituted fluoranthenyl group, a substituted or unsubstituted benzophenanthrenyl group, a substituted or unsubstituted picenyl group, a substituted or unsubstituted benzo[b]fluoranthenyl group, a substituted or unsubstituted phenanthrenyl group, a substituted or unsubstituted binaphthyl group, a substituted or unsubstituted dibenzophenanthrenyl group, a substituted or unsubstituted naphthotriphenylenyl group, or a substituted or unsubstituted benzofluorenyl group;
each of Y1a to Y16a independently represents C(R) or a nitrogen atom, and each of R groups independently represents a hydrogen atom, a substituent, or a valence bonded to a carbazole skeleton;
each of L1a and L2a independently represents a single bond, a substituted or unsubstituted, divalent aromatic hydrocarbon group having 6 to 30 ring carbon atoms, or a substituted or unsubstituted, divalent aromatic heterocyclic group having 2 to 30 ring carbon atoms: 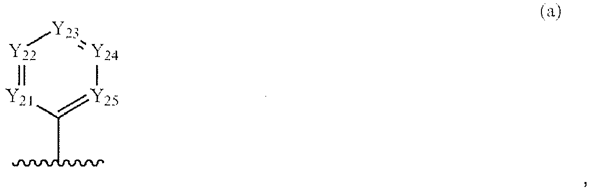
wherein each of Y21 and Y25 independently represents C(Ra) or a nitrogen atom, and each of Ra groups independently represents a hydrogen atom or a substituent.

The details of A1a, A2a, Y1a to Y16a, L1a, L2a, and Ra in formulae (1a) and (a) are the same as those of A1, A2, Y1 to Y16, L1, L2, and R in formula (1).

When one of A1a and A2a represents a group represented by formula (a) and the other represents a group including a large molecular weight fused ring, such as a triphenylenyl group and a chrysenyl group, the compound represented by formula (1a) has an excessively large molecular weight, increasing the vapor deposition temperature and therefore likely to increase the amount of thermally decomposed components. Therefore, when one of A1a and A2a represents a group represented by formula (a), the other preferably represents a substituted or unsubstituted fluoranthenyl group or a substituted or unsubstituted phenanthrenyl group. 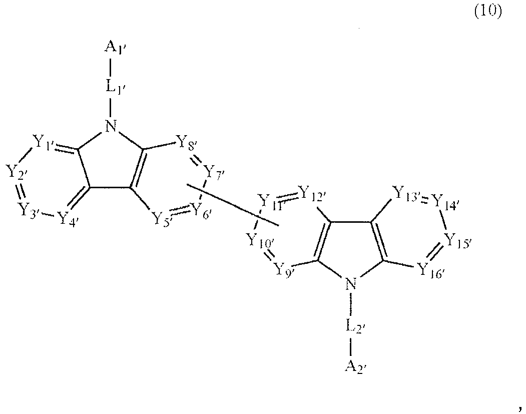 wherein:
one of A1' and A2' represents a substituted or unsubstituted naphthyl group or a substituted or unsubstituted fluorenyl group and the other represents a substituted or unsubstituted aromatic hydrocarbon group having 6 to 30 ring carbon atoms;
each of Y1' to Y16' independently represents C(R') or a nitrogen atom, and each of R' groups independently represents a hydrogen atom, a substituent, or a valence bonded to a carbazole skeleton; and
each of L1' and L2' independently represents a single bond, a substituted or unsubstituted, divalent aromatic hydrocarbon group having 6 to 30 ring carbon atoms, or a substituted or unsubstituted, divalent aromatic heterocyclic group having 2 to 30 ring carbon atoms.

The details of A1', A2', L1', L2', Y1' to Y16', and R' in formula (10) are the same as those of A1, A2, L1, L2, Y1 to Y16, and R in formula (1).

In formulae (1) to (4) and (1'), at least one of A1, A2 and R preferably represents a substituted or unsubstituted fluoranthenyl group, a substituted or unsubstituted triphenylenyl group, a substituted or unsubstituted benzophenanthrenyl group, a substituted or unsubstituted benzotriphenylenyl group, a substituted or unsubstituted dibenzotriphenylenyl group, a substituted or unsubstituted chrysenyl group, a substituted or unsubstituted benzochrysenyl group, a substituted or unsubstituted picenyl group, a substituted or unsubstituted benzo[b]fluoranthenyl group, a substituted or unsubstituted benzofuranyl group, a substituted or unsubstituted dibenzofuranyl group, a substituted or unsubstituted benzothiophenyl group, a substituted or unsubstituted dibenzothiophenyl group, a substituted or unsubstituted phenanthrenyl group, a substituted or unsubstituted fluorenyl group, or a substituted or unsubstituted binaphthyl group, because these groups are moderately bulky. More preferably, at least one of A1 and A2 represents a substituted or unsubstituted fluoranthenyl group, a substituted or unsubstituted triphenylenyl group, a substituted or unsubstituted benzophenanthrenyl group, a substituted or unsubstituted benzotriphenylenyl group, a substituted or unsubstituted dibenzotriphenylenyl group, a substituted or unsubstituted chrysenyl group, a substituted or unsubstituted benzochrysenyl group, a substituted or unsubstituted picenyl group, a substituted or unsubstituted benzo[b]fluoranthenyl group, a substituted or unsubstituted benzofuranyl group, a substituted or unsubstituted dibenzofuranyl group, a substituted or unsubstituted benzothiophenyl group, a substituted or unsubstituted dibenzothiophenyl group, or a substituted or unsubstituted binaphthyl group.

Also preferably, each of A1 and A2 in formulae (1) to (4) and (1') independently represents a substituted or unsubstituted fluoranthenyl group, a substituted or unsubstituted triphenylenyl group, a substituted or unsubstituted benzotriphenylenyl group, a substituted or unsubstituted benzophenanthrenyl group, a substituted or unsubstituted dibenzofuranyl group, or a substituted or unsubstituted dibenzothiophenyl group.

In addition, -L1-A1 and -L2-A2 in formulae (1) to (4) and (1') are preferably different from each other.

The substituted or unsubstituted phenyl group for any of A1, A2 and R is preferably a phenyl group substituted by an aromatic hydrocarbon group having 10 to 30 ring carbon atoms and particularly preferably a naphthylphenyl group.

When at least one of A1 and A2 in formulae (1) to (4) and (1') represents a group represented by formula (a), the biscarbazole derivative is particularly preferred as a host material to be used in combination with a green emitting dopant.

In formula (a), Y21 and/or Y25 preferably represents a nitrogen atom, and each of Y22 and Y24 more preferably represents C(Ra).

Specific examples of the substituent which A1 and A2 in formulae (1) to (4) and (1') may have and the substituents represented by R and Ra include a fluorine atom; a cyano group; a substituted or unsubstituted, linear, branched, or cyclic alkyl group having 1 to 20 carbon atoms; a linear, branched, or cyclic alkylene group having 1 to 20 carbon atoms; a linear, branched, or cyclic, divalent, unsaturated hydrocarbon group having 1 to 20 carbon atoms; a substituted or unsubstituted, linear, branched, or cyclic alkoxy group having 1 to 20 carbon atoms; a substituted or unsubstituted, linear, branched, or cyclic haloalkyl group having 1 to 20 carbon atoms; a substituted or unsubstituted, linear, branched, or cyclic haloalkoxy group having 1 to 20 carbon atoms; a substituted or unsubstituted, linear, branched, or cyclic alkylsilyl group having 1 to 10 carbon atoms; a substituted or unsubstituted arylsilyl group having 6 to 30 carbon atoms; a substituted or unsubstituted aromatic hydrocarbon group having 6 to 30 ring carbon atoms; and a substituted or unsubstituted aromatic heterocyclic group having 2 to 30 ring carbon atoms. In addition, a plurality of substituents of any such kind may exist, and when the plurality of substituents exist, the substituents may be the same or different from each other.

The R groups on adjacent ring carbon atoms may be bonded to each other to form a ring structure together with the ring carbon atoms.

Examples of the linear, branched, or cyclic alkyl group having 1 to 20 carbon atoms include a methyl group, an ethyl group, a propyl group, an isopropyl group, a n-butyl group, a s-butyl group, an isobutyl group, a t-butyl group, a n-pentyl group, a n-hexyl group, a n-heptyl group, a n-octyl group, a n-nonyl group, a n-decyl group, a n-undecyl group, a n-dodecyl group, a n-tridecyl group, a n-tetradecyl group, a n-pentadecyl group, a n-hexadecyl group, a n-heptadecyl group, a n-octadecyl group, a neopentyl group, a 1-methylpentyl group, a 2-methylpentyl group, a 1-pentylhexyl group, a 1-butylpentyl group, a 1-heptyloctyl group, a 3-methylpentyl group, a cyclopentyl group, a cyclohexyl group, a cyclooctyl group, a 3,5-tetramethylcyclohexyl group, a trifluoromethyl group, a 2,2,2-trifluoroethyl group, and a 1,1,1,3,3,3-hexafluoro-2-propyl group.

Examples of the linear, branched, or cyclic alkylene group having 1 to 20 carbon atoms include an ethylene group, a propylene group, and a butylene group.

Examples of the linear, branched, or cyclic, divalent unsaturated hydrocarbon group having 1 to 20 carbon atoms include a 1,3-butadiene-1,4-diyl group.

Examples of the linear, branched, or cyclic alkylsilyl group having 1 to 10 carbon atoms include a trimethylsilyl group, a triethylsilyl group, a tributylsilyl group, a dimethylethylsilyl group, a dimethylisopropylsilyl group, a dimethylpropylsilyl group, a dimethylbutylsilyl group, a dimethyl-t-butylsilyl group, and a diethylisopropylsilyl group.

Examples of the arylsilyl group having 6 to 30 carbon atoms include a phenyldimethylsilyl group, a diphenylmethylsilyl group, a diphenyl-t-butylsilyl group, and a triphenylsilyl group.

Examples of the halogen atom include a fluorine atom.

Examples of the aromatic heterocyclic group having 2 to 30 ring carbon atoms include non-fused aromatic heterocyclic and fused aromatic heterocyclic groups, more specifically, a pyrrolyl group, a pyrazinyl group, a pyridinyl group, an indolyl group, an isoindolyl group, a furyl group, a benzofuranyl group, an isobenzofuranyl group, a dibenzofuranyl group, a dibenzothiophenyl group, a quinolyl group, an isoquinolyl group, a quinoxalinyl group, a carbazolyl group, a phenanthridinyl group, an acridinyl group, a phenanthrolinyl group, a thienyl group, and residues of a pyridine ring, a pyrazine ring, a pyrimidine ring, a pyridazine ring, a triazine ring, an indole ring, a quinoline ring, an acridine ring, a pyrrolidine ring, a dioxane ring, a piperidine ring, a morpholine ring, a piperazine ring, a carbazole ring, a furan ring, a thiophene ring, an oxazole ring, an oxadiazole ring, a benzoxazole ring, a thiazole ring, a thiadiazole ring, a benzothiazole ring, a triazole ring, an imidazole ring, a benzimidazole ring, a pyran ring, a dibenzofuran ring, and a benzo[c]dibenzofuran ring.

Examples of the aromatic hydrocarbon group having 6 to 30 ring carbon atoms include non-fused aromatic hydrocarbon groups and fused aromatic hydrocarbon groups, more specifically, a phenyl group, a naphthyl group, a phenanthryl group, a biphenyl group, a terphenyl group, a quaterphenyl group, a fluoranthenyl group, a triphenylenyl group, a phenanthrenyl group, a 9,9-dimethylfluorenyl group, a benzo[c]phenanthrenyl group, a benzo[α]triphenylenyl group, a naphtho[1,2-c]phenanthrenyl group, a naphtho[1,2-a]triphenylenyl group, a dibenzo[a,c]triphenylenyl group, and a benzo[b]fluoranthenyl group.

Examples of the divalent linking group represented by L1 and L2 in formulae (1) to (4) and (1') include a substituted or unsubstituted, divalent aromatic hydrocarbon group having 6 to 30 ring carbon atoms and a substituted or unsubstituted, divalent aromatic heterocyclic group having 2 to 30 ring carbon atoms.

Examples of the divalent aromatic hydrocarbon group having 6 to 30 ring carbon atoms include groups obtained by making the examples of the aromatic hydrocarbon group having 6 to 30 ring carbon atoms mentioned above into divalent groups.

In addition, specific examples of the divalent aromatic heterocyclic group having 2 to 30 ring carbon atoms include groups obtained by making the examples of the aromatic heterocyclic group having 2 to 30 ring carbon atoms mentioned above into divalent groups.

In each of formulae (1) to (4) and (1'), Y1 to Y16 all preferably represent C(R).

In each of formulae (1) to (4) and (1'), the number of substituents represented by R in Y1 to Y8 or in Y9 to Y16 is preferably 0 to 2, more preferably 0 or 1.

Specific examples of the biscarbazole derivative represented by any one of formulae (1) to (4), (1'), and (10) include the following compounds. In the following structural formulae, D represents a heavy hydrogen (deuterium).

According to the present invention, the compounds according to general formulae (lib) and (IIc) are preferably be used as host material in the light emitting layer of the electronic device, preferably in a OLED, according to the present invention. The compounds according to general formulae (IIb) and (IIc) can be used (a) as single host materials or can be used (b) in combination with any compounds suitable as host materials as mentioned above. Embodiment (a) is preferred; if a red light emitting material is present in the light emitting layer. Embodiment (b) is preferred; if a green light emitting material is present in the light emitting layer.

Preferred host materials, which may be used, if blue dopants are present in the light emitting layer, are mentioned in US 2012/112169. Preferably, the anthracene derivative represented by the formula (5) is used as host material for blue dopants:

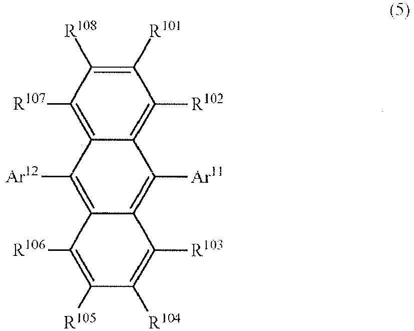

In the formula (5), Ar¹¹ and Ar¹² are independently a substituted or unsubstituted monocyclic group having 5 to 50 ring atoms, a substituted or unsubstituted fused ring group having 8 to 50 ring atoms, or a group formed by combination of a monocyclic group and a fused ring group and R¹⁰¹ to R¹⁰⁸ are independently a group selected from a hydrogen atom, a substituted or unsubstituted monocyclic group having 5 to 50 ring atoms, a substituted or unsubstituted fused ring group having 8 to 50 ring atoms, a group formed by combination of a monocyclic group and a fused ring group, a substituted or unsubstituted alkyl group having 1 to 50 carbon atoms, a substituted or unsubstituted cycloalkyl group having 3 to 50 ring carbon atoms, a substituted or unsubstituted alkoxy group having 1 to 50 carbon atoms, a substituted or unsubstituted aralkyl group having 7 to 50 carbon atoms, a substituted or unsubstituted aryloxy group having 6 to 50 ring carbon atoms, a substituted or unsubstituted silyl group, a halogen atom and a cyano group.

The monocyclic group in the formula (5) means a group which is composed only of ring structures having no fused structure.

As specific examples of the monocyclic group having 5 to 50 (preferably 5 to 30, more preferably 5 to 20) ring atoms, aromatic groups such as a phenyl group, biphenyl group, terphenyl group and quaterphenyl group, and heterocyclic groups such as a pyridyl group, pyradyl group, pyrimidyl group, triadinyl group, furyl group and thienyl group, can be given preferably.

Among these, a phenyl group, biphenyl group or terphenyl group is preferable.

The fused ring group in the formula (5) means a group formed by fusion of 2 or more ring structures.

As specific examples of the fused ring group having 8 to 50 (preferably 8 to 30, more preferably 8 to 20) ring atoms, fused aromatic ring groups such as a naphthyl group, phenanthryl group, anthryl group, chrysenyl group, benzanthryl group, benzophenanthryl group, triphenylenyl group, benzochrysenyl group, indenyl group, fluorenyl group, 9,9-dimethylfluorenyl group, benzofluorenyl group, dibenzofluorenyl group, fluoranthenyl group and benzofluoranthenyl group, and fused heterocyclic groups such as a benzofuranyl group, benzothiophenyl group, indolyl group, dibenzofuranyl group, dibenzothiophenyl group, carbazolyl group, quinolyl group and phenanthrolinyl group, can be given preferably.

Among these, a naphthyl group, phenanthryl group, anthryl group, 9,9-dimethylfluorenyl group, fluoranthenyl group, benzanthryl group, dibenzothiophenyl group, dibenzofuranyl group or carbazolyl group is preferable.

As preferable substituents of "substituted or unsubstituted ... " in Ar¹¹, Ar¹², and R¹⁰¹ to R¹⁰⁸, a monocyclic group, fused ring group, alkyl group, cycloalkyl group, silyl group, alkoxy group, cyano group and halogen atom (in particular, fluorine) can be given. A monocyclic group and fused ring group are particularly preferable.

It is preferred that the anthracene derivative represented by the formula (5) be any of the following anthracene derivatives (A), (B) and (C), which is selected depending on the constitution or demanded properties of an organic EL device to which it is applied.

### (Anthracene Derivative (A))

This anthracene derivative is derivatives of the formula (5) wherein Ar¹¹ and Ar¹² are independently a substituted or unsubstituted fused ring group having 8 to 50 ring atoms. This anthracene derivative can be classified into the case that Ar¹¹ and Ar¹² are the same substituted or unsubstituted fused ring group and the case that Ar¹¹ and Ar¹² are different substituted or unsubstituted fused ring groups.

Particularly preferred is the anthracene derivative of the formula (5) wherein Ar¹¹ and Ar¹² are different (including difference in substituted positions) substituted or unsubstituted fused ring groups. Preferable specific examples of the fused ring are the same as those described above. Among those, a naphthyl group, phenanthryl group, benzanthryl group, 9,9-dimethylfluorenyl group and dibenzofuranyl group are preferable.

### (Anthracene Derivative (B))

This anthracene derivative is derivatives of the formula (5) wherein one of Ar¹¹ and Ar¹² is a substituted or unsubstituted monocyclic group having 5 to 50 ring atoms, and the other is a substituted or unsubstituted fused ring group having 8 to 50 ring atoms.

As a preferred embodiment, Ar¹² is a naphthyl group, phenanthryl group, benzanthryl group, 9,9-dimethylfluorenyl group or dibenzofuranyl group, and Ar¹¹ is a phenyl group substituted by a monocyclic group or fused ring group.

As another preferred embodiment, Ar¹² is a fused ring group, and A¹¹ is an unsubstituted phenyl group. In this case, as the fused ring group, a phenanthryl group, 9,9-dimethylfluorenyl group, dibenzofuranyl group and benzoanthryl group are particularly preferable.

### (Anthracene Derivative (C))

This anthracene derivative is derivatives of formula (5) wherein Ar¹¹ and Ar¹² are independently a substituted or unsubstituted monocyclic group having 5 to 50 ring atoms.

As a preferred embodiment, both Ar¹¹ and Ar¹² are a substituted or unsubstituted phenyl group.

As a further preferred embodiment, Ar¹¹ is an unsubstituted phenyl group, and Ar¹² is a phenyl group having a monocyclic group or a fused ring group as a substituent, and Ar¹¹ and Ar¹² are independently a phenyl group having a monocyclic group or a fused ring group as a substituent.

The preferable specific examples of the monocyclic group and fused ring group as a substituent are the same as those described above. As the monocyclic group as a substituent, a phenyl group and biphenyl group are further preferable. As the fused ring group as a substituent, a naphthyl group, phenanthryl group, 9,9-dimethylfluorenyl group, dibenzofuranyl group and benzanthryl group are further preferable.

### Hole/exciton blocking layer (f):

Blocking layers may be used to reduce the number of charge carriers (electrons or holes) and/or excitons that leave the emissive layer. The hole blocking layer may be disposed between the emitting layer (e) and electron transport layer (g), to block holes from leaving layer (e) in the direction of electron transport layer (g). Blocking layers may also be used to block excitons from diffusing out of the emissive layer.

According to one embodiment of the present invention, at least one compound according to general formulae (IIb) and (IIc) is present in the hole/exciton blocking layer.

Additional hole blocker materials typically used in OLEDs are 2,6-bis(N-carbazolyl)pyridine (mCPy), 2,9-dimethyl-4,7-diphenyl-1,10-phenanthroline (bathocuproin, (BCP)), bis(2-methyl-8-quinolinato)-4-phenylphenylato)aluminum(III) (BAlq), phenothiazine *S,S-*dioxide derivates and 1,3,5-tris(N-phenyl-2-benzylimidazolyl)benzene) (TPBI), TPBI also being suitable as electron-transport material. Further suitable hole blockers and/or electron conductor materials are 2,2',2"-(1 ,3,5-benzenetriyl)tris(1-phenyl-1-H-benzimidazole), 2-(4-biphenylyl)-5-(4-tert-butylphenyl)-1,3,4-oxadiazole, 8-hydroxyquinolinolatolithium, 4-(naphthalen-1-yl)-3,5-diphenyl-4H-1,2,4-triazole, 1,3-bis[2-(2,2'-bipyridin-6-yl)-1,3,4-oxadiazo-5-yl]benzene, 4,7-diphenyl-1,10-phenanthroline, 3-(4-biphenylyl)-4-phenyl-5-tert-butylphenyl-1,2,4-triazole, 6,6'-bis[5-(biphenyl-4-yl)-1,3,4-oxadiazo-2-yl]-2,2'-bipyridyl, 2-phenyl-9,10-di(naphthalene-2-yl)anthracene, 2,7-bis[2-(2,2'-bipyridin-6-yl)-1,3,4-oxadiazo-5-yl]-9,9-dimethylfluorene, 1,3-bis[2-(4-tert-butylphenyl)-1,3,4-oxadiazo-5-yl]benzene, 2-(naphthalene-2-yl)-4,7-diphenyl-1,10-phenanthroline, tris(2,4,6-trimethyl-3-(pyridin-3-yl)phenyl)borane, 2,9-bis(naphthalene-2-yl)-4,7-diphenyl-1,10-phenanthroline, 1-methyl-2-(4-(naphthalene-2-yl)phenyl)-1H-imidazo[4,5-f][1,10]-phenanthroline. In a further embodiment, it is possible to use compounds which comprise aromatic or heteroaromatic rings joined via groups comprising carbonyl groups, as disclosed in WO2006/100298, disilyl compounds selected from the group consisting of disilylcarbazoles, disilylbenzofurans, disilylbenzothiophenes, disilylbenzophospholes, disilylbenzothiophene S-oxides and disilylbenzothiophene S,S-dioxides, as specified, for example, in PCT applications WO2009/003919 and WO2009003898 and disilyl compounds as disclosed in WO2008/034758, as a blocking layer for holes/excitons (f).

In another preferred embodiment compounds **(SH-1), (SH-2), (SH-3), SH-4, SH-5, SH-6, (SH-7), (SH-8), (SH-9), (SH-10)** and **(SH-11)** may be used as hole/exciton blocking materials.

### Electron transport layer (g):

Electron transport layer may include a material capable of transporting electrons. Electron transport layer may be intrinsic (undoped), or doped. Doping may be used to enhance conductivity.

The compounds according to general formulae (IIb) and (IIc) according to the present invention are suitable as electron transport material, either alone or in combination with one or more of the electron transport materials mentioned below. The compounds according to general formulae (IIb) and (IIc) according to the present invention are preferably suitable as electron transport material, if a blue fluorescent emitter is present in the emitting layer.

Further suitable electron-transporting materials for layer (g) of the inventive OLEDs, which may be used in combination with the compounds of general formulae (IIb) and (IIc) according to the present invention or in absence of the compounds of general formulae (IIb) and (IIc) according to the present invention as electron transport material, comprise metals chelated with oxinoid compounds, such as tris(8-hydroxyquinolato)aluminum (Alq₃), compounds based on phenanthroline such as 2,9-dimethyl-4,7-diphenyl-1,10-phenanthroline (DDPA = BCP), 4,7-diphenyl-1,10-phenanthroline (Bphen), 2,4,7,9-tetraphenyl-1,10-phenanthroline, 4,7-diphenyl-1,10-phenanthroline (DPA) or phenanthroline derivatives disclosed in EP1786050, in EP1970371, or in EP1097981, and azole compounds such as 2-(4-biphenylyl)-5-(4-t-butylphenyl)-1,3,4-oxadiazole (PBD) and 3-(4-biphenylyl)-4phenyl-5-(4-t-butylphenyl)-1,2,4-triazole (TAZ).

Further suitable electron transport materials, which may be used in combination with the compounds of general formulae (IIb) and (IIc) according to the present invention or in absence of the compounds of general formulae (lib) and (IIc) according to the present invention as electron transport material, are mentioned in Abhishek P. Kulkarni, Christopher J. Tonzola, Amit Babel, and Samson A. Jenekhe, Chem. Mater. 2004, 16, 4556-4573; G. Hughes, M. R. Bryce, J. Mater. Chem. 2005, 15, 94-107 and Yasuhiko Shirota and Hiroshi Kageyama, Chem. Rev. 2007, 107, 953-1010 *(ETM, HTM).*

It is likewise possible to use mixtures of at least two materials in the electron-transporting layer, in which case at least one material is electron-conducting. Preferably, in such mixed electron-transport layers, at least one phenanthroline compound is used, preferably BCP, or at least one pyridine compound according to the formula (**XVI**) below, preferably a compound of the formula (XVIa) below. More preferably, in mixed electron-transport layers, in addition to at least one phenanthroline compound, alkaline earth metal or alkali metal hydroxyquinolate complexes, for example Liq, are used. Suitable alkaline earth metal or alkali metal hydroxyquinolate complexes are specified below (**formula XVII**). Reference is made to WO2011/157779.

The electron-transport layer may also be electronically doped in order to improve the transport properties of the materials used, in order firstly to make the layer thicknesses more generous (avoidance of pinholes/short circuits) and in order secondly to minimize the operating voltage of the device. Electronic doping is known to those skilled in the art and is disclosed, for example, in W. Gao, A. Kahn, J. Appl. Phys., Vol. 94, No. 1, 1 July 2003 (p-doped organic layers); A. G. Werner, F. Li, K. Harada, M. Pfeiffer, T. Fritz, K. Leo, Appl. Phys. Lett., Vol. 82, No. 25, 23 June 2003 and Pfeiffer et al., Organic Electronics 2003, 4, 89 - 103 and K. Walzer, B. Maennig, M. Pfeiffer, K. Leo, Chem. Soc. Rev. 2007, 107, 1233. For example, it is possible to use mixtures which lead to electrical n-doping of the electron-transport layer. n-Doping is achieved by the addition of reducing materials. These mixtures may, for example, be mixtures of the abovementioned electron transport materials with alkali/alkaline earth metals or alkali/alkaline earth metal salts, for example Li, Cs, Ca, Sr, Cs₂CO₃, with alkali metal complexes, for example 8-hydroxyquinolatolithium (Liq), and with Y, Ce, Sm, Gd, Tb, Er, Tm, Yb, Li₃N, Rb₂CO₃, dipotassium phthalate, W(hpp)₄ from EP1786050, or with compounds described in EP1837926B1, EP1837927, EP2246862 and WO2010132236.

In a preferred embodiment, the electron-transport layer comprises at least one compound of the general formula (**XVII**) 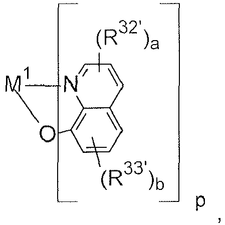 in which
R^{32'} and R^{33'} are each independently F, C₁-C₈-alkyl, or C₆-C₁₄-aryl, which is optionally substituted by one or more C₁-C₈-alkyl groups, or
two R^{32'} and/or R^{33'} substituents together form a fused benzene ring which is optionally substituted by one or more C₁-C₈-alkyl groups;
a and b are each independently 0, or 1, 2 or 3,
M¹ is an alkaline metal atom or alkaline earth metal atom,
p is 1 when M¹ is an alkali metal atom, p is 2 when M¹ is an earth alkali metal atom.

A very particularly preferred compound of the formula (**XVII**) is 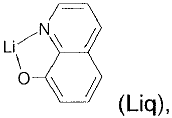 which may be present as a single species, or in other forms such as Li_{g}Q_{g} in which g is an integer, for example Li₆Q₆. Q is an 8-hydroxyquinolate ligand or an 8-hydroxyquinolate derivative.

In a further preferred embodiment, the electron-transport layer comprises at least one compound of the formula (**XVI**), 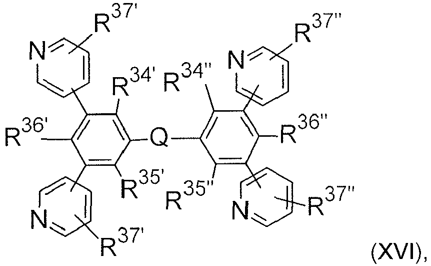 in which
R^{34"}, R^{35"}, R^{36"}, R^{37"}, R^{34'}, R^{35'} R^{36'} and R^{37'} are each independently H, C₁-C₁₈-alkyl, C₁-C₁₈-alkyl which is substituted by E' and/or interrupted by D', C₆-C₂₄-aryl, C₆-C₂₄-aryl which is substituted by G', C₂-C₂₀-heteroaryl or C₂-C₂₀-heteroaryl which is substituted by G',
Q is an arylene or heteroarylene group, each of which is optionally substituted by G';
D' is -CO-; -COO-; -S-; -SO-; -SO₂-; -O-; -NR^{40'}-; -SiR^{45'}R^{46'}-; -POR^{47'}-; -CR^{38'}=CR^{39'}-; or -C≡C-; E' is -OR^{44'}; -SR^{44'}; -NR^{40'}R^{41'}; -COR^{43'}; -COOR^{42'}; -CONR^{40'}R^{41'}; -CN; or F;
G' is E', C₁-C₁₈-alkyl, C₁-C₁₈-alkyl which is interrupted by D', C₁-C₁₈-perfluoroalkyl, C₁-C₁₈-alkoxy, or C₁-C₁₈-alkoxy which is substituted by E' and/or interrupted by D', in which R^{38'} and R^{39'} are each independently H, C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-;
R^{40'} and R^{41'} are each independently C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-; or
R^{40'} and R^{41'} together form a 6-membered ring;
R^{42'} and R^{43'} are each independently C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-,
R^{44'} is C₆-C₁₈-aryl; C₆-C₁₈-aryl which is substituted by C₁-C₁₈-alkyl or C₁-C₁₈-alkoxy; C₁-C₈-alkyl; or C₁-C₁₈-alkyl which is interrupted by -O-,
R^{45'} and R^{46'} are each independently C₁-C₁₈-alkyl, C₆-C₁₈-aryl or C₆-C₁₈-aryl which is substituted by C₁-C₁₈-alkyl,
R^{47'} is C₁-C₁₈-alkyl, C₆-C₁₈-aryl or C₆-C₁₈-aryl which is substituted by C₁-C₁₈-alkyl.

Preferred compounds of the formula (**XVI**) are compounds of the formula (**XVIa**) 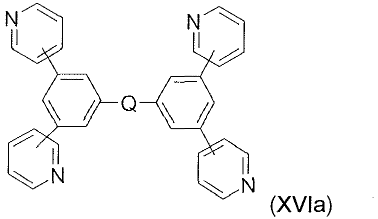 in which Q is: 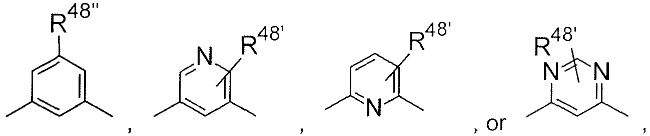
R^{48'} is H or C₁-C₁₈-alkyl and
R^{48"} is H, C₁-C₁₈-alkyl or

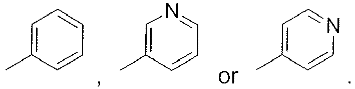

Particular preference is given to a compound of the formula

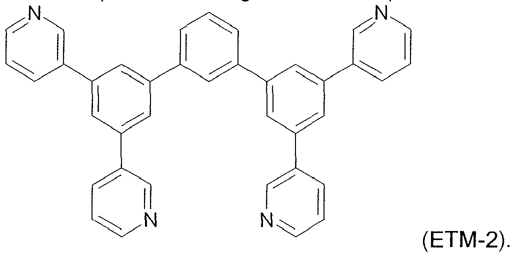

In a further, very particularly preferred embodiment, the electron-transport layer comprises a compound Liq and a compound **ETM-2.**

In a preferred embodiment, the electron-transport layer comprises at least one compound of the formula (**XVII**) in an amount of 99 to 1% by weight, preferably 75 to 25% by weight, more preferably about 50% by weight, and at least one compound of the formula (**XVI**) in an amount of 1 to 99% by weight, preferably 25 to 75% by weight, more preferably about 50% by weight, where the amount of the compounds of the formulae (**XVII**) and the amount of the compounds of the formulae (**XVI**) adds up to a total of 100% by weight.

The preparation of the compounds of the formula (**XVI**) is described in J. Kido et al., Chem. Commun. (2008) 5821-5823, J. Kido et al., Chem. Mater. 20 (2008) 5951-5953 and JP2008/127326, or the compounds can be prepared analogously to the processes disclosed in the aforementioned documents.

It is likewise possible to use mixtures of alkali metal hydroxyquinolate complexes, preferably Liq, and dibenzofuran compounds in the electron-transport layer. Reference is made to WO2011/157790. Dibenzofuran compounds **A-1** to **A-36** and **B-1** to **B-22** described in WO2011/157790 are preferred, wherein dibenzofuran compound 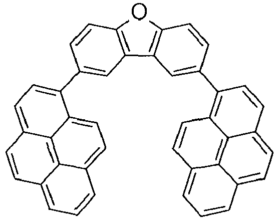 (**A-10;** = **ETM-1**) is most preferred.

In a preferred embodiment, the electron-transport layer comprises **Liq** in an amount of 99 to 1% by weight, preferably 75 to 25% by weight, more preferably about 50% by weight, and at least one dibenzofuran compound in an amount of 1 to 99% by weight, preferably 25 to 75% by weight, more preferably about 50% by weight, where the amount of **Liq** and the amount of the dibenzofuran compound(s), especially **ETM-1,** adds up to a total of 100% by weight.

In a preferred embodiment, the electron-transport layer comprises at least one phenanthroline derivative and/or pyridine derivative.

In a further preferred embodiment, the electron-transport layer comprises at least one phenanthroline derivative and/or pyridine derivative and at least one alkali metal hydroxyquinolate complex.

In a further preferred embodiment, the electron-transport layer comprises at least one of the dibenzofuran compounds **A-1** to **A-36** and **B-1** to **B-22** described in WO2011/157790, especially **ETM-1.**

In a further preferred embodiment, the electron-transport layer comprises a compound described in WO2012/111462, WO2012/147397, WO2012014621, such as, for example, a compound of formula 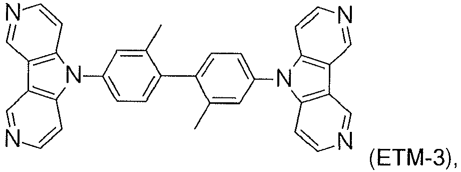 US2012/0261654 , such as, for example, a compound of formula 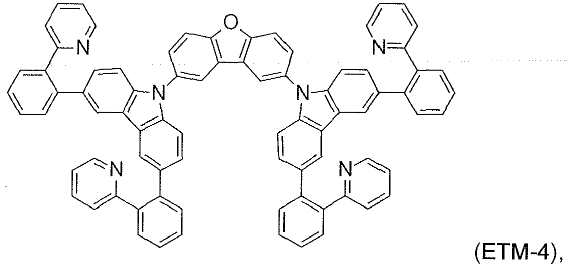 and WO2012/115034, such as for example, such as, for example, a compound of formula

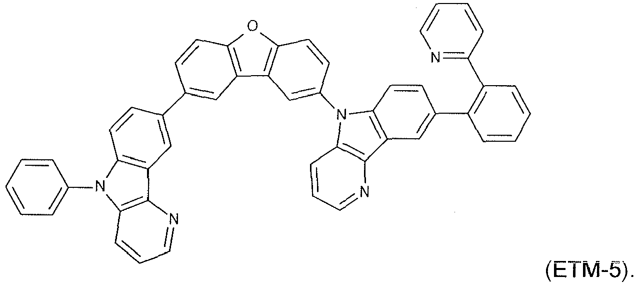

Further preferred embodiments of the electron injection layer of the OLED according to the present invention are mentioned in US 2013306955.

For example, the electron transporting material that may be present in the electron transporting layer of the OLED according to the present invention is an electron transporting material represented by formula (1):

A1(-L1-L2-L3-L4-Ar1)m (1)

wherein:
each of L1, L2, L3, and L4 independently represents a single bond, a substituted or unsubstituted alkylene group having 1 to 50 carbon atoms, a substituted or unsubstituted alkenylene group having 1 to 50 carbon atoms, a substituted or unsubstituted alkynylene group having 1 to 50 carbon atoms, a substituted or unsubstituted cycloalkylene group having 3 to 50 ring carbon atoms, a substituted or unsubstituted arylene group having 6 to 30 ring carbon atoms, or a substituted or unsubstituted heteroarylene group having 5 to 30 ring atoms;
Ar1 represents a substituted or unsubstituted alkyl group having 1 to 50 carbon atoms, a substituted or unsubstituted cycloalkyl group having 3 to 50 ring carbon atoms, a substituted or unsubstituted aryl group having 6 to 30 ring carbon atoms, or a substituted or unsubstituted heteroaryl group having 5 to 30 ring atoms;
A1 represents an m-valent residue of a ring-containing compound represented by formula (2); and
m represents an integer of 1 or more: 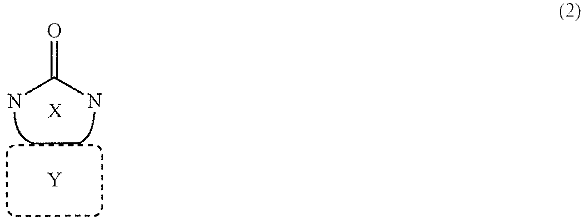 wherein:
   ring X is a substituted or unsubstituted, saturated or unsaturated 5- to 8-membered ring having a ring nitrogen atom and a ring carbon atom;
   the ring X may be fused to one or more rings Y; and
   the ring Y represents a substituted or unsubstituted hydrocarbon ring or a substituted or unsubstituted heteroring;

The ring Y preferably represents a substituted or unsubstituted non-fused aromatic hydrocarbon ring having 6 to 30 ring carbon atoms, a substituted or unsubstituted fused aromatic hydrocarbon ring having 10 to 30 ring carbon atoms, a substituted or unsubstituted non-fused heteroring having 5 to 30 ring atoms, or a substituted or unsubstituted fused heteroring having 10 to 30 ring atoms.

The electron transporting material of the invention is preferably represented by formula (1-1) or (1-2):

A11(-L11-L21-L31-L41-Ar11)p (1-1)

wherein:
each of L11, L21, L31, and L41 independently represents a single bond, a substituted or unsubstituted alkylene group having 1 to 50 carbon atoms, a substituted or unsubstituted alkenylene group having 1 to 50 carbon atoms, a substituted or unsubstituted alkynylene group having 1 to 50 carbon atoms, a substituted or unsubstituted cycloalkylene group having 3 to 50 ring carbon atoms, a substituted or unsubstituted arylene group having 6 to 30 ring carbon atoms, or a substituted or unsubstituted heteroarylene group having 5 to 30 ring atoms;
Ar11 represents a substituted or unsubstituted alkyl group having 1 to 50 carbon atoms, a substituted or unsubstituted cycloalkyl group having 3 to 50 ring carbon atoms, a substituted or unsubstituted aryl group having 6 to 30 ring carbon atoms, or a substituted or unsubstituted heteroaryl group having 5 to 30 ring atoms;
A11 represents a p-valent residue of a ring-containing compound represented by formula (2-1); and
p represents an integer of 1 or more: 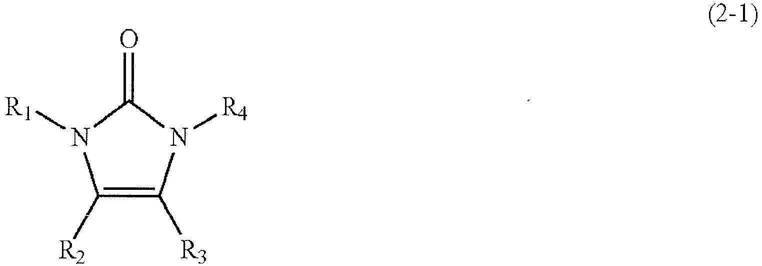 wherein;
   each of R1 to R4 independently represents a hydrogen atom, a substituted or unsubstituted alkyl group having 1 to 50 carbon atoms, a substituted or unsubstituted alkoxy group having 1 to 50 carbon atoms, a substituted or unsubstituted alkoxycarbonyl group having 1 to 50 carbon atoms, a substituted or unsubstituted cycloalkyl group having 3 to 50 ring carbon atoms, a substituted or unsubstituted aryl group having 6 to 30 ring carbon atoms, a substituted or unsubstituted aralkyl group having 6 to 50 ring carbon atoms, a substituted or unsubstituted aryloxy group having 6 to 50 ring carbon atoms, a substituted or unsubstituted heteroaryl group having 5 to 30 ring atoms, a substituted or unsubstituted alkylsilyl group having 1 to 50 carbon atoms, a substituted or unsubstituted arylsilyl group having 6 to 50 ring carbon atoms, a substituted or unsubstituted haloalkyl group having 1 to 50 carbon atoms, a substituted or unsubstituted alkenyl group having 1 to 50 carbon atoms, a substituted or unsubstituted alkynyl group having 1 to 50 carbon atoms, a substituted or unsubstituted alkylamino group having 1 to 50 carbon atoms, a substituted or unsubstituted arylamino group having 6 to 30 ring carbon atoms, a substituted or unsubstituted heteroarylamino group having 5 to 30 ring atoms; a substituted or unsubstituted acylamino group having 2 to 50 carbon atoms, a substituted or unsubstituted heteroaryloxy group having 5 to 30 ring atoms, a substituted or unsubstituted acyl group having 2 to 50 carbon atoms, a substituted or unsubstituted aryloxycarbonyl group having 6 to 30 ring carbon atoms, a substituted or unsubstituted alkylcarbonyl group having 1 to 50 carbon atoms, a mercapto group, a substituted or unsubstituted alkylthio group having 1 to 50 carbon atoms, a substituted or unsubstituted arylthio group having 6 to 30 ring carbon atoms, a sulfonyl group, a boryl group, a phosphino group, an amino group, a halogen atom, a cyano group, a nitro group, a hydroxyl group, a carboxyl group, or a valence bonded to L11; or a pair of R1 and R2, R2 and R3, or R3 and R4 are bonded to each other to form a ring Y represented by a substituted or unsubstituted hydrocarbon ring or a substituted or unsubstituted heteroring.

A11 of formula (1-1) preferably represents a p-valent residue of a compound represented by formula (2-1-1), (2-1-2), or (2-1-3): 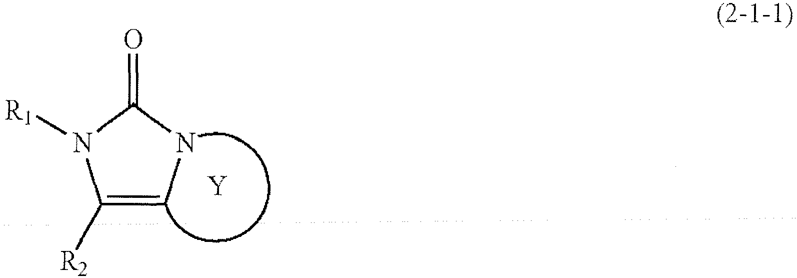 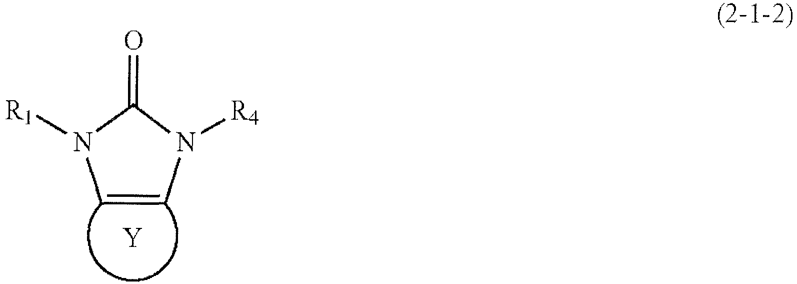 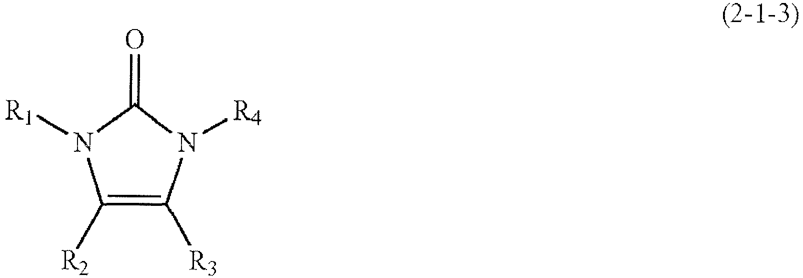 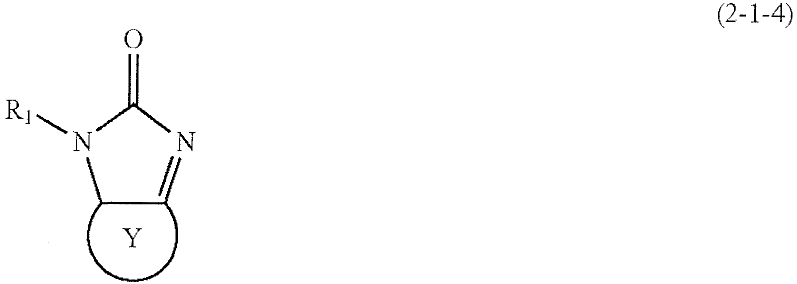 wherein:
each of R1 to R4 independently represents a hydrogen atom, a substituted or unsubstituted alkyl group having 1 to 50 carbon atoms, a substituted or unsubstituted alkoxy group having 1 to 50 carbon atoms, a substituted or unsubstituted alkoxycarbonyl group having 1 to 50 carbon atoms, a substituted or unsubstituted cycloalkyl group having 3 to 50 ring carbon atoms, a substituted or unsubstituted aryl group having 6 to 30 ring carbon atoms, a substituted or unsubstituted aralkyl group having 6 to 50 ring carbon atoms, a substituted or unsubstituted aryloxy group having 6 to 50 ring carbon atoms, a substituted or unsubstituted arylthio group having 6 to 50 ring carbon atoms, a substituted or unsubstituted heteroaryl group having 5 to 30 ring atoms, a substituted or unsubstituted alkylsilyl group having 1 to 50 carbon atoms, a substituted or unsubstituted arylsilyl group having 6 to 50 ring carbon atoms, an amino group substituted by a substituted or unsubstituted alkyl group having 1 to 50 carbon atoms, a substituted or unsubstituted haloalkyl group having 1 to 50 carbon atoms, a substituted or unsubstituted alkenyl group having 1 to 50 carbon atoms, a substituted or unsubstituted alkynyl group having 1 to 50 carbon atoms, a substituted or unsubstituted alkylamino group having 1 to 50 carbon atoms, a substituted or unsubstituted arylamino group having 6 to 30 ring carbon atoms, a substituted or unsubstituted heteroarylamino group having 5 to 30 ring atoms, a substituted or unsubstituted acylamino group having 2 to 50 carbon atoms, a substituted or unsubstituted heteroaryloxy group having 5 to 30 ring atoms, a substituted or unsubstituted acyl group having 2 to 50 carbon atoms, a substituted or unsubstituted aryloxycarbonyl group having 6 to 30 ring carbon atoms, a substituted or unsubstituted alkylcarbonyl group having 1 to 50 carbon atoms, mercapto group, a substituted or unsubstituted alkylthio group having 1 to 50 carbon atoms, a substituted or unsubstituted arylthio group having 6 to 30 ring carbon atoms, a sulfonyl group, an boryl group, a phosphino group, an amino group, a halogen atom, a cyano group, a nitro group, a hydroxyl group, a carboxyl group, or a valence bonded to L11; and
Y represents the ring Y.

Further, A11 preferably represents a p-valent residue of a compound represented by formula (2-1-2-1): 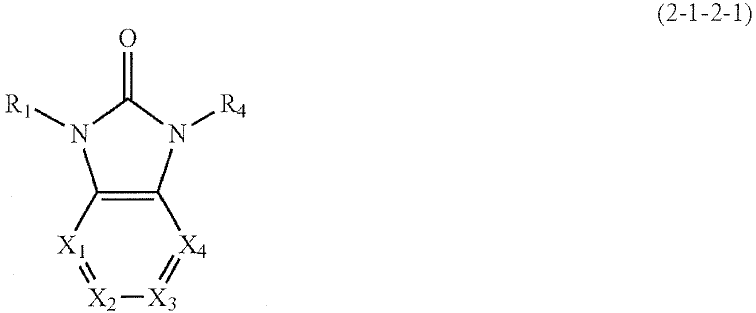 wherein:
each of X1 to X4 independently represents CR5 or N;
each of R1, R4, and R5 independently represents a hydrogen atom, a substituted or unsubstituted alkyl group having 1 to 50 carbon atoms, a substituted or unsubstituted alkoxy group having 1 to 50 carbon atoms, a substituted or unsubstituted alkoxycarbonyl group having 1 to 50 carbon atoms, a substituted or unsubstituted cycloalkyl group having 3 to 50 ring carbon atoms, a substituted or unsubstituted aryl group having 6 to 30 ring carbon atoms, a substituted or unsubstituted aralkyl group having 6 to 50 ring carbon atoms, a substituted or unsubstituted aryloxy group having 6 to 50 ring carbon atoms, a substituted or unsubstituted heteroaryl group having 5 to 30 ring atoms, a substituted or unsubstituted alkylsilyl group having 1 to 50 carbon atoms, a substituted or unsubstituted arylsilyl group having 6 to 50 ring carbon atoms, a substituted or unsubstituted haloalkyl group having 1 to 50 carbon atoms, a substituted or unsubstituted alkenyl group having 1 to 50 carbon atoms, a substituted or unsubstituted alkynyl group having 1 to 50 carbon atoms, a substituted or unsubstituted alkylamino group having 1 to 50 carbon atoms, a substituted or unsubstituted arylamino group having 6 to 30 ring carbon atoms, a substituted or unsubstituted heteroarylamino group having 5 to 30 ring atoms, a substituted or unsubstituted acylamino group having 2 to 50 carbon atoms, a substituted or unsubstituted heteroaryloxy group having 5 to 30 ring atoms, a substituted or unsubstituted acyl group having 2 to 50 carbon atoms, a substituted or unsubstituted aryloxycarbonyl group having 6 to 30 ring carbon atoms, a substituted or unsubstituted alkylcarbonyl group having 1 to 50 carbon atoms, a mercapto group, a substituted or unsubstituted alkylthio group having 1 to 50 carbon atoms, a substituted or unsubstituted arylthio group having 6 to 30 ring carbon atoms, a sulfonyl group, an boryl group, a phosphino group, an amino group, a halogen atom, a cyano group, a nitro group, a hydroxyl group, a carboxyl group, or a valence bonded to L11; or R1, R4, and R5 are each bonded to each other to form a ring which forms a part of the ring Y.

In particular, the electron transporting layer of the OLED according to the present invention, between the light emitting layer and the cathode, preferably comprises at least one compound of the general formula (IIb) or (IIc).

In a preferred embodiment, the electron transporting layer comprising at least one compound of the general formula (lib) or (IIc) further comprises a reducing dopant.

Examples of the reducing dopant include a donating metal, a donating metal compound, and a donating metal complex. The reducing dopant may be used alone or in combination of two or more.

The reducing dopant referred to herein is an electron-donating material. The electron-donating material is a material which generates radical anions by the interaction with a coexisting organic material in the electron transporting layer or an organic material in a layer adjacent to the electron transporting layer, or a material having an electron-donating radical.

The donating metal is a metal having a work function of 3.8 eV or less, preferably an alkali metal, an alkaline earth metal, or a rare earth metal, and more preferably Cs, Li, Na, Sr, K, Mg, Ca, Ba, Yb, Eu, or Ce.

The donating metal compound is a compound comprising the above donating metal, preferably a compound comprising an alkali metal, an alkaline earth metal, or a rare earth metal, and more preferably a halide, an oxide, a carbonate, or a borate of these metals, for example, a compound represented by MOₓ (M: donating metal, x: 0.5 to 1.5), MFₓ (x: 1 to 3), or M(CO₃)ₓ (x: 0.5 to 1.5).

The donating metal complex is a complex comprising the above donating metal, preferably an organic metal complex of an alkali metal, an alkaline earth metal or a rare earth metal, and more preferably an organic metal complex represented by formula (I):

MQₙ (I)

wherein M is a donating metal, Q is a ligand, preferably a carboxylic acid derivative, a diketone derivative, or a quinoline derivative, and n is an integer of 1 to 4.

Examples of the donating metal complex include watermill-shaped tungsten compounds described in JP 2005-72012A and phthalocyanine compounds having an alkali metal or an alkaline earth metal as the central metal, which are described in JP 11-345687A.

The reducing dopant is preferably at least one selected from the group consisting of an alkali metal, an alkaline earth metal, a rare earth metal, an alkali metal oxide, an alkali metal halide, an alkaline earth metal oxide, an alkaline earth metal halide, a rare earth metal oxide, a rare earth metal halide, an organic complex having an alkali metal, an organic complex having an alkaline earth metal, and an organic complex having a rare earth metal, and more preferably a 8-quinolinol complex of an alkali metal.

Examples of the alkali metal includes:
Li (lithium, work function: 2.93 eV),
Na (sodium, work function: 2.36 eV),
K (potassium, work function: 2.3 eV),
Rb (rubidium, work function: 2.16 eV), and
Cs (cesium, work function: 1.95 eV).

The values of work functions are based on Handbook of Chemistry (Pure Chemistry II, 1984, p. 493, edited by The Chemical Society of Japan). The same applies hereafter
Preferred examples of the alkaline earth metals are:
Ca (calcium, work function: 2.9 eV),
Mg (magnesium, work function: 3.66 eV),
Ba (barium, work function: 2.52 eV), and
Sr (strontium, work function: 2.0 to 2.5 eV).

The work function of strontium is based on Physics of Semiconductor Device (N.Y., Wiley, 1969, p. 366).

Preferred examples of the rare earth metal are:
Yb (ytterbium, work function: 2.6 eV),
Eu (europium, work function: 2.5 eV),
Gd (gadolinium, work function: 3.1 eV), and
Er (erbium, work function: 2.5 eV).

Examples of the alkali metal oxide include Li₂O, LiO, and NaO. The alkaline earth metal oxide is preferably CaO, BaO, SrO, BeO, or MgO.

Examples of the alkali metal halide include a fluoride, for example, LiF, NaF, CsF, and KF and a chloride, for example, LiCI, KCI, and NaCl.

The alkaline earth metal halide is preferably a fluoride, such as CaF₂, BaF₂, SrF₂, MgF₂, and BeF₂ and a halide other than fluoride.

An OLED wherein at least one compound according to general formula (lib) or (IIc) used in the electron transporting layer is particularly preferred because the driving voltage is reduced while increasing the efficiency.

The content of the at least one compound according to general formula (IIb) or (IIc) in the electron transporting layer is preferably 50% by mass or more and more preferably 60% by mass or more.

The electron transporting layer facilitates the injection of electrons into the light emitting layer and transports the electrons to the light emitting zone, and has a large electron mobility and an electron affinity generally as large as 2.5 eV or more. The electron transporting layer is preferably formed from a material capable of transporting electrons to the light emitting layer at a lower strength of electric field, preferably having an electron mobility of, for example, at least 10⁻⁶ cm²/V·s under an electric field of 10⁴ to 10⁶ V/cm.

When the of the at least one compound according to general formula (IIb) or (IIc) is used in the electron transporting layer, the electron transporting layer may be formed from the of the at least one compound according to general formula (lib) or (IIc) alone or in combination with another material.

The material for forming the electron injecting/transporting layer in combination with the of the at least one compound according to general formula (lib) or (IIc) is not particularly limited as long as having the preferred properties mentioned above and may be selected from those commonly used as the electron transporting material in the field of photoconductive materials and those known as the materials for the electron injecting/transporting layer of organic EL devices.

In the present invention, an electron injecting layer including an insulating material or a semiconductor may be disposed between the cathode and the organic layer. By such an electron injecting layer, the leak of electric current is effectively prevented to improve the electron injecting ability. Preferred examples of the insulating material include at least one metal compound selected from the group consisting of an alkali metal chalcogenide, an alkaline earth metal chalcogenide, an alkali metal halide, and an alkaline earth metal halide. An electron injecting layer including the above alkali metal chalcogenide is preferred because the electron injecting property is further improved. Preferred alkali metal chalcogenides include Li₂O, K₂O, Na₂S, Na₂Se, and Na₂O; preferred alkaline earth metal chalcogenides include CaO, BaO, SrO, BeO, BaS, and CaSe; preferred alkali metal halides include LiF, NaF, KF, LiCI, KCI, and NaCl; and preferred alkaline earth metal halides include fluoride such as CaF₂, BaF₂, SrF₂, MgF₂, and BeF₂ and halides other than fluoride.

Examples of the semiconductor for the electron transporting layer include an oxide, a nitride and an oxynitride of at least one element selected from Ba, Ca, Sr, Yb, Al, Ga, In, Li, Na, Cd, Mg, Si, Ta, Sb, and Zn, which are used singly or in combination of two or more. It is preferred that the inorganic compound constituting the electron transporting layer forms a microcrystalline or amorphous insulating thin film. When constituted of the insulating thin film described above, the electron injecting layer is made more uniform to reduce the pixel defect such as dark spots. Examples of such a inorganic compound include the alkali metal chalcogenide, the alkaline earth metal chalcogenide, the alkali metal halide and the alkaline earth metal halide which are described above.

### Electron injection layer (h):

The electron injection layer may be any layer that improves the injection of electrons into an adjacent organic layer.

Lithium-comprising organometallic compounds such as 8-hydroxyquinolatolithium (Liq), CsF, NaF, KF, Cs₂CO₃ or LiF may be applied between the electron transport layer (g) and the cathode (i) as an electron injection layer (h) in order to reduce the operating voltage.

### Cathode (i):

The cathode (i) is an electrode which serves to introduce electrons or negative charge carriers. The cathode may be any metal or nonmetal which has a lower work function than the anode. Suitable materials for the cathode are selected from the group consisting of alkali metals of group 1, for example Li, Cs, alkaline earth metals of group 2, metals of group 12 of the Periodic Table of the Elements, comprising the rare earth metals and the lanthanides and actinides. In addition, metals such as aluminum, indium, calcium, barium, samarium and magnesium, and combinations thereof, may be used.

In general, the different layers, if present, have the following thicknesses:
anode (a): 500 to 5000 Å (ångström), preferably 1000 to 2000 Å;
hole injection layer (b): 50 to 1000 Å, preferably 200 to 800 Å,
hole-transport layer (c): 50 to 1000 Å, preferably 100 to 800 Å,
exciton blocking layer (d): 10 to 500 Å, preferably 50 to 100 Å,
light-emitting layer (e): 10 to 1000 Å, preferably 50 to 600 Å,
hole/ exciton blocking layer (f): 10 to 500 Å, preferably 50 to 100 Å,
electron-transport layer (g): 50 to 1000 Å, preferably 200 to 800 Å,
electron injection layer (h): 10 to 500 Å, preferably 20 to 100 Å,
cathode (i): 200 to 10 000 Å, preferably 300 to 5000 Å.

The person skilled in the art is aware (for example on the basis of electrochemical studies) of how suitable materials have to be selected. Suitable materials for the individual layers are known to those skilled in the art and are disclosed, for example, in WO 00/70655.

In addition, it is possible that some of the layers used in the inventive OLED have been surface-treated in order to increase the efficiency of charge carrier transport. The selection of the materials for each of the layers mentioned is preferably determined by obtaining an OLED with a high efficiency and lifetime.

The inventive OLED can be produced by methods known to those skilled in the art. In general, the inventive OLED is produced by successive vapor deposition of the individual layers onto a suitable substrate. Suitable substrates are, for example, glass, inorganic semiconductors or polymer films. For vapor deposition, it is possible to use customary techniques, such as thermal evaporation, chemical vapor deposition (CVD), physical vapor deposition (PVD) and others. In an alternative process, the organic layers of the OLED can be applied from solutions or dispersions in suitable solvents, employing coating techniques known to those skilled in the art.

Use of the compounds according to general formulae (IIb) and (IIc) in at least one layer of the OLED, preferably in the light-emitting layer, preferably as a host material, a charge transporting material, particularly preferably as a host material and hole or electron transporting material, makes it possible to obtain OLEDs with high efficiency and with low use and operating voltage. Frequently, the OLEDs obtained by the use of the compounds according to general formulae (lib) and (IIc) additionally have high lifetimes. The efficiency of the OLEDs can additionally be improved by optimizing the other layers of the OLEDs. For example, high-efficiency cathodes such as Ca or Ba, if appropriate in combination with an intermediate layer of LiF, can be used. Moreover, additional layers may be present in the OLEDs in order to adjust the energy level of the different layers and to facilitate electroluminescence.

The OLEDs may further comprise at least one second light-emitting layer. The overall emission of the OLEDs may be composed of the emission of the at least two light-emitting layers and may also comprise white light.

The OLEDs can be used in all apparatus in which electroluminescence is useful. Suitable devices are preferably selected from stationary and mobile visual display units and illumination units. Stationary visual display units are, for example, visual display units of computers, televisions, visual display units in printers, kitchen appliances and advertising panels, illuminations and information panels. Mobile visual display units are, for example, visual display units in cellphones, tablet PCs, laptops, digital cameras, MP3 players, vehicles and destination displays on buses and trains. Further devices in which the inventive OLEDs can be used are, for example, keyboards; items of clothing; furniture; wallpaper. In addition, the present invention relates to a device selected from the group consisting of stationary visual display units such as visual display units of computers, televisions, visual display units in printers, kitchen appliances and advertising panels, illuminations, information panels, and mobile visual display units such as visual display units in cellphones, tablet PCs, laptops, digital cameras, MP3 players, vehicles and destination displays on buses and trains; illumination units; keyboards; items of clothing; furniture; wallpaper, comprising at least one inventive organic light-emitting diode or at least one inventive light-emitting layer.

The following examples are included for illustrative purposes. Unless otherwise stated, all parts and percentages are by weight.

### Examples

### Compounds synthesized

### Compound 1

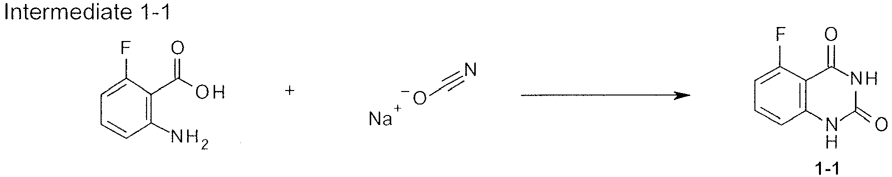

2-Amino-6-fluorobenzoic acid (35 g, 225.6 mmol) is dissolved in 885 ml of water and 26 mL of acetic acid. The mixture is stirred at 35 °C for 15 min. After that, sodium cyanate (36.67 g, 564 mmol) dissolved in 442 mL of water is added dropwise to the suspension, and the mixture is stirred at 35 °C for 30 min. Then, sodium hydroxide (180.49 g / 4.51 mol) is slowly added to the reaction mixture, the mixture is cooled at room temperature. After 374 mL of hydrogen chloride is added there, the precipitate is collected by filtration and washed with water. The solid is dried in a vacuum oven to yield 37.19 (91.5%) of 1-1 as white solid.
¹H NMR (300 MHz, DMSO-*d*₆) δ 11.25 (s, 1H), 7.65-7.57 (m, 1H), 6.99-6.88 (m, 2H). LC-MS (m/z): 179

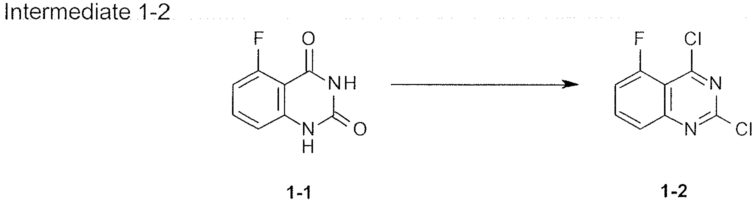

1-1 (2.6 g, 14.43 mmol) is suspended in 29 mL of toluene and heated to 50 °C. Phosphoryl chloride (9.88 mL, 108.25 mmol) is added dropwise, and then DBU (4.31 mL, 28.87 mmol) is added dropwise. The mixture is stirred vigorously at 120 °C for overnight. After the reaction mixture is cooled at room temperature, it is added dropwise to ice-water. The aqueous layer is extracted with ethyl acetate. After it is washed with brine and dried with Na₂SO₄, it is concentrated to give a solid. The crude product is purified by column chromatography on silica gel eluting with toluene to yield 3.41 g (80.8%) of 1-2 as a white powder.
¹H NMR (300 MHz, CDCl₃) δ 7.99-7.92 (m, 1H), 7.87 (dd, *J* = 8.3, 1.3 Hz, 1H), 7.41 (ddd, *J* = 8.1, 6.5, 1.3 Hz, 1H). LC-MS (m/z): 217

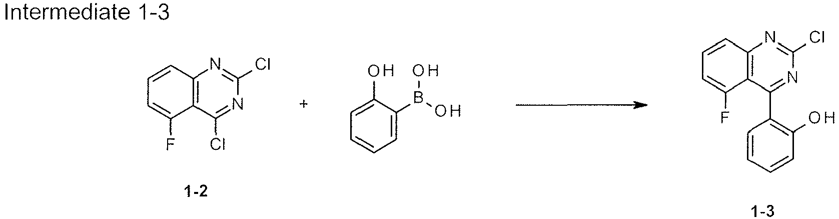

1-2 (2.17 g, 10.0 mmol) and 2-hydroxybenzene boronic acid (1.38 g, 10.0 mmol) are dissolved in 10 mL of THF. To the solution is added potassium fluoride (1.74 g, 30.0 mmol) dissolved in 5 mL, and the mixture is evacuated and purged with Argon gas. Then, tBu₃P-HBF₄ (290 mg, 1.00 mmol) and Pd(OAc)₂ (225 mg, 1.00 mmol) are added to the mixture, and the mixture is stirred at room temperature for 1.5 h. The reaction mixture is dried with MgSO₄, filtrated over Celite and washed with ethyl acetate. The crude is purified by column chromatography on silica gel eluting with a mixed solvent of heptane and ethyl acetate (3:1) to yield 2.27 g (96%) of 1-3 as a slightly yellow solid.
¹H NMR (300 MHz, CDCl₃) δ 9.98 (s, 1H), 8.00 - 7.89 (m, 2H), 7.51-7.43 (m, 2H), 7.36 (ddd, *J* = 8.5, 6.8, 1.4 Hz, 1H), 7.15 (dd, *J* = 8.1, 1.1 Hz, 1H), 7.06-7.01 (m 1H). LC-MS (m/z): 275

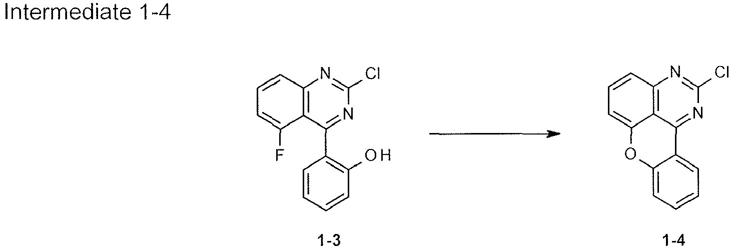

1-3 (3.02 g, 11 mmol) is dissolved in 55 mL of DMF and potassium carbonate (1.67 g, 12.1 mmol) is added. The mixture is stirred at room temperature for 5 min. The reaction mixture is diluted with 100 mL of water, and the solid is collected by filtration and washed with water. It is dried in vacuum oven to yield 2.60 g of 1-4 as a yellow powder.
¹H NMR (300 MHz, CDCl₃) δ 8.55 (ddd, *J* = 8.5, 6.8, 1.4 Hz, 1H), 7.94 (t, *J* = 8.2 H, 1H), 7.74-7.68 (m, 1H), 7.60 (dd, *J* = 8.4, 0.8 Hz, 1H), 7.44 - 7.40 (m, 2 H), 7.32 (dd, *J* = 8.1, 1.0 Hz, 1H) LC-MS (m/z): 255

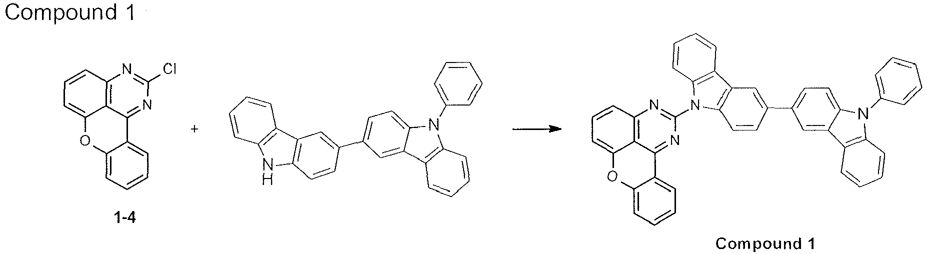

1-4 (1.10 g, 4.32 mmol), 3-(9H-carbazol-3-yl)-9-phenyl-carbazole (1.76 g, 4.32 mmol), and sodium tert-butoxide (581 mg, 6.05 mmol) are added to 43 ml of Toluene. The reaction mixture is evacuated and purged with Argon gas three times. Then, tBu₃P-HBF₄ (100 mg, 0.35 mmol) and Pd₂(dba)3 (79 mg, 0.02 mmol) are added to the mixture, and the reaction mixture is stirred at 80°C for 3 h. The reaction mixture is diluted with water, and the solid is collected by filtration. It is purified by flash chromatography using heptane/toluene = 1:1 as an eluent. The isolated product is suspended to toluene and the suspension is stirred at 110 °C overnight. Then the solid is collected by filtration to yield 1.88 g (69%) of Compound 1 as a yellow powder.
¹H NMR (300 MHz, DMSO-d₆) δ 9.12-9.05 (m, 2H), 8.78 (d, *J* = 1.8 Hz, 1H), 8.66 (d, *J* = 1.8 Hz, 1H), 8.56 (dd, *J* = 1.8, 8.2 Hz, 1H), 8.43-8.37 (m, 2H), 8.06-8.00 (m, 2H), 7.95 (dd, *J* = 2.0, 8.2 Hz, 1H), 7.87-7.81 (m, 1H), 7.77-7.64 (m, 5H), 7.63-7.42 (m, 8H), 7.38-7.33 (m, 2H)
LC-MS (m/z): 627

### Application examples

### Application Example 1

A glass substrate with 120 nm-thick indium-tin-oxide (ITO) transparent electrode (manufactured by Geomatec Co., Ltd.) used as an anode is first cleaned with isopropanol in an ultrasonic bath for 10 min. To eliminate any possible organic residues, the substrate is exposed to an ultraviolet light and ozone for further 30 min. This treatment also improves the hole injection properties of the ITO. The cleaned substrate is mounted on a substrate holder and loaded into a vacuum chamber. Thereafter, the organic materials specified below are applied by vapor deposition to the ITO substrate at a rate of approx. 0.2-1 Å/sec at about 10-6 -10-8 mbar. As the first layer, 5 nm-thick of electron accepting compound A is vapor-deposited. Then 220 nm-thick of aromatic amine compound B is applied as a hole transporting layer. Then, a mixture of 2% by weight of an emitter compound (Compound D), 98% by weight of a host (compound 1) is applied to form a 40 nm-thick phosphorescent-emitting layer. On the emitting layer, a mixture of 50% by weight of an electron transporting compound, Compound C, 50% by weight of Liq (8-Hydroxyquinolate lithium) is applied to form a 25 nm-thick electron transport layer. Finally, 1 nm-thick Liq is deposited as an electron injection layer and 80 nm-thick Al is then deposited as a cathode to complete the device. The device is sealed with a glass lid and a getter in an inert nitrogen atmosphere with less than 1 ppm of water and oxygen.

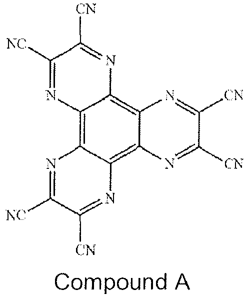

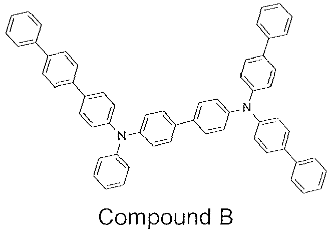

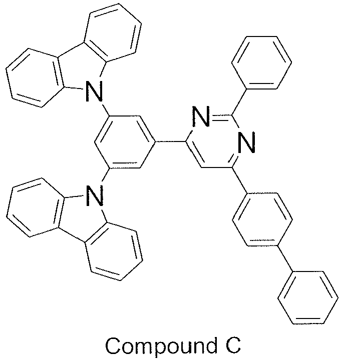

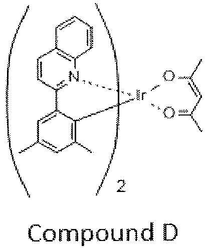

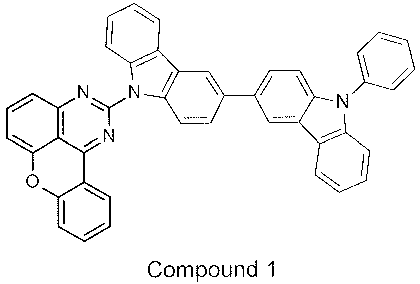

### Application Example 2

Application Example 2 is repeated except that the host (compound 1) is replaced by comparative compound (Comparative 1). The device results are shown in Table 1.

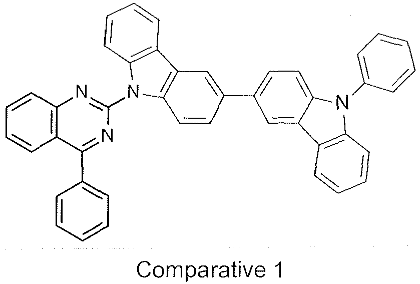

### OLED characterization

To characterize the OLED, electroluminescence spectra are recorded at various currents and voltages. In addition, the current-voltage characteristic is measured in combination with the luminance to determine luminous efficiency and external quantum efficiency (EQE). Driving voltage U, EQE and Commission Internationale de I'Eclairage (CIE) coordinate are given at 10mA/cm2 except otherwise stated.

**Table 1**

| Appl. Ex. | Host | U (V) | EQE (%) | CIE x, y |
|---|---|---|---|---|
| Appl. Ex. 1 | Compound 1 | 5.08 | 16.7 | 0.66, 0.34 |
| Appl. Ex. 2 | Comparative 1 | 5.16 | 16.0 | 0.66, 0.34 |

The results are shown in Table 1. The CIE values show that the electroluminescence is originated from the red emitter compound (Compound D). The compound 1 can show lower driving voltage and higher EQE than the comparative compound (Comparative 1).

### Application Example 3

A glass substrate with 120 nm-thick indium-tin-oxide (ITO) transparent electrode (manufactured by Geomatec Co., Ltd.) used as an anode is first cleaned with isopropanol in an ultrasonic bath for 10 min. To eliminate any possible organic residues, the substrate is exposed to an ultraviolet light and ozone for further 30 min. This treatment also improves the hole injection properties of the ITO. The cleaned substrate is mounted on a substrate holder and loaded into a vacuum chamber. Thereafter, the organic materials specified below are applied by vapor deposition to the ITO substrate at a rate of approx. 0.2-1 Å/sec at about 10-6 -10-8 mbar. As a hole injection layer, 50 nm-thick of compound E is applied. Then 45 nm-thick of aromatic amine compound F is applied as a hole transporting layer. Then, a mixture of 3% by weight of an emitter compound G, 97% by weight of a host compound H are applied to form a 20 nm-thick fluorescent-emitting layer. On the emitting layer, 5 nm-thick compound 1 is applied as a first electron transport layer. Then 25 nm-thick compound I is applied as a second electron transport layer. Finally, 1 nm-thick LiF is deposited as an electron injection layer and 80 nm-thick Al is then deposited as a cathode to complete the device. The device is sealed with a glass lid and a getter in an inert nitrogen atmosphere with less than 1 ppm of water and oxygen.

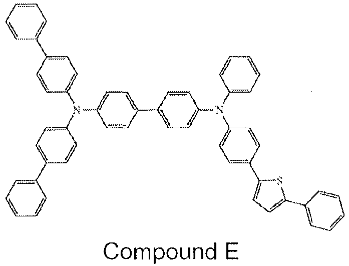

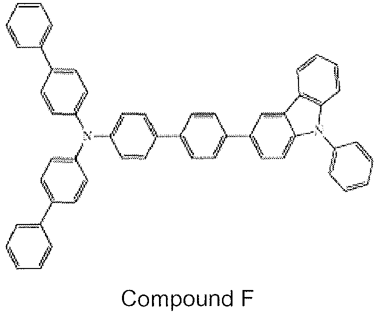

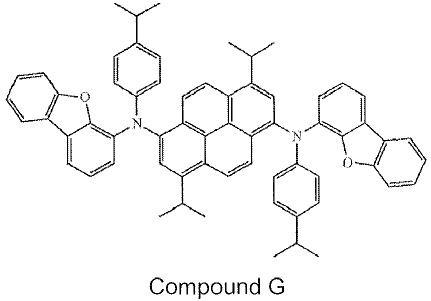

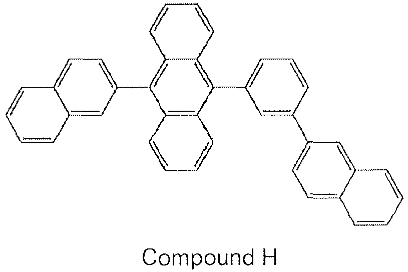

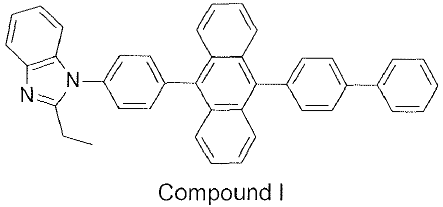

**Table 2**

| Appl. Ex. | Electron transporting layer | U (V) | EQE (%) | CIE x, y |
|---|---|---|---|---|
| Appl. Ex. 3 | Compound 1 | 4.5 | 6.4 | 0.14, 0.10 |

The results are shown in Table 2. The CIE values show that the electroluminescence is originated from the blue emitter compound G. In addition, application examples 3 show EQEs of more than 6%, which exceeds the theoretical limit of 5%. The results demonstrate that the compound 1 can overcome the pure theoretical limit of 5% by confining triplet excitons in the emitting layer, which enhances triplet-triplet fusion.

### Further synthesis examples:

### Compounds synthesized

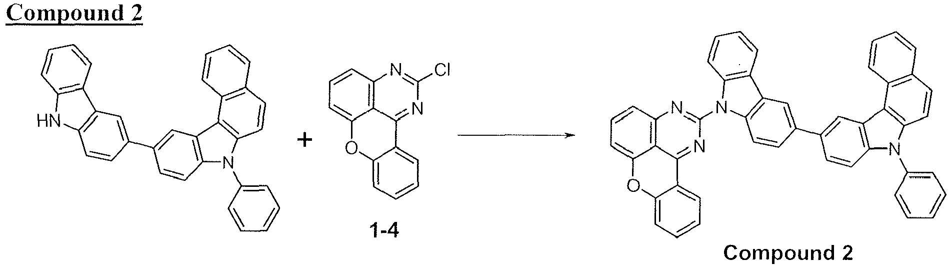

**1-4** (1.21 g, 4.73 mmol), 10-(9H-carbazol-3-yl)-7-phenyl-7H-benzo[c]carbazole (1.67 g, 3.64 mmol), and sodium *tert*-butoxide (490 mg, 5.10 mmol) are added to 36 mL of Toluene. The mixture reaction is evacuated and purged with argon gas three times. Then, ^{t}Bu₃P-HBF₄ (85 mg, 0.29 mmol) and Pd₂(dba)₃ (67 mg, 0.07 mmol) are added to the mixture, and the reaction mixture is stirred at 80°C for 5 h. The reaction mixture is cooled to room temperature. Then a solid is collected by filtration, and washed with toluene and ethanol. The product is recrystallized with dichlorobenzene to yield 1.49 g (41%) of **Compound 2** as a yellow powder.
¹H NMR (300 MHz, DMSO-*d₆*) δ 9.15-9.01 (m, 4H), 8.69 (d, *J* = 1.9 Hz, 1H), 8.52 (dd, *J* = 8.3, 1.6 Hz, 1H), 8.42 (dd, *J* = 7.8, 1.4 Hz, 1H), 8.16-8.03 (m, 2H), 7.89 (m, 3H), 7.88-7.70 (m, 6H), 7.69-7.49 (m, 8H), 7.49-7.40 (m, 1H), 7.28 (dd, *J* = 8.1, 0.7 Hz, 1H)
LC-MS (m/z): 677 [M+1]

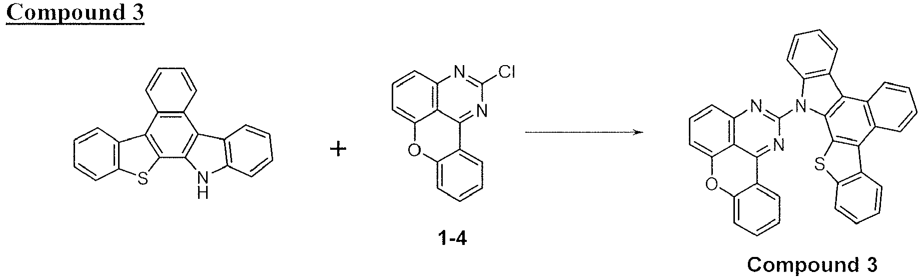

**1-4** (1.55 g, 6.09 mmol), 14H-benzo[c]benzo[4,5]thieno[2,3-a]carbazole (1.79 g, 5.54 mmol), which is prepared according to the procedure mentioned in US20160028020, and sodium *tert-*butoxide (745 mg, 7.75 mmol) are added to 55 mL of Toluene. The mixture is evacuated and purged with argon gas three times. Then, ^{t}Bu₃P-HBF₄ (128 mg, 0.44 mmol) and Pd₂(dba)₃ (101 mg, 0.11 mmol) are added to the mixture, and the reaction mixture is stirred at 80°C for 3.5 h. The reaction mixture is cooled to room temperature. Then, a solid is collected by filtration, and washed with toluene and ethanol. The product is recrystallized with dichlorobenzene to yield 1.97 g (66%) of **Compound 3** as a yellow powder.
¹H NMR (300 MHz, DMSO-*d₆*) δ 9.39-9.29 (m, 1H), 9.22 (dd, *J* = 8.3, 1.5 Hz, 1H), 9.06 (d, *J* = 8.4 Hz, 1H), 8.93-8.83 (m, 1H), 8.46-8.35 (m, 2H), 8.19 (t, *J* = 8.2 Hz, 1H), 8.01 (dd, *J* = 8.0, 1.1 Hz, 1H), 8.00-7.79 (m, 3H), 7.77 (dd, *J=* 8.4, 0.8 Hz, 1H), 7.72-7.52 (m, 5H), 7.53-7.43 (m, 2H)
LC-MS (m/z): 542 [M+1]

### Compound 4

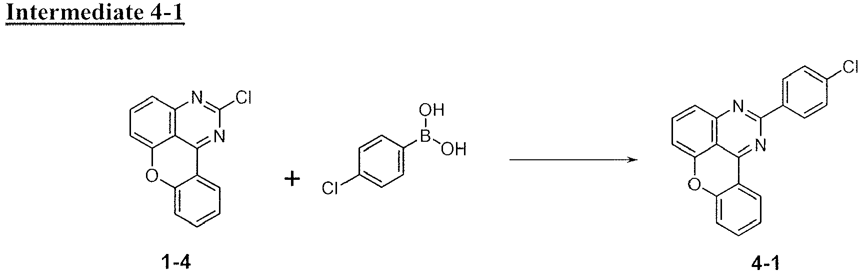

**1-4** (6.60 g, 25.92 mmol), 4-chlorobenzene boronic acid (6.08 g, 38.87 mmol), and potassium fluoride (4.52 g, 77.7 mmol) are suspended in 260 mL of THF and 5 mL of water. The reaction mixture is evacuated and purged with Argon gas three times. Then, ^{t}Bu₃P-HBF₄ (752 mg, 2.59 mmol) and Palladium acetate (582 mg, 2.59 mmol) are added to the mixture, and the reaction mixture is stirred at 60°C for 15 h. The reaction mixture is cooled to room temperature, and a solid is collected by filtration, and washed with ethanol. The product is extracted in Soxhlet extractor with a mixed solvent of THF, chloroform, and ethanol. The solid formed in the solvent is collected by filtration to yield 5.07 g (59%) of **4-1** as a yellow powder.
LC-MS: 331, 333

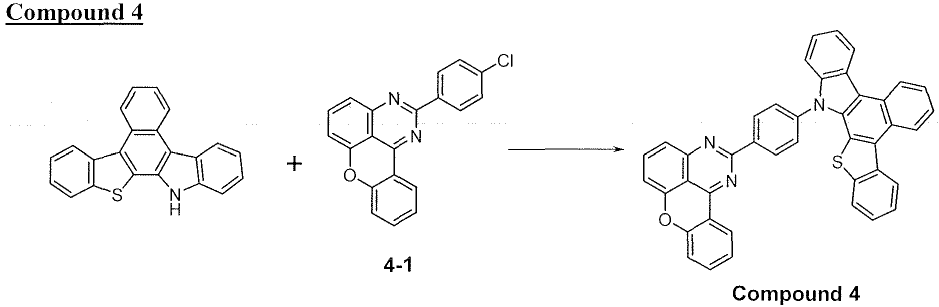

**4-1** (1.59 g, 4.80 mmol), 14H-benzo[c]benzo[4,5]thieno[2,3-a]carbazole (1.55 g, 4.80 mmol), and sodium *tert*-butoxide (646 mg, 6.72 mmol) are added to 48 mL of Toluene. The mixture is evacuated and purged with argon gas three times. Then, Di-*tert*-butyl(2,2-diplienyl-1-methyl-1-cyclopropyl)phosphine (135 mg, 0.38 mmol) and Pd₂(dba)₃ (88 mg, 0.10 mmol) are added to the mixture, and the reaction mixture is stirred at 110°C for 1 h. The reaction mixture is cooled to room temperature. Then a solid is collected by filtration, and washed with toluene and ethanol. The product is recrystallized with dichlorobenzene to yield 2.15 g (72%) of **Compound 4** as a yellow powder.
¹H NMR (400 MHz, DMSO-*d₆*) δ 9.24-9.09 (m, 2H), 9.05-8.92 (m, 3H), 8.87-8.77 (m, 1H), 8.63 (dd, *J* = 7.9, 1.7 Hz, 1H), 8.05-7.95 (m, 4H), 7.91 (ddd, *J=* 8.2, 7.0, 1.1 Hz, 1H), 7.81-7.73 (m, 2H), 7.67-7.58 (m, 2H), 7.57-7.44 (m, 4H), 7.44-7.33 (m, 3H)
LC-MS (m/z): 618 [M+1]

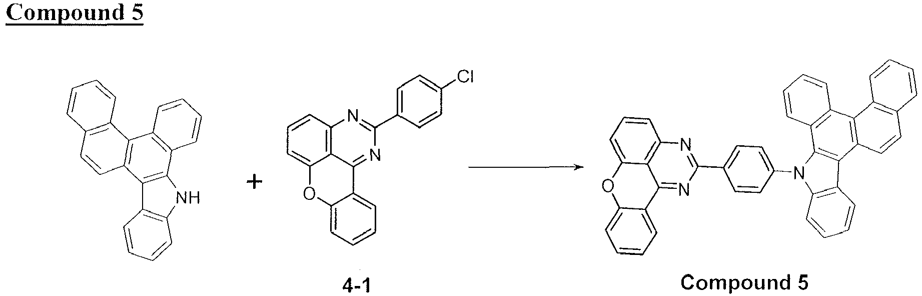

**4-1** (1.67 g, 5.04 mmol), 9H-benzo[a]naphtho[1,2-c]carbazole (1.60 g, 5.04 mmol), which is prepared according to the procedure mentioned in WO2014196580, and sodium *tert*-butoxide (678 mg, 7.06 mmol) are added to 50 mL of Toluene. The mixture is evacuated and purged with argon gas three times. Then, Di-*tert*-butyl(2,2-diphenyl-1-methyl-1-cyclopropyl)phosphine (142 mg, 0.40 mmol) and Pd₂(dba)₃ (92 mg, 0.10 mmol) are added to the mixture, and the reaction mixture is stirred at 110°C for 3.5 h. The reaction mixture is cooled to room temperature. Then a solid is collected by filtration, and washed with toluene and ethanol. The product is recrystallized with dichlorobenzene to yield 2.83 g (92%) of **Compound 5** as a yellow powder.
LC-MS (m/z): 612 [M+1]

### Compound 6

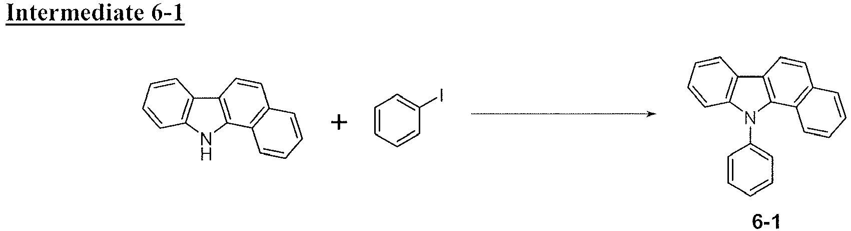

Benzo[a]carbazole (13.04 g, 60.00 mmol), iodobenzene (18.36 g, 90.00 mmol), and sodium *tert-*butoxide (8.07 g, 84.00 mmol) are added to 300 mL of toluene. To this mixture are cBRIDP (846 mg, 2.4 mmol) and Pd₂(dba)₃ added. Then, the mixture is stirred at 120 °C overnight. After the reaction mixture is cooled at room temperature, it is diluted with toluene. The solid is removed by filtration. The crude product is purified by column chromatography on silica gel eluting with heptane to yield 16.6 g (94%) of **6-1** as a brown resin.
LC-MS (m/z): 294

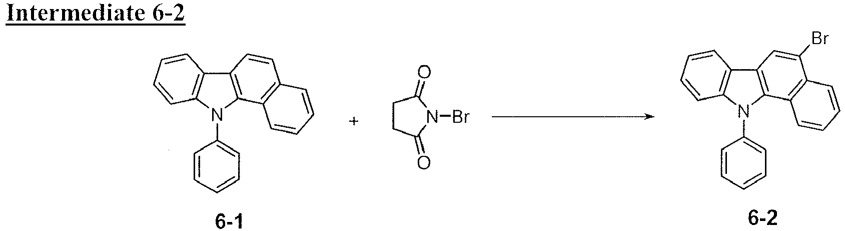

**6-1** (16.39 g, 55.87 mmol) is dissolved in 100 mL of THF. A suspension of N-bromosuccinimide (9.94 g, 55.87 mmol) in 40 mL of THF is added there, and then the mixture is stirred at room temperature for 1h. The reaction mixture is diluted with ethanol. Then, the solid is collected by filtration, and washed with ethanol to yield 18.26 g (88%) of **6-2** as an off-white solid. It is used for next reaction without purification.

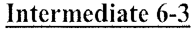

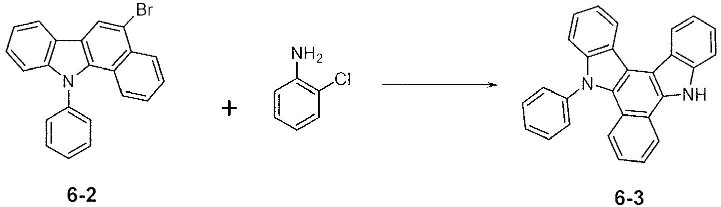

**6-2** (0.93 g, 2.50 mmol), 2-chloroaniline (319 mg, 2.5 mmol), and sodium *tert*-butoxide (480 mg, 5.00 mmol) are suspended in 25 mL of toluene. Then, ^{t}Bu₃P-HBF₄ (73 mg, 0.25 mmol) and Pd₂(dba)₃ (114 mg, 0.125 mmol) are added to the mixture, and the reaction mixture is stirred at 100°C for 2.5 h. After the reaction mixture is cooled at room temperature, it is diluted with chlroform, and the solid is removed by filtration. The crude product is purified by column chromatography on silica gel eluting with a mixture of heptane and toluene to yield 497 mg (52%) of **6-3** as a beige solid.
LC-MS (m/z): 381

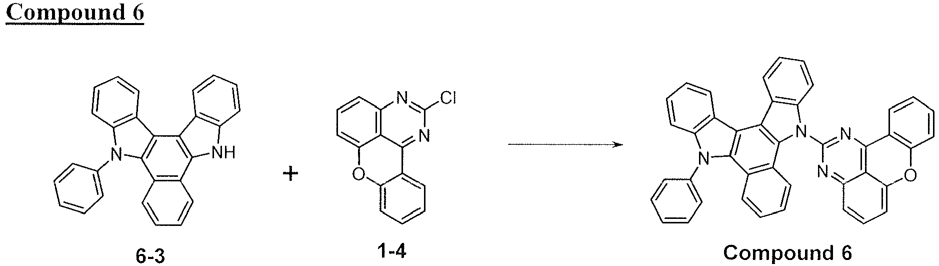

**1-4** (1.32 g, 5.17 mmol), **6-3** (1.80 g, 4.706 mmol), and sodium *tert*-butoxide (633 mg, 6.59 mmol) are added to 47 mL of Toluene. The mixture is evacuated and purged with Argon gas three times. Then, 4,5-Bis(diphenylphosphino)-9,9-dimethylxanthene (120 mg, 0.21 mmol) and Pd₂(dba)₃ (86 mg, 0.09 mmol) are added to the mixture, and the reaction mixture is stirred at 80°C for 1.5 h. The reaction mixture is cooled to room temperature. Then a solid is collected by filtration, and washed with toluene and ethanol. The product is recrystallized with dichlorobenzene to yield 2.39 g (75%) of **Compound 6** as a yellow powder.
¹H NMR (300 MHz, DMSO-*d₆*) δ 9.05-8.96 (m, 2H), 8.34-8.26 (m, 1H), 8.20 (dd, *J* = 8.0, 1.6 Hz, 1H), 8.11 (t, *J* = 8.3, 1H), 7.86-7.75 (m, 4H), 7.74-7.66 (m, 3H), 7.66-7.49 (m, 8H), 7.40 (ddd, *J* = 8.1, 7.2, 1.1 Hz, 1H), 7.28-7.17 (m, 3H)
LC-MS (m/z): 601 [M+1]

### Compound 7

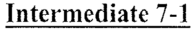

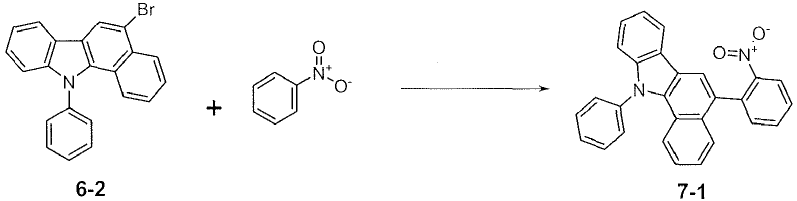

**6-2** (5.00 g, 13.43 mmol), nitrobenzene (16.54 g, 134.31 mmol), pivalic acid (412 mg, 4.03 mmol), and potassium carbonate (2.41 g, 17.46 mmol) is suspended in 90 mL of xylene. Then, Pd₂(dba)₃ and di-tert-butyl(methyl)phosphonium tetrafluoroborate (0.50 g, 2.02 mmol) are added to the mixture, and the mixture is stirred at 140 °C for 48 h. the solid is removed by filtration, and the filtrate was concentrated. The crude product is purified by column chromatography on silica gel eluting with a mixed solvent of heptane and toluene to yield 2.63 g (47%) of **7-1** as an orange solid.
LC-MS (m/z): 415

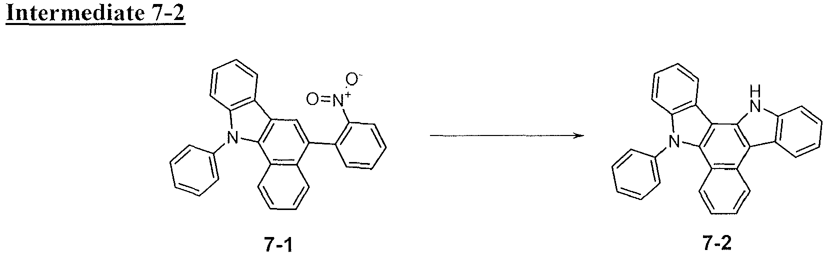

**7-1** (2.60 g, 6.27 mmol) and triphenyl phosphine (4.11 g, 15.68 mmol) are suspended in 13 mL of 1,2-dichlorobenzene, and the mixture is stirred at 180 °C overnight. The reaction mixture is cooled at room temperature, and concentrated. The crude product is purified by column chromatography on silica gel eluting with a mixed solvent of heptane and toluene to yield 1.92 g (80%) of **7-2** as a brown solid.

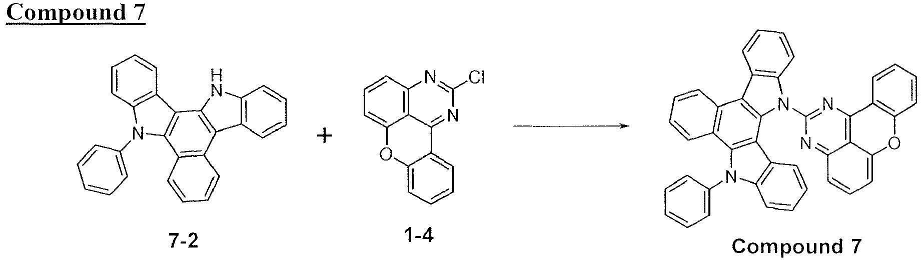

**7-2** (1.35 g, 3.53 mmol), **1-4** (0.98 g, 3.88 mmol), and sodium *tert*-butoxide (475 mg, 4.94 mmol) are added to 35 mL of toluene. The mixture is evacuated and purged with Argon gas three times. Then, 4,5-Bis(diphenylphosphino)-9,9-dimethylxanthene (90 mg, 0.16 mmol) and Pd₂(dba)₃ (65 mg, 0.07 mmol) are added to the mixture, and the reaction mixture is stirred at 80°C for 3 h. The reaction mixture is cooled to room temperature. Then a solid is collected by filtration, and washed with toluene. The crude product is purified by column chromatography on silica gel eluting with a mixed solvent of heptane and toluene (1:2) to yield 2.12 g (75%) of **Compound 7** as a yellow powder.
¹H NMR (300 MHz, DMSO-*d₆*) δ 9.11 (d, *J* = 8.4 Hz, 1H), 8.82-8.71 (m, 1H), 8.60-8.50 (m, 1H), 8.16 (dd, *J* = 7.9, 1.6 Hz, 1H), 8.04 (t, *J* = 8.2 Hz, 1H), 7.84-7.73 (m, 5H), 7.70-7.67 (m, 2H), 7.64-7.43 (m, 6H), 7.40-7.31 (m, 2H), 7.13 (ddd, *J* = 8.2, 6.9, 1.2 Hz, 1H), 7.03 (dt, *J* = 8.3, 1.0 Hz, 1H), 6.69 (ddd, *J* = 8.1, 6.9, 1.1 Hz, 1H), 6.58 (dt, *J* = 8.1, 1.0 Hz, 1H)
LC-MS (m/z): 601 [M+1]

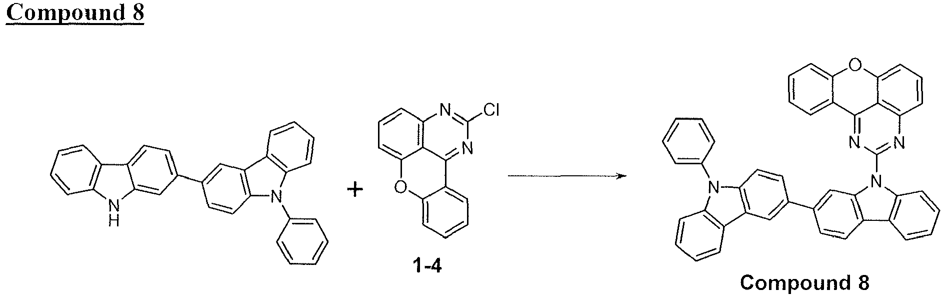

**1-4** (1.50 g, 5.87 mmol), 9'-phenyl-9H,9'H-2,3'-bicarbazole (2.00 g, 4.90 mmol), and sodium *tert*-butoxide (659 mg, 6.85 mmol) are added to 33 mL of Toluene. The mixture is evacuated and purged with Argon gas three times. Then, ^{t}Bu₃P-HBF₄ (114 mg, 0.39 mmol) and Pd₂(dba)₃ (90 mg, 0.10 mmol) are added to the mixture, and the reaction mixture is stirred at 80°C for 17 h. The reaction mixture is cooled to room temperature. Then a solid is collected by filtration, and washed with toluene and ethanol. The crude product is purified by sublimation twice to yield 3.02 g (98%) of **Compound 8** as a yellow powder.
¹H NMR (300 MHz, DMSO-*d₆*) δ 9.46 (d, *J* = 1.5 Hz, 1H), 9.09-9.01 (m, 1H), 8.73 (d, *J* = 1.8 Hz, 1H), 8.61 (dd, *J* = 7.9, 1.6 Hz, 1H), 8.42-8.23 (m, 3H), 8.03 (t, *J* = 8.2 Hz, 1H), 7.93 (dd, *J* = 8.6, 1.8 Hz, 1H), 7.89-7.65 (m, 7H), 7.64-7.40 (m, 8H), 7.37-7.32 (m, 2H)
LC-MS (m/z): 627 [M+1]

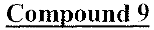

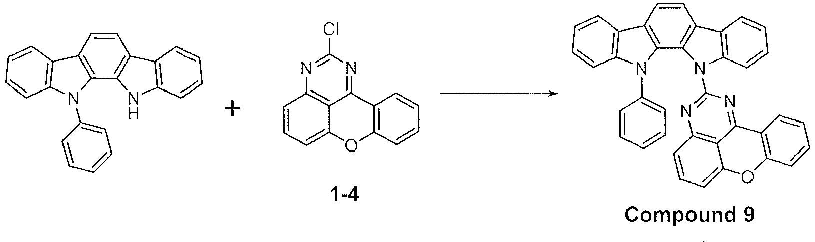

**1-4** (1.96 g, 7.70 mmol), 11-phenyl-11,12-dihydroindolo[2,3-a]carbazole (1.79 g, 7.00 mmol) which is prepared according to the procedure mentioned in WO2009116377, and sodium *tert-*butoxide (942 mg, 9.80 mmol) are added to 50 mL of Toluene. The mixture is evacuated and purged with Argon gas three times. Then, ^{t}Bu₃P-HBF₄ (162 mg, 0.56 mmol) and Pd₂(dba)₃ (128 mg, 0.14 mmol) are added to the mixture, and the reaction mixture is stirred at 80°C for 17 h. The reaction mixture is cooled to room temperature. Then a solid is collected by filtration, and washed with toluene and ethanol. The crude product is column chromatography by eluting with dichloromethane to yield 1.05 g (27%) of **Compound 9** as a yellow powder.
¹H NMR (400 MHz, DMSO-*d₆*) δ 8.42-8.31 (m, 4H), 8.27 (d, *J* = 8.1 Hz, 1H), 8.16 (dd, *J* = 7.9, 1.6 Hz, 1H), 7.98 (t, *J* = 8.2 Hz, 1H), 7.81 (ddd, *J* = 8.8, 7.2, 1.7 Hz, 1H), 7.59 (dd, *J* = 8.4 Hz, 1.0 Hz, 1H), 7.53-7.31 (m, 8H), 7.07-6.57 (m, 5H)
LC-MS (m/z): 550 [M]

### Compound 10

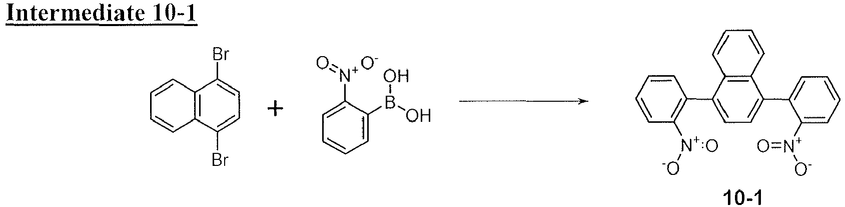

1,4-Dibromonaphthalene (5.71 g, 19.97 mmol), 2-nitrobenzene boronic acid (7.00 g, 41.93 mmol) are dissolved in 100 mL of THF. To the solution is a solution of potassium fluoride (6.99 g, 119.81 mmol) dissolved in 10 mL of water added. Then, ^{t}Bu₃P-HBF₄ (1.16 g, 3.99 mmol) and Pd₂(dba)₃ (1.82 g, 2.00 mmol) are there, and the mixture is stirred at room temperature overnight. The solid is removed by filtrataion, and the filtrate is extracted with ethyl acetate. The solution is partly concentrated to give a beige powder, and it is collected by filtration. And further powder from the filtrate is collected by filtration. Total, 6.77 g (91%) of **10-1** is obtained as a beige powder. It is used for next reaction without further purification.

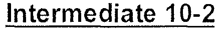

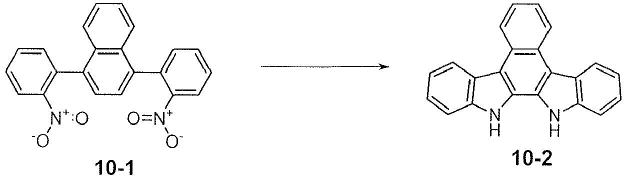

**10-1** (6.72 g, 18.16 mmol) and triphenyl phosphine (28.57 g, 108.95 mmol) are dissolved in 91 mL of 1,2-dichlorobenzene, and the solution is stirred at 180 °C overnight. The solvent is removed by distillation, and the crude product is chromatographed eluting with a mixed solvent of heptane and dichloromethane to yield 4.74 g (85%) of **10-2** as a beige powder. it is used for next reaction without further purification.

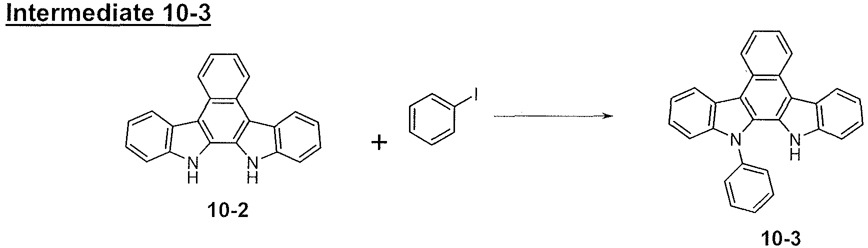

**10-2** (3.89 g, 12.71 mmol) and iodobenzene (2.59 g, 12.71 mmol) are suspended in 65 mL of toulene. To the suspension are ^{t}Bu₃P-HBF₄ (295 mg, 1.02 mmol) and Pd₂(dba)₃ (233 mg, 0.25 mmol) added, and the mixture is refluxed under argon atomosphere overnight. The reaction mixture is cooled at room temperature, and the solid is removed by filtration. The filtrate is concentrated, and the crude product is chromatographed eluting with a mixed solvent of heptane and dichloromethane to yield 3.40 g (85%) of **10-3** as a beige powder. it is used for next reaction without further purification.

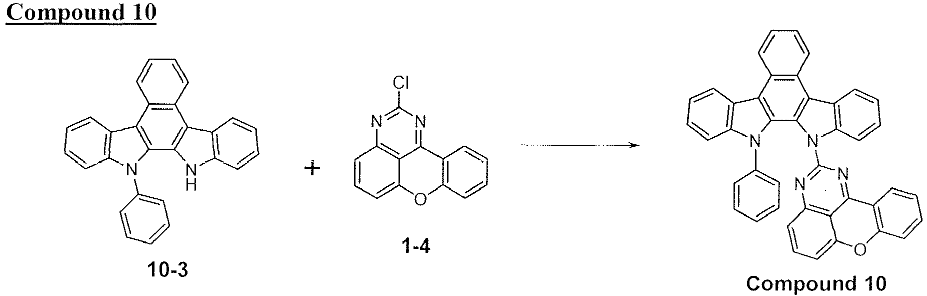

**10-3** (1.45 g, 3.79 mmol), **1-4** (1.07 g, 4.20 mmol), and sodium *tert*-butoxide (510 mg, 5.31 mmol) are added to 55 mL of Toluene. The mixture is evacuated and purged with Argon gas three times. Then, 4,5-Bis(diphenylphosphino)-9,9-dimethylxanthene (175 mg, 0.30 mmol) and Pd₂(dba)₃ (174 mg, 0.19 mmol) are added to the mixture, and the reaction mixture is stirred at 80°C for 7 h. The reaction mixture is cooled to room temperature. Then a solid is collected by filtration, and washed with toluene. The crude product is chromatographed eluting with a mixed solvent of heptane and dichloromethane to yield 1.58 g (70%) of **Compound 10** as a yellow powder.
¹H NMR (300 MHz, DMSO-*d₆*) δ 9.21-9.17 (m, 2H), 8.95-8.76 (m, 2H), 8.43-8.23 (m,1H), 8.13-7.72 (m, 5H), 7.67-7.20 (m, 9H), 7.05-6.72 (m, 5H)
LC-MS (m/z): 601 [M+1]

### Compound 11

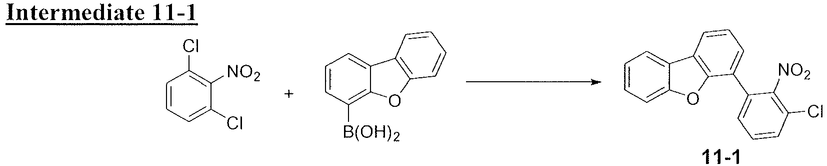

1,3-Dichloro-2-nitrobenzene (5.07 g, 26.54 mmol), dibenzofurane-4-boronic acid (5.628 g, 26.54 mmol), Pd(PPh₃)₄ (610 mg, 0.53 mmol), and Na₂CO₃ (8.51 g, 80.29 mmol) are added to 25 mL of toluene, 5 mL of 1,2-dimethoxyethane, and 5 mL of water. The mixture is refluxed for 12 h. After the reaction mixture is cooled to room temperature, the aqueous layer is extracted with toluene. After the organic layer is dried with MgSO4, the solvent is evaporated. The crude product is chromatographed to yield 6.1 g (71%) of **11-1** as a white solid.

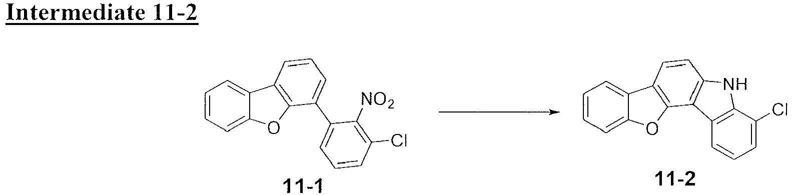

**11-1** (1.98 g, 6.15 mmol) and triphenyl phosphine (4.06 g, 15.49 mmol) are added to 50 mL of 1,2-dichlorobenzene, and the mixture is stirred at 180 °C for 12 h. The reaction mixture is cooled to room temperature. The crude product is chromatographed to yield 1.2 g (67%) of **11-2** as a white solid.

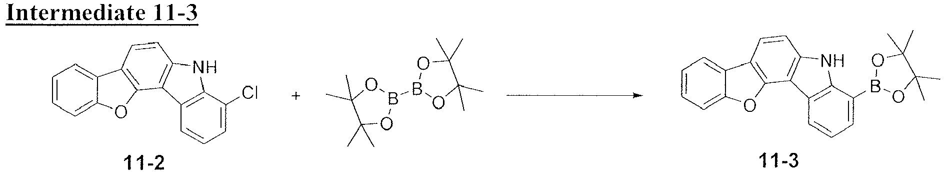

**11-2** (2.6 g, 8.93 mmol), bispinacolate diboron (6.8 g, 26.78 mmol), Pd₂(dba)₃ (246 mg, 0.26 mmol), 2-dicyclohexylphosphino-2',4',6'-triisopropylbiphenyl (248 mg, 0.52 mmol), and potassium acetate (256 mg, 2.6 mmol) are added to 45 mL of 1,4-dioxane. The mixture is refluxed for 12 h. After the reaction mixture is cooled to room temperature, the solvent is evaporated. The crude product is chromatographed to yield 2.5 g (74%) of **11-3** as a white solid.

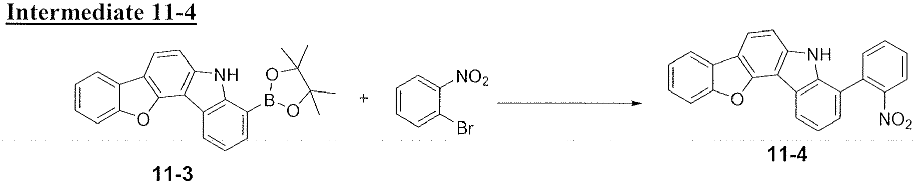

**11-3** (2.52 g, 6.53 mmol), 2-bromonitrobenzene (1.31 g, 6.53 mmol), Pd(PPh₃)₄ (130 mg, 0.53 mmol), and Na₂CO₃ (1.93 g, 19.75 mmol) are added to 15 mL of toluene, 3 mL of 1,2-dimethoxyethane, and 3 mL of water under argon atmosphere. The mixture is refluxed for 12 h. After the reaction mixture is cooled to room temperature, the aqueous layer is extracted with toluene. After the organic layer is dried with MgSO4, the solvent is evaporated. The crude product is chromatographed to yield 2.0 g (81%) of **11-4** as a pale yellow solid.

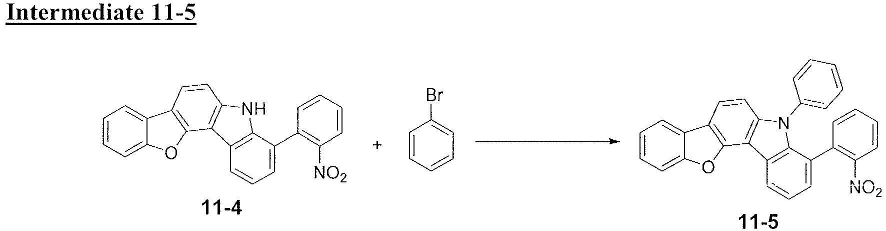

**11-4** (2.00 g, 5.30 mmol), bromobenzene (827 mg, 5.30 mmol), and sodium *tert*-butoxide (713 mg, 7.42 mmol) are added to 30 mL of Toluene. The reaction mixture is evacuated and purged with argon gas three times. Then, ^{t}Bu₃P-HBF₄ (123 mg, 0.42 mmol) and Pd₂(dba)₃ (98 mg, 0.11 mmol) are added to the mixture, and the reaction mixture is stirred at 80°C overnight. The reaction mixture is cooled to room temperature. Then a solid is collected by filtration, and washed with toluene and ethanol. The crude product is purified by sublimation to yield 1.30 g (54%) of **11-5** as a pale yellow powder.

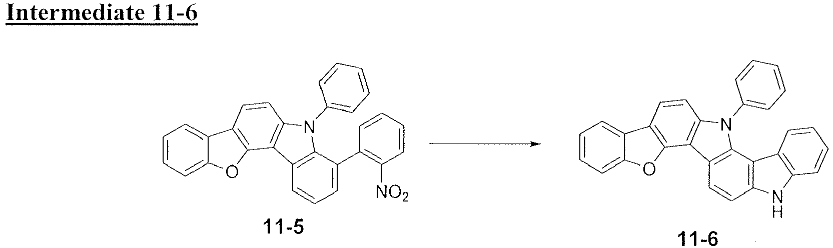

**11-5** (1.98 g, 6.15 mmol) and triphenyl phosphine (4.06 g, 15.49 mmol) are added to 50 mL of 1,2-dichlorobenzene, and the mixture is stirred at 180 °C for 12 h. The reaction mixture is cooled to room temperature. The crude product is chromatographed to yield 262 mg (62%) of **11-6** as a white solid.

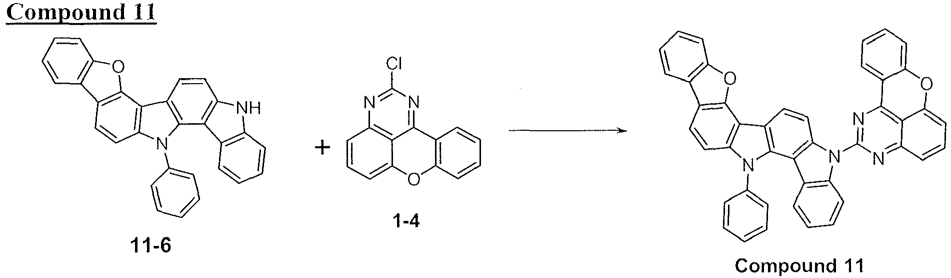

**1-4** (1.40 g, 5.50 mmol), **11-6** (2.10 g, 5.00 mmol), and sodium *tert*-butoxide (673 mg, 7.00 mmol) are added to 60 mL of Toluene. The mixture is evacuated and purged with argon gas three times. Then, ^{t}Bu₃P-HBF₄ (116 mg, 0.40 mmol) and Pd₂(dba)₃ (92 mg, 0.10 mmol) are added to the mixture, and the reaction mixture is stirred at 80°C overnight. The reaction mixture is cooled to room temperature. Then a solid is collected by filtration, and washed with toluene and ethanol. The crude product is purified by sublimation to yield 2.30 g (72%) of **Compound 11** as a yellow powder.
¹H NMR (300 MHz, DMSO-*d₆*) δ 8.92-8.85 (m, 2H), 8.69-8.60 (m, 1H), 8.50-8.40 (m,1H), 8.18-8.05 (m, 2H), 7.99 (t, *J* = 8.2 Hz, 1H), 7.81 (ddd, *J* = 8.8, 7.3, 1.7 Hz, 1H), 7.74-7.29 (m, 11H), 6.75 (br, 5H) LC-MS (m/z): 640 [M]

### Compound 12

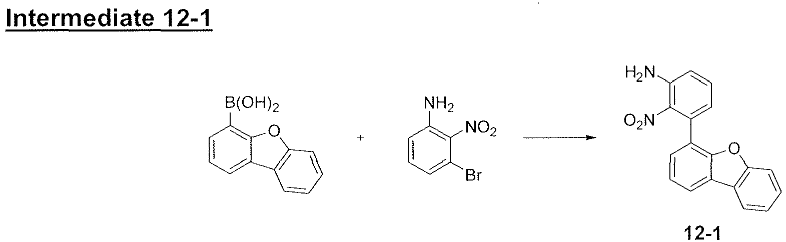

3-Bromo-2-nitroaniline (21.70 g, 100.0 mmol), dibenzofurane-4-boronic acid (21.20 g, 100.0 mmol) and potassium phosphate (42.45 g, 200.0 mmol) are dissolved in 200 mL of tetrahydrofuran and 50 mL of water. The reaction mixture is evacuated and purged with argon gas three times. Tri-t-butylphosphoniumtetrafluoroborat (290 mg, 1.0 mmol) and Pd₂(dba)₃ (458 mg, 0.5 mmol) are added and the mixture is refluxed for 1.5 h. After cooling to room temperature, 200 mL of water and 200 mL of toluene are added and the reaction mixture is filtered. The filtrate is transferred to a separation funnel, the phases are separated and the aqueous layer is extracted with toluene. The organic layer is washed with brine, dried with Na₂SO₄ and the solvent is evaporated. The crude product is chromatographed with heptane/toluene as eluent to yield 29.57 g (97%) of **12-1** as a solid.

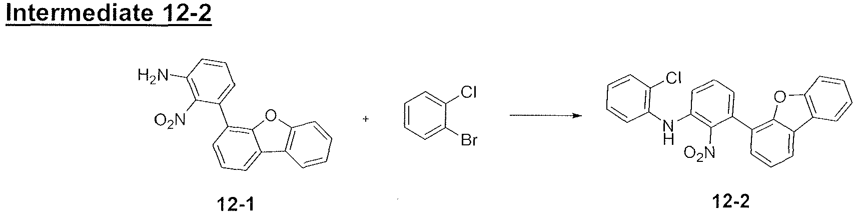

**12-1** (13.99 g, 46.0 mmol) and cesium carbonate (29.97 g, 92.0 mmol) are suspended in 150 mL of toluene and evacuated and purged with argon. 1-Bromo-2-chlorobenzene (8.81 g, 46.0 mmol) is added, then argon is bubbled through for 15 minutes. Rac-BINAP (859 mg, 1.38 mmol) and Pd₂(dba)₃ (842 mg, 0.92 mmol) are added and the reaction mixture is refluxed for 15 hours. After cooling to room temperature the suspension is filtered and the filtrate is evaporated. The crude product is chromatographed with heptane/toluene as eluent to yield 17.40 g (91%) of **12-2** as a solid.

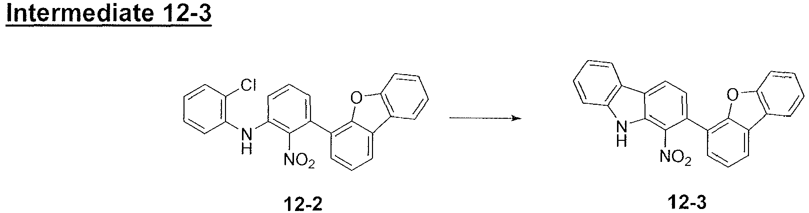

**12-2** (415 mg, 1.00 mmol) and potassium carbonate (276 mg, 2.00 mmol) are suspended in 8 mL of o-xylene and evacuated and purged with argon. Then argon is bubbled through for 15 minutes. Tricyclohexylphosphine (34 mg, 0.12 mmol) and palladium(II)acetate (13 mg, 0.06 mmol) are added and the reaction mixture is refluxed for 21 hours. After cooling to room temperature the suspension is filtered and the filtrate is evaporated. The crude product is chromatographed with heptane/toluene as eluent to yield 216 mg (57%) of **12-3** as a solid.

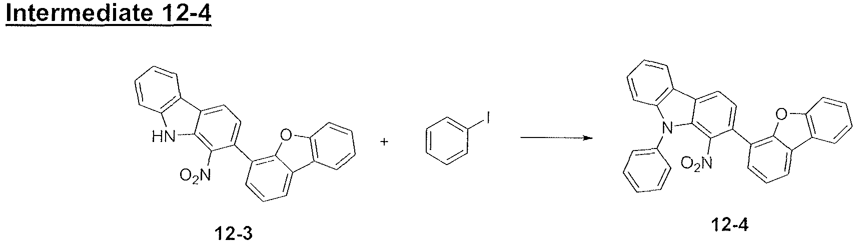

**12-3** (5.11 g, 13.50 mmol), iodobenzene (12.39 g, 60.75 mmol), copper (642 mg, 10.10 mmol) and potassium carbonate (5.59 g, 40.50 mmol) are suspended in 55 mL of nitrobenzene. The reaction mixture is stirred at 200°C for 43 hours, then cooled to room temperature and the solvent is evaporated in vacuo. The residue is stirred with 100 mL of water and 130 mL of toluene and filtered. The filtrate is transferred to a separation funnel and the phases are separated. The water phase is extracted with toluene, the organic phase is washed with brine, dried with Na₂SO₄, filtered and the solvent is evaporated. The crude product is chromatographed with heptane/toluene as eluent to yield 4.97 g (81%) of **12-4** as a solid.

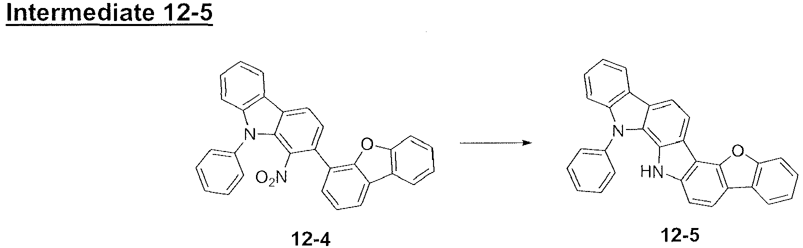

**12-4** (4.99 g, 11.00 mmol) and triphenyl phosphine (43.28 g, 165.0 mmol) are mixed and heated to 200°C. The solution is stirred at 200 °C for 64 hours, then cooled to room temperature. The brown solid is chromatographed with heptane/toluene as eluent to yield 3.96 g (85%) of **12-5** as a solid.

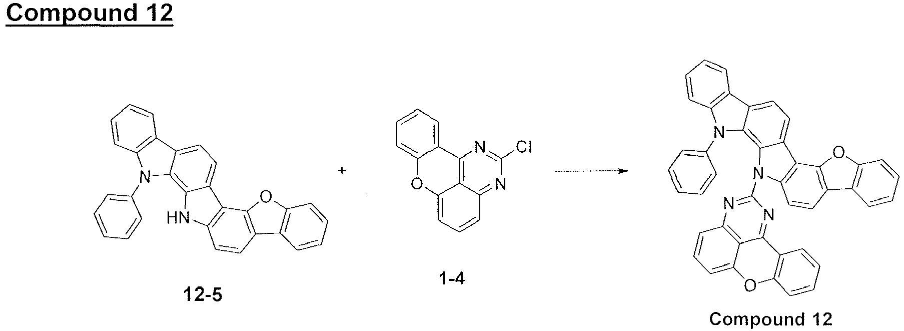

**1-4** (1.17 g, 4.60 mmol), **12-5** (1.69 g, 4.00 mmol) and sodium tert-butoxide (589 mg, 6.00 mmol) are suspended in 30 mL of toluene. The mixture is evacuated and purged with argon, then argon is bubbled through for 15 minutes. 4,5-Bis(diphenylphosphino)-9,9-dimethylxanthene (139 mg, 0.24 mmol) and Pd₂(dba)₃ (110 mg, 0.12 mmol) are added and the reaction mixture is refluxed for 1 h. After cooling to room temperature, 100 mL of water and 30 mL of toluene are added and the reaction mixture is filtered. The filtrate is transferred to a separation funnel and the phases are separated. The water phase is extracted with toluene, the organic phase is washed with brine, dried with Na₂SO₄, filtered and the solvent is evaporated. The crude product is chromatographed with heptane/toluene as eluent to yield 2.33 g (91%) of **compound 12** as a yellow solid.
LC-MS (m/z): 640 [M]

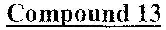

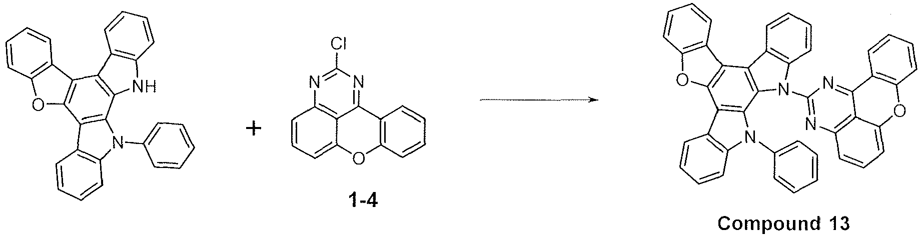

**1-4** (1.40 g, 5.50 mmol), 6-phenyl-5,6-dihydrobenzofuro[2,3-c]indolo[2,3-a]carbazole (2.10 g, 5.00 mmol), and sodium *tert*-butoxide (673 mg, 7.00 mmol) are added to 50 mL of Toluene. The mixture is evacuated and purged with Argon gas three times. Then, 4,5-Bis(diphenylphosphino)-9,9-dimethylxanthene (231 mg, 0.40 mmol) and Pd₂(dba)₃ (230 mg, 0.25 mmol) are added to the mixture, and the reaction mixture is stirred at 80°C overnight. The reaction mixture is cooled to room temperature. Then a solid is collected by filtration, and washed with toluene and ethanol. The crude product is purified by column chromatography by eluting with a mixed solvent of heptane and dichloromethane to yield 1.94 g (60%) of **Compound 13** as a yellow powder.
LC-MS (m/z): 641 [M+1]

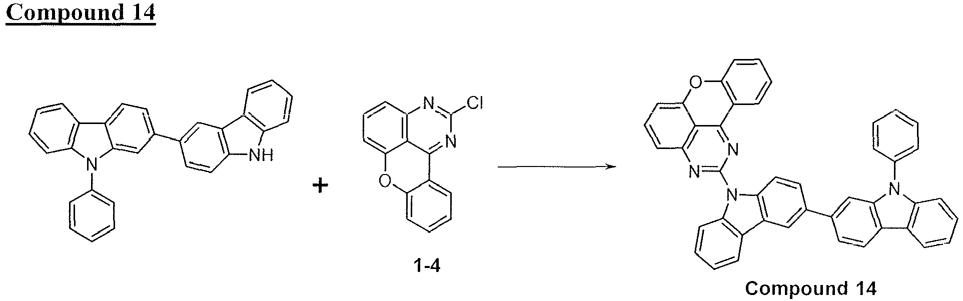

**1-4** (1.50 g, 5.87 mmol), 9-phenyl-9H,9'H-2,3'-bicarbazole (2.00 g, 4.90 mmol), and sodium *tert-*butoxide (659 mg, 6.85 mmol) are added to 33 mL of Toluene. The mixture is evacuated and purged with Argon gas three times. The reaction mixture is evacuated and purged with Argon gas three times. Then, ^{t}Bu₃P-HBF₄ (114 mg, 0.39 mmol) and Pd₂(dba)₃ (90 mg, 0.10 mmol) are added to the mixture, and the reaction mixture is stirred at 80°C for 17 h. The reaction mixture is cooled to room temperature. Then a solid is collected by filtration, and washed with toluene and ethanol. The crude product is recrystallized with chloroform and ethanol to yield 3.02 g (98%) of **Compound 14** as a yellow powder.
¹H NMR (300 MHz, CDCl₃) δ 9.14-9.04 (m, 2H), 8.67 (dd, *J* = 8.0, 1.7 Hz, 1H), 8.36 (dd, *J* = 1.9, 0.6 Hz, 1H), 8.28 (dd, *J* = 8.1, 0.7 Hz, 1H), 8.24-8.14 (m, 2H), 7.96-7.84 (m, 2H), 7.80-7.67 (m, 7H), 7.66-7.31 (m, 9H), 7.21 (dd, *J* = 8.0, 0.8 Hz, 1H)
LC-MS (m/z): 627 [M+1]

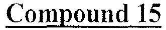

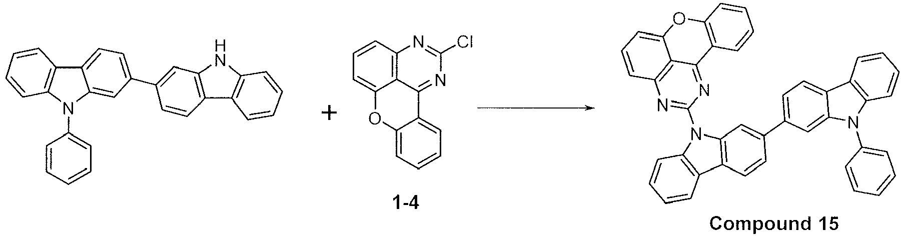

**1-4** (1.01 g, 3.97 mmol), 9-phenyl-9H,9'H-2,2'-bicarbazole (1.35 g, 3.31 mmol), and sodium *tert-*butoxide (445 mg, 4.63 mmol) are added to 22 mL of Toluene. The mixture is evacuated and purged with Argon gas three times. Then, ^{t}Bu₃P-HBF₄ (77 mg, 0.26 mmol) and Pd₂(dba)₃ (61 mg, 0.07 mmol) are added to the mixture, and the reaction mixture is stirred at 80°C overnight. The reaction mixture is cooled to room temperature. Then a solid is collected by filtration, and washed with toluene and ethanol. The crude product is recrystallized with chloroform and ethanol to yield 1.69 g (67%) of **Compound 15** as a yellow powder.
LC-MS (m/z): 627 [M+1]

### Compound 16

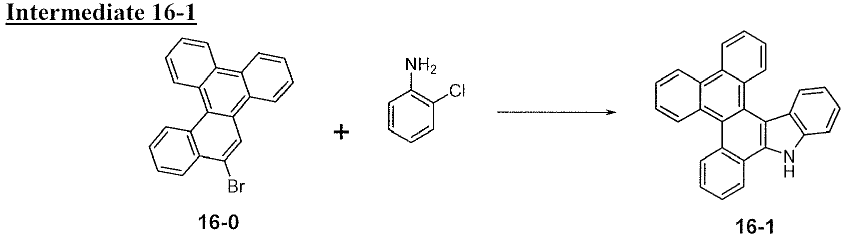

16-0 (3.00 g, 8.40 mmol) which prepared according to the procedure mentioned in WO2011086941, 2-chloroaniline (1.07 g, 8.40 mmol), and sodium *tert*-butoxide (1.61 g, 16.80 mmol) are added to 20 mL of toluene. The mixture is evacuated and purged with Argon gas three times. Then, ^{t}Bu₃P-HBF₄ (244 mg, 0.84 mmol) and Pd₂(dba)₃ (384 mg, 0.42 mmol) are added to the mixture, and the reaction mixture is refluxed overnight. The reaction mixture is cooled to room temperature, and diluted with chloroform. The solid is removed by filtration, and the crude product is chromatographed eluting with a mixed solvent of heptane and dichloromethane to yield 2.47 g (80%) of **16-1** as a beige powder.
LC-MS (m/z): 369 [M+1]

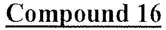

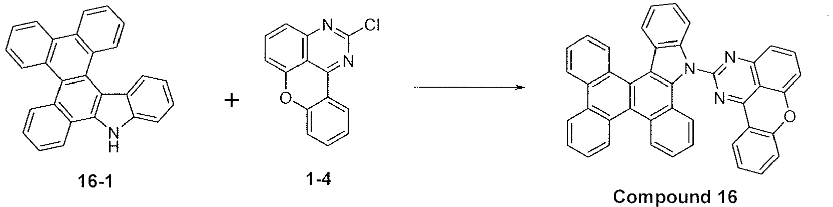

**16-1** (1.47 g, 4.00 mmol), **1-4** (1.22 g, 4.80 mmol), and sodium *tert*-butoxide (538 mg, 5.60 mmol) are added to 27 mL of toluene. The mixture is evacuated and purged with Argon gas three times. Then, 4,5-Bis(diphenylphosphino)-9,9-dimethylxanthene (93 mg, 0.16 mmol) and Pd₂(dba)₃ (73 mg, 0.07 mmol) are added to the mixture, and the reaction mixture is stirred at 80°C overnight. The reaction mixture is cooled to room temperature. Then a solid is collected by filtration, and washed with toluene and ethanol. The crude product is recrystallized with chloroform and ethanol to yield 2.33 g (99%) of **Compound 16** as a yellow powder.
¹H NMR (300 MHz, CDCl₃) δ 9.14-9.06 (m, 1H), 8.94-8.86 (m, 1H), 8.81-8.74 (m, 2H), 8.52 (td, *J* = 8.1, 1.0 Hz, 1H), 8.25 (d, *J* = 7.9 Hz, 1H), 8.21-8.10 (m, 2H), 8.05-7.94 (m, 3H), 7.89-7.59 (m, 7H), 7.51(ddd, J = 8.4, 6.9, 1.3 Hz, 1H), 7.44-7.36 (m, 2H), 7.35-7.18 (m, 2H)
LC-MS (m/z): 586 [M+1]

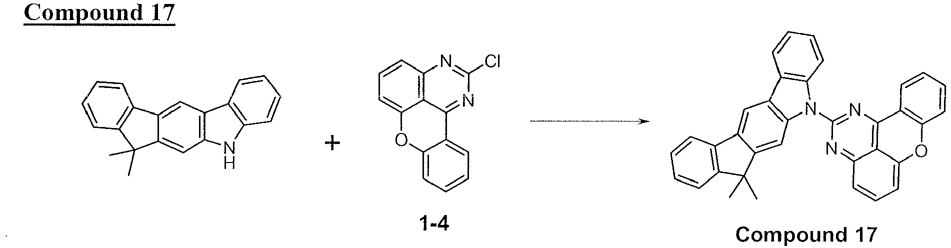

**1-4** (1.77 g, 6.95 mmol), 7,7-dimethyl-5,7-dihydroindeno[2,1-b]carbazole (1.79 g, 6.32 mmol), and sodium *tert*-butoxide (850 mg, 8.84 mmol) are added to 50 mL of Toluene. The mixture is evacuated and purged with Argon gas three times. Then, ^{t}Bu₃P-HBF₄ (147 mg, 0.51 mmol) and Pd₂(dba)₃ (116 mg, 0.13 mmol) are added to the mixture, and the reaction mixture is stirred at 80°C for 4 h. The reaction mixture is cooled to room temperature. Then a solid is collected by filtration, and washed with toluene and ethanol. The crude product is recrystallized with chloroform and heptane to yield 1.8 g (57%) of **Compound 17** as a yellow powder.
¹H NMR (300 MHz, DMSO-*d₆*) δ 9.09 (d, *J* = 2.6 Hz, 1H), 8.97 (dd, *J* = 8.3, 2.8 Hz, 1H), 8.63 (d, *J* = 3.4 Hz, 1H), 8.26 (dd, *J* = 8.0, 2.6 Hz, 1H), 8.03-7.88 (m, 2H), 7.83-7.67 (m, 1H), 7.67-7.31 (m, 8H), 7.25-7.23 (br, 1H), 1.61 (s, 6H)
LC-MS (m/z): 501 [M]

### Compound 18

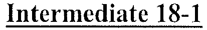

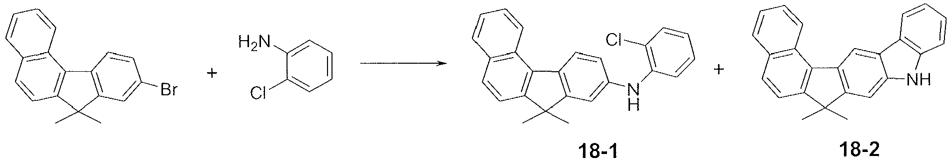

9-Bromo-7,7-domethyl-7H-benzo[c]fluorene (10.70 g, 33.10 mmol), 2-chloroaniline (4.23 g, 33.10 mmol), and sodium *tert*-butoxide (9.34 g, 66.20 mmol) are added to 110 mL of toluene. The mixture is evacuated and purged with Argon gas. Then, ^{t}Bu₃P-HBF₄ (418 mg, 1.44 mmol) and Pd₂(dba)₃ (659 mg, 0.72 mmol) are added to the mixture, and the reaction mixture is stirred at 110°C for 1 h. The reaction mixture is cooled at room temperature, and diluted with toluene. The solid is removed by filtration, and the filtrate is concentrated. The crude product is chromatographed eluting with a mixed solvent of heptane and toluene to yield 4.44 g (36%) of **18-1** as a beige powder and 6.21 g (56%) of **18-2** as a beige powder.

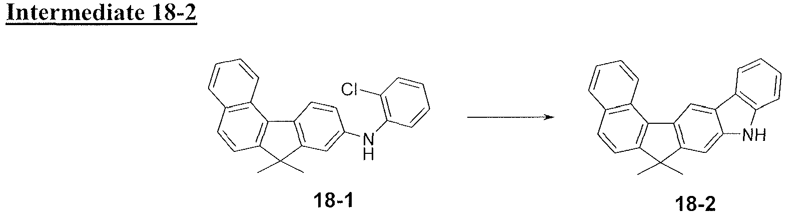

**18-1** (6.2 g, 13.95 mmol) and potassium carbonate (4.65 g, 33.65 mmol) are suspended in 90 mL of dimethylacetamide. To the suspension are PCy₃-HBF₄ (248 mg, 0.67 mmol) and Pd(OAc)₂ (62 mg, 0.27 mmol) added, and the mixture is stirred at 135 °C for 4.5 h. The reaction mixture is cooled at room temperature, and the solid is removed by filtration. The filtrate is concentrated, and the crude product is purified by recrystallization with a mixed solvent of heptane and ethyl acetate to yield 3.54 g (63%) of **18-2** as a beige powder.

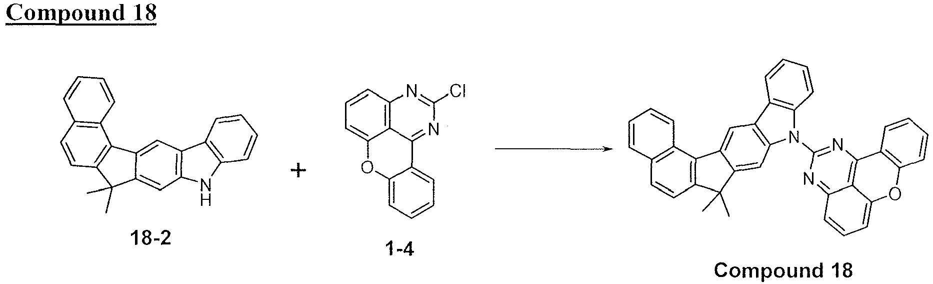

**1-4** (2.80 g, 11.00 mmol), **18-2** (3.33 g, 10.00 mmol) and sodium *tert*-butoxide (1.06 g, 11.00 mmol) are added in 50 mL of toluene. The mixture is evacuated and purged with argon gas three times. Then, ^{t}Bu₃P-HBF₄ (232 mg, 0.80 mmol) and Pd₂(dba)₃ (183 mg, 0.20 mmol) are added to the mixture, and the reaction mixture is stirred at 80°C for 17 h. The reaction mixture is cooled to room temperature. Then a solid is collected by filtration, and washed with methanol. The crude product is recrystallized with dimethylsulfoxide under nitrogen atmosphere to yield 4.27 g (78%) of **Compound 18** as a yellow powder.
¹H NMR (300 MHz, CDCl₃) δ 9.21 (s, 1H), 9.13-9.06 (m, 1H), 9.04-9.02 (m, 2H), 8.77-8.69 (m, 1H), 8.36-8.29 (m, 1H), 8.02 (dd, *J* = 8.3, 1.3 Hz, 1H), 7.98-7.85 (m, 2H), 7.83-7.67 (m, 4H), 7.63-7.57 (m, 2H), 7.54-7.41 (m, 3H), 7.24 (dd, *J* = 8.1, 0.8 Hz, 1H)), 1.75 (s, 6H)
LC-MS (m/z): 551 [M]

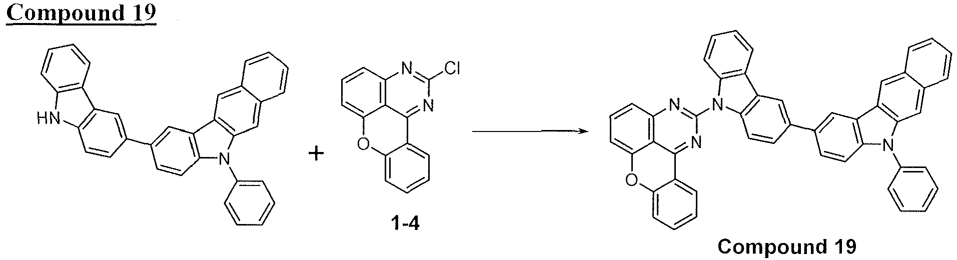

**1-4** (0.64 g, 2.52 mmol), 2-(9H-carbazol-3-yl)5-phenyl-5H-benzo[b]carbazole (1.05 g, 2.29 mmol), and sodium *tert*-butoxide (308 mg, 3.2 mmol) are added to 23 mL of Toluene. The mixture is evacuated and purged with argon gas three times. Then, ^{t}Bu₃P-HBF₄ (53 mg, 0.18 mmol) and Pd₂(dba)₃ (42 mg, 0.05 mmol) are added to the mixture, and the reaction mixture is stirred at 80°C for overnight. The reaction mixture is cooled to room temperature. Then a solid is collected by filtration, and washed with methanol. The crude product is recrystallized with chloroform and ethanol to yield 1.45 g (94%) of **Compound 19** as a yellow powder.
¹H NMR (300 MHz, CDCl₃) δ 9.17-9.06 (m, 2H), 8.73 (d, *J* = 1.3 Hz, 1H), 8.70-8.61 (m, 2H), 8.42 (dd, *J* = 2.0, 0.6 Hz, 1H), 8.21 (ddd, *J* = 7.7, 1.3, 0.6 Hz, 1H), 8.16-8.08 (m, 1H), 8.00-7.83 (m, 4H), 7.82-7.36 (m, 15H), 7.17 (dd, *J* = 8.0, 0.8 Hz, 1H)
LC-MS (m/z): 677 [M+1]

### Compound 20

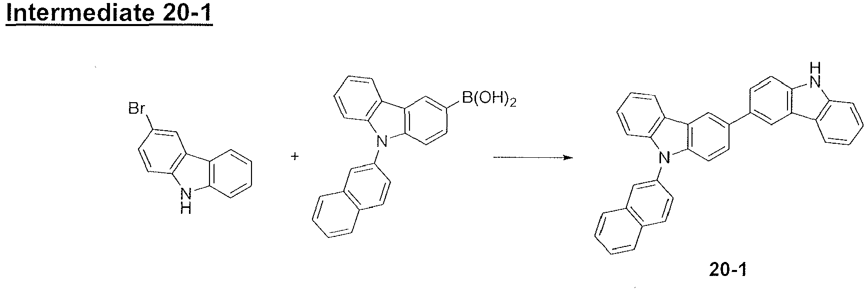

3-Bromocarbazole (7,87 g, 32.00 mmol), [9-(2-naphthyl)carbazole-3-yl]boronic acid (11.33 g, 33.60 mmol) and potassium phosphate (16.98 g, 80.00 mmol) are dissolved in 120 mL of tetra-hydrofuran and 30 mL of water. The mixture is evacuated and purged with argon, then argon is bubbled through for 15 minutes. Tri-t-butylphosphoniumtetrafluoroborat (371 mg, 1.28 mmol) and Pd₂(dba)₃ (586 mg, 0.64 mmol) are added and the mixture is refluxed for 2 hours. After cooling to room temperature, 100 mL of water are added and the reaction mixture is filtered. The filtrate is transferred to a separation funnel and the product is extracted twice with toluene. The organic layer is washed with brine, dried with Na₂SO₄ and the solvent is evaporated. The crude product is suspended in THF and heated to reflux for 15 minutes, then cooled to room temperature, filtered and the residue dried in vacuo to yield 9.84 g (67%) of 20-1 as a white powder.
LC-MS: 458 [M]

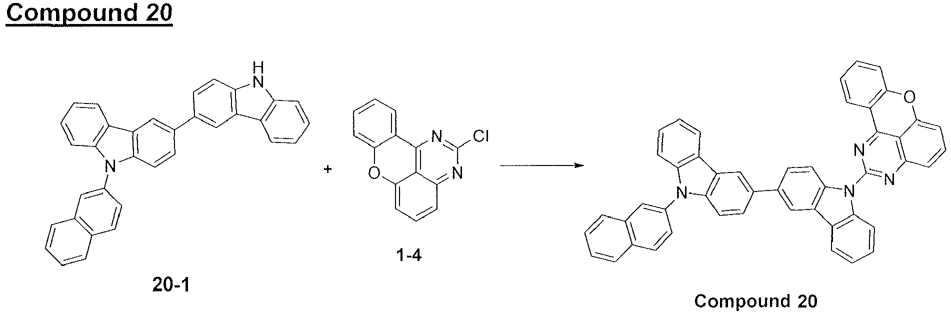

**1-4** (1.54 g, 6.05 mmol), 20-1 (2.52 g, 5.50 mmol) and sodium tert-butoxide (809 mg, 8.25 mmol) are suspended in 50 mL of toluene. The mixture is evacuated and purged with argon, then argon is bubbled through. 4,5-Bis(diphenylphosphino)-9,9-dimethylxanthene (127 mg, 0.22 mmol) and Pd₂(dba)₃ (101 mg, 0.11 mmol) are added and the reaction mixture is heated to 80°C for 2 hours. After cooling to room temperature, the solid is collected by filtration and washed with toluene and ethanol. The crude product is crystallized from chlorobenzene to yield 2.57 g (69%) of **compound 20** as a yellow solid.
¹H NMR (300 MHz, CDCl₃) δ 9.10-9.07 (m, 2H), 8.58 (dd, *J* = 7.9, 1.7 Hz, 1H), 8.54 (d, *J* = 1.8 Hz, 1H), 8.37 (d, *J* = 1.9 Hz, 1H), 8.30 (dd, *J* = 7.7, 1.0 Hz, 1H), 8.17 (dd, *J* = 7.8, 1.3 Hz, 1H), 8.13-8.11 (m, 2H), 8.02 (dt, *J* = 7.0, 3.6 Hz, 1H), 7.96 (dt, *J* = 7.0, 3.4 Hz, 1H), 7.90 (dd, *J* = 8.7, 2.0 Hz, 1H), 7.84-7.82 (m, 2H), 7.75 (dd, *J* = 8.7, 2.0 Hz, 1H), 7.68-7.32 (m, 12H), 7.11 (d, *J* = 8.0 Hz, 1H)
LC-MS (m/z): 676 [M]

### Compound 21

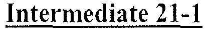

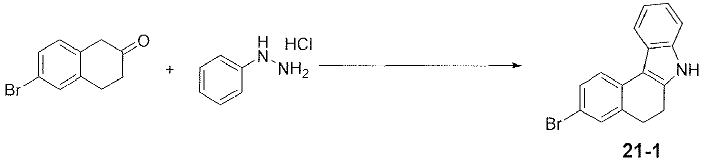

Phenylhydrazine hydrochloride (8.03 g, 55.5 mmol) is dissolved in 280 ml of EtOH and 32 mL of acetic acid. The mixture is stirred at 80 °C for 15 min. After that, 6-Bromo-2-tetralone (12.5 g, 55.5 mmol) is added dropwise to the solution, and the mixture is stirred at 85 °C for 20 hours. The reaction mixture is evaporated to remove EtOH. 100ml of H₂O is added to the resulting solution, which is filtrated to give a crude material. The crude material is purified by column chromatography by eluting with a mixed solvent of heptane and dichloromethane, and is then washed with Hexane to yield 11.7 g (71%) of **21-1** as a white powder.
LC-MS (m/z): 298 [M+1]

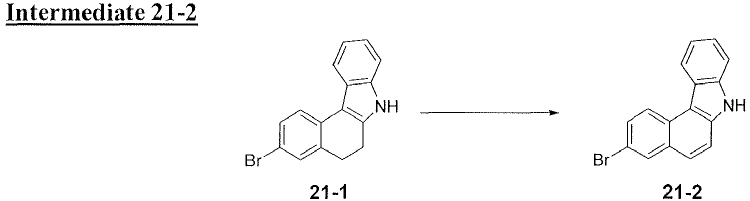

**21-1** (11.7 g, 39.2 mmol) and chloranil (13.5 g, 54.9 mmol) are added to 80 mL of Xylene. The resulting mixture is stirred at 130 °C for 20 hours. The reaction mixture is cooled at room temperature and is then filtered through a pad of celite washing well with CH₂Cl₂. The resulting solution is evaporated to give a crude material. The crude material is purified by CombiFlash eluting with a mixed solvent of heptane and dichloromethane, and is then washed with Hexane to yield 9.87 g (85%) of **21-2** as a white solid. LC-MS (m/z): 296 [M+1]

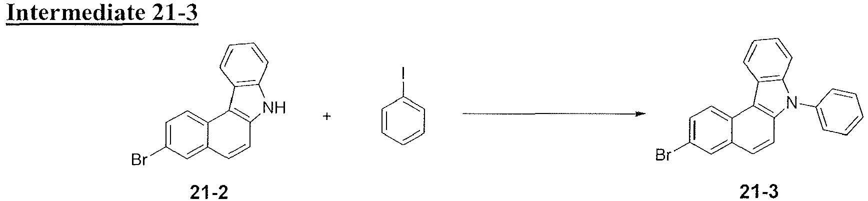

**21-2** (8.00 g, 27.0 mmol), iodobenzene (11.0 g, 54.0 mmol), CuI (5.14 g, 54.0 mmol), K₃PO₄ (11.5 g, 54.0 mmol) and cyclohexanediamine (6.17 g, 54.0 mmol) are added in 135 mL of 1,4-dioxane, and degassed under N₂ for 5 minutes. The mixture is heated at 115 °C for 24 hours. The reaction mixture is cooled to room temperature and is then filtered through a pad of celite washing well with ethyl acetate. The filtrate is evaporated to give a crude material as a black oil. The crude product i purified by CombiFlash eluting with a mixed solvent of heptane and dichloromethane, and is then washed with Hexane to yield 5.56 g (55%) of **21-3** as a white solid.
LC-MS (m/z): 372 [M+1]

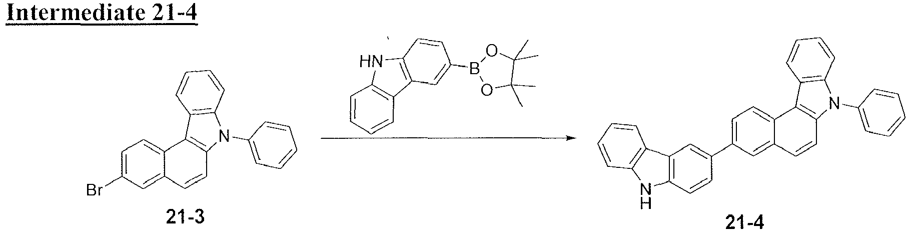

**21-3** (5.50 g, 14.8 mmol) is combined with 3-(4,4,5,5-tetramethyl-1,3,2-dioxaborolan-2-yl)carbazole (3.91 g, 13.3 mmol), Pd(dppf)Cl₂ (0.604 g, 0.740 mmol), and 2M Na₂CO₃ aq. (14 ml) in Toluene/EtOH (30 ml, 2:1 mixed solution) and degassed under N₂ for 5 minutes. The resulting mixture is heated at 90 °C for 12 hours. The reaction mixture is cooled to room temperature and is then filtered through a pad of celite washing well with dichloromethane. The resulting solution is evaporated to give a crude material as a black oil. The crude material is purified by CombiFlash eluting with a mixed solvent of heptane and dichloromethane, and is then washed with methanol to yield 4.25 g (70%) of **21-4** as a white solid. LC-MS (m/z): 459 [M+1]

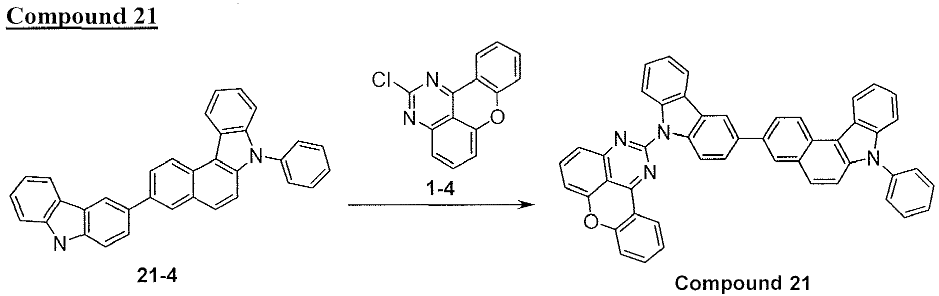

**21-4** (2.35 g, 5.12 mmol), **1-4** (1.10 g, 4.32 mmol), and sodium *tert*-butoxide (984 mg, 10.2 mmol) are added to 25 mL of xylene. The mixture is evacuated and purged with argon gas three times. Then, ^{t}Bu₃P-HBF₄ (297 mg, 1.02 mmol) and Pd₂(dba)₃ (234 mg, 0.256 mmol) are added to the mixture, and the reaction mixture is stirred at 130°C for 24 h. The reaction mixture is cooled to room temperature and is then filtered through a pad of celite washing well with THF. The filtrate is evaporated to give a crude material as a black oil. The crude material is purified by CombiFlash eluting with a mixed solvent of heptane and THF, and is then washed with MeOH to yield 2.50 g (72%) of compound **21** as a yellow solid.
LC-MS (m/z): 677 [M+1]

### Compound 22

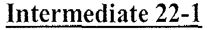

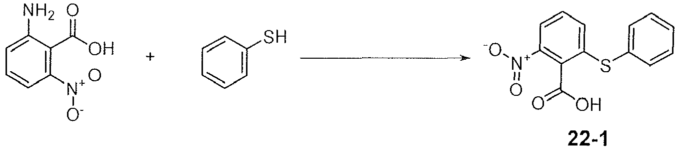

2-Amino-6-nitrobenzoic acid (8.38 g, 46.00 mmol) is dissolved in 35 mL of sulfuric acid, and the solution is cooled at 5 °C. To the solution is a solution of 4.4 g of sodium nitrite dissolved in 26 mL of water added dropwise. The reaction mixture is then poured into a stirred solution of benzenethiol ([4.97 g, 45.08 mmol] in 85 mL of 35% NaOH solution), and the mixture is stirred at 95 °C overnight. The reaction mixture is extracted with toluene, and the aqueous layer is acidified with conc. HCl. Then, it is extracted with ethyl acetate, and the organic layer is evaporated after it is dried with MgSO₄. The crude is purified by column chromatography by eluting with a mixed solvent of toluene, ethanol, and acetic acid to yield 5.66 g (41%) of the intermediate **22-1.**

It is used for next reaction without further purification.

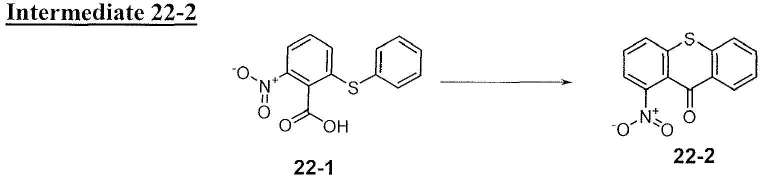

**22-1** (5.77 g, 20.96 mmol) is suspended in poly phosphoric acid (42 g) and 14 mL of acetic acid, and the mixture is stirred at 140 °C for 1h. The reaction mixture is poured into ice water and basified with 50% NaOH solution. The formed solid is collected by filtration, and washed out with chloroform. After the filtrated is concentrated, the crude product is purified by column chromatography by eluting with chloroform to yield 1.17 g (22%) of the intermediate **22-2.**
LC-MS (m/z) 258

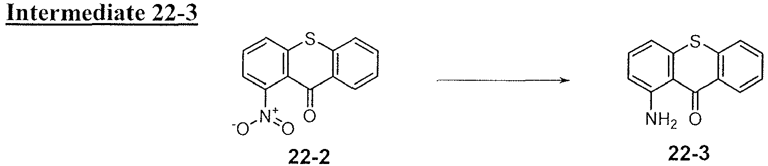

**22-2** (1.6 g, 6.13 mmol) is supended in 25 mL of acetic acid, and the mixture is heated to 50 °C. To this mixture are iron (1.74 g, 31.10 mmol) and 6 mL of water, and then the mixture is stirred at 100 °C for 35 min. After the reaction mixture is cooled at room temperature, it is basified with 2 M Na₂CO₃, and extracted with chloroform. After the organic layer is dried with Na2SO4, it is concentrated. The crude product is purified by column chromatography by eluting with chloroform to yield 1.74 g (92%) of the intermediate **22-3.**
LC-MS (m/z) 228

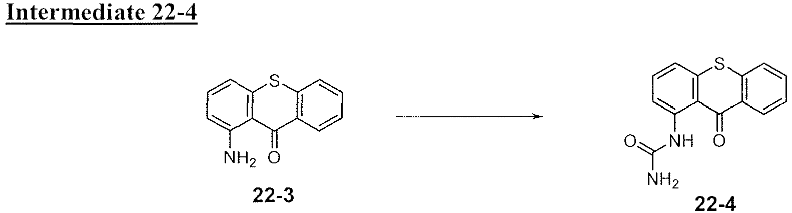

**22-3** (1.61 g, 7.08 mmol) is suspended in 71 mL of acetic acid and 6 mL of water. Sodium cyanate (0.69 g, 10.63 mmol) is added there, and the mixture is stirred at room temperature for 45 min. The reaction mixture is diluted with 142 mL of water to give a solid. The solid is collected by filtration to yield 1.74 g (85%) of the intermeidate **23-4.** It is used for the next reaction without further purification.

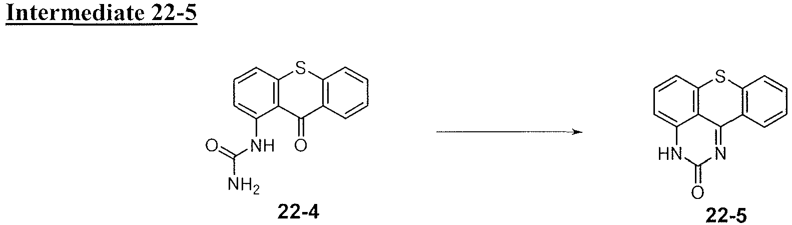

**22-4** (1.74 g, 6.44 mmol) is suspended in 805 mL of ethanol and potassium hydroxide (0.80 g, 14.16 mmol) is added. The mixture is stirred at 85 °C for 35 min. The reaction mixture is cooled at room temperature, and 14 mL of 1N HCl is added. Then, the solvent is evaporated to give the intermediate **22-5.** The crude product is used for the next reaction without purification.

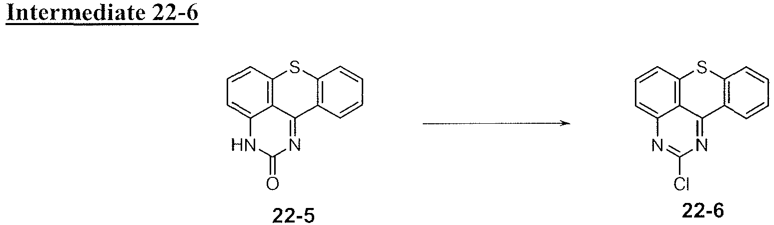

**22-5** (1.63 g, 6.47 mmol) is suspended in 24 mL of toluene, and heated at 50 °C. Phosphoryl chloride (7.44 g, 48.52 mmol) is added dropwise, and then DBU (1.97 g, 12.94 mmol) is added dropwise there. The mixture is refluxed overnight. The reaction mixture is cooled at room temperature, and poured into ice water. The mixture is stirred for 30 min. The aqueous layer is extracted with CHCl3. The organic layer is washed with brine, dried with Na2SO4, and then the solvent is evaporated. The crude product is purified by column chromatography by eluting with chloroform to yield 901 mg (51%) of the intermediate **22-6.**
LC-MS (m/z) 271/273

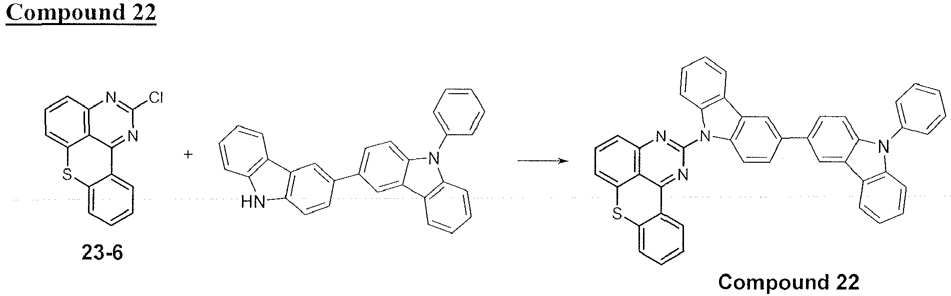

**23-6** (898 mg, 3.32 mmol), 3-(9H-carbazol-3-yl)-9-phenyl-carbazole (1.36 g, 3.32 mmol), and sodium tert-butoxide (446 mg, 4.64 mmol) are added to 27 mL of Toluene. The mixture is evacuated and purged with argon gas three times. Then, ^{t}Bu₃P-HBF₄ (77 mg, 0.27 mmol) and Pd₂(dba)₃ (61 mg, 0.06 mmol) are added to the mixture, and the reaction mixture is stirred at 80°C for over the weekend. The reaction mixture is cooled to room temperature, and the solid is collected by filtration. The solid is washed out with toluene and ethanol. It is purified by flash chromatography eluting with a mixed solvent of heptane and chloroform to yield 1.09 g (51%) of **Compound 22** as a yellow powder.
¹H NMR (300 MHz, DMSO-*d*₆) δ 9.01-8.86 (m, 3H), 8.81-8.67 (m, 2H), 8.42-8.40 (m, 2H), 8.04 (dd, *J* = 8.8, 2.0 Hz, 1H), 8.00-7.88 (m, 2H), 7.82-7.55 (m, 11H), 7.55-7.41 (m, 4H), 7.37-7.35 (m, 1H)
LC-MS (m/z): 677 [M+1]

Further application examples:

### Application Example 4

According to Application Example 1, Application example 4 is repeated except that the host (Compound 1) is replaced by Compound 3. The device result is shown in Table 3.

### Comparative application example 1

According to Application Example 1, Comparative application example 1 is repeated except that the host (Compound 1) is replaced by Comparative 2. The device result is shown in Table 3.

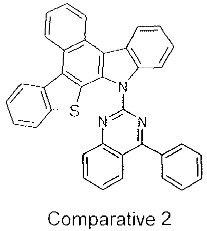

### OLED characterization

To characterize the OLED, electroluminescence spectra are recorded at various currents and voltages. In addition, the current-voltage characteristic is measured in combination with the luminance to determine luminous efficiency and external quantum efficiency (EQE). Driving voltage U, EQE, Commission Internationale de l'Éclairage (CIE) coordinate are given at 10mA/cm² except otherwise stated.

**Table 3**

| Appl. Ex. | Host | U(V) | EQE (%) | CIE x, y |
|---|---|---|---|---|
| Appl. Ex. 4 | Compound 3 | 4.93 | 15.0 | 0.67, 0.33 |
| Comp. Appl. Ex. 1 | Comparative 2 | 5.30 | 13.8 | 0.66, 0.33 |

The results are shown in Table 3. The CIE values show that the electroluminescence is originated from the red emitter compound (Compound D). Compound 3 can show lower driving voltage and higher EQE than Comparative 2.

### Application Example 5

According to Application Example 1, Application Example 5 is repeated except that the hole transporting layer is replaced by 210 nm-thick of Compound B as the first layer and 10 nm-thick of Compound B' as the second layer.

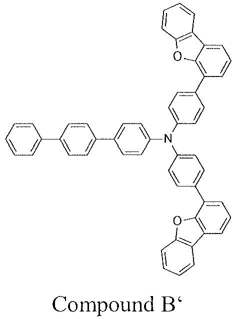

### Application Examples 6 to 21

According to Application Example 5, Application Examples 6 to 21 are repeated except that the host (Compound 1) is replaced by Compound 6 to 13 and 15 to 22. The device results are shown in Table 4.

### Comparative application example 2

According to Application Example 5, Comparative application example 2 is repeated except that the host (Compound 1) is replaced by Comparative 1. The device result is shown in Table 4.

**Table 4**

| Appl. Ex. | Host | U (V) | CIE (x,y) |
|---|---|---|---|
| Appl. Ex. 5 | Compound 1 | 5.36 | 0.66, 0.33 |
| Appl. Ex. 6 | Compound 6 | 4.52 | 0.66, 0.33 |
| Appl. Ex. 7 | Compound 7 | 4.52 | 0.66, 0.33 |
| Appl. Ex. 8 | Compound 8 | 5.13 | 0.66, 0.33 |
| Appl. Ex. 9 | Compound 9 | 4.89 | 0.66, 0.33 |
| Appl. Ex. 10 | Compound 10 | 4.89 | 0.66, 0.33 |
| Appl. Ex. 11 | Compound 11 | 5.08 | 0.66, 0.33 |
| Appl. Ex. 12 | Compound 12 | 5.11 | 0.66, 0.33 |
| Appl. Ex. 13 | Compound 13 | 4.66 | 0.66, 0.33 |
| Appl. Ex. 14 | Compound 15 | 5.40 | 0.66, 0.33 |
| Appl. Ex. 15 | Compound 16 | 4.88 | 0.66, 0.33 |
| Appl. Ex. 16 | Compound 17 | 5.11 | 0.66, 0.33 |
| Appl. Ex. 17 | Compound 18 | 4.88 | 0.66, 0.33 |
| Appl. Ex. 18 | Compound 19 | 4.68 | 0.66, 0.33 |
| Appl. Ex. 19 | Compound 20 | 5.12 | 0.66, 0.33 |
| Appl. Ex. 20 | Compound 21 | 5.10 | 0.66, 0.33 |
| Appl. Ex. 21 | Compound 22 | 5.34 | 0.66, 0.33 |
| Comp. Appl. Ex. 2 | Comparative 1 | 5.42 | 0.66, 0.34 |

The results are shown in Table 4. The CIE values show that the electroluminescence is originated from the red emitter compound (Compound D). The results of Table 4 demonstrate that the new compounds listed on the table show lower driving voltage than Comparative 1.

## Claims

1. A compound of general formula (IIb) or (IIc) 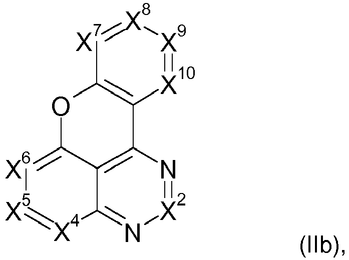 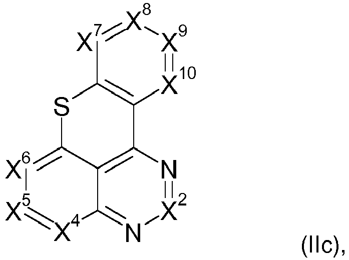 wherein
X² is CR²,
X⁴ is CR⁴,
X⁵ is CR⁵,
X⁶ is CR⁶,
X⁷ is CR⁷,
X⁸ is CR⁸,
X⁹ is CR⁹,
X¹⁰ is CR¹⁰,
R⁴, R⁵, R⁶, R⁷, R⁸, R⁹ and R¹⁰ are H,
R² is a group of formula -(L¹)ₒ₋(L²)ₚ₋(L³)_{q}-(L⁴)ᵣ-R¹⁴, wherein L¹, L², L³ and L⁴ are independently of each other selected from a C₆-C₄₀aryl group which is unsubstituted or substituted by at least one group E and a C₁-C₂₄heteroaryl group which is unsubstituted or substituted by at least one group E, and R¹⁴ is independently of each other selected from H or a C₁-C₂₅alkyl group which unsubstituted or substituted by at least one group E and/or interrupted by at least one group D,
o is 1, p is independently of each other 0 or 1, q is independently of each other 0 or 1 and r is independently of each other 0 or 1,
D is independently of each other -CO-, -COO-, -S-, -SO-, -SO₂-, -O-,-CR¹⁵=CR¹⁶-, -NR¹⁷-, -SiR²²R²³-, -POR²⁵-, -C≡C-,
E is independently of each other -OR²¹,-SR²¹, -NR¹⁷R¹⁸, -COR²⁰, -COOR¹⁹, -CONR¹⁷R¹⁸, -CN, -SiR²²R²³R²⁴, POR²⁵R²⁷, halogen, a C₆-C₆₀aryl group which is unsubstituted or is substituted by at least one -F, - CF₃, -CF₂CF₃, -CF₂CF₂CF₃, -CF(CF₃)₂, -(CF₂)₃CF₃ or -C(CF₃)₃, a C₁-C₁₈ alkyl or a C₁-C₁₈alkyl group which is interrupted by at least one O, a C₁-C₆₀heteroaryl group which is unsubstituted or substituted by at least one -F, - CF₃, -CF₂CF₃, -CF₂CF₂CF₃, -CF(CF₃)₂, -(CF₂)₃CF₃ or -C(CF₃)₃,
R¹⁵ and R¹⁶ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group which is interrupted by at least one O,
R¹⁷ and R¹⁸ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O, or
R¹⁷ and R¹⁸ together form a five or six membered aliphatic, aromatic or heteroaromatic ring,
R¹⁹ is H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O,
R²⁰ is H or a C₆-C₁₈aryl group which is unsubstituted or substituted by at least one C₁-C₁₈alkyl group or at least one C₁-C₁₈alkoxy group, a C₁-C₁₈ alkyl group or a C₁-C₁₈alkyl group, which is interrupted by at least one O,
R²¹ is independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O,
R²², R²³ and R²⁴ are independently of each other H, a C₁-C₁₈alkyl group, a C₆-C₁₈aryl group which is unsubstituted or substituted by at least one C₁-C₁₈alkyl group, and
R²⁵ and R²⁷ are independently of each other H, a C₁-C₁₈alkyl group, a C₆-C₁₈aryl group which is unsubstituted or substituted by at least one C₁-C₁₈alkyl group, a C₇-C₂₅aralkyl which is unsubstituted or substituted by at least one group E, a C₅-C₁₂cycloalkyl group which is unsubstituted or substituted by at least one group E.

2. The compound according to claim 1, wherein R² is a C₆-C₄₀aryl group which is unsubstituted or substituted by at least one group E or a C₁-C₂₄ N-comprising heteroaryl group which is unsubstituted or substituted by at least one group E, wherein E has the meanings as defined in claim 1.

3. The compound according to claim 1 or 2, wherein R² is a N-heteroaryl group according to general formula (XII) 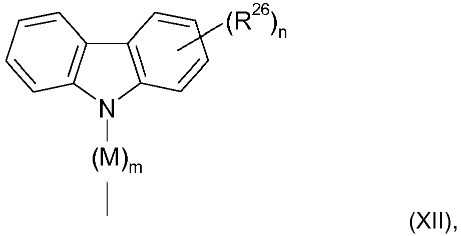 wherein
n is an integer of 0 to 8,
m is an integer of 0 to 4,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which unsubstituted or substituted by at least one group E and/or interrupted by at least one group D,
R²⁶ is independently of each other selected from E, a group of formula -(L¹)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, wherein o, p, q, r, L¹, L², L³, L⁴, R¹⁴, E and D have the meanings as defined in claim 1,
or at least two of R²⁶, if present at adjacent carbon atoms, may form at least one five or six membered, substituted or unsubstituted, saturated, unsaturated, aromatic or heteroaromatic ring or ring system, to which at least one further aromatic and/or heteroaromatic ring or ring system may be fused.

4. The compound according to claim 1 or 2, wherein R² is a N-heteroaryl group according to general formula (XIII) or (XV) 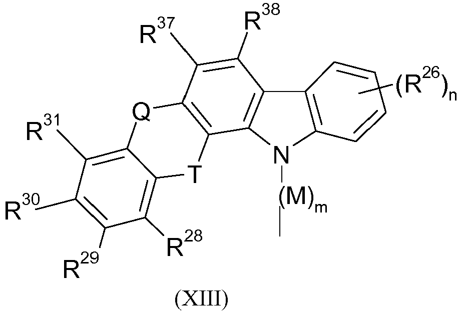
wherein M, m and R²⁶ have the meanings as defined in claim 3, n is 0 to 4,
R²⁸, R²⁹, R³⁰, R³¹, R³⁷ and R³⁸ are independently of each other selected from H, E, a group of formula -(L¹)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
or
at least two of R²⁸, R²⁹, R³⁰, R³¹, R³⁷ or R³⁸, if present at adjacent carbon atoms, together form at least one C₆-C₁₈aryl or C₂-C₁₈heteroaryl ring or ring system, and
Q and T are independently of each other selected from direct bond, S, O, SiR³²R³³, CR³⁴R³⁵ or NR³⁶,
wherein R³² and R³³ are independently of each other H, a C₁-C₁₈alkyl group, a C₆-C₁₈aryl group which is unsubstituted or substituted by at least one C₁-C₁₈alkyl group,
R³⁴ and R³⁵ are independently of each other H, E, a C₆-C₂₄aryl group which is unsubstituted or substituted by at least one group E, C₁-C₂₄heteroaryl group which is unsubstituted or substituted by at least one group E, a C₁-C₂₅alkyl group which unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, or spiro group, wherein R³⁴ and R³⁵ together form a five or six membered, substituted or unsubstituted, aliphatic ring,
R³⁶ is H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O,
wherein o, p, q, r, L¹, L², L³, L⁴, R¹⁴, D and E have the same meanings as defined in claim 1, 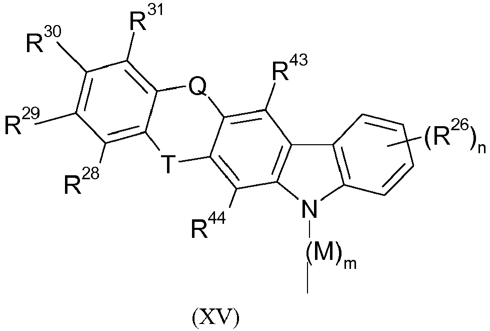
wherein M, m and R²⁶ have the meanings as defined in claim 3, n is 0 to 4,
R²⁸, R²⁹, R³⁰, R³¹, R⁴³ and R⁴⁴ are independently of each other selected from H, E, a group of formula -(L¹)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
or
at least two of R²⁸, R²⁹, R³⁰ or R³¹, if present at adjacent carbon atoms, together form at least one C₆-C₁₈aryl or C₂-C₁₈heteroaryl ring or ring system, and
Q and T are independently of each other selected from direct bond, S, O, SiR³²R³³, CR³⁴R³⁵ or NR³⁶,
wherein R³² and R³³ are independently of each other H, a C₁-C₁₈alkyl group, a C₆-C₁₈aryl group which is unsubstituted or substituted by at least one C₁-C₁₈alkyl group, ,
R³⁴ and R³⁵ are independently of each other H, E, a C₆-C₂₄aryl group which is unsubstituted or substituted by at least one group E, C₁-C₂₄heteroaryl group which is unsubstituted or substituted by at least one group E, a C₁-C₂₅alkyl group which unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, or spiro group, wherein R³⁴ and R³⁵ together form a five or six membered, substituted or unsubstituted, aliphatic ring,
R³⁶ is H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O,
wherein o, p, q, r, L¹, L², L³, L⁴, R¹⁴, D and E have the meanings as defined in claim 1.

5. The compound according to claim 4, wherein R² is a N-heteroaryl group according to general formula (XIV) 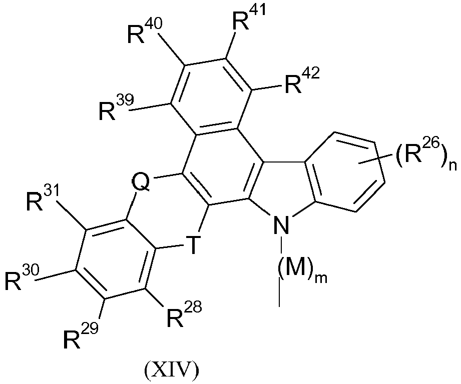
wherein R²⁶, R²⁸, R²⁹, R³⁰, R³¹, M, Q, T, n and m have the same meanings as defined in claim 3 or 4 and
R³⁹, R⁴⁰, R⁴¹, R⁴² are independently of each other selected from H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O.

6. The compound according to any of claims 3 to 5, wherein m is 1, M is a C₆-C₄₀arylene group which is unsubstituted or substituted by at least one group E, and at least two of R²⁶, if present at adjacent carbon atoms, form at least one five or six membered, substituted or unsubstituted, saturated, unsaturated, aromatic or heteroaromatic ring, wherein E has the meanings as defined in claim 1.

7. The compound according to claim 1 or 2, wherein R² is a heterocyclic group represented by general formula (XXI) 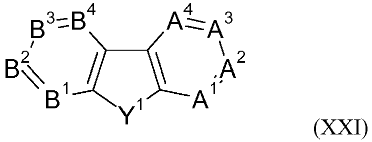 wherein
A¹ is CR⁶² or N,
A² is CR⁶³ or N,
A³ is CR⁶⁴ or N,
A⁴ is CR⁶⁵ or N,
B¹ is CR⁶⁶ or N,
B² is CR⁶⁷ or N,
B³ is CR⁶⁸ or N,
B⁴ is CR⁶⁹ or N,
Y¹ is independently of each other NR⁷⁰, CR⁷¹R⁷², O or S
R⁶², R⁶³, R⁶⁴, R⁶⁵, R⁶⁶, R⁶⁷, R⁶⁸ and R⁶⁹ are independently of each other selected from H, direct bond, E, a group of formula -(L¹)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
and/or
at least two of R⁶², R⁶³, R⁶⁴ and R⁶⁵, if present are directly bonded to the moiety represented by the general formula (XXII) by the two *-locations. 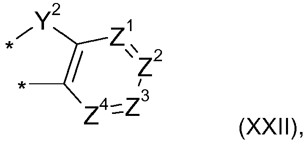 wherein
Y² is independently of each other NR⁷³, CR⁷⁴R⁷⁵, O or S,
Z¹ is CR⁷⁶,
Z² is CR⁷⁷,
Z³ is CR⁷⁸
Z⁴ is CR⁷⁹,
and/or
R⁶², R⁶³, R⁶⁴ or R⁶⁵, if present at adjacent carbon atoms, together form at least one C⁶-C₁₈aryl or C₂-C₁₈heteroaryl ring or ring system,
and/or
at least two of R⁶⁶, R⁶⁷, R⁶⁸ and R⁶⁹, if present are directly bonded to the moiety represented by the general formula (XXIII) by the two *-locations. 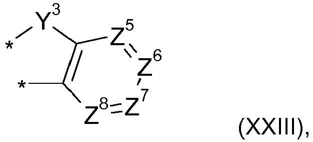 wherein
Y³ is independently of each other NR⁸⁰, CR⁸¹R⁸², O or S,
Z⁵ is CR⁸³,
Z⁶ is CR⁸⁴,
Z⁷ is CR⁸⁵,
Z⁸ is CR⁸⁶,
and/or
at least two of R⁶⁶, R⁶⁷, R⁶⁸ or R⁶⁹, if present at adjacent carbon atoms, together form at least one C₆-C₁₈aryl or C₂-C₁₈heteroaryl ring or ring system,
R⁷⁰, R⁷³ and R⁸⁰ are independently of each other selected from direct bond, H, E, a group of formula -(L¹)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
R⁷¹, R⁷², R⁷⁴, R75, R⁸¹ and R⁸² are, independently of each other selected from H, direct bond, E, a group of formula -(L¹)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, or R⁷¹ and R⁷², R⁷⁴ and R⁷⁵ and/or R⁸¹ and R⁸² together form at least one C₃-C₁₈-alkyl ring or ring system to which at least one C₆-C₁₈aryl ring or ring system may be attached,
R⁷⁶, R⁷⁷, R⁷⁸, R⁷⁹, R⁸³, R⁸⁴, R⁸⁵ and R⁸⁶ are independently of each other selected from H, direct bond, E, a group of formula -(L¹)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
and/or
at least two of R⁷⁶, R⁷⁷, R⁷⁸, R⁷⁹, R⁸³, R⁸⁴, R⁸⁵ or R⁸⁶, if present at adjacent carbon atoms, together form at least one C₆-C₁₈aryl or C₂-C₁₈heteroaryl ring or ring system,
wherein the substituent according to general formula (XXI) is connected to the compound according to general formula (IIb) or (IIc) via one of R⁷⁰, R⁷³, R⁸⁰, R⁶², R⁶³, R⁶⁴, R⁶⁵, R⁶⁶, R⁶⁷, R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁸⁴, R⁸⁵ or R⁸⁶, if present, wherein this respective R⁷⁰, R⁷³, R⁸⁰, R⁶², R⁶³, R⁶⁴, R⁶⁵, R⁶⁶, R⁶⁷, R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁸⁴, R⁸⁵ or R⁸⁶ is a direct bond, optionally interrupted by a group of formula -(M)ₘ-, in this case,
m is an integer of 0 to 4,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which unsubstituted or substituted by at least one group E and/or interrupted by at least one group D,
o is independently of each other 0 or 1, p is independently of each other 0 or 1, q is independently of each other 0 or 1 and r is independently of each other 0 or 1,
L¹, L², L³ and L⁴ are independently of each other selected from a C₆-C₄₀aryl group which is unsubstituted or substituted by at least one group E, C₁-C₂₄heteroaryl group which is unsubstituted or substituted by at least one group E,
R¹⁴ is independently of each other selected from H, E, a C₆-C₂₄aryl group which is unsubstituted or substituted by at least one group E, C₁-C₂₄heteroaryl group which is unsubstituted or substituted by at least one group E, a C₁-C₂₅alkyl group which unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
D is independently of each other -CO-, -COO-, -S-, -SO-, -SO₂-, -O-,-CR¹⁵=CR¹⁶-, -NR¹⁷-, ⁻SiR²²R²³-, -POR²⁵-, -C≡C-,
E is independently of each other -OR²¹,-SR²¹, -NR¹⁷R¹⁸, -COR²⁰, -COOR¹⁹, -CONR¹⁷R¹⁸, -CN, -SiR²²R²³R²⁴, POR²⁵R²⁷, halogen, a C₆-C₆₀aryl group which is unsubstituted or is substituted by at least one -F, - CF₃, -CF₂CF₃, -CF₂CF₂CF₃, -CF(CF₃)₂, -(CF₂)₃CF₃ or -C(CF₃)₃, a C₁-C₁₈ alkyl or a C₁-C₁₈alkyl group which is interrupted by at least one O, a C₁-C₆₀heteroaryl group which is unsubstituted or substituted by at least one -F, - CF₃, -CF₂CF₃, -CF₂CF₂CF₃, -CF(CF₃)₂, -(CF₂)₃CF₃ or -C(CF₃)₃,
R¹⁵ and R¹⁶ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group which is interrupted by at least one O,
R¹⁷ and R¹⁸ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O, or
R¹⁷ and R¹⁸ together form a five or six membered aliphatic, aromatic or heteroaromatic ring,
R¹⁹ is H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O,
R²⁰ is H or a C₆-C₁₈aryl group which is unsubstituted or substituted by at least one C₁-C₁₈alkyl group or at least one C₁-C₁₈alkoxy group, a C₁-C₁₈ alkyl group or a C₁-C₁₈alkyl group, which is interrupted by at least one O,
R²¹ is independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O,
R²², R²³ and R²⁴ are independently of each other H, a C₁-C₁₈alkyl group, a C₆-C₁₈aryl group which is unsubstituted or substituted by at least one C₁-C₁₈alkyl group, and
R²⁵ and R²⁷ are independently of each other H, a C₁-C₁₈alkyl group, a C₆-C₁₈aryl group which is unsubstituted or substituted by at least one C₁-C₁₈alkyl group, a C₇-C₂₅aralkyl which is unsubstituted or substituted by at least one group E, a C₅-C₁₂cycloalkyl group which is unsubstituted or substituted by at least one group E.

8. The compound according to claim 7, wherein the heterocyclic group according to general formula (XXI) is represented by any one of general formula (XXIa), (XXIb) or (XXIc) 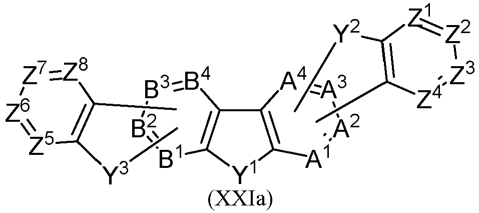 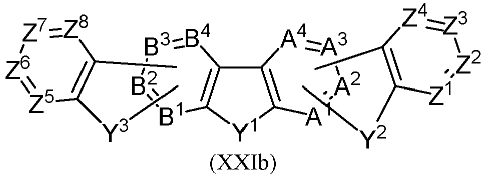 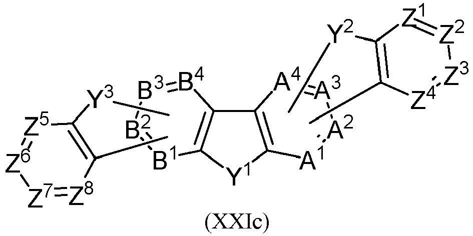 wherein A¹, A², A3, A4, B¹, B², B³, B⁴, Z¹, Z², Z³, Z⁴, Z⁵, Z⁶, Z⁷, Z⁸, Y¹, Y² and Y³ have the same meanings as defined in claim 7.

9. The compound according to claim 7 or 8 wherein one of Y¹, Y² and Y³ is NR⁷⁰, NR⁷³ or NR⁸⁰ respectively.

10. The compound according to any of claims 7 to 9, wherein two of Y¹, Y² and Y³ are NR⁷⁰, NR⁷³ or NR⁸⁰ respectively.

11. The compound according to any of claim 7 to 9 wherein the substituent according to general formula (XXI) is connected to the compound according to general formula (IIb) or (IIc) via R⁷⁰, R⁷³ or R⁸⁰, if present, wherein this respective R⁷⁰, R⁷³ or R⁸⁰ is a direct bond, optionally interrupted by a group of formula -(M)m-, wherein M and m have the meanings as defined in claim 3.

12. The compound according to claim 3, wherein the N-heteroaryl group according to general formula (XII) corresponds to a group according to general formula (XX) 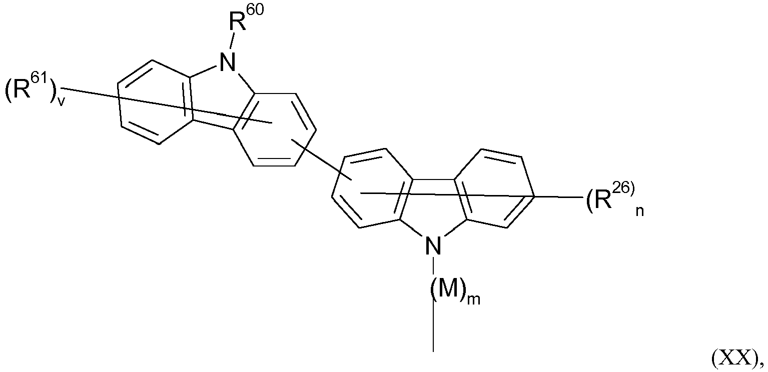 wherein
m, M, R²⁶ have the same meanings as defined in claim 3 ,
n is an integer of 0 to 7,
v is an integer of 0 to 7,
R⁶⁰ is independently of each other selected from H, E, a group of formula -(L¹)ₒ₋(L²)ₚ-(L³)_{q}-(L⁴)ᵣ-R¹⁴, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, R²⁶ is independently of each other selected from E, a group of formula -(L¹)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, wherein o, p, q, r, L¹, L², L³, L⁴, R¹⁴, E and D have the meanings as defined in claim 1,
R⁶¹ is independently of each other selected from E, a group of formula -(L¹)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, wherein o, p, q, r, L¹, L², L³, L⁴, R¹⁴, E and D have the meanings as defined in claim 1,
or at least two of R⁶¹, if present at adjacent carbon atoms, may form at least one five or six membered, substituted or unsubstituted, saturated, unsaturated, aromatic or heteroaromatic ring or ring system, to which at least one further aromatic and/or heteroaromatic ring or ring system may be fused.

13. A process for the preparation of a compound according to general formula (IIb) or (IIc) as defined in any of claims 1 to 12, at least comprising step (A)
(A) coupling of a compound according to general formula (Vaa) or (Vab) 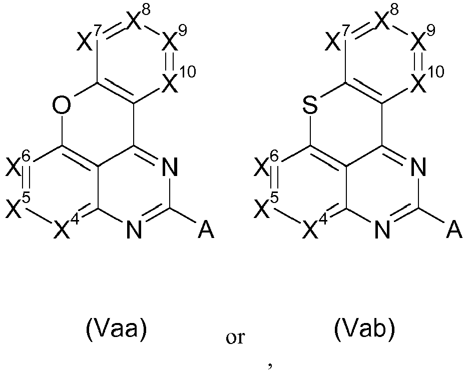 with a compound of formula R²-H to obtain a compound according to general formula (IIb) or (IIc), wherein X⁴, X⁵, X⁶, X⁷, X⁸, X⁹, X¹⁰ and R² have the same meanings as defined in any of claims 1 to 12 and A is a selected from Cl, Br, I, F, OSO₂CH₃, and OSO₂CF₃ or OSO₂C₆H₄CH₃.

14. An electronic device comprising at least one compound as defined in any of claims 1 to 12.

15. The electronic device, preferably an organic electroluminescence device, according to claim 14, comprising a cathode, an anode, and a plurality of organic thin film layers provided between the cathode and the anode, the organic thin film layers comprising an emitting layer comprising the at least one compound of general formula (IIb) or (IIc).

16. The electronic device, preferably an organic electroluminescence device, according to claim 14 or 15, wherein the emitting layer comprises a phosphorescent material, which is an ortho-metallated complex comprising a metal atom selected from iridium (Ir), osmium (Os) and platinum (Pt).

17. An electronic equipment comprising the organic electroluminescence device according to claims 15 or 16.

18. An emitting layer comprising at least one compound of general formula (IIb) or (IIc) as defined in any of claims 1 to 12.

19. Use of a compound according to general formula (IIb) or (IIc) as defined in any of claims 1 to 12 in an electronic device as a host material, a charge transporting material, charge and/or exciton blocking material.

20. A material for an organic electroluminescence device comprising at least one compound according to any one of claims 1 to 12.

## Patentansprüche

1. Verbindung der allgemeinen Formel (IIb) oder (IIc) 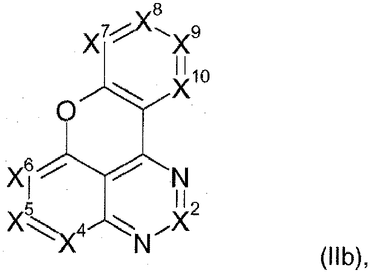 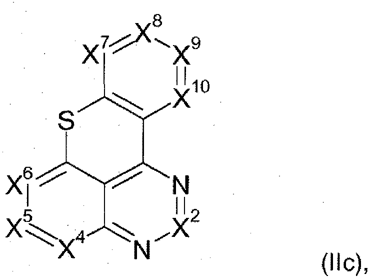 wobei
X² für CR² steht,
X⁴ für CR⁴ steht,
X⁵ für CR⁵ steht,
X⁶ für CR⁶ steht,
X⁷ für CR⁷ steht,
X⁸ für CR⁸ steht,
X⁹ für CR⁹ steht,
X¹⁰ für CR¹⁰ steht,
R⁴, R⁵, R⁶, R⁷, R⁸, R⁹ und R¹⁰ für H stehen,
R² für eine Gruppe der Formel - (L¹)ₒ-(L²)ₚ-(L³)_{q}-(L⁴)ᵣ-R¹⁴ steht, wobei L¹, L², L³ und L⁴ unabhängig voneinander aus einer C₆-C₄₀-Arylgruppe, die unsubstituiert oder durch mindestens eine Gruppe E substituiert ist, und einer C₁-C₂₄-Heteroaryl-gruppe, die unsubstituiert oder durch mindestens eine Gruppe E substituiert ist, ausgewählt sind und R¹⁴ unabhängig voneinander aus H oder einer C₁-C₂₅-Alkylgruppe, die unsubstituiert oder durch mindestens eine Gruppe E substituiert und/oder durch mindestens eine Gruppe D unterbrochen ist, ausgewählt ist,
o für 1 steht, p unabhängig voneinander für 0 oder 1 steht, q unabhängig voneinander für 0 oder 1 steht und r unabhängig voneinander für 0 oder 1 steht,
D unabhängig voneinander für -CO-, -COO-, -S-, -SO-, -SO₂-, -O-, -CR¹⁵=CR¹⁶- -NR¹⁷-, -SiR²²R²³-, -POR²⁵- oder -C≡C- steht;
E unabhängig voneinander für -OR²¹, -SR²¹, -NR¹⁷R¹⁸, -COR²⁰, -COOR¹⁹, -CONR¹⁷R¹⁸, -CN, -SiR²²R²³R²⁴, POR²⁵R²⁷, Halogen, eine C₆-C₆₀-Arylgruppe, die unsubstituiert oder durch mindestens ein -F, -CF₃, -CF₂CF₃, -CF₂CF₂CF₃, -CF(CF₃)₂, -(CF₂)₃CF₃ oder -C(CF₃)₃ substituiert ist, eine C₁-C₁₈-Alkylgruppe oder eine C₁-C₁₈-Alkylgruppe, die durch mindestens ein O unterbrochen ist, oder eine C₁-C₆₀-Heteroarylgruppe, die unsubstituiert oder durch mindestens ein -F, -CF₃, -CF₂CF₃, -CF₂CF₂CF₃, -CF(CF₃)₂, -(CF₂)₃CF₃ oder -C(CF₃)₃ substituiert ist, steht;
R¹⁵ und R¹⁶ unabhängig voneinander für H, eine C₆-C₁₈-Arylgruppe, die unsubstituiert oder durch mindestens eine C₁-C₁₈-Alkylgruppe oder mindestens eine C₁-C₁₈-Alkoxygruppe substituiert ist, eine C₁-C₁₈-Alkylgruppe oder eine C₁-C₁₈-Alkylgruppe, die durch mindestens ein O unterbrochen ist, stehen, R¹⁷ und R¹⁸ unabhängig voneinander für H, eine C₆-C₁₈-Arylgruppe, die unsubstituiert oder durch mindestens eine C₁-C₁₈-Alkylgruppe oder mindestens eine C₁-C₁₈-Alkoxygruppe substituiert ist, eine C₁-C₁₈-Alkylgruppe oder eine C₁-C₁₈-Alkylgruppe, die durch mindestens ein O unterbrochen ist, stehen oder
R¹⁷ und R¹⁸ zusammen einen fünf- oder sechsgliedrigen aliphatischen, aromatischen oder heteroaromatischen Ring bilden,
R¹⁹ für H, eine C₆-C₁₈-Arylgruppe, die unsubstituiert oder durch mindestens eine C₁-C₁₈-Alkylgruppe oder mindestens eine C₁-C₁₈-Alkoxygruppe substituiert ist, eine C₁-C₁₈-Alkylgruppe oder eine C₁-C₁₈-Alkylgruppe, die durch mindestens ein O unterbrochen ist, steht,
R²⁰ für H, eine C₆-C₁₈-Arylgruppe, die unsubstituiert oder durch mindestens eine C₁-C₁₈-Alkylgruppe oder mindestens eine C₁-C₁₈-Alkoxygruppe substituiert ist, eine C₁-C₁₈-Alkylgruppe oder eine C₁-C₁₈-Alkylgruppe, die durch mindestens ein O unterbrochen ist, steht,
R²¹ unabhängig voneinander für H, eine C₆-C₁₈-Arylgruppe, die unsubstituiert oder durch mindestens eine C₁-C₁₈-Alkylgruppe oder mindestens eine C₁-C₁₈-Alkoxygruppe substituiert ist, eine C₁-C₁₈-Alkylgruppe oder eine C₁-C₁₈-Alkylgruppe, die durch mindestens ein O unterbrochen ist, steht, R²², R²³ und R²⁴ unabhängig voneinander für H, eine C₁-C₁₈-Alkylgruppe oder eine C₆-C₁₈-Arylgruppe, die unsubstituiert oder durch mindestens eine C₁-C₁₈-Alkylgruppe substituiert ist, stehen und
R²⁵ und R²⁷ unabhängig voneinander für H, eine C₁-C₁₈-Alkylgruppe, eine C₆-C₁₈-Arylgruppe, die unsubstituiert oder durch mindestens eine C₁-C₁₈-Alkylgruppe substituiert ist, ein C₇-C₂₅-Aralkyl, das unsubstituiert oder durch mindestens eine Gruppe E substituiert ist, oder eine C₅-C₁₂-Cycloalkylgruppe, die unsubstituiert oder durch mindestens eine Gruppe E substituiert ist, stehen.

2. Verbindung nach Anspruch 1, wobei R² für eine C₆-C₄₀-Arylgruppe, die unsubstituiert oder durch mindestens eine Gruppe E substituiert ist, oder eine N-haltige C₁-C₂₄-Heteroarylgruppe, die unsubstituiert oder durch mindestens eine Gruppe E substituiert ist, steht, wobei E die in Anspruch 1 angegebenen Bedeutungen besitzt.

3. Verbindung nach Anspruch 1 oder 2, wobei R² für eine N-Heteroarylgruppe gemäß der allgemeinen Formel (XII) steht 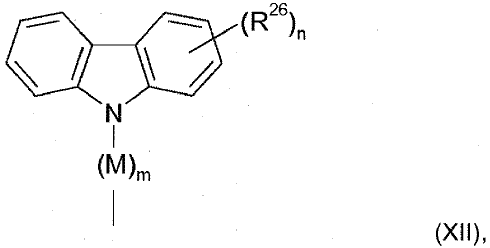 wobei
n für eine ganze Zahl von 0 bis 8 steht,
m für eine ganze Zahl von 0 bis 4 steht,
M für eine C₆-C₄₀-Arylengruppe, die unsubstituiert oder durch mindestens eine Gruppe E substituiert ist, eine C₁-C₂₄-Heteroarylengruppe, die unsubstituiert oder durch mindestens eine Gruppe E substituiert ist, oder eine C₁-C₂₅-Alkylengruppe, die unsubstituiert oder durch mindestens eine Gruppe E substituiert und/oder durch mindestens eine Gruppe D unterbrochen ist, steht,
R²⁶ unabhängig voneinander aus E, einer Gruppe der Formel -(L¹)ₒ-(L²)ₚ-(L³)-(L⁴)ᵣ-R¹⁴ und einer C₁-C₂₅-Alkylgruppe, die unsubstituiert oder durch mindestens eine Gruppe E substituiert und/oder durch mindestens eine Gruppe D unterbrochen ist, ausgewählt ist, wobei o, p, q, r, L¹, L², L³, L⁴, R¹⁴, E und D die in Anspruch 1 angegebenen Bedeutungen besitzen,
oder mindestens zwei von R²⁶ dann, wenn sie an benachbarten Kohlenstoffatomen vorliegen, mindestens einen fünf- oder sechsgliedrigen, substituierten oder unsubstituierten, gesättigten, ungesättigten, aromatischen oder heteroaromatischen Ring bzw. ein solches Ringsystem, woran mindestens ein weiterer aromatischer und/oder heteroaromatischer Ring bzw. ein solches Ringsystem anelliert sein kann, bilden können.

4. Verbindung nach Anspruch 1 oder 2, wobei R² für eine N-Heteroarylgruppe gemäß der allgemeinen Formel (XIII) oder (XV) steht 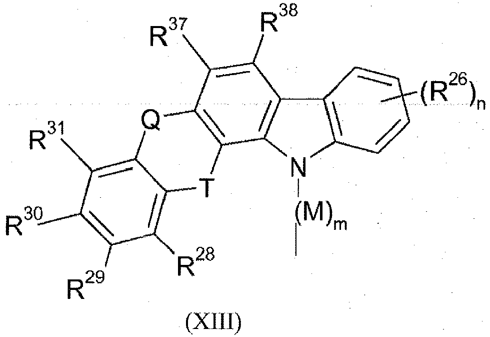 wobei
M, m und R²⁶ die in Anspruch 3 angegebenen Bedeutungen besitzen, n für 0 bis 4 steht,
R²⁸, R²⁹, R³⁰, R³¹, R³⁷ und R³⁸ unabhängig voneinander aus H, E, einer Gruppe der Formel -(L¹)ₒ-(L²)ₚ-(L³)_{q}-(L⁴)ᵣ-R¹⁴, einer C₁-C₂₅-Alkylgruppe, die unsubstituiert oder durch mindestens eine Gruppe E substituiert und/oder durch mindestens eine Gruppe D unterbrochen ist, einem C₇-C₂₅-Aralkyl, das unsubstituiert oder durch mindestens eine Gruppe E substituiert ist, oder einer C₅-C₁₂-Cycloalkylgruppe, die unsubstituiert oder durch mindestens eine Gruppe E substituiert ist, ausgewählt sind, oder
mindestens zwei von R²⁸, R²⁹, R³⁰, R³¹, R³⁷ und R³⁸ dann, wenn sie an benachbarten Kohlenstoffatomen vorliegen, zusammen mindestens einen C₆-C₁₈-Aryl- oder C₂-C₁₈-Heteroarylring bzw. ein solches Ringsystem bilden und
Q und T unabhängig voneinander aus einer direkten Bindung, S, O, SiR³²R³³, CR³⁴R³⁵ oder NR³⁶ ausgewählt sind,
wobei R³² und R³³ unabhängig voneinander für H, eine C₁-C₁₈-Alkylgruppe oder eine C₆-C₁₈-Arylgruppe, die unsubstituiert oder durch mindestens eine C₁-C₁₈-Alkylgruppe substituiert ist, stehen,
R³⁴ und R³⁵ unabhängig voneinander für H, E, eine C₆-C₂₄-Arylgruppe, die unsubstituiert oder durch mindestens eine Gruppe E substituiert ist, eine C₁-C₂₄-Heteroarylgruppe, die unsubstituiert oder durch mindestens eine Gruppe E substituiert ist, eine C₁-C₂₅-Alkylgruppe, die unsubstituiert oder durch mindestens eine Gruppe E substituiert und/oder durch mindestens eine Gruppe D unterbrochen ist, ein C₇-C₂₅-Aralkyl, das unsubstituiert oder durch mindestens eine Gruppe E substituiert ist, oder eine C₅-C₁₂-Cycloalkylgruppe, die unsubstituiert oder durch mindestens eine Gruppe E substituiert ist, oder eine Spirogruppe, wobei R³⁴ und R³⁵ zusammen einen fünf- oder sechsgliedrigen, substituierten oder unsubstituierten aliphatischen Ring bilden, stehen,
R³⁶ für H, eine C₆-C₁₈-Arylgruppe, die unsubstituiert oder durch mindestens eine C₁-C₁₈-Alkylgruppe oder mindestens eine C₁-C₁₈-Alkoxygruppe substituiert ist, eine C₁-C₁₈-Alkylgruppe oder eine C₁-C₁₈-Alkylgruppe, die durch mindestens ein O unterbrochen ist, steht,
wobei o, p, q, r, L¹, L², L³, L⁴, R¹⁴, D und E die in Anspruch 1 angegebenen Bedeutungen besitzen, 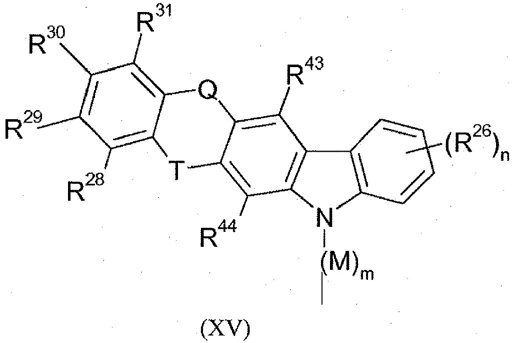 wobei
M, m und R²⁶ die in Anspruch 3 angegebenen Bedeutungen besitzen, n für 0 bis 4 steht,
R²⁸, R²⁹, R³⁰, R³¹, R⁴³ und R⁴⁴ unabhängig voneinander aus H, E, einer Gruppe der Formel -(L¹)ₒ-(L²)ₚ-(L³)_{q}-(L⁴)ᵣ-R¹⁴, einer C₁-C₂₅-Alkylgruppe, die unsubstituiert oder durch mindestens eine Gruppe E substituiert und/oder durch mindestens eine Gruppe D unterbrochen ist, einer C₇-C₂₅-Aralkylgruppe, die unsubstituiert oder durch mindestens eine Gruppe E substituiert ist, oder einer C₅-C₁₂-Cycloalkylgruppe, die unsubstituiert oder durch mindestens eine Gruppe E substituiert ist, ausgewählt sind, oder
mindestens zwei von R²⁸, R²⁹, R³⁰ und R³¹ dann, wenn sie an benachbarten Kohlenstoffatomen vorliegen, zusammen mindestens einen C₆-C₁₈-Aryl- oder C₂-C₁₈-Heteroarylring bzw. ein solches Ringsystem bilden und
Q und T unabhängig voneinander aus einer direkten Bindung, S, O, SiR³²R³³, CR³⁴R³⁵ oder NR³⁶ ausgewählt sind,
wobei R³² und R³³ unabhängig voneinander für H, eine C₁-C₁₈-Alkylgruppe oder eine C₆-C₁₈-Arylgruppe, die unsubstituiert oder durch mindestens eine C₁-C₁₈-Alkylgruppe substituiert ist, stehen,
R³⁴ und R³⁵ unabhängig voneinander für H, E, eine C₆-C₂₄-Arylgruppe, die unsubstituiert oder durch mindestens eine Gruppe E substituiert ist, eine C₁-C₂₄-Heteroarylgruppe, die unsubstituiert oder durch mindestens eine Gruppe E substituiert ist, eine C₁-C₂₅-Alkylgruppe, die unsubstituiert oder durch mindestens eine Gruppe E substituiert und/oder durch mindestens eine Gruppe D unterbrochen ist, ein C₇-C₂₅-Aralkyl, das unsubstituiert oder durch mindestens eine Gruppe E substituiert ist, oder eine C₅-C₁₂-Cycloalkylgruppe, die unsubstituiert oder durch mindestens eine Gruppe E substituiert ist, oder eine Spirogruppe, wobei R³⁴ und R³⁵ zusammen einen fünf- oder sechsgliedrigen, substituierten oder unsubstituierten aliphatischen Ring bilden, stehen,
R³⁶ für H, eine C₆-C₁₈-Arylgruppe, die unsubstituiert oder durch mindestens eine C₁-C₁₈-Alkylgruppe oder mindestens eine C₁-C₁₈-Alkoxygruppe substituiert ist, eine C₁-C₁₈-Alkylgruppe oder eine C₁-C₁₈-Alkylgruppe, die durch mindestens ein O unterbrochen ist, steht,
wobei o, p, q, r, L¹, L², L³, L⁴, R¹⁴, D und E die in Anspruch 1 angegebenen Bedeutungen besitzen.

5. Verbindung nach Anspruch 4, wobei R² für eine N-Heteroarylgruppe gemäß der allgemeinen Formel (XIV) steht 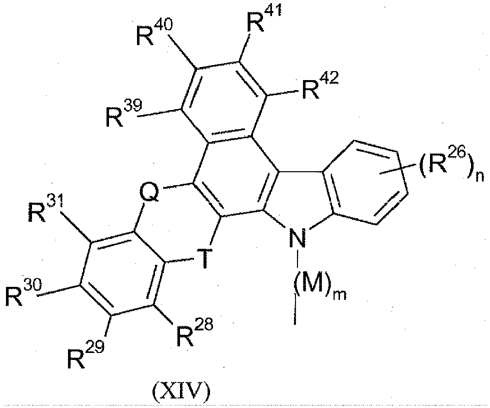
wobei R²⁶, R²⁸, R²⁹, R³⁰, R³¹, M, Q, T, n und m die gleichen Bedeutungen wie in Anspruch 3 oder 4 besitzen und
R³⁹, R⁴⁰, R⁴¹ und R⁴² unabhängig voneinander aus H, einer C₆-C₁₈-Arylgruppe, die unsubstituiert oder durch mindestens eine C₁-C₁₈-Alkylgruppe oder mindestens eine C₁-C₁₈-Alkoxygruppe substituiert ist, eine C₁-C₁₈-Alkylgruppe oder eine C₁-C₁₈-Alkylgruppe, die durch mindestens ein O unterbrochen ist, ausgewählt sind.

6. Verbindung nach einem der Ansprüche 3 bis 5, wobei m für 1 steht, M für eine C₆-C₄₀-Arylengruppe, die unsubstituiert oder durch mindestens eine Gruppe E substituiert ist, steht und mindestens zwei von R²⁶ dann, wenn sie an benachbarten Kohlenstoffatomen vorliegen, mindestens einen fünf- oder sechsgliedrigen, substituierten oder unsubstituierten, gesättigten, ungesättigten, aromatischen oder heteroaromatischen Ring bilden, wobei E die in Anspruch 1 angegebenen Bedeutungen besitzt.

7. Verbindung nach Anspruch 1 oder 2, wobei R² für eine heterocyclische Gruppe steht, die durch die allgemeine Formel (XXI) wiedergegeben wird 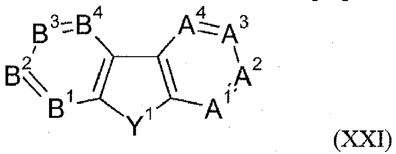 wobei
A¹ für CR⁶² oder N steht,
A² für CR⁶³ oder N steht,
A³ für CR⁶⁴ oder N steht,
A⁴ für CR⁶⁵ oder N steht,
B¹ für CR⁶⁶ oder N steht,
B² für CR⁶⁷ oder N steht,
B³ für CR⁶⁸ oder N steht,
B⁴ für CR⁶⁹ oder N steht,
Y¹ unabhängig voneinander für NR⁷⁰, CR⁷¹R⁷², O oder S steht,
R⁶², R⁶³, R⁶⁴, R⁶⁵, R⁶⁶, R⁶⁷, R⁶⁸ und R⁶⁹ unabhängig voneinander aus H, einer direkten Bindung, E, einer Gruppe der Formel -(L¹)ₒ-(L²)ₚ-(L³)_{q}-(L⁴)ᵣ-R¹⁴, einer C₁-C₂₅-Alkylgruppe, die unsubstituiert oder durch mindestens eine Gruppe E substituiert und/oder durch mindestens eine Gruppe D unterbrochen ist, einem C₇-C₂₅-Aralkyl, das unsubstituiert oder durch mindestens eine Gruppe E unterbrochen ist, und einer C₅-C₁₂-Cycloalkylgruppe, die unsubstituiert oder durch mindestens eine Gruppe E substituiert ist, ausgewählt sind
und/oder
mindestens zwei von R⁶², R⁶³, R⁶⁴ und R⁶⁵, sofern vorhanden, über die beiden *-Positionen direkt an die durch die allgemeine Formel (XXII) wiedergegebene Gruppierung gebunden sind 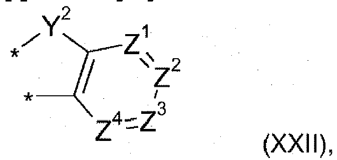 wobei
Y² unabhängig voneinander für NR⁷³, CR⁷⁴R⁷⁵, O oder S steht,
Z¹ für CR⁷⁶ steht,
Z² für CR⁷⁷ steht,
Z³ für CR⁷⁸ steht,
Z⁴ für CR⁷⁹ steht,
und/oder
R⁶², R⁶³, R⁶⁴ oder R⁶⁵ dann, wenn sie an benachbarten Kohlenstoffatomen vorliegen, zusammen mindestens einen C₆-C₁₈-Aryl- oder C₂-C₁₈-Heteroarylring bzw. ein solches Ringsystem bilden,
und/oder
mindestens zwei von R⁶⁶, R⁶⁷, R⁶⁸ und R⁶⁹, sofern vorhanden, über die beiden *-Positionen direkt an die durch die allgemeine Formel (XXIII) wiedergegebene Gruppierung gebunden sind 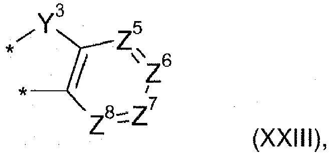 wobei
Y³ unabhängig voneinander für NR⁸⁰, CR⁸¹R⁸², O oder S steht,
Z⁵ für CR⁸³ steht,
Z⁶ für CR⁸⁴ steht,
Z⁷ für CR⁸⁵ steht,
Z⁸ für CR⁸⁶ steht,
und/oder
mindestens zwei von R⁶⁶, R⁶⁷, R⁶⁸ oder R⁶⁹ dann, wenn sie an benachbarten Kohlenstoffatomen vorliegen, zusammen mindestens einen C₆-C₁₈-Aryl- oder C₂-C₁₈-Heteroarylring bzw. ein solches Ringsystem bilden, R⁷⁰, R⁷³ und R⁸⁰ unabhängig voneinander aus einer direkten Bindung, H, einer Gruppe der Formel (L¹)ₒ-(L²)ₚ-(L³)_{q}-(L⁴)ᵣ-R¹⁴, einer C₁-C₂₅-Alkylgruppe, die unsubstituiert oder durch mindestens eine Gruppe E substituiert und/oder durch mindestens eine Gruppe D substituiert ist, einem C₇-C₂₅-Aralkyl, das unsubstituiert oder durch mindestens eine Gruppe E substituiert ist, und einer C₅-C₁₂-Cycloalkylgruppe, die unsubstituiert oder durch mindestens eine Gruppe E substituiert ist, ausgewählt sind, R⁷¹, R⁷², R⁷⁴, R⁷⁵, R⁸¹ und R⁸² unabhängig voneinander aus H, einer direkten Bindung, einer Gruppe der Formel (L¹)ₒ-(L²)ₚ-(L³)_{q}-(L⁴)ᵣ-R¹⁴, einer C₁-C₂₅-Alkylgruppe, die unsubstituiert oder durch mindestens eine Gruppe E substituiert und/oder durch mindestens eine Gruppe D substituiert ist, einem C₇-C₂₅-Aralkyl, das unsubstituiert oder durch mindestens eine Gruppe E substituiert ist, und einer C₅-C₁₂-Cycloalkylgruppe, die unsubstituiert oder durch mindestens eine Gruppe E substituiert ist, ausgewählt sind
oder R⁷¹ und R⁷², R⁷⁴ und R⁷⁵ und/oder R⁸¹ und R⁸² zusammen mindestens einen C₃-C₁₈-Alkylring bzw. ein solches Ringsystem, woran mindestens ein C₆-C₁₈-Arylring bzw. ein solches Ringsystem gebunden sein kann, bilden,
R⁷⁶, R⁷⁷, R⁷⁸, R⁷⁹, R⁸³, R⁸⁴, R⁸⁵ und R⁸⁶ unabhängig voneinander aus H, einer direkten Bindung, einer Gruppe der Formel (L¹)ₒ-(L²)ₚ-(L³)_{q}-(L⁴)ᵣ-R¹⁴, einer C₁-C₂₅-Alkylgruppe, die unsubstituiert oder durch mindestens eine Gruppe E substituiert und/oder durch mindestens eine Gruppe D substituiert ist, einem C₇-C₂₅-Aralkyl, das unsubstituiert oder durch mindestens eine Gruppe E substituiert ist, und einer C₅-C₁₂-Cycloalkylgruppe, die unsubstituiert oder durch mindestens eine Gruppe E substituiert ist, ausgewählt sind und/oder
mindestens zwei von R⁷⁶, R⁷⁷, R⁷⁸, R⁷⁹, R⁸³, R⁸⁴, R⁸⁵ und R⁸⁶ dann, wenn sie an benachbarten Kohlenstoffatomen vorliegen, zusammen mindestens einen C₆-C₁₈-Aryl- oder C₂-C₁₈-Heteroarylring bzw. ein solches Ringsystem bilden,
wobei der Substituent gemäß der allgemeinen Formel (XXI) über eines von R⁷⁰, R⁷³, R⁸⁰, R⁶², R⁶³, R⁶⁴, R⁶⁵, R⁶⁶, R⁶⁷, R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁸⁴, R⁸⁵ oder R⁸⁶, sofern vorhanden, an die Verbindung gemäß des all gemeinen Formel (IIb) oder (IIc) gebunden ist, wobei die jeweilige R⁷⁰, R⁷³, R⁸⁰, R⁶², R⁶³, R⁶⁴, R⁶⁵, R⁶⁶, R⁶⁷, R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁸⁴, R⁸⁵ oder R⁸⁶ in diesem Fall für eine direkte Bindung, die gegebenenfalls durch eine Gruppe der Formel -(M)ₘ- unterbrochen ist, steht,
m für eine ganze Zahl von 0 bis 4 steht,
M für eine C₆-C₄₀-Arylengruppe, die unsubstituiert oder durch mindestens eine Gruppe E substituiert ist, eine C₁-C₂₄-Heteroarylengruppe, die unsubstituiert oder durch mindestens eine Gruppe E substituiert ist, oder eine C₁-C₂₅-Alkylengruppe, die unsubstituiert oder durch mindestens eine Gruppe E substituiert und/oder durch mindestens eine Gruppe D unterbrochen ist, steht,
o unabhängig voneinander für 0 oder 1 steht, p unabhängig voneinander für 0 oder 1 steht, q unabhängig voneinander für 0 oder 1 steht und r unabhängig voneinander für 0 oder 1 steht,
L¹, L², L³ und L⁴ unabhängig voneinander aus einer C₆-C₄₀-Arylgruppe, die unsubstituiert oder durch mindestens eine Gruppe E substituiert ist, und einer C₁-C₂₄-Heteroarylgruppe, die unsubstituiert oder durch mindestens eine Gruppe E substituiert ist, ausgewählt sind,
R¹⁴ unabhängig voneinander aus H, E, einer C₆-C₂₄-Arylgruppe, die unsubstituiert oder durch mindestens eine Gruppe E substituiert ist, einer C₁-C₂₄-Heteroarylgruppe, die unsubstituiert oder durch mindestens eine Gruppe E substituiert ist, einer C₁-C₂₅-Alkylgruppe, die unsubstituiert oder durch mindestens eine Gruppe E substituiert und/oder durch mindestens eine Gruppe D unterbrochen ist, einem C₇-C₂₅-Aralkyl, das unsubstituiert oder durch mindestens eine Gruppe E substituiert ist, oder einer C₅-C₁₂-Cycloalkylgruppe, die unsubstituiert oder durch mindestens eine Gruppe E substituiert ist, ausgewählt ist,
D unabhängig voneinander für -CO-, -COO-, -S-, -SO-, -SO₂-, -O-, -CR¹⁵=CR¹⁶- -NR¹⁷-, -SiR²²R²³-, -POR²⁵- oder -C≡C- steht;
E unabhängig voneinander für -OR²¹, -SR²¹, -NR¹⁷R¹⁸, -COR²⁰, -COOR¹⁹, -CONR¹⁷R¹⁸ -CN, -SiR²²R²³R²⁴, POR²⁵R²⁷, Halogen, eine C₆-C₆₀-Arylgruppe, die unsubstituiert oder durch mindestens ein -F, -CF₃, -CF₂CF₃, -CF₂CF₂CF₃, -CF(CF₃)₂, -(CF₂)₃CF₃ oder -C(CF₃)₃ substituiert ist, ein C₁-C₁₈-Alkyl oder eine C₁-C₁₈-Alkylgruppe, die durch mindestens ein 0 unterbrochen ist, oder eine C₁-C₆-Heteroarylgruppe, die unsubstituiert oder durch mindestens ein -F, -CF₃, -CF₂CF₃, -CF₂CF₂CF₃, -CF(CF₃)₂, -(CF₂)₃CF₃ oder -C(CF₃)₃ substituiert ist, steht;
R¹⁵ und R¹⁶ unabhängig voneinander für H, eine C₆-C₁₈-Arylgruppe, die unsubstituiert oder durch mindestens eine C₁-C₁₈-Alkylgruppe oder mindestens eine C₁-C₁₈-Alkoxygruppe substituiert ist, eine C₁-C₁₈-Alkylgruppe oder eine C₁-C₁₈-Alkylgruppe, die durch mindestens ein O unterbrochen ist, stehen, R¹⁷ und R¹⁸ unabhängig voneinander für H, eine C₆-C₁₈-Arylgruppe, die unsubstituiert oder durch mindestens eine C₁-C₁₈-Alkylgruppe oder mindestens eine C₁-C₁₈-Alkoxygruppe substituiert ist, eine C₁-C₁₈-Alkylgruppe oder eine C₁-C₁₈-Alkylgruppe, die durch mindestens ein O unterbrochen ist, stehen oder
R¹⁷ und R¹⁸ zusammen einen fünf- oder sechsgliedrigen aliphatischen, aromatischen oder heteroaromatischen Ring bilden,
R¹⁹ für H, eine C₆-C₁₈-Arylgruppe, die unsubstituiert oder durch mindestens eine C₁-C₁₈-Alkylgruppe oder mindestens eine C₁-C₁₈-Alkoxygruppe substituiert ist, eine C₁-C₁₈-Alkylgruppe oder eine C₁-C₁₈-Alkylgruppe, die durch mindestens ein O unterbrochen ist, steht,
R²⁰ für H, eine C₆-C₁₈-Arylgruppe, die unsubstituiert oder durch mindestens eine C₁-C₁₈-Alkylgruppe oder mindestens eine C₁-C₁₈-Alkoxygruppe substituiert ist, eine C₁-C₁₈-Alkylgruppe oder eine C₁-C₁₈-Alkylgruppe, die durch mindestens ein O unterbrochen ist, steht,
R²¹ unabhängig voneinander für H, eine C₆-C₁₈-Arylgruppe, die unsubstituiert oder durch mindestens eine C₁-C₁₈-Alkylgruppe oder mindestens eine C₁-C₁₈-Alkoxygruppe substituiert ist, eine C₁-C₁₈-Alkylgruppe oder eine C₁-C₁₈-Alkylgruppe, die durch mindestens ein O unterbrochen ist, steht, R²², R²³ und R²⁴ unabhängig voneinander für H, eine C₁-C₁₈-Alkylgruppe oder eine C₆-C₁₈-Arylgruppe, die unsubstituiert oder durch mindestens eine C₁-C₁₈-Alkylgruppe substituiert ist, stehen und
R²⁵ und R²⁷ unabhängig voneinander für H, eine C₁-C₁₈-Alkylgruppe, eine C₆-C₁₈-Arylgruppe, die unsubstituiert oder durch mindestens eine C₁-C₁₈-Alkylgruppe substituiert ist, ein C₇-C₂₅-Aralkyl, das unsubstituiert oder durch mindestens eine Gruppe E substituiert ist, oder eine C₅-C₁₂-Cycloalkylgruppe, die unsubstituiert oder durch mindestens eine Gruppe E substituiert ist, stehen.

8. Verbindung nach Anspruch 7, wobei die heterocyclische Gruppe gemäß der allgemeinen Formel (XXI) durch eine der allgemeinen Formeln (XXIa), (XXIb) oder (XXIc) wiedergegeben wird 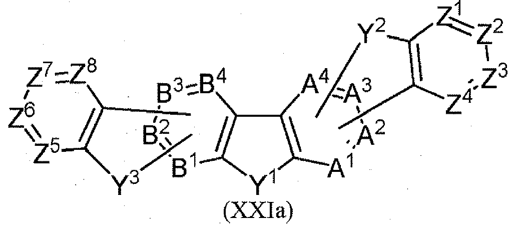 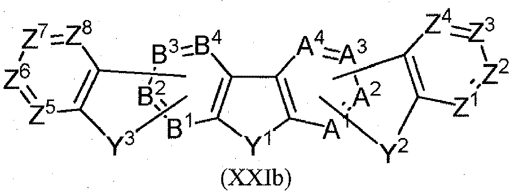 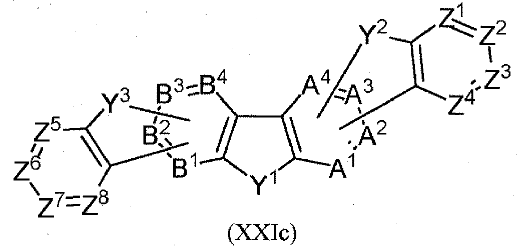 wobei A¹, A², A³, A⁴, B¹, B², B³, B⁴, Z¹, Z², Z³ Z⁴, Z⁵, Z⁶, Z⁷, Z⁸, Y¹, Y² und Y³ die in Anspruch 7 definierten Bedeutungen besitzen.

9. Verbindung nach Anspruch 7 oder 8, wobei eines von Y¹, Y² und Y³ für NR⁷⁰, NR⁷³ bzw. NR⁸⁰ steht.

10. Verbindung nach einem der Ansprüche 7 bis 9, wobei zwei von Y¹, Y² und Y³ für NR⁷⁰, NR⁷³ bzw. NR⁸⁰ stehen.

11. Verbindung nach einem der Ansprüche 7 bis 9, wobei der Substituent gemäß der allgemeinen Formel (XXI) über R⁷⁰, R⁷³ oder R⁸⁰, sofern vorhanden, mit der Verbindung gemäß der allgemeinen Formel (IIb) oder (IIc) verknüpft ist, wobei dieses jeweilige R⁷⁰, R⁷³ oder R⁸⁰ für eine direkte Bindung, die gegebenenfalls durch eine Gruppe der Formel -(M)ₘ-unterbrochen ist, steht, wobei M und m die in Anspruch 3 angegebenen Bedeutungen besitzen.

12. Verbindung nach Anspruch 3, wobei die N-Heteroarylgruppe gemäß der allgemeinen Formel (XII) einer Gruppe gemäß der allgemeinen Formel (XX) entspricht 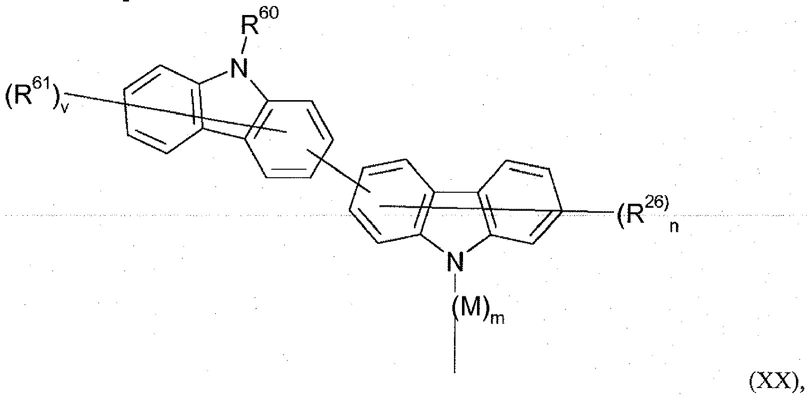 wobei
m, M und R²⁶ die in Anspruch 3 angegebenen Bedeutungen besitzen,
n für eine ganze Zahl von 0 bis 7 steht,
v für eine ganze Zahl von 0 bis 7 steht,
R⁶⁰ unabhängig voneinander aus H, E, einer Gruppe der Formel -(L¹)ₒ-(L²)ₚ-(L³)_{q}-(L⁴)ᵣ-R¹⁴, einer C₁-C₂₅-Alkylgruppe, die unsubstituiert oder durch mindestens eine Gruppe E substituiert und/oder durch mindestens eine Gruppe D unterbrochen ist, einem C₇-C₂₅-Aralkyl, das unsubstituiert oder durch mindestens eine Gruppe E substituiert ist, und einer C₅-C₁₂-Cycloalkylgruppe, die unsubstituiert oder durch mindestens eine Gruppe E substituiert ist, ausgewählt ist, R²⁶ unabhängig voneinander aus E, einer Gruppe der Formel -(L¹)ₒ-(L²)ₚ-(L³)_{q}-(L⁴)ᵣ-R¹⁴ und einer C₁-C₂₅-Alkylgruppe, die unsubstituiert oder durch mindestens eine Gruppe E substituiert und/oder durch mindestens eine Gruppe D unterbrochen ist, ausgewählt ist, wobei o, p, q, r, L¹, L², L³, L⁴, R¹⁴, E und D die in Anspruch 1 angegebenen Bedeutungen besitzen,
R⁶¹ unabhängig voneinander aus E, einer Gruppe der Formel -(L¹)ₒ-(L²)ₚ-(L³)_{q}-(L⁴)ᵣ-R¹⁴ und einer C₁-C₂₅-Alkylgruppe, die unsubstituiert oder durch mindestens eine Gruppe E substituiert und/oder durch mindestens eine Gruppe D unterbrochen ist, ausgewählt ist, wobei o, p, q, r, L¹, L², L³, L⁴, R¹⁴, E und D die in Anspruch 1 angegebenen Bedeutungen besitzen,
oder mindestens zwei von R⁶¹ dann, wenn sie an benachbarten Kohlenstoffatomen vorliegen, mindestens einen fünf- oder sechsgliedrigen, substituierten oder unsubstituierten, gesättigten, ungesättigten, aromatischen oder heteroaromatischen Ring bzw. ein solches Ringsystem, woran mindestens ein weiterer aromatischer und/oder heteroaromatischen Ring bzw. ein solches Ringsystem anelliert sein kann, bilden können.

13. Verfahren zur Herstellung einer Verbindung gemäß der allgemeinen Formel (IIb) oder (IIc) gemäß einem der Ansprüche 1 bis 12, umfassend mindestens Schritt (A)
(A) Kuppeln einer Verbindung der allgemeinen Formel (Vaa) oder (Vab) 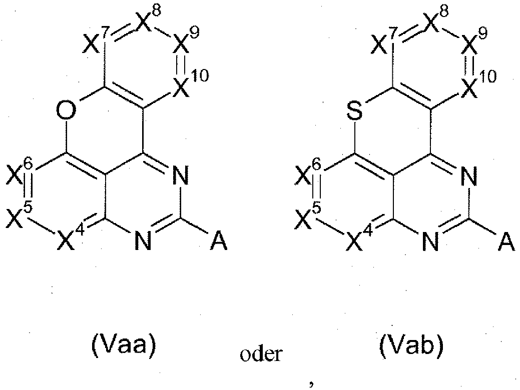 mit einer Verbindung der Formel R²-H zum Erhalt einer Verbindung gemäß der allgemeinen Formel (IIb) oder (IIc), wobei X⁴, X⁵, X⁶, X⁷, X⁸, X⁹, X¹⁰ und R² die in einem der Ansprüche 1 bis 12 angegebenen Bedeutungen besitzen und A aus Cl, Br, I, F, OSO₂CH₃ und OSO₂CF₃ oder OSO₂C₆H₄CH₃ ausgewählt ist.

14. Elektronische Vorrichtung, umfassend mindestens eine Verbindung gemäß einem der Ansprüche 1 bis 12.

15. Elektronische Vorrichtung, vorzugsweise organische Elektrolumineszenzvorrichtung, nach Anspruch 14 mit einer Kathode, einer Anode und mehreren organischen Dünnfilmschichten zwischen der Kathode und der Anode, wobei die organischen Dünnfilmschichten eine Emissionsschicht, die mindestens eine Verbindung der allgemeinen Formel (IIb) oder (IIc) umfasst, umfassen.

16. Elektronische Vorrichtung, vorzugsweise organische Elektrolumineszenzvorrichtung, nach Anspruch 14 oder 15, wobei die Emissionsschicht ein phosphoreszierendes Material umfasst, bei dem es sich um einen ortho-metallierten Komplex mit einem Metallatom, das aus Iridium (Ir) Osmium (Os) und Platin (Pt) ausgewählt ist, handelt.

17. Elektronisches Gerät, umfassend die organische Elektrolumineszenzvorrichtung nach Anspruch 15 oder 16.

18. Emissionsschicht, umfassend mindestens eine Verbindung der allgemeinen Formel (IIb) oder (IIc) gemäß einem der Ansprüche 1 bis 12.

19. Verwendung einer Verbindung der allgemeinen Formel (IIb) oder (IIc) gemäß einem der Ansprüche 1 bis 12 in einer elektronischen Vorrichtung als Wirtsmaterial, Ladungstransportmaterial oder Ladungs- und/oder Exzitonen-Blockiermaterial.

20. Material für eine organische Elektrolumineszenzvorrichtung, umfassend mindestens eine Verbindung nach einem der Ansprüche 1 bis 12.

## Revendications

1. Composé de formule générale (IIb) ou (IIc) 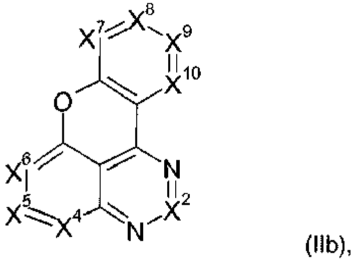 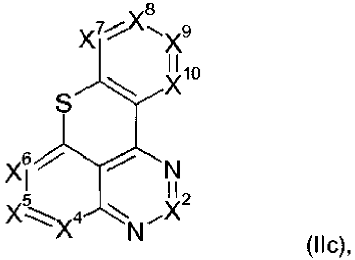 dans lequel
X² est CR²,
X⁴ est CR⁴,
X⁵ est CR⁵,
X⁶ est CR⁶,
X⁷ est CR⁷,
X⁸ est CR⁸,
X⁹ est CR⁹,
X¹⁰ est CR¹⁰,
R⁴, R⁵, R⁶, R⁷, R⁸, R⁹ et R¹⁰ sont H,
R² est un groupe de formule -(L¹)ₒ-(L²)ₚ-(L³)_{q}-(L⁴)-R¹⁴, dans lequel L¹, L², L³ et L⁴ sont, indépendamment les uns des autres, choisis parmi un groupe aryle en C₆-C₄₀ qui est non substitué ou substitué par au moins un groupe E et un groupe hétéroaryle en C₁-C₂₄ qui est non substitué ou substitué par au moins un groupe E, et R¹⁴ est, indépendamment les uns des autres, choisi parmi H ou un groupe alkyle en C₁-C₂₅ qui est non substitué ou substitué par au moins un groupe E et/ou interrompu par au moins un groupe D,
o est 1, p est, indépendamment les uns des autres, 0 ou 1, q est, indépendamment les uns des autres, 0 ou 1 et r est, indépendamment les uns des autres, 0 ou 1,
D est, indépendamment les uns des autres, -CO-, -COO-, -S-, -SO-, -SO₂-, -O-, -CR¹⁵=CR¹⁶-, -NR¹⁷-, -SiR²²R²³-, -POR²⁵-, -C≡C-,
E est, indépendamment les uns des autres, -OR²¹, -SR²¹, -NR¹⁷R¹⁸, -COR²⁰, -COOR¹⁹, -CONR¹⁷R¹⁸, -CN, -SiR²²R²³R²⁴, POR²⁵R²⁷, halogène, un groupe aryle en C₆-C₆₀ qui est non substitué ou est substitué par au moins
un -F, -CF₃, -CF₂CF₃, -CF₂CF₂CF₃, -CF (CF₃)₂, -(CF₂)₃CF₃ ou -C(CF₃)₃, un groupe alkyle en C₁-C₁₈ ou un groupe alkyle en C₁-C₁₈ qui est interrompu par au moins un O, un groupe hétéroaryle en C₁-C₆₀ qui est non substitué ou substitué par au moins un -F, -CF₃, -CF₂CF₃, -CF₂CF₂CF₃, -CF(CF₃)₂, -(CF₂)₃CF₃ ou -C(CF₃)₃,
R¹⁵ et R¹⁶ sont, indépendamment l'un de l'autre, H, un groupe aryle en C₆-C₁₈ qui est non substitué ou substitué par au moins un groupe alkyle en C₁-C₁₈ ou au moins un groupe alcoxy en C₁-C₁₈, un groupe alkyle en C₁-C₁₈ ou un groupe alkyle en C₁-C₁₈ qui est interrompu par au moins un O,
R¹⁷ et R¹⁸ sont, indépendamment l'un de l'autre, H, un groupe aryle en C₆-C₁₈ qui est non substitué ou substitué par au moins un groupe alkyle en C₁-C₁₈ ou au moins un groupe alcoxy en C₁-C₁₈, un groupe alkyle en C₁-C₁₈ ou un groupe alkyle en C₁-C₁₈, qui est interrompu par au moins un O, ou
R¹⁷ et R¹⁸ forment conjointement un cycle aliphatique, aromatique ou hétéroaromatique de cinq ou six chaînons, R¹⁹ est H, un groupe aryle en C₆-C₁₈ qui est non substitué ou substitué par au moins un groupe alkyle en C₁-C₁₈ ou au moins un groupe alcoxy en C₁-C₁₈, un groupe alkyle en C₁-C₁₈ ou un groupe alkyle en C₁-C₁₈, qui est interrompu par au moins un O,
R²⁰ est H ou un groupe aryle en C₆-C₁₈ qui est non substitué ou substitué par au moins un groupe alkyle en C₁-C₁₈ ou au moins un groupe alcoxy en C₁-C₁₈, un groupe alkyle en C₁-C₁₈ ou un groupe alkyle en C₁-C₁₈, qui est interrompu par au moins un O,
R²¹ est, indépendamment les uns des autres, H, un groupe aryle en C₆-C₁₈ qui est non substitué ou substitué par au moins un groupe alkyle en C₁-C₁₈ ou au moins un groupe alcoxy en C₁-C₁₈, un groupe alkyle en C₁-C₁₈ ou un groupe alkyle en C₁-C₁₈, qui est interrompu par au moins un O,
R²², R²³ et R²⁴ sont, indépendamment les uns des autres, H, un groupe alkyle en C₁-C₁₈, un groupe aryle en C₆-C₁₈ qui est non substitué ou substitué par au moins un groupe alkyle en C₁-C₁₈, et
R²⁵ et R²⁷ sont, indépendamment l'un de l'autre, H, un groupe alkyle en C₁-C₁₈, un groupe aryle en C₆-C₁₈ qui est non substitué ou substitué par au moins un groupe alkyle en C₁-C₁₈, un aralkyle en C₇-C₂₅ qui est non substitué ou substitué par au moins un groupe E, un groupe cycloalkyle en C₅-C₁₂ qui est non substitué ou substitué par au moins un groupe E.

2. Composé selon la revendication 1, dans lequel R² est un groupe aryle en C₆-C₄₀ qui est non substitué ou substitué par au moins un groupe E ou un groupe hétéroaryle comprenant N en C₁-C₂₄ qui est non substitué ou substitué par au moins un groupe E, dans lequel E a la signification telle que définie dans la revendication 1.

3. Composé selon la revendication 1 ou 2, dans lequel R² est un groupe N-hétéroaryle selon la formule générale (XII) 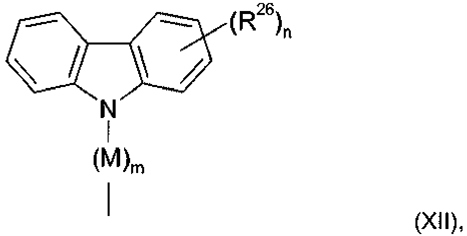 dans lequel
n est un entier de 0 à 8,
m est un entier de 0 à 4,
M est un groupe arylène en C₆-C₄₀ qui est non substitué ou substitué par au moins un groupe E, un groupe hétéroarylène en C₁-C₂₄ qui est non substitué ou substitué par au moins un groupe E ou un groupe alkylène en C₁-C₂₅ qui est non substitué ou substitué par au moins un groupe E et/ou interrompu par au moins un groupe D,
R²⁶ est, indépendamment les uns des autres, choisi parmi E, un groupe de formule -(L¹)ₒ-(L²)ₚ-(L³)_{q}-(L⁴)ᵣ-R¹⁴, un groupe alkyle en C₁-C₂₅, qui est non substitué ou substitué par au moins un groupe E et/ou interrompu par au moins un groupe D, dans lequel o, p, q, r, L¹, L², L³, L⁴, R¹⁴, E et D ont les significations telles que définies dans la revendication 1,
ou au moins deux des R²⁶, s'ils sont présents sur des atomes de carbone adjacents, peuvent former au moins un cycle ou système cyclique saturé, insaturé, aromatique ou hétéroaromatique, substitué ou non substitué, de cinq ou six chaînons auquel au moins un cycle ou système cyclique aromatique et/ou hétéroaromatique supplémentaire peut être condensé.

4. Composé selon la revendication 1 ou 2, dans lequel R² est un groupe N-hétéroaryle selon la formule générale (XIII) ou (XV) 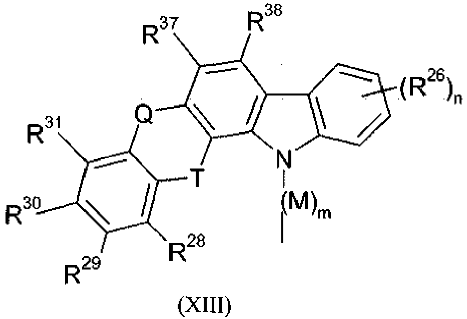
dans lequel M, m et R²⁶ ont les significations telles que définies dans la revendication 3, n est 0 à 4,
R²⁸, R²⁹, R³⁰, R³¹, R³⁷ et R³⁸ sont, indépendamment les uns des autres, choisis parmi H, E, un groupe de formule -(L¹)ₒ-(L²)ₚ-(L³)_{q}-(L⁴)ᵣ-R¹⁴, un groupe alkyle en C₁-C₂₅, qui est non substitué ou substitué par au moins un groupe E et/ou interrompu par au moins un groupe D, un aralkyle en C₇-C₂₅ qui est non substitué ou substitué par au moins un groupe E, un groupe cycloalkyle en C₅-C₁₂ qui est non substitué ou substitué par au moins un groupe E,
ou
au moins deux parmi R²⁸, R²⁹, R³⁰, R³¹, R³⁷ ou R³⁸, s'ils sont présents sur des atomes de carbone adjacents, forment conjointement au moins un cycle ou système cyclique aryle en C₆-C₁₈ ou hétéroaryle en C₂-C₁₈, et
Q et T sont, indépendamment l'un de l'autre, choisis parmi une liaison directe, S, O, SiR³²R³³, CR³⁴R³⁵ ou NR³⁶, dans lequel R³² et R³³ sont, indépendamment l'un de l'autre, H, un groupe alkyle en C₁-C₁₈, un groupe aryle en C₆-C₁₈ qui est non substitué ou substitué par au moins un groupe alkyle en C₁-C₁₈,
R³⁴ et R³⁵ sont, indépendamment l'un de l'autre, H, E, un groupe aryle en C₆-C₂₄ qui est non substitué ou substitué par au moins un groupe E, un groupe hétéroaryle en C₁-C₂₄ qui est non substitué ou substitué par au moins un groupe E, un groupe alkyle en C₁-C₂₅ qui est non substitué ou substitué par au moins un groupe E et/ou interrompu par au moins un groupe D, un aralkyle en C₇-C₂₅ qui est non substitué ou substitué par au moins un groupe E, un groupe cycloalkyle en C₅-C₁₂ qui est non substitué ou substitué par au moins un groupe E, ou un groupe spiro, dans lequel R³⁴ et R³⁵ forment conjointement un cycle aliphatique, substitué ou non substitué, de cinq ou six chaînons,
R³⁶ est H, un groupe aryle en C₆-C₁₈ qui est non substitué ou substitué par au moins un groupe alkyle en C₁-C₁₈ ou au moins un groupe alcoxy en C₁-C₁₈, un groupe alkyle en C₁-C₁₈ ou un groupe alkyle en C₁-C₁₈, qui est interrompu par au moins un O,
dans lequel o, p, q, r, L¹, L², L³, L⁴, R¹⁴, D et E ont les significations telles que définies dans la revendication 1, 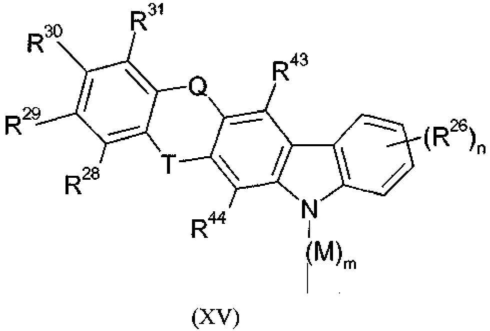
dans lequel M, m et R²⁶ ont les significations telles que définies dans la revendication 3, n est 0 à 4,
R²⁸, R²⁹, R³⁰, R³¹, R⁴³ et R⁴⁴ sont, indépendamment les uns des autres, choisis parmi H, E, un groupe de formule -(L¹)ₒ-(L²)ₚ-(L³)_{q}-(L⁴)ᵣ-R¹⁴, un groupe alkyle en C₁-C₂₅, qui est non substitué ou substitué par au moins un groupe E et/ou interrompu par au moins un groupe D, un groupe aralkyle en C₇-C₂₅ qui est non substitué ou substitué par au moins un groupe E, un groupe cycloalkyle en C₅-C₁₂ qui est non substitué ou substitué par au moins un groupe E, ou
au moins deux parmi R²⁸, R²⁹, R³⁰ ou R³¹, s'ils sont présents sur des atomes de carbone adjacents, forment conjointement au moins un cycle ou système cyclique aryle en C₆-C₁₈ ou hétéroaryle en C₂-C₁₈, et
Q et T sont, indépendamment l'un de l'autre, choisis parmi une liaison directe, S, O, SiR³²R³³, CR³⁴R³⁵ ou NR³⁶, dans lequel R³² et R³³ sont, indépendamment l'un de l'autre, H, un groupe alkyle en C₁-C₁₈, un groupe aryle en C₆-C₁₈ qui est non substitué ou substitué par au moins un groupe alkyle en C₁-C₁₈,
R³⁴ et R³⁵ sont, indépendamment l'un de l'autre, H, E, un groupe aryle en C₆-C₂₄ qui est non substitué ou substitué par au moins un groupe E, un groupe hétéroaryle en C₁-C₂₄ qui est non substitué ou substitué par au moins un groupe E, un groupe alkyle en C₁-C₂₅ qui est non substitué ou substitué par au moins un groupe E et/ou interrompu par au moins un groupe D, un aralkyle en C₇-C₂₅ qui est non substitué ou substitué par au moins un groupe E, un groupe cycloalkyle en C₅-C₁₂ qui est non substitué ou substitué par au moins un groupe E, ou un groupe spiro, dans lequel R³⁴ et R³⁵ forment conjointement un cycle aliphatique, substitué ou non substitué, de cinq ou six chaînons,
R³⁶ est H, un groupe aryle en C₆-C₁₈ qui est non substitué ou substitué par au moins un groupe alkyle en C₁-C₁₈ ou au moins un groupe alcoxy en C₁-C₁₈, un groupe alkyle en C₁-C₁₈ ou un groupe alkyle en C₁-C₁₈, qui est interrompu par au moins un O,
dans lequel o, p, q, r, L¹, L², L³, L⁴, R¹⁴, D et E ont les significations telles que définies dans la revendication 1.

5. Composé selon la revendication 4, dans lequel R² est un groupe N-hétéroaryle selon la formule générale (XIV) 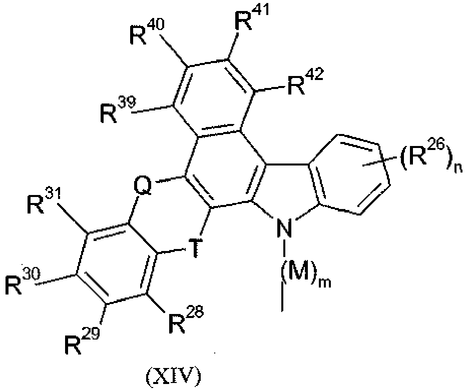
dans lequel R²⁶, R²⁸, R²⁹, R³⁰, R³¹, M, Q, T, n et m ont les significations telles que définies dans la revendication 3 ou 4 et
R³⁹, R⁴⁰, R⁴¹, R⁴² sont, indépendamment les uns des autres, choisis parmi H, un groupe aryle en C₆-C₁₈ qui est non substitué ou substitué par au moins un groupe alkyle en C₁-C₁₈ ou au moins un groupe alcoxy en C₁-C₁₈, un groupe alkyle en C₁-C₁₈ ou un groupe alkyle en C₁-C₁₈ qui est interrompu par au moins un O.

6. Composé selon l'une quelconque des revendications 3 à 5, dans lequel m est 1, M est un groupe arylène en C₆-C₄₀ qui est non substitué ou substitué par au moins un groupe E, et au moins deux des R²⁶, s'ils sont présents sur des atomes de carbone adjacents, forment au moins un cycle saturé, insaturé, aromatique ou hétéroaromatique substitué ou non substitué, de cinq ou six chaînons, dans lequel E a les significations telles que définies dans la revendication 1.

7. Composé selon la revendication 1 ou 2, dans lequel R² est un groupe hétérocyclique représenté par la formule générale (XXI) 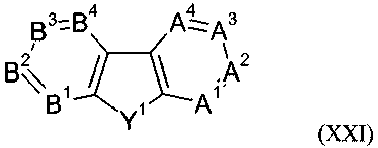 dans lequel
A¹ est CR⁶² ou N,
A² est CR⁶³ ou N,
A³ est CR⁶⁴ ou N,
A⁴ est CR⁶⁵ ou N,
B¹ est CR⁶⁶ ou N,
B² est CR⁶⁷ ou N,
B³ est CR⁶⁸ ou N,
B⁴ est CR⁶⁹ ou N,
Y¹ est, indépendamment les uns des autres, NR⁷⁰, CR⁷¹R⁷², O ou S
R⁶², R⁶³, R⁶⁴, R⁶⁵, R⁶⁶, R⁶⁷, R⁶⁸ et R⁶⁹ sont, indépendamment les uns des autres, choisis parmi H, une liaison directe, E, un groupe de formule -(L¹)ₒ-(L²)ₚ-(L³)_{q}-(L⁴)ᵣ-R¹⁴, un groupe alkyle en C₁-C₂₅, qui est non substitué ou substitué par au moins un groupe E et/ou interrompu par au moins un groupe D, un groupe aralkyle en C₇-C₂₅ qui est non substitué ou substitué par au moins un groupe E, un groupe cycloalkyle en C₅-C₁₂ qui est non substitué ou substitué par au moins un groupe E, et/ou au moins deux parmi R⁶², R⁶³, R⁶⁴ et R⁶⁵, s'ils sont présents, sont directement liés au fragment représenté par la formule générale (XXII) par les deux emplacements *. 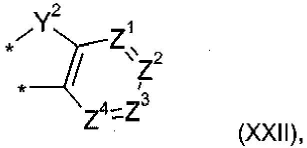 dans lequel
Y² est, indépendamment les uns des autres, NR⁷³, CR⁷⁴R⁷⁵, O ou S,
Z¹ est CR⁷⁶,
Z² est CR⁷⁷,
Z³ est CR⁷⁸,
Z⁴ est CR⁷⁹,
et/ou
R⁶², R⁶³, R⁶⁴ ou R⁶⁵, s'ils sont présents sur des atomes de carbone adjacents, forment conjointement au moins un cycle ou système cyclique aryle en C₆-C₁₈ ou hétéroaryle en C₂-C₁₈,
et/ou
au moins deux parmi R⁶⁶, R⁶⁷, R⁶⁸ et R⁶⁹, s'ils sont présents, sont directement liés au fragment représenté par la formule générale (XXIII) par les deux emplacements *, 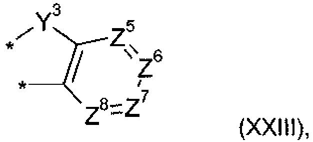 dans lequel
Y³ est, indépendamment les uns des autres, NR⁸⁰, CR⁸¹R⁸², O ou S,
Z⁵ est CR⁸³,
Z⁶ est CR⁸⁴,
Z⁷ est CR⁸⁵,
Z⁸ est CR⁸⁶,
et/ou
au moins deux parmi R⁶⁶, R⁶⁷, R⁶⁸ ou R⁶⁹, s'ils sont présents sur des atomes de carbone adjacents, forment conjointement au moins un cycle ou système cyclique aryle en C₆-C₁₈ ou hétéroaryle en C₂-C₁₈,
R⁷⁰, R⁷³ et R⁸⁰ sont, indépendamment les uns des autres, choisis parmi une liaison directe, H, E, un groupe de formule -(L¹)ₒ-(L²)ₚ-(L³)_{q}-(L⁴)ᵣ-R¹⁴, un groupe alkyle en C₁-C₂₅, qui est non substitué ou substitué par au moins un groupe E et/ou interrompu par au moins un groupe D, un aralkyle en C₇-C₂₅ qui est non substitué ou substitué par au moins un groupe E, un groupe cycloalkyle en C₅-C₁₂ qui est non substitué ou substitué par au moins un groupe E,
R⁷¹, R⁷², R⁷⁴, R⁷⁵, R⁸¹ et R⁸² sont, indépendamment les uns des autres, choisis parmi H, une liaison directe, E, un groupe de formule -(L¹)ₒ-(L²)ₚ-(L³)_{q}-(L⁴)ᵣ-R¹⁴, un groupe alkyle en C₁-C₂₅, qui est non substitué ou substitué par au moins un groupe E et/ou interrompu par au moins un groupe D, un aralkyle en C₇-C₂₅ qui est non substitué ou substitué par au moins un groupe E, un groupe cycloalkyle en C₅-C₁₂ qui est non substitué ou substitué par au moins un groupe E,
ou R⁷¹ et R⁷², R⁷⁴ et R⁷⁵ et/ou R⁸¹ et R⁸² forment conjointement au moins un cycle ou système cyclique alkyle en C₃-C₁₈ auquel au moins un cycle ou système cyclique aryle en C₆-C₁₈ peut être lié,
R⁷⁶, R⁷⁷, R⁷⁸, R⁷⁹, R⁸³, R⁸⁴, R⁸⁵ et R⁸⁶ sont, indépendamment les uns des autres, choisis parmi H, une liaison directe, E, un groupe de formule - (L¹)ₒ-(L²)ₚ-(L³)_{q}-(L⁴)ᵣ-R¹⁴, un groupe alkyle en C₁-C₂₅, qui est non substitué ou substitué par au moins un groupe E et/ou interrompu par au moins un groupe D, un aralkyle en C₇-C₂₅ qui est non substitué ou substitué par au moins un groupe E, un groupe cycloalkyle en C₅-C₁₂ qui est non substitué ou substitué par au moins un groupe E,
et/ou
au moins deux parmi R⁷⁶, R⁷⁷, R⁷⁸, R⁷⁹, R⁸³, R⁸⁴, R⁸⁵ ou R⁸⁶, s'ils sont présents sur des atomes de carbone adjacents, forment conjointement au moins un cycle ou système cyclique aryle en C₆-C₁₈ ou hétéroaryle en C₂-C₁₈,
dans lequel le substituant selon la formule générale (XXI) est relié au composé selon la formule générale (IIb) ou (IIc) par l'intermédiaire de l'un parmi R⁷⁰, R⁷³, R⁸⁰, R⁶², R⁶³, R⁶⁴, R⁶⁵, R⁶⁶, R⁶⁷, R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁸⁴, R⁸⁵ ou R⁸⁶, s'ils sont présents, dans lequel ces R⁷⁰, R⁷³, R⁸⁰, R⁶², R⁶³, R⁶⁴, R⁶⁵, R⁶⁶, R⁶⁷, R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁸⁴, R⁸⁵ ou R⁸⁶ respectifs sont une liaison directe, facultativement interrompue par un groupe de formule -(M)ₘ-, dans ce cas,
m est un entier de 0 à 4,
M est un groupe arylène en C₆-C₄₀ qui est non substitué ou substitué par au moins un groupe E, un groupe hétéroarylène en C₁-C₂₄ qui est non substitué ou substitué par au moins un groupe E ou un groupe alkylène en C₁-C₂₅ qui est non substitué ou substitué par au moins un groupe E et/ou interrompu par au moins un groupe D,
o est, indépendamment les uns des autres, 0 ou 1, p est, indépendamment les uns des autres, 0 ou 1, q est, indépendamment les uns des autres, 0 ou 1 et r est, indépendamment les uns des autres, 0 ou 1, L¹, L², L³ et L⁴ sont, indépendamment les uns des autres, choisis parmi un groupe aryle en C₆-C₄₀ qui est non substitué ou substitué par au moins un groupe E, un groupe hétéroaryle en C₁-C₂₄ qui est non substitué ou substitué par au moins un groupe E,
R¹⁴ est, indépendamment les uns des autres, choisi parmi H, E, un groupe aryle en C₆-C₂₄ qui est non substitué ou substitué par au moins un groupe E, un groupe hétéroaryle en C₁-C₂₄ qui est non substitué ou substitué par au moins un groupe E, un groupe alkyle en C₁-C₂₅ qui est non substitué ou substitué par au moins un groupe E et/ou interrompu par au moins un groupe D, un aralkyle en C₇-C₂₅ qui est non substitué ou substitué par au moins un groupe E, un groupe cycloalkyle en C₅-C₁₂ qui est non substitué ou substitué par au moins un groupe E,
D est, indépendamment les uns des autres, -CO-, -COO-, -S-, -SO-, -SO₂-, -O-, -CR¹⁵=CR¹⁶-, -NR¹⁷-, -SiR²²R²³-, -POR²⁵-, -C≡C-,
E est, indépendamment les uns des autres, -OR²¹, -SR²¹, -NR¹⁷R¹⁸, -COR²⁰, -COOR¹⁹, -CONR¹⁷R¹⁸, -CN, -SiR²²R²³R²⁴, POR²⁵R²⁷, halogène, un groupe aryle en C₆-C₆₀ qui est non substitué ou est substitué par au moins un -F, -CF₃, -CF₂CF₃, -CF₂CF₂CF₃, -CF(CF₃)₂, -(CF₂)₃CF₃ ou -C(CF₃)₃, un alkyle en C₁-C₁₈ ou un groupe alkyle en C₁-C₁₈ qui est interrompu par au moins un O, un groupe hétéroaryle en C₁-C₆₀ qui est non substitué ou substitué par au moins un -F, -CF₃, -CF₂CF₃, -CF₂CF₂CF₃, -CF(CF₃)₂, - (CF₂)₃CF₃ ou-C(CF₃)₃,
R¹⁵ et R¹⁶ sont, indépendamment l'un de l'autre, H, un groupe aryle en C₆-C₁₈ qui est non substitué ou substitué par au moins un groupe alkyle en C₁-C₁₈ ou au moins un groupe alcoxy en C₁-C₁₈, un groupe alkyle en C₁-C₁₈ ou un groupe alkyle en C₁-C₁₈ qui est interrompu par au moins un O,
R¹⁷ et R¹⁸ sont, indépendamment l'un de l'autre, H, un groupe aryle en C₆-C₁₈ qui est non substitué ou substitué par au moins un groupe alkyle en C₁-C₁₈ ou au moins un groupe alcoxy en C₁-C₁₈, un groupe alkyle en C₁-C₁₈ ou un groupe alkyle en C₁-C₁₈, qui est interrompu par au moins un O, ou
R¹⁷ et R¹⁸ forment conjointement un cycle aliphatique, aromatique ou hétéroaromatique de cinq ou six chaînons, R¹⁹ est H, un groupe aryle en C₆-C₁₈ qui est non substitué ou substitué par au moins un groupe alkyle en C₁-C₁₈ ou au moins un groupe alcoxy en C₁-C₁₈, un groupe alkyle en C₁-C₁₈ ou un groupe alkyle en C₁-C₁₈, qui est interrompu par au moins un O,
R²⁰ est H ou un groupe aryle en C₆-C₁₈ qui est non substitué ou substitué par au moins un groupe alkyle en C₁-C₁₈ ou au moins un groupe alcoxy en C₁-C₁₈, un groupe alkyle en C₁-C₁₈ ou un groupe alkyle en C₁-C₁₈, qui est interrompu par au moins un O,
R²¹ est, indépendamment les uns des autres, H, un groupe aryle en C₆-C₁₈ qui est non substitué ou substitué par au moins un groupe alkyle en C₁-C₁₈ ou au moins un groupe alcoxy en C₁-C₁₈, un groupe alkyle en C₁-C₁₈ ou un groupe alkyle en C₁-C₁₈, qui est interrompu par au moins un O,
R²², R²³ et R²⁴ sont, indépendamment les uns des autres, H, un groupe alkyle en C₁-C₁₈, un groupe aryle en C₆-C₁₈ qui est non substitué ou substitué par au moins un groupe alkyle en C₁-C₁₈, et
R²⁵ et R²⁷ sont, indépendamment l'un de l'autre, H, un groupe alkyle en C₁-C₁₈, un groupe aryle en C₆-C₁₈ qui est non substitué ou substitué par au moins un groupe alkyle en C₁-C₁₈, un aralkyle en C₇-C₂₅ qui est non substitué ou substitué par au moins un groupe E, un groupe cycloalkyle en C₅-C₁₂ qui est non substitué ou substitué par au moins un groupe E.

8. Composé selon la revendication 7, dans lequel le groupe hétérocyclique selon la formule générale (XXI) est représenté par l'une quelconque des formules générales (XXIa), (XXIb) ou (XXIc) 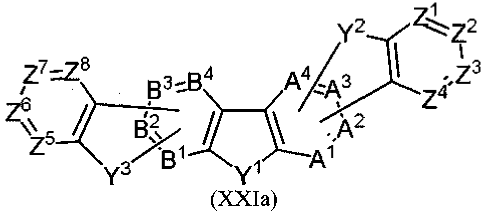 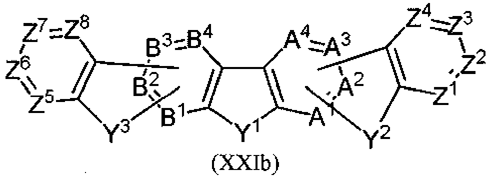 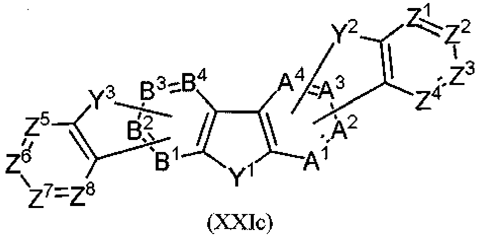 dans lesquelles A¹, A², A³, A⁴, B¹, B², B³, B⁴, Z¹, Z², Z³, Z⁴, Z⁵, Z⁶, Z⁷, Z⁸, Y¹, Y² et Y³ ont les significations telles que définies dans la revendication 7.

9. Composé selon la revendication 7 ou 8, dans lequel l'un de Y¹, Y² et Y³ est NR⁷⁰, NR⁷³ ou NR⁸⁰, respectivement.

10. Composé selon l'une quelconque des revendications 7 à 9, dans lequel, deux parmi Y¹, Y² et Y³ sont NR⁷⁰, NR⁷³ ou NR⁸⁰ respectivement.

11. Composé selon l'une quelconque des revendications 7 à 9, dans lequel le substituant selon la formule générale (XXI) est lié au composé selon la formule générale (IIb) ou (IIc) par l'intermédiaire de R⁷⁰, R⁷³ ou R⁸⁰, s'ils sont présents, dans lequel ces R⁷⁰, R⁷³ ou R⁸⁰ respectifs sont une liaison directe, facultativement interrompue par un groupe de formule -(M)m-, dans laquelle M et m ont les significations telles que définies dans la revendication 3.

12. Composé selon la revendication 3, dans lequel le groupe N-hétéroaryle selon la formule générale (XII) correspond à un groupe selon la formule générale (XX) 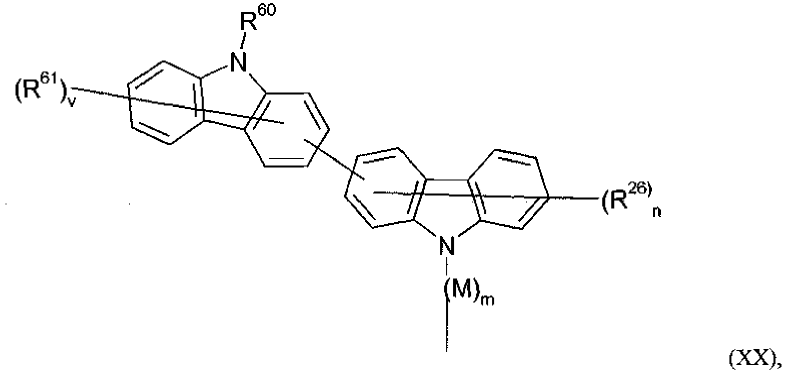 dans lequel
m, M, R²⁶ ont les significations telles que définies dans la revendication 3,
n est un entier de 0 à 7,
v est un entier de 0 à 7,
R⁶⁰ est, indépendamment les uns des autres, choisi parmi H, E, un groupe de formule - (L¹)ₒ-(L²)ₚ-(L³)_{q}-(L⁴)ᵣ-R¹⁴, un groupe alkyle en C₁-C₂₅, qui est non substitué ou substitué par au moins un groupe E et/ou interrompu par au moins un groupe D, un aralkyle en C₇-C₂₅ qui est non substitué ou substitué par au moins un groupe E, un groupe cycloalkyle en C₅-C₁₂ qui est non substitué ou substitué par au moins un groupe E, R²⁶ est, indépendamment les uns des autres, choisi parmi E, un groupe de formule -(L¹)ₒ-(L²)ₚ-(L³)_{q}-(L⁴)ᵣ-R¹⁴, un groupe alkyle en C₁-C₂₅, qui est non substitué ou substitué par au moins un groupe E et/ou interrompu par au moins un groupe D, dans lequel o, p, q, r, L¹, L², L³, L⁴, R¹⁴, E et D ont les significations telles que définies dans la revendication 1,
R⁶¹ est, indépendamment les uns des autres, choisi parmi E, un groupe de formule -(L¹)ₒ-(L²)ₚ-(L³)_{q}-(L⁴)ᵣ-R¹⁴, un groupe alkyle en C₁-C₂₅, qui est non substitué ou substitué par au moins un groupe E et/ou interrompu par au moins un groupe D, dans lequel o, p, q, r, L¹, L², L³, L⁴, R¹⁴, E et D ont les significations telles que définies dans la revendication 1,
ou au moins deux des R⁶¹, s'ils sont présents sur des atomes de carbone adjacents, peuvent former au moins un cycle ou système cyclique saturé, insaturé, aromatique ou hétéroaromatique substitué ou non substitué, de cinq ou six chaînons, auquel au moins un cycle ou système cyclique aromatique et/ou hétéroaromatique supplémentaire peut être condensé.

13. Procédé de préparation d'un composé selon la formule générale (IIb) ou (IIc) tel que défini dans l'une quelconque des revendications 1 à 12, comprenant au moins l'étape (A)
(A) couplage d'un composé selon la formule générale (Vaa) ou (Vab) 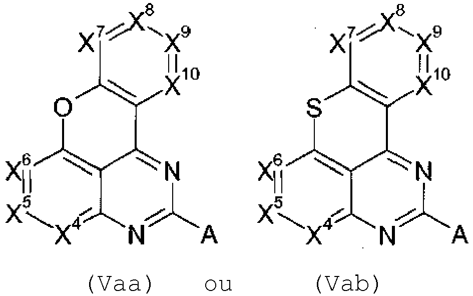 avec un composé de formule R²-H pour obtenir un composé selon la formule générale (IIb) ou (IIc), dans lequel X⁴, X⁵, X⁶, X⁷, X⁸, X⁹, X¹⁰ et R² ont les significations telles que définies dans l'une quelconque des revendications 1 à 12 et A est choisi parmi Cl, Br, I, F, OSO₂CH₃, et OSO₂CF₃ ou OSO₂C₆H₄CH₃.

14. Dispositif électronique comprenant au moins un composé tel que défini dans l'une quelconque des revendications 1 à 12.

15. Dispositif électronique, de préférence un dispositif à électroluminescence organique, selon la revendication 14, comprenant une cathode, une anode, et une pluralité de couches de film mince organiques disposées entre la cathode et l'anode, les couches de film mince organiques comprenant une couche émettrice comprenant l'au moins un composé de formule générale (IIb) ou (IIc).

16. Dispositif électronique, de préférence un dispositif à électroluminescence organique, selon la revendication 14 ou 15, dans lequel la couche émettrice comprend un matériau phosphorescent, qui est un complexe ortho-métallisé comprenant un atome de métal choisi parmi l'iridium (Ir), l'osmium (Os) et le platine (Pt).

17. Équipement électronique comprenant le dispositif à électroluminescence organique selon les revendications 15 ou 16.

18. Couche émettrice comprenant au moins un composé de formule générale (IIb) ou (IIc) tel que défini dans l'une quelconque des revendications 1 à 12.

19. Utilisation d'un composé selon la formule générale (IIb) ou (IIc) tel que défini dans l'une quelconque des revendications 1 à 12 dans un dispositif électronique en tant que matériau hôte, un matériau de transport de charge, un matériau de blocage de charge et/ou exciton.

20. Matériau pour un dispositif à électroluminescence organique comprenant au moins un composé selon l'une quelconque des revendications 1 à 12.
